# EUROPEAN PATENT SPECIFICATION

(11) **EP 3 323 885 B1**
(45) Date of publication and mention of the grant of the patent: **24.03.2021**
(21) Application number: 16823785.7
(22) Date of filing: 29.06.2016
(51) Int. Cl.: C12N 7/04, C07K 19/00, C12N 15/62, A61K 39/00, A61K 39/385, A61P 25/28, C12N 15/70, C12R 1/93

(54) **CHIMERIC NOROVIRUS P PARTICLE AND PREPARATION AND USE THEREOF**
CHIMÄRE NOROVIRUS-P-PARTIKEL UND HERSTELLUNG UND VERWENDUNG DAVON
PARTICULE P DE NOROVIRUS CHIMÉRIQUE AINSI QUE SA PRÉPARATION ET SON UTILISATION

(30) Priority: 15.07.2015 CN 201510415556
(43) Date of publication of application: 23.05.2018
(73) Proprietor: Changchun Bcht Biotechnology Co., Jilin 130012 (CN); Jilin University, Changchun, Jilin 130021 (CN)
(72) Inventor: KONG, Wei, Changchun Jilin 130012 (CN); WU, Hui, Changchun Jilin 130012 (CN); JIANG, Chunlai, Changchun Jilin 130012 (CN); YU, Xianghui, Changchun Jilin 130012 (CN); FU, Lu, Changchun Jilin 130012 (CN); LI, Yingnan, Changchun Jilin 130012 (CN)
(74) Representative: Gill Jennings & Every LLP
(86) International application number: PCT/CN2016/087643
(87) International publication number: WO 2017/008638

(56) References cited:
- WO-A2-2006/112553
- CN-A- 1 504 240
- CN-A- 101 538 558
- CN-A- 102 272 301
- C. WIESSNER ET AL: "The Second-Generation Active A Immunotherapy CAD106 Reduces Amyloid Accumulation in APP Transgenic Mice While Minimizing Potential Side Effects", THE JOURNAL OF NEUROSCIENCE, vol. 31, no. 25, 22 June 2011 (2011-06-22) , pages 9323-9331, XP055541157, US ISSN: 0270-6474, DOI: 10.1523/JNEUROSCI.0293-11.2011
- MING TAN ET AL: "Norovirus P particle: a subviral nanoparticle for vaccine development against norovirus, rotavirus and influenza virus", NANOMEDICINE, vol. 7, no. 6, 1 June 2012 (2012-06-01), pages 889-897, XP055541116, GB ISSN: 1743-5889, DOI: 10.2217/nnm.12.62
- LI YINGNAN ET AL: "Establishment of a novel method without sequence modification for developing NoV P particle-based chimeric vaccines", PROTEIN EXPRESSION AND PURIFICATION, vol. 121, 22 June 2011 (2011-06-22), pages 73-80, XP029465982, ISSN: 1046-5928, DOI: 10.1016/J.PEP.2016.01.003
- LU FU ET AL: "Norovirus P particle-based active A[beta] immunotherapy elicits sufficient immunogenicity and improves cognitive capacity in a mouse model of Alzheimer's disease", SCIENTIFIC REPORTS, vol. 7, no. 1, 1 December 2017 (2017-12-01), XP055541106, DOI: 10.1038/srep41041
- FU, LU ET AL.: 'Norovirus P particle: an Excellent Vaccine Platform for Antibody Production Against Alzheimer's Disease' IMMUNOLOGY LETTERS vol. 168, 05 September 2015, ISSN 0165-2478 pages 22 - 30, XP055344814
- TAN, MING ET AL.: 'Norovirus P Particle, a Novel Platform for Vaccine Development and Antibody Production' JOURNAL OF VIROLOGY vol. 85, no. 2, 31 January 2011, ISSN 0022-538X pages 753 - 764, XP002669772
- FENG, GAIFENG ET AL.: 'Prokaryotic Expression and Immunogenicity of The Chimeric HBcAg Containing Abeta1-15' JOURNAL OF CENTRAL SOUTH UNIVERSITY (MEDICIAL SCIENCES) vol. 37, no. 3, 01 January 2012, pages 290 - 295, XP055509188 DOI: 10.3969/J.ISSN.1672-7347.2012.03.014

### sequence listing

<110> CHANGCHUN BCHT BIOTECHNOLOGY CO. JILIN UNIVERSITY
<120> CHIMERIC NOROVIRUS P PARTICLE AND PREPARATION AND USE THEREOF
<130> JWJ02424EP
<140> EP16823785.7
   <141>
<150> PCT/CN2016/087643
   <151> 2016-06-29
<160> 152
<170> PatentIn version 3.3
<210> 1
   <211> 317
   <212> PRT
   <213> NOROVIRUS
<400> 1
   Met Lys Pro Phe Ser Val Pro Ile Leu Thr Val Glu Glu Met Thr Asn
   1               5                   10                  15
   Ser Arg Phe Pro Ile Pro Leu Glu Lys Leu Phe Thr Gly Pro Ser Ser
               20                  25                  30
   Ala Phe Val Val Gln Pro Gln Asn Gly Arg Cys Thr Thr Asp Gly Val
           35                  40                  45
   Leu Leu Gly Thr Thr Gln Leu Ser Pro Val Asn Ile Cys Thr Phe Arg
       50                  55                  60
   Gly Asp Val Thr His Ile Ala Gly Thr Gln Asn Tyr Thr Met Asn Leu
   65                  70                  75                  80
   Ala Ser Gln Asn Trp Asn Asn Tyr Asp Pro Thr Glu Glu Ile Pro Ala
                   85                  90                  95
   Pro Leu Gly Thr Pro Asp Phe Val Gly Arg Ile Gln Gly Val Leu Thr
               100                 105                 110
   Gln Thr Thr Arg Arg Asp Gly Ser Thr Arg Gly His Lys Ala Thr Val
           115                 120                 125
   Ser Thr Gly Ser Val His Phe Thr Pro Lys Leu Gly Ser Val Gln Phe
       130                 135                 140
   Ser Thr Asp Thr Ser Asn Asp Phe Glu Thr Gly Gln Asn Thr Arg Phe
   145                 150                 155                 160
   Thr Pro Val Gly Val Val Gln Asp Gly Ser Thr Thr His Gln Asn Glu
                   165                 170                 175
   Pro Gln Gln Trp Val Leu Pro Asp Tyr Ser Gly Arg Asp Ser His Asn
               180                 185                 190
   Val His Leu Ala Pro Ala Val Ala Pro Thr Phe Pro Gly Glu Gln Leu
           195                 200                 205
   Leu Phe Phe Arg Ser Thr Met Pro Gly Cys Ser Gly Tyr Pro Asn Met
       210                 215                 220
   Asn Leu Asp Cys Leu Leu Pro Gln Glu Trp Val Gln His Phe Tyr Gln
   225                 230                 235                 240
   Glu Ser Ala Pro Ala Gln Ser Asp Val Ala Leu Leu Arg Phe Val Asn
                   245                 250                 255
   Pro Asp Thr Gly Arg Val Leu Phe Glu Cys Lys Leu His Lys Ser Gly
               260                 265                 270
   Tyr Val Thr Val Ala His Thr Gly Gln His Asp Leu Val Ile Pro Pro
           275                 280                 285
   Asn Gly Tyr Phe Arg Phe Asp Ser Trp Val Asn Gln Phe Tyr Thr Leu
       290                 295                 300
   Ala Pro Met Gly Asn Gly Ala Gly Arg Arg Arg Ala Leu
   305                 310                 315
<210> 2
   <211> 15
   <212> PRT
   <213> Homo sapiens
<400> 2
   Asp Ala Glu Phe Arg His Asp Ser Gly Tyr Glu Val His His Gln
   1               5                   10                  15
<210> 3
   <211> 15
   <212> PRT
   <213> Mus musculus
<400> 3
   Asp Ala Glu Phe Gly His Asp Ser Gly Phe Glu Val Arg His Gln
   1 5 10 15
<210> 4
   <211> 15
   <212> PRT
   <213> Primates
<400> 4
   Asp Ala Glu Phe Arg His Asp Ser Gly Tyr Glu Val His His Gln
   1 5 10 15
<210> 5
   <211> 15
   <212> PRT
   <213> Oryctolagus cuniculus
<400> 5
   Asp Ala Glu Phe Arg His Asp Ser Gly Tyr Glu Val His His Gln
   1 5 10 15
<210> 6
   <211> 15
   <212> PRT
   <213> Xenopus laevis
<400> 6
   Asp Ser Glu Tyr Arg His Asp Thr Ala Tyr Glu Val His His Gln
   1 5 10 15
<210> 7
   <211> 15
   <212> PRT
   <213> Rattus norvegicus
<400> 7
   Asp Ser Glu Tyr Arg His Asp Thr Ala Tyr Glu Val His His Gln
   1 5 10 15
<210> 8
   <211> 15
   <212> PRT
   <213> Cavia porcellus
<400> 8
   Asp Ala Glu Phe Arg His Asp Ser Gly Tyr Glu Val His His Gln
   1 5 10 15
<210> 9
   <211> 954
   <212> DNA
   <213> artificial sequence
<400> 9
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggcacaaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac gatttcgaga ctggccagaa caccaggttc 480
   actcccgtgg gtgtcgttca agacggcagc accactcacc agaacgaacc ccagcaatgg 540
   gtcctccctg actactcggg cagggattcc cacaacgttc acttggctcc cgccgtggct 600
   ccaaccttcc ctggagagca gttgctgttc ttcagatcca ctatgccagg ctgctctgga 660
   tacccgaaca tgaacctcga ctgtctcttg cctcaggagt gggtgcaaca cttctaccag 720
   gaatctgccc cagctcaaag cgacgtcgct ctgctccgtt tcgttaaccc cgataccggt 780
   cgcgtgctct tcgagtgtaa gttgcacaag tctggttacg tcactgttgc ccacacaggc 840
   cagcacgacc tggtcatccc tcccaacggc tacttccgct tcgatagctg ggtcaaccag 900
   ttctacacac tcgccccgat gggaaacgga gccggtcgtc gcagagcctt gtaa 954
<210> 10
   <211> 45
   <212> DNA
   <213> artificial sequence
<400> 10
   gatgcagaat tccgacatga ctcaggatat gaagttcatc atcaa 45
<210> 11
   <211> 31
   <212> DNA
   <213> artificial sequence
<400> 11
   cgttcacttg gctccggccg tggctccaac c 31
<210> 12
   <211> 31
   <212> DNA
   <213> artificial sequence
<400> 12
   ggttggagcc acggccggag ccaagtgaac g 31
<210> 13
   <211> 32
   <212> DNA
   <213> artificial sequence
<400> 13
   ggtgtcctgc tcggtaccac ccagctctca cc 32
<210> 14
   <211> 32
   <212> DNA
   <213> artificial sequence
<400> 14
   ggtgagagct gggtggtacc gagcaggaca cc 32
<210> 15
   <211> 990
   <212> DNA
   <213> artificial sequence
<400> 15
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc ggaggaggag atgcagaatt ccgacatgga 240
   ggaggagctg gaacacaaaa ctacaccatg aacttggcct cgcagaactg gaacaactac 300
   gatccaaccg aggaaatccc cgctcctttg ggaactcccg acttcgtggg acgtatccaa 360
   ggtgtcctga cacagactac acgtcgcgac ggctctactc gcggacacaa ggccactgtg 420
   tcgacaggtt ccgtccactt cacccctaag ctgggctctg tgcaattcag cacagacacc 480
   tcaaacgatt tcgagactgg ccagaacacc aggttcactc ccgtgggtgt cgttcaagac 540
   ggcagcacca ctcaccagaa cgaaccccag caatgggtcc tccctgacta ctcgggcagg 600
   gattcccaca acgttcactt ggctccggcc gtggctccaa ccttccctgg agagcagttg 660
   ctgttcttca gatccactat gccaggctgc tctggatacc cgaacatgaa cctcgactgt 720
   ctcttgcctc aggagtgggt gcaacacttc taccaggaat ctgccccagc tcaaagcgac 780
   gtcgctctgc tccgtttcgt taaccccgat accggtcgcg tgctcttcga gtgtaagttg 840
   cacaagtctg gttacgtcac tgttgcccac acaggccagc acgacctggt catccctccc 900
   aacggctact tccgcttcga tagctgggtc aaccagttct acacactcgc cccgatggga 960
   aacggagccg gtcgtcgcag agccttgtaa 990
<210> 16
   <211> 1323
   <212> DNA
   <213> artificial sequence
<400> 16
   atgaagccct tctcggtccc tatcctgaca gttgaggaaa tgaccaactc tagattccc 60
<210> 17
   <211> 1863
   <212> DNA
   <213> artificial sequence
<400> 17
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc ggaggaggag atgcagaatt ccgacatgac 240
   tcaggatatg aagttggagg aggagatgca gaattccgac atgactcagg atatgaagtt 300
   ggaggaggag atgcagaatt ccgacatgac tcaggatatg aagttggagg aggagatgca 360
   gaattccgac atgactcagg atatgaagtt ggaggaggag atgcagaatt ccgacatgac 420
   tcaggatatg aagttggagg aggagatgca gaattccgac atgactcagg atatgaagtt 480
   ggaggaggag atgcagaatt ccgacatgac tcaggatatg aagttggagg aggagatgca 540
   gaattccgac atgactcagg atatgaagtt ggaggaggag atgcagaatt ccgacatgac 600
   tcaggatatg aagttggagg aggagatgca gaattccgac atgactcagg atatgaagtt 660
   ggaggaggag atgcagaatt ccgacatgac tcaggatatg aagttggagg aggagatgca 720
   gaattccgac atgactcagg atatgaagtt ggaggaggag atgcagaatt ccgacatgac 780
   tcaggatatg aagttggagg aggagatgca gaattccgac atgactcagg atatgaagtt 840
   ggaggaggag atgcagaatt ccgacatgac tcaggatatg aagttggagg aggagatgca 900
   gaattccgac atgactcagg atatgaagtt ggaggaggag atgcagaatt ccgacatgac 960
   tcaggatatg aagttggagg aggagatgca gaattccgac atgactcagg atatgaagtt 1020
   ggaggaggag atgcagaatt ccgacatgac tcaggatatg aagttggagg aggagatgca 1080
   gaattccgac atgactcagg atatgaagtt ggaggaggag ctggaacaca aaactacacc 1140
   atgaacttgg cctcgcagaa ctggaacaac tacgatccaa ccgaggaaat ccccgctcct 1200
   ttgggaactc ccgacttcgt gggacgtatc caaggtgtcc tgacacagac tacacgtcgc 1260
   gacggctcta ctcgcggaca caaggccact gtgtcgacag gttccgtcca cttcacccct 1320
   aagctgggct ctgtgcaatt cagcacagac acctcaaacg atttcgagac tggccagaac 1380
   accaggttca ctcccgtggg tgtcgttcaa gacggcagca ccactcacca gaacgaaccc 1440
   cagcaatggg tcctccctga ctactcgggc agggattccc acaacgttca cttggctccg 1500
   gccgtggctc caaccttccc tggagagcag ttgctgttct tcagatccac tatgccaggc 1560
   tgctctggat acccgaacat gaacctcgac tgtctcttgc ctcaggagtg ggtgcaacac 1620
   ttctaccagg aatctgcccc agctcaaagc gacgtcgctc tgctccgttt cgttaacccc 1680
   gataccggtc gcgtgctctt cgagtgtaag ttgcacaagt ctggttacgt cactgttgcc 1740
   cacacaggcc agcacgacct ggtcatccct cccaacggct acttccgctt cgatagctgg 1800
   gtcaaccagt tctacacact cgccccgatg ggaaacggag ccggtcgtcg cagagccttg 1860
   taa 1863
<210> 18
   <211> 3123
   <212> DNA
   <213> artificial sequence
<400> 18
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc ggaggaggag atgcagaatt ccgacatgac 240
   tcaggatatg aagttcatca tcaaggagga ggagatgcag aattccgaca tgactcagga 300
   tatgaagttc atcatcaagg aggaggagat gcagaattcc gacatgactc aggatatgaa 360
   gttcatcatc aaggaggagg agatgcagaa ttccgacatg actcaggata tgaagttcat 420
   catcaaggag gaggagatgc agaattccga catgactcag gatatgaagt tcatcatcaa 480
   ggaggaggag atgcagaatt ccgacatgac tcaggatatg aagttcatca tcaaggagga 540
   ggagatgcag aattccgaca tgactcagga tatgaagttc atcatcaagg aggaggagat 600
   gcagaattcc gacatgactc aggatatgaa gttcatcatc aaggaggagg agatgcagaa 660
   ttccgacatg actcaggata tgaagttcat catcaaggag gaggagatgc agaattccga 720
   catgactcag gatatgaagt tcatcatcaa ggaggaggag atgcagaatt ccgacatgac 780
   tcaggatatg aagttcatca tcaaggagga ggagatgcag aattccgaca tgactcagga 840
   tatgaagttc atcatcaagg aggaggagat gcagaattcc gacatgactc aggatatgaa 900
   gttcatcatc aaggaggagg agatgcagaa ttccgacatg actcaggata tgaagttcat 960
   catcaaggag gaggagatgc agaattccga catgactcag gatatgaagt tcatcatcaa 1020
   ggaggaggag atgcagaatt ccgacatgac tcaggatatg aagttcatca tcaaggagga 1080
   ggagatgcag aattccgaca tgactcagga tatgaagttc atcatcaagg aggaggagat 1140
   gcagaattcc gacatgactc aggatatgaa gttcatcatc aaggaggagg agatgcagaa 1200
   ttccgacatg actcaggata tgaagttcat catcaaggag gaggagatgc agaattccga 1260
   catgactcag gatatgaagt tcatcatcaa ggaggaggag atgcagaatt ccgacatgac 1320
   tcaggatatg aagttcatca tcaaggagga ggagatgcag aattccgaca tgactcagga 1380
   tatgaagttc atcatcaagg aggaggagat gcagaattcc gacatgactc aggatatgaa 1440
   gttcatcatc aaggaggagg agatgcagaa ttccgacatg actcaggata tgaagttcat 1500
   catcaaggag gaggagatgc agaattccga catgactcag gatatgaagt tcatcatcaa 1560
   ggaggaggag atgcagaatt ccgacatgac tcaggatatg aagttcatca tcaaggagga 1620
   ggagatgcag aattccgaca tgactcagga tatgaagttc atcatcaagg aggaggagat 1680
   gcagaattcc gacatgactc aggatatgaa gttcatcatc aaggaggagg agatgcagaa 1740
   ttccgacatg actcaggata tgaagttcat catcaaggag gaggagatgc agaattccga 1800
   catgactcag gatatgaagt tcatcatcaa ggaggaggag atgcagaatt ccgacatgac 1860
   tcaggatatg aagttcatca tcaaggagga ggagatgcag aattccgaca tgactcagga 1920
   tatgaagttc atcatcaagg aggaggagat gcagaattcc gacatgactc aggatatgaa 1980
   gttcatcatc aaggaggagg agatgcagaa ttccgacatg actcaggata tgaagttcat 2040
   catcaaggag gaggagatgc agaattccga catgactcag gatatgaagt tcatcatcaa 2100
   ggaggaggag atgcagaatt ccgacatgac tcaggatatg aagttcatca tcaaggagga 2160
   ggagatgcag aattccgaca tgactcagga tatgaagttc atcatcaagg aggaggagat 2220
   gcagaattcc gacatgactc aggatatgaa gttcatcatc aaggaggagg agatgcagaa 2280
   ttccgacatg actcaggata tgaagttcat catcaaggag gaggagatgc agaattccga 2340
   catgactcag gatatgaagt tcatcatcaa ggaggaggag ctggaacaca aaactacacc 2400
   atgaacttgg cctcgcagaa ctggaacaac tacgatccaa ccgaggaaat ccccgctcct 2460
   ttgggaactc ccgacttcgt gggacgtatc caaggtgtcc tgacacagac tacacgtcgc 2520
   gacggctcta ctcgcggaca caaggccact gtgtcgacag gttccgtcca cttcacccct 2580
   aagctgggct ctgtgcaatt cagcacagac acctcaaacg atttcgagac tggccagaac 2640
   accaggttca ctcccgtggg tgtcgttcaa gacggcagca ccactcacca gaacgaaccc 2700
   cagcaatggg tcctccctga ctactcgggc agggattccc acaacgttca cttggctccg 2760
   gccgtggctc caaccttccc tggagagcag ttgctgttct tcagatccac tatgccaggc 2820
   tgctctggat acccgaacat gaacctcgac tgtctcttgc ctcaggagtg ggtgcaacac 2880
   ttctaccagg aatctgcccc agctcaaagc gacgtcgctc tgctccgttt cgttaacccc 2940
   gataccggtc gcgtgctctt cgagtgtaag ttgcacaagt ctggttacgt cactgttgcc 3000
   cacacaggcc agcacgacct ggtcatccct cccaacggct acttccgctt cgatagctgg 3060
   gtcaaccagt tctacacact cgccccgatg ggaaacggag ccggtcgtcg cagagccttg 3120
   taa 3123
<210> 19
   <211> 999
   <212> DNA
   <213> artificial sequence
<400> 19
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggag gaggaggagg aacacaaaac tacaccatga acttggcctc gcagaactgg     300
   aacaactacg atccaaccga ggaaatcccc gctcctttgg gaactcccga cttcgtggga     360
   cgtatccaag gtgtcctgac acagactaca cgtcgcgacg gctctactcg cggacacaag     420
   gccactgtgt cgacaggttc cgtccacttc acccctaagc tgggctctgt gcaattcagc    480
   acagacacct caaacgattt cgagactggc cagaacacca ggttcactcc cgtgggtgtc     540
   gttcaagacg gcagcaccac tcaccagaac gaaccccagc aatgggtcct ccctgactac     600
   tcgggcaggg attcccacaa cgttcacttg gctccggccg tggctccaac cttccctgga     660
   gagcagttgc tgttcttcag atccactatg ccaggctgct ctggataccc gaacatgaac     720
   ctcgactgtc tcttgcctca ggagtgggtg caacacttct accaggaatc tgccccagct     780
   caaagcgacg tcgctctgct ccgtttcgtt aaccccgata ccggtcgcgt gctcttcgag     840
   tgtaagttgc acaagtctgg ttacgtcact gttgcccaca caggccagca cgacctggtc     900
   atccctccca acggctactt ccgcttcgat agctgggtca accagttcta cacactcgcc     960
   ccgatgggaa acggagccgg tcgtcgcaga gccttgtaa                            999
<210> 20
   <211> 1413
   <212> DNA
   <213> artificial sequence
<400> 20
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggat atgaagttgg aggaggagat gcagaattcc gacatgactc aggatatgaa     300
   gttggaggag gagatgcaga attccgacat gactcaggat atgaagttgg aggaggagat     360
   gcagaattcc gacatgactc aggatatgaa gttggaggag gagatgcaga attccgacat     420
   gactcaggat atgaagttgg aggaggagat gcagaattcc gacatgactc aggatatgaa     480
   gttggaggag gagatgcaga attccgacat gactcaggat atgaagttgg aggaggagat     540
   gcagaattcc gacatgactc aggatatgaa gttggaggag gagatgcaga attccgacat     600
   gactcaggat atgaagttgg aggaggagat gcagaattcc gacatgactc aggatatgaa     660
   gttggaggag gaggaacaca aaactacacc atgaacttgg cctcgcagaa ctggaacaac     720
   tacgatccaa ccgaggaaat ccccgctcct ttgggaactc ccgacttcgt gggacgtatc     780
   caaggtgtcc tgacacagac tacacgtcgc gacggctcta ctcgcggaca caaggccact     840
   gtgtcgacag gttccgtcca cttcacccct aagctgggct ctgtgcaatt cagcacagac     900
   acctcaaacg atttcgagac tggccagaac accaggttca ctcccgtggg tgtcgttcaa     960
   gacggcagca ccactcacca gaacgaaccc cagcaatggg tcctccctga ctactcgggc    1020
   agggattccc acaacgttca cttggctccg gccgtggctc caaccttccc tggagagcag    1080
   ttgctgttct tcagatccac tatgccaggc tgctctggat acccgaacat gaacctcgac    1140
   tgtctcttgc ctcaggagtg ggtgcaacac ttctaccagg aatctgcccc agctcaaagc    1200
   gacgtcgctc tgctccgttt cgttaacccc gataccggtc gcgtgctctt cgagtgtaag    1260
   ttgcacaagt ctggttacgt cactgttgcc cacacaggcc agcacgacct ggtcatccct    1320
   cccaacggct acttccgctt cgatagctgg gtcaaccagt tctacacact cgccccgatg    1380
   ggaaacggag ccggtcgtcg cagagccttg taa                                 1413
<210> 21
   <211> 2043
   <212> DNA
   <213> artificial sequence
<400> 21
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggat atgaagttca tcatcaagga ggaggagatg cagaattccg acatgactca     300
   ggatatgaag ttcatcatca aggaggagga gatgcagaat tccgacatga ctcaggatat     360
   gaagttcatc atcaaggagg aggagatgca gaattccgac atgactcagg atatgaagtt     420
   catcatcaag gaggaggaga tgcagaattc cgacatgact caggatatga agttcatcat     480
   caaggaggag gagatgcaga attccgacat gactcaggat atgaagttca tcatcaagga     540
   ggaggagatg cagaattccg acatgactca ggatatgaag ttcatcatca aggaggagga     600
   gatgcagaat tccgacatga ctcaggatat gaagttcatc atcaaggagg aggagatgca     660
   gaattccgac atgactcagg atatgaagtt catcatcaag gaggaggaga tgcagaattc     720
   cgacatgact caggatatga agttcatcat caaggaggag gagatgcaga attccgacat     780
   gactcaggat atgaagttca tcatcaagga ggaggagatg cagaattccg acatgactca     840
   ggatatgaag ttcatcatca aggaggagga gatgcagaat tccgacatga ctcaggatat     900
   gaagttcatc atcaaggagg aggagatgca gaattccgac atgactcagg atatgaagtt     960
   catcatcaag gaggaggaga tgcagaattc cgacatgact caggatatga agttcatcat    1020
   caaggaggag gagatgcaga attccgacat gactcaggat atgaagttca tcatcaagga    1080
   ggaggagatg cagaattccg acatgactca ggatatgaag ttcatcatca aggaggagga    1140
   gatgcagaat tccgacatga ctcaggatat gaagttcatc atcaaggagg aggagatgca    1200
   gaattccgac atgactcagg atatgaagtt catcatcaag gaggaggaga tgcagaattc    1260
   cgacatgact caggatatga agttcatcat caaggaggag gaggaacaca aaactacacc    1320
   atgaacttgg cctcgcagaa ctggaacaac tacgatccaa ccgaggaaat ccccgctcct    1380
   ttgggaactc ccgacttcgt gggacgtatc caaggtgtcc tgacacagac tacacgtcgc   1440
   gacggctcta ctcgcggaca caaggccact gtgtcgacag gttccgtcca cttcacccct   1500
   aagctgggct ctgtgcaatt cagcacagac acctcaaacg atttcgagac tggccagaac   1560
   accaggttca ctcccgtggg tgtcgttcaa gacggcagca ccactcacca gaacgaaccc   1620
   cagcaatggg tcctccctga ctactcgggc agggattccc acaacgttca cttggctccg   1680
   gccgtggctc caaccttccc tggagagcag ttgctgttct tcagatccac tatgccaggc   1740
   tgctctggat acccgaacat gaacctcgac tgtctcttgc ctcaggagtg ggtgcaacac   1800
   ttctaccagg aatctgcccc agctcaaagc gacgtcgctc tgctccgttt cgttaacccc   1860
   gataccggtc gcgtgctctt cgagtgtaag ttgcacaagt ctggttacgt cactgttgcc   1920
   cacacaggcc agcacgacct ggtcatccct cccaacggct acttccgctt cgatagctgg   1980
   gtcaaccagt tctacacact cgccccgatg ggaaacggag ccggtcgtcg cagagccttg   2040
   taa                                                                 2043
<210> 22
   <211> 2043
   <212> DNA
   <213> artificial sequence
<400> 22
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   ggaggaggag atgcagaatt ccgacatgga ggaggagatg cagaattccg acatggagga     300
   ggagatgcag aattccgaca tggaggagga gatgcagaat tccgacatgg aggaggagat     360
   gcagaattcc gacatggagg aggagatgca gaattccgac atggaggagg agatgcagaa     420
   ttccgacatg gaggaggaga tgcagaattc cgacatggag gaggagatgc agaattccga     480
   catggaggag gagatgcaga attccgacat ggaggaggag atgcagaatt ccgacatgga     540
   ggaggagatg cagaattccg acatggagga ggagatgcag aattccgaca tggaggagga     600
   gatgcagaat tccgacatgg aggaggagat gcagaattcc gacatggagg aggagatgca     660
   gaattccgac atggaggagg agatgcagaa ttccgacatg gaggaggaga tgcagaattc     720
   cgacatggag gaggagatgc agaattccga catggaggag gagatgcaga attccgacat     780
   ggaggaggag atgcagaatt ccgacatgga ggaggagatg cagaattccg acatggagga     840
   ggagatgcag aattccgaca tggaggagga gatgcagaat tccgacatgg aggaggagat     900
   gcagaattcc gacatggagg aggagatgca gaattccgac atggaggagg agatgcagaa     960
   ttccgacatg gaggaggaga tgcagaattc cgacatggag gaggagatgc agaattccga 1020
   catggaggag gagatgcaga attccgacat ggaggaggag atgcagaatt ccgacatgga 1080
   ggaggagatg cagaattccg acatggagga ggagatgcag aattccgaca tggaggagga 1140
   gatgcagaat tccgacatgg aggaggagat gcagaattcc gacatggagg aggagatgca 1200
   gaattccgac atggaggagg agatgcagaa ttccgacatg gaggaggaga tgcagaattc 1260
   cgacatggag gaggagatgc agaattccga catggaggag gaggaacaca aaactacacc 1320
   atgaacttgg cctcgcagaa ctggaacaac tacgatccaa ccgaggaaat ccccgctcct 1380
   ttgggaactc ccgacttcgt gggacgtatc caaggtgtcc tgacacagac tacacgtcgc 1440
   gacggctcta ctcgcggaca caaggccact gtgtcgacag gttccgtcca cttcacccct 1500
   aagctgggct ctgtgcaatt cagcacagac acctcaaacg atttcgagac tggccagaac 1560
   accaggttca ctcccgtggg tgtcgttcaa gacggcagca ccactcacca gaacgaaccc 1620
   cagcaatggg tcctccctga ctactcgggc agggattccc acaacgttca cttggctccg 1680
   gccgtggctc caaccttccc tggagagcag ttgctgttct tcagatccac tatgccaggc 1740
   tgctctggat acccgaacat gaacctcgac tgtctcttgc ctcaggagtg ggtgcaacac 1800
   ttctaccagg aatctgcccc agctcaaagc gacgtcgctc tgctccgttt cgttaacccc 1860
   gataccggtc gcgtgctctt cgagtgtaag ttgcacaagt ctggttacgt cactgttgcc 1920
   cacacaggcc agcacgacct ggtcatccct cccaacggct acttccgctt cgatagctgg 1980
   gtcaaccagt tctacacact cgccccgatg ggaaacggag ccggtcgtcg cagagccttg 2040
   taa 2043
<210> 23
   <211> 990
   <212> DNA
   <213> artificial sequence
<400> 23
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catggaggag gaacacaaaa ctacaccatg aacttggcct cgcagaactg gaacaactac     300
   gatccaaccg aggaaatccc cgctcctttg ggaactcccg acttcgtggg acgtatccaa     360
   ggtgtcctga cacagactac acgtcgcgac ggctctactc gcggacacaa ggccactgtg     420
   tcgacaggtt ccgtccactt cacccctaag ctgggctctg tgcaattcag cacagacacc     480
   tcaaacgatt tcgagactgg ccagaacacc aggttcactc ccgtgggtgt cgttcaagac     540
   ggcagcacca ctcaccagaa cgaaccccag caatgggtcc tccctgacta ctcgggcagg     600
   gattcccaca acgttcactt ggctccggcc gtggctccaa ccttccctgg agagcagttg     660
   ctgttcttca gatccactat gccaggctgc tctggatacc cgaacatgaa cctcgactgt     720
   ctcttgcctc aggagtgggt gcaacacttc taccaggaat ctgccccagc tcaaagcgac     780
   gtcgctctgc tccgtttcgt taaccccgat accggtcgcg tgctcttcga gtgtaagttg     840
   cacaagtctg gttacgtcac tgttgcccac acaggccagc acgacctggt catccctccc     900
   aacggctact tccgcttcga tagctgggtc aaccagttct acacactcgc cccgatggga     960
   aacggagccg gtcgtcgcag agccttgtaa                                      990
<210> 24
   <211> 1233
   <212> DNA
   <213> artificial sequence
<400> 24
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catggaggag gagatgcaga attccgacat ggaggaggag atgcagaatt ccgacatgga 300
   ggaggagatg cagaattccg acatggagga ggagatgcag aattccgaca tggaggagga 360
   gatgcagaat tccgacatgg aggaggagat gcagaattcc gacatggagg aggagatgca 420
   gaattccgac atggaggagg agatgcagaa ttccgacatg gaggaggaga tgcagaattc 480
   cgacatggag gaggaacaca aaactacacc atgaacttgg cctcgcagaa ctggaacaac 540
   tacgatccaa ccgaggaaat ccccgctcct ttgggaactc ccgacttcgt gggacgtatc 600
   caaggtgtcc tgacacagac tacacgtcgc gacggctcta ctcgcggaca caaggccact 660
   gtgtcgacag gttccgtcca cttcacccct aagctgggct ctgtgcaatt cagcacagac 720
   acctcaaacg atttcgagac tggccagaac accaggttca ctcccgtggg tgtcgttcaa 780
   gacggcagca ccactcacca gaacgaaccc cagcaatggg tcctccctga ctactcgggc 840
   agggattccc acaacgttca cttggctccg gccgtggctc caaccttccc tggagagcag 900
   ttgctgttct tcagatccac tatgccaggc tgctctggat acccgaacat gaacctcgac 960
   tgtctcttgc ctcaggagtg ggtgcaacac ttctaccagg aatctgcccc agctcaaagc 1020
   gacgtcgctc tgctccgttt cgttaacccc gataccggtc gcgtgctctt cgagtgtaag 1080
   ttgcacaagt ctggttacgt cactgttgcc cacacaggcc agcacgacct ggtcatccct 1140
   cccaacggct acttccgctt cgatagctgg gtcaaccagt tctacacact cgccccgatg 1200
   ggaaacggag ccggtcgtcg cagagccttg taa 1233
<210> 25
   <211> 1503
   <212> DNA
   <213> artificial sequence
<400> 25
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catggaggag gagatgcaga attccgacat ggaggaggag atgcagaatt ccgacatgga     300
   ggaggagatg cagaattccg acatggagga ggagatgcag aattccgaca tggaggagga     360
   gatgcagaat tccgacatgg aggaggagat gcagaattcc gacatggagg aggagatgca     420
   gaattccgac atggaggagg agatgcagaa ttccgacatg gaggaggaga tgcagaattc     480
   cgacatggag gaggagatgc agaattccga catggaggag gagatgcaga attccgacat     540
   ggaggaggag atgcagaatt ccgacatgga ggaggagatg cagaattccg acatggagga     600
   ggagatgcag aattccgaca tggaggagga gatgcagaat tccgacatgg aggaggagat     660
   gcagaattcc gacatggagg aggagatgca gaattccgac atggaggagg agatgcagaa     720
   ttccgacatg gaggaggaga tgcagaattc cgacatggag gaggaacaca aaactacacc     780
   atgaacttgg cctcgcagaa ctggaacaac tacgatccaa ccgaggaaat ccccgctcct     840
   ttgggaactc ccgacttcgt gggacgtatc caaggtgtcc tgacacagac tacacgtcgc     900
   gacggctcta ctcgcggaca caaggccact gtgtcgacag gttccgtcca cttcacccct     960
   aagctgggct ctgtgcaatt cagcacagac acctcaaacg atttcgagac tggccagaac    1020
   accaggttca ctcccgtggg tgtcgttcaa gacggcagca ccactcacca gaacgaaccc    1080
   cagcaatggg tcctccctga ctactcgggc agggattccc acaacgttca cttggctccg    1140
   gccgtggctc caaccttccc tggagagcag ttgctgttct tcagatccac tatgccaggc    1200
   tgctctggat acccgaacat gaacctcgac tgtctcttgc ctcaggagtg ggtgcaacac    1260
   ttctaccagg aatctgcccc agctcaaagc gacgtcgctc tgctccgttt cgttaacccc    1320
   gataccggtc gcgtgctctt cgagtgtaag ttgcacaagt ctggttacgt cactgttgcc    1380
   cacacaggcc agcacgacct ggtcatccct cccaacggct acttccgctt cgatagctgg    1440
   gtcaaccagt tctacacact cgccccgatg ggaaacggag ccggtcgtcg cagagccttg    1500
   taa                                                                  1503
<210> 26
   <211> 2043
   <212> DNA
   <213> artificial sequence
<400> 26
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catggaggag gagatgcaga attccgacat ggaggaggag atgcagaatt ccgacatgga 300
   ggaggagatg cagaattccg acatggagga ggagatgcag aattccgaca tggaggagga 360
   gatgcagaat tccgacatgg aggaggagat gcagaattcc gacatggagg aggagatgca 420
   gaattccgac atggaggagg agatgcagaa ttccgacatg gaggaggaga tgcagaattc 480
   cgacatggag gaggagatgc agaattccga catggaggag gagatgcaga attccgacat 540
   ggaggaggag atgcagaatt ccgacatgga ggaggagatg cagaattccg acatggagga 600
   ggagatgcag aattccgaca tggaggagga gatgcagaat tccgacatgg aggaggagat 660
   gcagaattcc gacatggagg aggagatgca gaattccgac atggaggagg agatgcagaa 720
   ttccgacatg gaggaggaga tgcagaattc cgacatggag gaggagatgc agaattccga 780
   catggaggag gagatgcaga attccgacat ggaggaggag atgcagaatt ccgacatgga 840
   ggaggagatg cagaattccg acatggagga ggagatgcag aattccgaca tggaggagga 900
   gatgcagaat tccgacatgg aggaggagat gcagaattcc gacatggagg aggagatgca 960
   gaattccgac atggaggagg agatgcagaa ttccgacatg gaggaggaga tgcagaattc 1020
   cgacatggag gaggagatgc agaattccga catggaggag gagatgcaga attccgacat 1080
   ggaggaggag atgcagaatt ccgacatgga ggaggagatg cagaattccg acatggagga 1140
   ggagatgcag aattccgaca tggaggagga gatgcagaat tccgacatgg aggaggagat 1200
   gcagaattcc gacatggagg aggagatgca gaattccgac atggaggagg agatgcagaa 1260
   ttccgacatg gaggaggaga tgcagaattc cgacatggag gaggaacaca aaactacacc 1320
   atgaacttgg cctcgcagaa ctggaacaac tacgatccaa ccgaggaaat ccccgctcct 1380
   ttgggaactc ccgacttcgt gggacgtatc caaggtgtcc tgacacagac tacacgtcgc 1440
   gacggctcta ctcgcggaca caaggccact gtgtcgacag gttccgtcca cttcacccct 1500
   aagctgggct ctgtgcaatt cagcacagac acctcaaacg atttcgagac tggccagaac 1560
   accaggttca ctcccgtggg tgtcgttcaa gacggcagca ccactcacca gaacgaaccc 1620
   cagcaatggg tcctccctga ctactcgggc agggattccc acaacgttca cttggctccg 1680
   gccgtggctc caaccttccc tggagagcag ttgctgttct tcagatccac tatgccaggc 1740
   tgctctggat acccgaacat gaacctcgac tgtctcttgc ctcaggagtg ggtgcaacac 1800
   ttctaccagg aatctgcccc agctcaaagc gacgtcgctc tgctccgttt cgttaacccc 1860
   gataccggtc gcgtgctctt cgagtgtaag ttgcacaagt ctggttacgt cactgttgcc 1920
   cacacaggcc agcacgacct ggtcatccct cccaacggct acttccgctt cgatagctgg 1980
   gtcaaccagt tctacacact cgccccgatg ggaaacggag ccggtcgtcg cagagccttg 2040
   taa 2043
<210> 27
   <211> 1008
   <212> DNA
   <213> artificial sequence
<400> 27
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacag gaggaggaga tgcagaattc 240
   cgacatgact caggatatga agttggagga ggacaaaact acaccatgaa cttggcctcg 300
   cagaactgga acaactacga tccaaccgag gaaatccccg ctcctttggg aactcccgac 360
   ttcgtgggac gtatccaagg tgtcctgaca cagactacac gtcgcgacgg ctctactcgc 420
   ggacacaagg ccactgtgtc gacaggttcc gtccacttca cccctaagct gggctctgtg 480
   caattcagca cagacacctc aaacgatttc gagactggcc agaacaccag gttcactccc 540
   gtgggtgtcg ttcaagacgg cagcaccact caccagaacg aaccccagca atgggtcctc 600
   cctgactact cgggcaggga ttcccacaac gttcacttgg ctccggccgt ggctccaacc 660
   ttccctggag agcagttgct gttcttcaga tccactatgc caggctgctc tggatacccg 720
   aacatgaacc tcgactgtct cttgcctcag gagtgggtgc aacacttcta ccaggaatct 780
   gccccagctc aaagcgacgt cgctctgctc cgtttcgtta accccgatac cggtcgcgtg 840
   ctcttcgagt gtaagttgca caagtctggt tacgtcactg ttgcccacac aggccagcac 900
   gacctggtca tccctcccaa cggctacttc cgcttcgata gctgggtcaa ccagttctac 960
   acactcgccc cgatgggaaa cggagccggt cgtcgcagag ccttgtaa 1008
<210> 28
   <211> 1503
   <212> DNA
   <213> artificial sequence
<400> 28
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacag gaggaggaga tgcagaattc     240
   cgacatgact caggatatga agttcatcat caaggaggag gagatgcaga attccgacat     300
   gactcaggat atgaagttca tcatcaagga ggaggagatg cagaattccg acatgactca     360
   ggatatgaag ttcatcatca aggaggagga gatgcagaat tccgacatga ctcaggatat     420
   gaagttcatc atcaaggagg aggagatgca gaattccgac atgactcagg atatgaagtt     480
   catcatcaag gaggaggaga tgcagaattc cgacatgact caggatatga agttcatcat     540
   caaggaggag gagatgcaga attccgacat gactcaggat atgaagttca tcatcaagga     600
   ggaggagatg cagaattccg acatgactca ggatatgaag ttcatcatca aggaggagga     660
   gatgcagaat tccgacatga ctcaggatat gaagttcatc atcaaggagg aggagatgca     720
   gaattccgac atgactcagg atatgaagtt catcatcaag gaggaggaca aaactacacc     780
   atgaacttgg cctcgcagaa ctggaacaac tacgatccaa ccgaggaaat ccccgctcct     840
   ttgggaactc ccgacttcgt gggacgtatc caaggtgtcc tgacacagac tacacgtcgc     900
   gacggctcta ctcgcggaca caaggccact gtgtcgacag gttccgtcca cttcacccct     960
   aagctgggct ctgtgcaatt cagcacagac acctcaaacg atttcgagac tggccagaac    1020
   accaggttca ctcccgtggg tgtcgttcaa gacggcagca ccactcacca gaacgaaccc    1080
   cagcaatggg tcctccctga ctactcgggc agggattccc acaacgttca cttggctccg    1140
   gccgtggctc caaccttccc tggagagcag ttgctgttct tcagatccac tatgccaggc    1200
   tgctctggat acccgaacat gaacctcgac tgtctcttgc ctcaggagtg ggtgcaacac    1260
   ttctaccagg aatctgcccc agctcaaagc gacgtcgctc tgctccgttt cgttaacccc    1320
   gataccggtc gcgtgctctt cgagtgtaag ttgcacaagt ctggttacgt cactgttgcc    1380
   cacacaggcc agcacgacct ggtcatccct cccaacggct acttccgctt cgatagctgg    1440
   gtcaaccagt tctacacact cgccccgatg ggaaacggag ccggtcgtcg cagagccttg    1500
   taa                                                                  1503
<210> 29
   <211> 1503
   <212> DNA
   <213> artificial sequence
<400> 29
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacag gaggaggaga tgcagaattc 240
   cgacatggag gaggagatgc agaattccga catggaggag gagatgcaga attccgacat 300
   ggaggaggag atgcagaatt ccgacatgga ggaggagatg cagaattccg acatggagga 360
   ggagatgcag aattccgaca tggaggagga gatgcagaat tccgacatgg aggaggagat 420
   gcagaattcc gacatggagg aggagatgca gaattccgac atggaggagg agatgcagaa 480
   ttccgacatg gaggaggaga tgcagaattc cgacatggag gaggagatgc agaattccga 540
   catggaggag gagatgcaga attccgacat ggaggaggag atgcagaatt ccgacatgga 600
   ggaggagatg cagaattccg acatggagga ggagatgcag aattccgaca tggaggagga 660
   gatgcagaat tccgacatgg aggaggagat gcagaattcc gacatggagg aggagatgca 720
   gaattccgac atggaggagg agatgcagaa ttccgacatg gaggaggaca aaactacacc 780
   atgaacttgg cctcgcagaa ctggaacaac tacgatccaa ccgaggaaat ccccgctcct 840
   ttgggaactc ccgacttcgt gggacgtatc caaggtgtcc tgacacagac tacacgtcgc 900
   gacggctcta ctcgcggaca caaggccact gtgtcgacag gttccgtcca cttcacccct 960
   aagctgggct ctgtgcaatt cagcacagac acctcaaacg atttcgagac tggccagaac 1020
   accaggttca ctcccgtggg tgtcgttcaa gacggcagca ccactcacca gaacgaaccc 1080
   cagcaatggg tcctccctga ctactcgggc agggattccc acaacgttca cttggctccg 1140
   gccgtggctc caaccttccc tggagagcag ttgctgttct tcagatccac tatgccaggc 1200
   tgctctggat acccgaacat gaacctcgac tgtctcttgc ctcaggagtg ggtgcaacac 1260
   ttctaccagg aatctgcccc agctcaaagc gacgtcgctc tgctccgttt cgttaacccc 1320
   gataccggtc gcgtgctctt cgagtgtaag ttgcacaagt ctggttacgt cactgttgcc 1380
   cacacaggcc agcacgacct ggtcatccct cccaacggct acttccgctt cgatagctgg 1440
   gtcaaccagt tctacacact cgccccgatg ggaaacggag ccggtcgtcg cagagccttg 1500
   taa 1503
<210> 30
   <211> 2403
   <212> DNA
   <213> artificial sequence
<400> 30
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacag gaggaggaga tgcagaattc     240
   cgacatgact caggaggagg aggagatgca gaattccgac atgactcagg aggaggagga     300
   gatgcagaat tccgacatga ctcaggagga ggaggagatg cagaattccg acatgactca     360
   ggaggaggag gagatgcaga attccgacat gactcaggag gaggaggaga tgcagaattc     420
   cgacatgact caggaggagg aggagatgca gaattccgac atgactcagg aggaggagga     480
   gatgcagaat tccgacatga ctcaggagga ggaggagatg cagaattccg acatgactca     540
   ggaggaggag gagatgcaga attccgacat gactcaggag gaggaggaga tgcagaattc     600
   cgacatgact caggaggagg aggagatgca gaattccgac atgactcagg aggaggagga     660
   gatgcagaat tccgacatga ctcaggagga ggaggagatg cagaattccg acatgactca     720
   ggaggaggag gagatgcaga attccgacat gactcaggag gaggaggaga tgcagaattc     780
   cgacatgact caggaggagg aggagatgca gaattccgac atgactcagg aggaggagga     840
   gatgcagaat tccgacatga ctcaggagga ggaggagatg cagaattccg acatgactca     900
   ggaggaggag gagatgcaga attccgacat gactcaggag gaggaggaga tgcagaattc     960
   cgacatgact caggaggagg aggagatgca gaattccgac atgactcagg aggaggagga    1020
   gatgcagaat tccgacatga ctcaggagga ggaggagatg cagaattccg acatgactca    1080
   ggaggaggag gagatgcaga attccgacat gactcaggag gaggaggaga tgcagaattc    1140
   cgacatgact caggaggagg aggagatgca gaattccgac atgactcagg aggaggagga    1200
   gatgcagaat tccgacatga ctcaggagga ggaggagatg cagaattccg acatgactca    1260
   ggaggaggag gagatgcaga attccgacat gactcaggag gaggaggaga tgcagaattc    1320
   cgacatgact caggaggagg aggagatgca gaattccgac atgactcagg aggaggagga    1380
   gatgcagaat tccgacatga ctcaggagga ggaggagatg cagaattccg acatgactca    1440
   ggaggaggag gagatgcaga attccgacat gactcaggag gaggaggaga tgcagaattc    1500
   cgacatgact caggaggagg aggagatgca gaattccgac atgactcagg aggaggagga    1560
   gatgcagaat tccgacatga ctcaggagga ggaggagatg cagaattccg acatgactca    1620
   ggaggaggag gagatgcaga attccgacat gactcaggag gaggaggaca aaactacacc    1680
   atgaacttgg cctcgcagaa ctggaacaac tacgatccaa ccgaggaaat ccccgctcct    1740
   ttgggaactc ccgacttcgt gggacgtatc caaggtgtcc tgacacagac tacacgtcgc    1800
   gacggctcta ctcgcggaca caaggccact gtgtcgacag gttccgtcca cttcacccct    1860
   aagctgggct ctgtgcaatt cagcacagac acctcaaacg atttcgagac tggccagaac    1920
   accaggttca ctcccgtggg tgtcgttcaa gacggcagca ccactcacca gaacgaaccc    1980
   cagcaatggg tcctccctga ctactcgggc agggattccc acaacgttca cttggctccg    2040
   gccgtggctc caaccttccc tggagagcag ttgctgttct tcagatccac tatgccaggc    2100
   tgctctggat acccgaacat gaacctcgac tgtctcttgc ctcaggagtg ggtgcaacac    2160
   ttctaccagg aatctgcccc agctcaaagc gacgtcgctc tgctccgttt cgttaacccc    2220
   gataccggtc gcgtgctctt cgagtgtaag ttgcacaagt ctggttacgt cactgttgcc 2280
   cacacaggcc agcacgacct ggtcatccct cccaacggct acttccgctt cgatagctgg 2340
   gtcaaccagt tctacacact cgccccgatg ggaaacggag ccggtcgtcg cagagccttg 2400
   taa 2403
<210> 31
   <211> 1017
   <212> DNA
   <213> artificial sequence
<400> 31
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaggaggagg agatgcagaa 240
   ttccgacatg actcaggata tgaagttcat catcaaggag gaggaaacta caccatgaac 300
   ttggcctcgc agaactggaa caactacgat ccaaccgagg aaatccccgc tcctttggga 360
   actcccgact tcgtgggacg tatccaaggt gtcctgacac agactacacg tcgcgacggc 420
   tctactcgcg gacacaaggc cactgtgtcg acaggttccg tccacttcac ccctaagctg 480
   ggctctgtgc aattcagcac agacacctca aacgatttcg agactggcca gaacaccagg 540
   ttcactcccg tgggtgtcgt tcaagacggc agcaccactc accagaacga accccagcaa 600
   tgggtcctcc ctgactactc gggcagggat tcccacaacg ttcacttggc tccggccgtg 660
   gctccaacct tccctggaga gcagttgctg ttcttcagat ccactatgcc aggctgctct 720
   ggatacccga acatgaacct cgactgtctc ttgcctcagg agtgggtgca acacttctac 780
   caggaatctg ccccagctca aagcgacgtc gctctgctcc gtttcgttaa ccccgatacc 840
   ggtcgcgtgc tcttcgagtg taagttgcac aagtctggtt acgtcactgt tgcccacaca 900
   ggccagcacg acctggtcat ccctcccaac ggctacttcc gcttcgatag ctgggtcaac 960
   cagttctaca cactcgcccc gatgggaaac ggagccggtc gtcgcagagc cttgtaa 1017
<210> 32
   <211> 1233
   <212> DNA
   <213> artificial sequence
<400> 32
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaggaggagg agatgcagaa     240
   ttccgacatg gaggaggaga tgcagaattc cgacatggag gaggagatgc agaattccga     300
   catggaggag gagatgcaga attccgacat ggaggaggag atgcagaatt ccgacatgga     360
   ggaggagatg cagaattccg acatggagga ggagatgcag aattccgaca tggaggagga     420
   gatgcagaat tccgacatgg aggaggagat gcagaattcc gacatggagg aggagatgca     480
   gaattccgac atggaggagg aaactacacc atgaacttgg cctcgcagaa ctggaacaac     540
   tacgatccaa ccgaggaaat ccccgctcct ttgggaactc ccgacttcgt gggacgtatc     600
   caaggtgtcc tgacacagac tacacgtcgc gacggctcta ctcgcggaca caaggccact     660
   gtgtcgacag gttccgtcca cttcacccct aagctgggct ctgtgcaatt cagcacagac     720
   acctcaaacg atttcgagac tggccagaac accaggttca ctcccgtggg tgtcgttcaa     780
   gacggcagca ccactcacca gaacgaaccc cagcaatggg tcctccctga ctactcgggc     840
   agggattccc acaacgttca cttggctccg gccgtggctc caaccttccc tggagagcag     900
   ttgctgttct tcagatccac tatgccaggc tgctctggat acccgaacat gaacctcgac     960
   tgtctcttgc ctcaggagtg ggtgcaacac ttctaccagg aatctgcccc agctcaaagc    1020
   gacgtcgctc tgctccgttt cgttaacccc gataccggtc gcgtgctctt cgagtgtaag    1080
   ttgcacaagt ctggttacgt cactgttgcc cacacaggcc agcacgacct ggtcatccct    1140
   cccaacggct acttccgctt cgatagctgg gtcaaccagt tctacacact cgccccgatg    1200
   ggaaacggag ccggtcgtcg cagagccttg taa                                 1233
<210> 33
   <211> 1683
   <212> DNA
   <213> artificial sequence
<400> 33
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaggaggagg agatgcagaa    240
   ttccgacatg actcaggagg aggaggagat gcagaattcc gacatgactc aggaggagga    300
   ggagatgcag aattccgaca tgactcagga ggaggaggag atgcagaatt ccgacatgac    360
   tcaggaggag gaggagatgc agaattccga catgactcag gaggaggagg agatgcagaa    420
   ttccgacatg actcaggagg aggaggagat gcagaattcc gacatgactc aggaggagga    480
   ggagatgcag aattccgaca tgactcagga ggaggaggag atgcagaatt ccgacatgac    540
   tcaggaggag gaggagatgc agaattccga catgactcag gaggaggagg agatgcagaa    600
   ttccgacatg actcaggagg aggaggagat gcagaattcc gacatgactc aggaggagga    660
   ggagatgcag aattccgaca tgactcagga ggaggaggag atgcagaatt ccgacatgac    720
   tcaggaggag gaggagatgc agaattccga catgactcag gaggaggagg agatgcagaa    780
   ttccgacatg actcaggagg aggaggagat gcagaattcc gacatgactc aggaggagga    840
   ggagatgcag aattccgaca tgactcagga ggaggaggag atgcagaatt ccgacatgac    900
   tcaggaggag gaggagatgc agaattccga catgactcag gaggaggagg aaactacacc    960
   atgaacttgg cctcgcagaa ctggaacaac tacgatccaa ccgaggaaat ccccgctcct   1020
   ttgggaactc ccgacttcgt gggacgtatc caaggtgtcc tgacacagac tacacgtcgc   1080
   gacggctcta ctcgcggaca caaggccact gtgtcgacag gttccgtcca cttcacccct   1140
   aagctgggct ctgtgcaatt cagcacagac acctcaaacg atttcgagac tggccagaac   1200
   accaggttca ctcccgtggg tgtcgttcaa gacggcagca ccactcacca gaacgaaccc   1260
   cagcaatggg tcctccctga ctactcgggc agggattccc acaacgttca cttggctccg   1320
   gccgtggctc caaccttccc tggagagcag ttgctgttct tcagatccac tatgccaggc   1380
   tgctctggat acccgaacat gaacctcgac tgtctcttgc ctcaggagtg ggtgcaacac   1440
   ttctaccagg aatctgcccc agctcaaagc gacgtcgctc tgctccgttt cgttaacccc   1500
   gataccggtc gcgtgctctt cgagtgtaag ttgcacaagt ctggttacgt cactgttgcc   1560
   cacacaggcc agcacgacct ggtcatccct cccaacggct acttccgctt cgatagctgg   1620
   gtcaaccagt tctacacact cgccccgatg ggaaacggag ccggtcgtcg cagagccttg   1680
   taa                                                                 1683
<210> 34
   <211> 2763
   <212> DNA
   <213> artificial sequence
<400> 34
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaggaggagg agatgcagaa    240
   ttccgacatg actcaggata tgaagttgga ggaggagatg cagaattccg acatgactca    300
   ggatatgaag ttggaggagg agatgcagaa ttccgacatg actcaggata tgaagttgga    360
   ggaggagatg cagaattccg acatgactca ggatatgaag ttggaggagg agatgcagaa    420
   ttccgacatg actcaggata tgaagttgga ggaggagatg cagaattccg acatgactca    480
   ggatatgaag ttggaggagg agatgcagaa ttccgacatg actcaggata tgaagttgga    540
   ggaggagatg cagaattccg acatgactca ggatatgaag ttggaggagg agatgcagaa    600
   ttccgacatg actcaggata tgaagttgga ggaggagatg cagaattccg acatgactca 660
   ggatatgaag ttggaggagg agatgcagaa ttccgacatg actcaggata tgaagttgga 720
   ggaggagatg cagaattccg acatgactca ggatatgaag ttggaggagg agatgcagaa 780
   ttccgacatg actcaggata tgaagttgga ggaggagatg cagaattccg acatgactca 840
   ggatatgaag ttggaggagg agatgcagaa ttccgacatg actcaggata tgaagttgga 900
   ggaggagatg cagaattccg acatgactca ggatatgaag ttggaggagg agatgcagaa 960
   ttccgacatg actcaggata tgaagttgga ggaggagatg cagaattccg acatgactca 1020
   ggatatgaag ttggaggagg agatgcagaa ttccgacatg actcaggata tgaagttgga 1080
   ggaggagatg cagaattccg acatgactca ggatatgaag ttggaggagg agatgcagaa 1140
   ttccgacatg actcaggata tgaagttgga ggaggagatg cagaattccg acatgactca 1200
   ggatatgaag ttggaggagg agatgcagaa ttccgacatg actcaggata tgaagttgga 1260
   ggaggagatg cagaattccg acatgactca ggatatgaag ttggaggagg agatgcagaa 1320
   ttccgacatg actcaggata tgaagttgga ggaggagatg cagaattccg acatgactca 1380
   ggatatgaag ttggaggagg agatgcagaa ttccgacatg actcaggata tgaagttgga 1440
   ggaggagatg cagaattccg acatgactca ggatatgaag ttggaggagg agatgcagaa 1500
   ttccgacatg actcaggata tgaagttgga ggaggagatg cagaattccg acatgactca 1560
   ggatatgaag ttggaggagg agatgcagaa ttccgacatg actcaggata tgaagttgga 1620
   ggaggagatg cagaattccg acatgactca ggatatgaag ttggaggagg agatgcagaa 1680
   ttccgacatg actcaggata tgaagttgga ggaggagatg cagaattccg acatgactca 1740
   ggatatgaag ttggaggagg agatgcagaa ttccgacatg actcaggata tgaagttgga 1800
   ggaggagatg cagaattccg acatgactca ggatatgaag ttggaggagg agatgcagaa 1860
   ttccgacatg actcaggata tgaagttgga ggaggagatg cagaattccg acatgactca 1920
   ggatatgaag ttggaggagg agatgcagaa ttccgacatg actcaggata tgaagttgga 1980
   ggaggagatg cagaattccg acatgactca ggatatgaag ttggaggagg aaactacacc 2040
   atgaacttgg cctcgcagaa ctggaacaac tacgatccaa ccgaggaaat ccccgctcct 2100
   ttgggaactc ccgacttcgt gggacgtatc caaggtgtcc tgacacagac tacacgtcgc 2160
   gacggctcta ctcgcggaca caaggccact gtgtcgacag gttccgtcca cttcacccct 2220
   aagctgggct ctgtgcaatt cagcacagac acctcaaacg atttcgagac tggccagaac 2280
   accaggttca ctcccgtggg tgtcgttcaa gacggcagca ccactcacca gaacgaaccc 2340
   cagcaatggg tcctccctga ctactcgggc agggattccc acaacgttca cttggctccg 2400
   gccgtggctc caaccttccc tggagagcag ttgctgttct tcagatccac tatgccaggc 2460
   tgctctggat acccgaacat gaacctcgac tgtctcttgc ctcaggagtg ggtgcaacac 2520
   ttctaccagg aatctgcccc agctcaaagc gacgtcgctc tgctccgttt cgttaacccc 2580
   gataccggtc gcgtgctctt cgagtgtaag ttgcacaagt ctggttacgt cactgttgcc 2640
   cacacaggcc agcacgacct ggtcatccct cccaacggct acttccgctt cgatagctgg 2700
   gtcaaccagt tctacacact cgccccgatg ggaaacggag ccggtcgtcg cagagccttg 2760
   taa 2763
<210> 35
   <211> 999
   <212> DNA
   <213> artificial sequence
<400> 35
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga caccggagga ggagatgcag aattccgaca tgactcagga 480
   ggaggaggat caaacgattt cgagactggc cagaacacca ggttcactcc cgtgggtgtc 540
   gttcaagacg gcagcaccac tcaccagaac gaaccccagc aatgggtcct ccctgactac 600
   tcgggcaggg attcccacaa cgttcacttg gctccggccg tggctccaac cttccctgga 660
   gagcagttgc tgttcttcag atccactatg ccaggctgct ctggataccc gaacatgaac 720
   ctcgactgtc tcttgcctca ggagtgggtg caacacttct accaggaatc tgccccagct 780
   caaagcgacg tcgctctgct ccgtttcgtt aaccccgata ccggtcgcgt gctcttcgag 840
   tgtaagttgc acaagtctgg ttacgtcact gttgcccaca caggccagca cgacctggtc 900
   atccctccca acggctactt ccgcttcgat agctgggtca accagttcta cacactcgcc 960
   ccgatgggaa acggagccgg tcgtcgcaga gccttgtaa 999
<210> 36
   <211> 1323
   <212> DNA
   <213> artificial sequence
<400> 36
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga caccggagga ggagatgcag aattccgaca tgactcagga 480
   ggaggaggag atgcagaatt ccgacatgac tcaggaggag gaggagatgc agaattccga 540
   catgactcag gaggaggagg agatgcagaa ttccgacatg actcaggagg aggaggagat 600
   gcagaattcc gacatgactc aggaggagga ggagatgcag aattccgaca tgactcagga 660
   ggaggaggag atgcagaatt ccgacatgac tcaggaggag gaggagatgc agaattccga 720
   catgactcag gaggaggagg agatgcagaa ttccgacatg actcaggagg aggaggagat 780
   gcagaattcc gacatgactc aggaggagga ggatcaaacg atttcgagac tggccagaac 840
   accaggttca ctcccgtggg tgtcgttcaa gacggcagca ccactcacca gaacgaaccc 900
   cagcaatggg tcctccctga ctactcgggc agggattccc acaacgttca cttggctccg 960
   gccgtggctc caaccttccc tggagagcag ttgctgttct tcagatccac tatgccaggc 1020
   tgctctggat acccgaacat gaacctcgac tgtctcttgc ctcaggagtg ggtgcaacac 1080
   ttctaccagg aatctgcccc agctcaaagc gacgtcgctc tgctccgttt cgttaacccc 1140
   gataccggtc gcgtgctctt cgagtgtaag ttgcacaagt ctggttacgt cactgttgcc 1200
   cacacaggcc agcacgacct ggtcatccct cccaacggct acttccgctt cgatagctgg 1260
   gtcaaccagt tctacacact cgccccgatg ggaaacggag ccggtcgtcg cagagccttg 1320
   taa 1323
<210> 37
   <211> 1683
   <212> DNA
   <213> artificial sequence
<400> 37
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga caccggagga ggagatgcag aattccgaca tgactcagga 480
   ggaggaggag atgcagaatt ccgacatgac tcaggaggag gaggagatgc agaattccga 540
   catgactcag gaggaggagg agatgcagaa ttccgacatg actcaggagg aggaggagat 600
   gcagaattcc gacatgactc aggaggagga ggagatgcag aattccgaca tgactcagga 660
   ggaggaggag atgcagaatt ccgacatgac tcaggaggag gaggagatgc agaattccga 720
   catgactcag gaggaggagg agatgcagaa ttccgacatg actcaggagg aggaggagat 780
   gcagaattcc gacatgactc aggaggagga ggagatgcag aattccgaca tgactcagga 840
   ggaggaggag atgcagaatt ccgacatgac tcaggaggag gaggagatgc agaattccga 900
   catgactcag gaggaggagg agatgcagaa ttccgacatg actcaggagg aggaggagat 960
   gcagaattcc gacatgactc aggaggagga ggagatgcag aattccgaca tgactcagga 1020
   ggaggaggag atgcagaatt ccgacatgac tcaggaggag gaggagatgc agaattccga 1080
   catgactcag gaggaggagg agatgcagaa ttccgacatg actcaggagg aggaggagat 1140
   gcagaattcc gacatgactc aggaggagga ggatcaaacg atttcgagac tggccagaac 1200
   accaggttca ctcccgtggg tgtcgttcaa gacggcagca ccactcacca gaacgaaccc 1260
   cagcaatggg tcctccctga ctactcgggc agggattccc acaacgttca cttggctccg 1320
   gccgtggctc caaccttccc tggagagcag ttgctgttct tcagatccac tatgccaggc 1380
   tgctctggat acccgaacat gaacctcgac tgtctcttgc ctcaggagtg ggtgcaacac 1440
   ttctaccagg aatctgcccc agctcaaagc gacgtcgctc tgctccgttt cgttaacccc 1500
   gataccggtc gcgtgctctt cgagtgtaag ttgcacaagt ctggttacgt cactgttgcc 1560
   cacacaggcc agcacgacct ggtcatccct cccaacggct acttccgctt cgatagctgg 1620
   gtcaaccagt tctacacact cgccccgatg ggaaacggag ccggtcgtcg cagagccttg 1680
   taa 1683
<210> 38
   <211> 2403
   <212> DNA
   <213> artificial sequence
<400> 38
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga caccggagga ggagatgcag aattccgaca tgactcagga    480
   ggaggaggag atgcagaatt ccgacatgac tcaggaggag gaggagatgc agaattccga    540
   catgactcag gaggaggagg agatgcagaa ttccgacatg actcaggagg aggaggagat    600
   gcagaattcc gacatgactc aggaggagga ggagatgcag aattccgaca tgactcagga    660
   ggaggaggag atgcagaatt ccgacatgac tcaggaggag gaggagatgc agaattccga    720
   catgactcag gaggaggagg agatgcagaa ttccgacatg actcaggagg aggaggagat    780
   gcagaattcc gacatgactc aggaggagga ggagatgcag aattccgaca tgactcagga    840
   ggaggaggag atgcagaatt ccgacatgac tcaggaggag gaggagatgc agaattccga    900
   catgactcag gaggaggagg agatgcagaa ttccgacatg actcaggagg aggaggagat    960
   gcagaattcc gacatgactc aggaggagga ggagatgcag aattccgaca tgactcagga   1020
   ggaggaggag atgcagaatt ccgacatgac tcaggaggag gaggagatgc agaattccga   1080
   catgactcag gaggaggagg agatgcagaa ttccgacatg actcaggagg aggaggagat   1140
   gcagaattcc gacatgactc aggaggagga ggagatgcag aattccgaca tgactcagga   1200
   ggaggaggag atgcagaatt ccgacatgac tcaggaggag gaggagatgc agaattccga   1260
   catgactcag gaggaggagg agatgcagaa ttccgacatg actcaggagg aggaggagat   1320
   gcagaattcc gacatgactc aggaggagga ggagatgcag aattccgaca tgactcagga   1380
   ggaggaggag atgcagaatt ccgacatgac tcaggaggag gaggagatgc agaattccga   1440
   catgactcag gaggaggagg agatgcagaa ttccgacatg actcaggagg aggaggagat   1500
   gcagaattcc gacatgactc aggaggagga ggagatgcag aattccgaca tgactcagga   1560
   ggaggaggag atgcagaatt ccgacatgac tcaggaggag gaggagatgc agaattccga   1620
   catgactcag gaggaggagg agatgcagaa ttccgacatg actcaggagg aggaggagat   1680
   gcagaattcc gacatgactc aggaggagga ggagatgcag aattccgaca tgactcagga   1740
   ggaggaggag atgcagaatt ccgacatgac tcaggaggag gaggagatgc agaattccga   1800
   catgactcag gaggaggagg agatgcagaa ttccgacatg actcaggagg aggaggagat   1860
   gcagaattcc gacatgactc aggaggagga ggatcaaacg atttcgagac tggccagaac   1920
   accaggttca ctcccgtggg tgtcgttcaa gacggcagca ccactcacca gaacgaaccc   1980
   cagcaatggg tcctccctga ctactcgggc agggattccc acaacgttca cttggctccg   2040
   gccgtggctc caaccttccc tggagagcag ttgctgttct tcagatccac tatgccaggc   2100
   tgctctggat acccgaacat gaacctcgac tgtctcttgc ctcaggagtg ggtgcaacac   2160
   ttctaccagg aatctgcccc agctcaaagc gacgtcgctc tgctccgttt cgttaacccc   2220
   gataccggtc gcgtgctctt cgagtgtaag ttgcacaagt ctggttacgt cactgttgcc 2280
   cacacaggcc agcacgacct ggtcatccct cccaacggct acttccgctt cgatagctgg   2340
   gtcaaccagt tctacacact cgccccgatg ggaaacggag ccggtcgtcg cagagccttg   2400
   taa 2403
<210> 39
   <211> 990
   <212> DNA
   <213> artificial sequence
<400> 39
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatggagga 480
   ggaaacgatt tcgagactgg ccagaacacc aggttcactc ccgtgggtgt cgttcaagac 540
   ggcagcacca ctcaccagaa cgaaccccag caatgggtcc tccctgacta ctcgggcagg 600
   gattcccaca acgttcactt ggctccggcc gtggctccaa ccttccctgg agagcagttg 660
   ctgttcttca gatccactat gccaggctgc tctggatacc cgaacatgaa cctcgactgt 720
   ctcttgcctc aggagtgggt gcaacacttc taccaggaat ctgccccagc tcaaagcgac 780
   gtcgctctgc tccgtttcgt taaccccgat accggtcgcg tgctcttcga gtgtaagttg 840
   cacaagtctg gttacgtcac tgttgcccac acaggccagc acgacctggt catccctccc 900
   aacggctact tccgcttcga tagctgggtc aaccagttct acacactcgc cccgatggga 960
   aacggagccg gtcgtcgcag agccttgtaa 990
<210> 40
   <211> 1233
   <212> DNA
   <213> artificial sequence
<400> 40
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagga ggaggagatg cagaattccg acatggagga     480
   ggagatgcag aattccgaca tggaggagga gatgcagaat tccgacatgg aggaggagat     540
   gcagaattcc gacatggagg aggagatgca gaattccgac atggaggagg agatgcagaa     600
   ttccgacatg gaggaggaga tgcagaattc cgacatggag gaggagatgc agaattccga     660
   catggaggag gagatgcaga attccgacat ggaggaggag atgcagaatt ccgacatgga     720
   ggaggaaacg atttcgagac tggccagaac accaggttca ctcccgtggg tgtcgttcaa     780
   gacggcagca ccactcacca gaacgaaccc cagcaatggg tcctccctga ctactcgggc     840
   agggattccc acaacgttca cttggctccg gccgtggctc caaccttccc tggagagcag     900
   ttgctgttct tcagatccac tatgccaggc tgctctggat acccgaacat gaacctcgac     960
   tgtctcttgc ctcaggagtg ggtgcaacac ttctaccagg aatctgcccc agctcaaagc    1020
   gacgtcgctc tgctccgttt cgttaacccc gataccggtc gcgtgctctt cgagtgtaag    1080
   ttgcacaagt ctggttacgt cactgttgcc cacacaggcc agcacgacct ggtcatccct    1140
   cccaacggct acttccgctt cgatagctgg gtcaaccagt tctacacact cgccccgatg    1200
   ggaaacggag ccggtcgtcg cagagccttg taa                                  1233
<210> 41
   <211> 1503
   <212> DNA
   <213> artificial sequence
<400> 41
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatggagga 480
   ggagatgcag aattccgaca tggaggagga gatgcagaat tccgacatgg aggaggagat 540
   gcagaattcc gacatggagg aggagatgca gaattccgac atggaggagg agatgcagaa 600
   ttccgacatg gaggaggaga tgcagaattc cgacatggag gaggagatgc agaattccga 660
   catggaggag gagatgcaga attccgacat ggaggaggag atgcagaatt ccgacatgga 720
   ggaggagatg cagaattccg acatggagga ggagatgcag aattccgaca tggaggagga 780
   gatgcagaat tccgacatgg aggaggagat gcagaattcc gacatggagg aggagatgca 840
   gaattccgac atggaggagg agatgcagaa ttccgacatg gaggaggaga tgcagaattc 900
   cgacatggag gaggagatgc agaattccga catggaggag gagatgcaga attccgacat 960
   ggaggaggag atgcagaatt ccgacatgga ggaggaaacg atttcgagac tggccagaac 1020
   accaggttca ctcccgtggg tgtcgttcaa gacggcagca ccactcacca gaacgaaccc 1080
   cagcaatggg tcctccctga ctactcgggc agggattccc acaacgttca cttggctccg 1140
   gccgtggctc caaccttccc tggagagcag ttgctgttct tcagatccac tatgccaggc 1200
   tgctctggat acccgaacat gaacctcgac tgtctcttgc ctcaggagtg ggtgcaacac 1260
   ttctaccagg aatctgcccc agctcaaagc gacgtcgctc tgctccgttt cgttaacccc 1320
   gataccggtc gcgtgctctt cgagtgtaag ttgcacaagt ctggttacgt cactgttgcc 1380
   cacacaggcc agcacgacct ggtcatccct cccaacggct acttccgctt cgatagctgg 1440
   gtcaaccagt tctacacact cgccccgatg ggaaacggag ccggtcgtcg cagagccttg 1500
   taa 1503
<210> 42
   <211> 2043
   <212> DNA
   <213> artificial sequence
<400> 42
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga cacctcagga ggaggagatg cagaattccg acatggagga    480
   ggagatgcag aattccgaca tggaggagga gatgcagaat tccgacatgg aggaggagat    540
   gcagaattcc gacatggagg aggagatgca gaattccgac atggaggagg agatgcagaa    600
   ttccgacatg gaggaggaga tgcagaattc cgacatggag gaggagatgc agaattccga    660
   catggaggag gagatgcaga attccgacat ggaggaggag atgcagaatt ccgacatgga    720
   ggaggagatg cagaattccg acatggagga ggagatgcag aattccgaca tggaggagga    780
   gatgcagaat tccgacatgg aggaggagat gcagaattcc gacatggagg aggagatgca     840
   gaattccgac atggaggagg agatgcagaa ttccgacatg gaggaggaga tgcagaattc     900
   cgacatggag gaggagatgc agaattccga catggaggag gagatgcaga attccgacat     960
   ggaggaggag atgcagaatt ccgacatgga ggaggagatg cagaattccg acatggagga    1020
   ggagatgcag aattccgaca tggaggagga gatgcagaat tccgacatgg aggaggagat    1080
   gcagaattcc gacatggagg aggagatgca gaattccgac atggaggagg agatgcagaa    1140
   ttccgacatg gaggaggaga tgcagaattc cgacatggag gaggagatgc agaattccga    1200
   catggaggag gagatgcaga attccgacat ggaggaggag atgcagaatt ccgacatgga    1260
   ggaggagatg cagaattccg acatggagga ggagatgcag aattccgaca tggaggagga    1320
   gatgcagaat tccgacatgg aggaggagat gcagaattcc gacatggagg aggagatgca    1380
   gaattccgac atggaggagg agatgcagaa ttccgacatg gaggaggaga tgcagaattc    1440
   cgacatggag gaggagatgc agaattccga catggaggag gagatgcaga attccgacat    1500
   ggaggaggag atgcagaatt ccgacatgga ggaggaaacg atttcgagac tggccagaac    1560
   accaggttca ctcccgtggg tgtcgttcaa gacggcagca ccactcacca gaacgaaccc    1620
   cagcaatggg tcctccctga ctactcgggc agggattccc acaacgttca cttggctccg    1680
   gccgtggctc caaccttccc tggagagcag ttgctgttct tcagatccac tatgccaggc    1740
   tgctctggat acccgaacat gaacctcgac tgtctcttgc ctcaggagtg ggtgcaacac    1800
   ttctaccagg aatctgcccc agctcaaagc gacgtcgctc tgctccgttt cgttaacccc    1860
   gataccggtc gcgtgctctt cgagtgtaag ttgcacaagt ctggttacgt cactgttgcc    1920
   cacacaggcc agcacgacct ggtcatccct cccaacggct acttccgctt cgatagctgg    1980
   gtcaaccagt tctacacact cgccccgatg ggaaacggag ccggtcgtcg cagagccttg    2040
   taa                                                                  2043
<210> 43
   <211> 1008
   <212> DNA
   <213> artificial sequence
<400> 43
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac ggaggaggag atgcagaatt ccgacatgac     480
   tcaggatatg aagttggagg aggagatttc gagactggcc agaacaccag gttcactccc     540
   gtgggtgtcg ttcaagacgg cagcaccact caccagaacg aaccccagca atgggtcctc     600
   cctgactact cgggcaggga ttcccacaac gttcacttgg ctccggccgt ggctccaacc     660
   ttccctggag agcagttgct gttcttcaga tccactatgc caggctgctc tggatacccg    720
   aacatgaacc tcgactgtct cttgcctcag gagtgggtgc aacacttcta ccaggaatct     780
   gccccagctc aaagcgacgt cgctctgctc cgtttcgtta accccgatac cggtcgcgtg     840
   ctcttcgagt gtaagttgca caagtctggt tacgtcactg ttgcccacac aggccagcac     900
   gacctggtca tccctcccaa cggctacttc cgcttcgata gctgggtcaa ccagttctac     960
   acactcgccc cgatgggaaa cggagccggt cgtcgcagag ccttgtaa                 1008
<210> 44
   <211> 1413
   <212> DNA
   <213> artificial sequence
<400> 44
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac ggaggaggag atgcagaatt ccgacatgac 480
   tcaggatatg aagttggagg aggagatgca gaattccgac atgactcagg atatgaagtt 540
   ggaggaggag atgcagaatt ccgacatgac tcaggatatg aagttggagg aggagatgca 600
   gaattccgac atgactcagg atatgaagtt ggaggaggag atgcagaatt ccgacatgac 660
   tcaggatatg aagttggagg aggagatgca gaattccgac atgactcagg atatgaagtt 720
   ggaggaggag atgcagaatt ccgacatgac tcaggatatg aagttggagg aggagatgca 780
   gaattccgac atgactcagg atatgaagtt ggaggaggag atgcagaatt ccgacatgac 840
   tcaggatatg aagttggagg aggagatgca gaattccgac atgactcagg atatgaagtt 900
   ggaggaggag atttcgagac tggccagaac accaggttca ctcccgtggg tgtcgttcaa 960
   gacggcagca ccactcacca gaacgaaccc cagcaatggg tcctccctga ctactcgggc 1020
   agggattccc acaacgttca cttggctccg gccgtggctc caaccttccc tggagagcag 1080
   ttgctgttct tcagatccac tatgccaggc tgctctggat acccgaacat gaacctcgac 1140
   tgtctcttgc ctcaggagtg ggtgcaacac ttctaccagg aatctgcccc agctcaaagc 1200
   gacgtcgctc tgctccgttt cgttaacccc gataccggtc gcgtgctctt cgagtgtaag 1260
   ttgcacaagt ctggttacgt cactgttgcc cacacaggcc agcacgacct ggtcatccct 1320
   cccaacggct acttccgctt cgatagctgg gtcaaccagt tctacacact cgccccgatg 1380
   ggaaacggag ccggtcgtcg cagagccttg taa 1413
<210> 45
   <211> 1863
   <212> DNA
   <213> artificial sequence
<400> 45
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga cacctcaaac ggaggaggag atgcagaatt ccgacatgac    480
   tcaggatatg aagttggagg aggagatgca gaattccgac atgactcagg atatgaagtt    540
   ggaggaggag atgcagaatt ccgacatgac tcaggatatg aagttggagg aggagatgca    600
   gaattccgac atgactcagg atatgaagtt ggaggaggag atgcagaatt ccgacatgac    660
   tcaggatatg aagttggagg aggagatgca gaattccgac atgactcagg atatgaagtt    720
   ggaggaggag atgcagaatt ccgacatgac tcaggatatg aagttggagg aggagatgca    780
   gaattccgac atgactcagg atatgaagtt ggaggaggag atgcagaatt ccgacatgac    840
   tcaggatatg aagttggagg aggagatgca gaattccgac atgactcagg atatgaagtt    900
   ggaggaggag atgcagaatt ccgacatgac tcaggatatg aagttggagg aggagatgca    960
   gaattccgac atgactcagg atatgaagtt ggaggaggag atgcagaatt ccgacatgac   1020
   tcaggatatg aagttggagg aggagatgca gaattccgac atgactcagg atatgaagtt   1080
   ggaggaggag atgcagaatt ccgacatgac tcaggatatg aagttggagg aggagatgca   1140
   gaattccgac atgactcagg atatgaagtt ggaggaggag atgcagaatt ccgacatgac   1200
   tcaggatatg aagttggagg aggagatgca gaattccgac atgactcagg atatgaagtt   1260
   ggaggaggag atgcagaatt ccgacatgac tcaggatatg aagttggagg aggagatgca 1320
   gaattccgac atgactcagg atatgaagtt ggaggaggag atttcgagac tggccagaac 1380
   accaggttca ctcccgtggg tgtcgttcaa gacggcagca ccactcacca gaacgaaccc 1440
   cagcaatggg tcctccctga ctactcgggc agggattccc acaacgttca cttggctccg 1500
   gccgtggctc caaccttccc tggagagcag ttgctgttct tcagatccac tatgccaggc 1560
   tgctctggat acccgaacat gaacctcgac tgtctcttgc ctcaggagtg ggtgcaacac 1620
   ttctaccagg aatctgcccc agctcaaagc gacgtcgctc tgctccgttt cgttaacccc 1680
   gataccggtc gcgtgctctt cgagtgtaag ttgcacaagt ctggttacgt cactgttgcc 1740
   cacacaggcc agcacgacct ggtcatccct cccaacggct acttccgctt cgatagctgg 1800
   gtcaaccagt tctacacact cgccccgatg ggaaacggag ccggtcgtcg cagagccttg 1860
   taa 1863
<210> 46
   <211> 2763
   <212> DNA
   <213> artificial sequence
<400> 46
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac ggaggaggag atgcagaatt ccgacatgac     480
   tcaggatatg aagttggagg aggagatgca gaattccgac atgactcagg atatgaagtt     540
   ggaggaggag atgcagaatt ccgacatgac tcaggatatg aagttggagg aggagatgca     600
   gaattccgac atgactcagg atatgaagtt ggaggaggag atgcagaatt ccgacatgac     660
   tcaggatatg aagttggagg aggagatgca gaattccgac atgactcagg atatgaagtt     720
   ggaggaggag atgcagaatt ccgacatgac tcaggatatg aagttggagg aggagatgca     780
   gaattccgac atgactcagg atatgaagtt ggaggaggag atgcagaatt ccgacatgac     840
   tcaggatatg aagttggagg aggagatgca gaattccgac atgactcagg atatgaagtt     900
   ggaggaggag atgcagaatt ccgacatgac tcaggatatg aagttggagg aggagatgca     960
   gaattccgac atgactcagg atatgaagtt ggaggaggag atgcagaatt ccgacatgac    1020
   tcaggatatg aagttggagg aggagatgca gaattccgac atgactcagg atatgaagtt 1080
   ggaggaggag atgcagaatt ccgacatgac tcaggatatg aagttggagg aggagatgca 1140
   gaattccgac atgactcagg atatgaagtt ggaggaggag atgcagaatt ccgacatgac 1200
   tcaggatatg aagttggagg aggagatgca gaattccgac atgactcagg atatgaagtt 1260
   ggaggaggag atgcagaatt ccgacatgac tcaggatatg aagttggagg aggagatgca 1320
   gaattccgac atgactcagg atatgaagtt ggaggaggag atgcagaatt ccgacatgac 1380
   tcaggatatg aagttggagg aggagatgca gaattccgac atgactcagg atatgaagtt 1440
   ggaggaggag atgcagaatt ccgacatgac tcaggatatg aagttggagg aggagatgca 1500
   gaattccgac atgactcagg atatgaagtt ggaggaggag atgcagaatt ccgacatgac 1560
   tcaggatatg aagttggagg aggagatgca gaattccgac atgactcagg atatgaagtt 1620
   ggaggaggag atgcagaatt ccgacatgac tcaggatatg aagttggagg aggagatgca 1680
   gaattccgac atgactcagg atatgaagtt ggaggaggag atgcagaatt ccgacatgac 1740
   tcaggatatg aagttggagg aggagatgca gaattccgac atgactcagg atatgaagtt 1800
   ggaggaggag atgcagaatt ccgacatgac tcaggatatg aagttggagg aggagatgca 1860
   gaattccgac atgactcagg atatgaagtt ggaggaggag atgcagaatt ccgacatgac 1920
   tcaggatatg aagttggagg aggagatgca gaattccgac atgactcagg atatgaagtt 1980
   ggaggaggag atgcagaatt ccgacatgac tcaggatatg aagttggagg aggagatgca 2040
   gaattccgac atgactcagg atatgaagtt ggaggaggag atgcagaatt ccgacatgac 2100
   tcaggatatg aagttggagg aggagatgca gaattccgac atgactcagg atatgaagtt 2160
   ggaggaggag atgcagaatt ccgacatgac tcaggatatg aagttggagg aggagatgca 2220
   gaattccgac atgactcagg atatgaagtt ggaggaggag atttcgagac tggccagaac 2280
   accaggttca ctcccgtggg tgtcgttcaa gacggcagca ccactcacca gaacgaaccc 2340
   cagcaatggg tcctccctga ctactcgggc agggattccc acaacgttca cttggctccg 2400
   gccgtggctc caaccttccc tggagagcag ttgctgttct tcagatccac tatgccaggc 2460
   tgctctggat acccgaacat gaacctcgac tgtctcttgc ctcaggagtg ggtgcaacac 2520
   ttctaccagg aatctgcccc agctcaaagc gacgtcgctc tgctccgttt cgttaacccc 2580
   gataccggtc gcgtgctctt cgagtgtaag ttgcacaagt ctggttacgt cactgttgcc 2640
   cacacaggcc agcacgacct ggtcatccct cccaacggct acttccgctt cgatagctgg 2700
   gtcaaccagt tctacacact cgccccgatg ggaaacggag ccggtcgtcg cagagccttg 2760
   taa 2763
<210> 47
   <211> 1017
   <212> DNA
   <213> artificial sequence
<400> 47
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatggaggag gagatgcaga attccgacat     480
   gactcaggat atgaagttca tcatcaagga ggaggattcg agactggcca gaacaccagg     540
   ttcactcccg tgggtgtcgt tcaagacggc agcaccactc accagaacga accccagcaa     600
   tgggtcctcc ctgactactc gggcagggat tcccacaacg ttcacttggc tccggccgtg     660
   gctccaacct tccctggaga gcagttgctg ttcttcagat ccactatgcc aggctgctct     720
   ggatacccga acatgaacct cgactgtctc ttgcctcagg agtgggtgca acacttctac     780
   caggaatctg ccccagctca aagcgacgtc gctctgctcc gtttcgttaa ccccgatacc     840
   ggtcgcgtgc tcttcgagtg taagttgcac aagtctggtt acgtcactgt tgcccacaca     900
   ggccagcacg acctggtcat ccctcccaac ggctacttcc gcttcgatag ctgggtcaac     960
   cagttctaca cactcgcccc gatgggaaac ggagccggtc gtcgcagagc cttgtaa       1017
<210> 48
   <211> 1503
   <212> DNA
   <213> artificial sequence
<400> 48
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatggaggag gagatgcaga attccgacat     480
   gactcaggat atgaagttca tcatcaagga ggaggagatg cagaattccg acatgactca     540
   ggatatgaag ttcatcatca aggaggagga gatgcagaat tccgacatga ctcaggatat     600
   gaagttcatc atcaaggagg aggagatgca gaattccgac atgactcagg atatgaagtt     660
   catcatcaag gaggaggaga tgcagaattc cgacatgact caggatatga agttcatcat     720
   caaggaggag gagatgcaga attccgacat gactcaggat atgaagttca tcatcaagga     780
   ggaggagatg cagaattccg acatgactca ggatatgaag ttcatcatca aggaggagga     840
   gatgcagaat tccgacatga ctcaggatat gaagttcatc atcaaggagg aggagatgca     900
   gaattccgac atgactcagg atatgaagtt catcatcaag gaggaggaga tgcagaattc     960
   cgacatgact caggatatga agttcatcat caaggaggag gattcgagac tggccagaac    1020
   accaggttca ctcccgtggg tgtcgttcaa gacggcagca ccactcacca gaacgaaccc    1080
   cagcaatggg tcctccctga ctactcgggc agggattccc acaacgttca cttggctccg    1140
   gccgtggctc caaccttccc tggagagcag ttgctgttct tcagatccac tatgccaggc    1200
   tgctctggat acccgaacat gaacctcgac tgtctcttgc ctcaggagtg ggtgcaacac    1260
   ttctaccagg aatctgcccc agctcaaagc gacgtcgctc tgctccgttt cgttaacccc    1320
   gataccggtc gcgtgctctt cgagtgtaag ttgcacaagt ctggttacgt cactgttgcc    1380
   cacacaggcc agcacgacct ggtcatccct cccaacggct acttccgctt cgatagctgg    1440
   gtcaaccagt tctacacact cgccccgatg ggaaacggag ccggtcgtcg cagagccttg    1500
   taa                                                                  1503
<210> 49
   <211> 2043
   <212> DNA
   <213> artificial sequence
<400> 49
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac gatggaggag gagatgcaga attccgacat 480
   gactcaggat atgaagttca tcatcaagga ggaggagatg cagaattccg acatgactca 540
   ggatatgaag ttcatcatca aggaggagga gatgcagaat tccgacatga ctcaggatat 600
   gaagttcatc atcaaggagg aggagatgca gaattccgac atgactcagg atatgaagtt 660
   catcatcaag gaggaggaga tgcagaattc cgacatgact caggatatga agttcatcat     720
   caaggaggag gagatgcaga attccgacat gactcaggat atgaagttca tcatcaagga     780
   ggaggagatg cagaattccg acatgactca ggatatgaag ttcatcatca aggaggagga     840
   gatgcagaat tccgacatga ctcaggatat gaagttcatc atcaaggagg aggagatgca     900
   gaattccgac atgactcagg atatgaagtt catcatcaag gaggaggaga tgcagaattc     960
   cgacatgact caggatatga agttcatcat caaggaggag gagatgcaga attccgacat    1020
   gactcaggat atgaagttca tcatcaagga ggaggagatg cagaattccg acatgactca    1080
   ggatatgaag ttcatcatca aggaggagga gatgcagaat tccgacatga ctcaggatat    1140
   gaagttcatc atcaaggagg aggagatgca gaattccgac atgactcagg atatgaagtt    1200
   catcatcaag gaggaggaga tgcagaattc cgacatgact caggatatga agttcatcat    1260
   caaggaggag gagatgcaga attccgacat gactcaggat atgaagttca tcatcaagga    1320
   ggaggagatg cagaattccg acatgactca ggatatgaag ttcatcatca aggaggagga    1380
   gatgcagaat tccgacatga ctcaggatat gaagttcatc atcaaggagg aggagatgca    1440
   gaattccgac atgactcagg atatgaagtt catcatcaag gaggaggaga tgcagaattc    1500
   cgacatgact caggatatga agttcatcat caaggaggag gattcgagac tggccagaac    1560
   accaggttca ctcccgtggg tgtcgttcaa gacggcagca ccactcacca gaacgaaccc    1620
   cagcaatggg tcctccctga ctactcgggc agggattccc acaacgttca cttggctccg    1680
   gccgtggctc caaccttccc tggagagcag ttgctgttct tcagatccac tatgccaggc    1740
   tgctctggat acccgaacat gaacctcgac tgtctcttgc ctcaggagtg ggtgcaacac    1800
   ttctaccagg aatctgcccc agctcaaagc gacgtcgctc tgctccgttt cgttaacccc    1860
   gataccggtc gcgtgctctt cgagtgtaag ttgcacaagt ctggttacgt cactgttgcc    1920
   cacacaggcc agcacgacct ggtcatccct cccaacggct acttccgctt cgatagctgg    1980
   gtcaaccagt tctacacact cgccccgatg ggaaacggag ccggtcgtcg cagagccttg    2040
   taa                                                                  2043
<210> 50
   <211> 3123
   <212> DNA
   <213> artificial sequence
<400> 50
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac gatggaggag gagatgcaga attccgacat 480
   gactcaggat atgaagttca tcatcaagga ggaggagatg cagaattccg acatgactca 540
   ggatatgaag ttcatcatca aggaggagga gatgcagaat tccgacatga ctcaggatat 600
   gaagttcatc atcaaggagg aggagatgca gaattccgac atgactcagg atatgaagtt 660
   catcatcaag gaggaggaga tgcagaattc cgacatgact caggatatga agttcatcat 720
   caaggaggag gagatgcaga attccgacat gactcaggat atgaagttca tcatcaagga 780
   ggaggagatg cagaattccg acatgactca ggatatgaag ttcatcatca aggaggagga 840
   gatgcagaat tccgacatga ctcaggatat gaagttcatc atcaaggagg aggagatgca 900
   gaattccgac atgactcagg atatgaagtt catcatcaag gaggaggaga tgcagaattc 960
   cgacatgact caggatatga agttcatcat caaggaggag gagatgcaga attccgacat 1020
   gactcaggat atgaagttca tcatcaagga ggaggagatg cagaattccg acatgactca 1080
   ggatatgaag ttcatcatca aggaggagga gatgcagaat tccgacatga ctcaggatat 1140
   gaagttcatc atcaaggagg aggagatgca gaattccgac atgactcagg atatgaagtt 1200
   catcatcaag gaggaggaga tgcagaattc cgacatgact caggatatga agttcatcat 1260
   caaggaggag gagatgcaga attccgacat gactcaggat atgaagttca tcatcaagga 1320
   ggaggagatg cagaattccg acatgactca ggatatgaag ttcatcatca aggaggagga 1380
   gatgcagaat tccgacatga ctcaggatat gaagttcatc atcaaggagg aggagatgca 1440
   gaattccgac atgactcagg atatgaagtt catcatcaag gaggaggaga tgcagaattc 1500
   cgacatgact caggatatga agttcatcat caaggaggag gagatgcaga attccgacat 1560
   gactcaggat atgaagttca tcatcaagga ggaggagatg cagaattccg acatgactca 1620
   ggatatgaag ttcatcatca aggaggagga gatgcagaat tccgacatga ctcaggatat 1680
   gaagttcatc atcaaggagg aggagatgca gaattccgac atgactcagg atatgaagtt 1740
   catcatcaag gaggaggaga tgcagaattc cgacatgact caggatatga agttcatcat 1800
   caaggaggag gagatgcaga attccgacat gactcaggat atgaagttca tcatcaagga 1860
   ggaggagatg cagaattccg acatgactca ggatatgaag ttcatcatca aggaggagga 1920
   gatgcagaat tccgacatga ctcaggatat gaagttcatc atcaaggagg aggagatgca 1980
   gaattccgac atgactcagg atatgaagtt catcatcaag gaggaggaga tgcagaattc 2040
   cgacatgact caggatatga agttcatcat caaggaggag gagatgcaga attccgacat 2100
   gactcaggat atgaagttca tcatcaagga ggaggagatg cagaattccg acatgactca   2160
   ggatatgaag ttcatcatca aggaggagga gatgcagaat tccgacatga ctcaggatat   2220
   gaagttcatc atcaaggagg aggagatgca gaattccgac atgactcagg atatgaagtt   2280
   catcatcaag gaggaggaga tgcagaattc cgacatgact caggatatga agttcatcat   2340
   caaggaggag gagatgcaga attccgacat gactcaggat atgaagttca tcatcaagga   2400
   ggaggagatg cagaattccg acatgactca ggatatgaag ttcatcatca aggaggagga   2460
   gatgcagaat tccgacatga ctcaggatat gaagttcatc atcaaggagg aggagatgca   2520
   gaattccgac atgactcagg atatgaagtt catcatcaag gaggaggaga tgcagaattc   2580
   cgacatgact caggatatga agttcatcat caaggaggag gattcgagac tggccagaac   2640
   accaggttca ctcccgtggg tgtcgttcaa gacggcagca ccactcacca gaacgaaccc   2700
   cagcaatggg tcctccctga ctactcgggc agggattccc acaacgttca cttggctccg   2760
   gccgtggctc caaccttccc tggagagcag ttgctgttct tcagatccac tatgccaggc   2820
   tgctctggat acccgaacat gaacctcgac tgtctcttgc ctcaggagtg ggtgcaacac   2880
   ttctaccagg aatctgcccc agctcaaagc gacgtcgctc tgctccgttt cgttaacccc   2940
   gataccggtc gcgtgctctt cgagtgtaag ttgcacaagt ctggttacgt cactgttgcc   3000
   cacacaggcc agcacgacct ggtcatccct cccaacggct acttccgctt cgatagctgg   3060
   gtcaaccagt tctacacact cgccccgatg ggaaacggag ccggtcgtcg cagagccttg   3120
   taa                                                                 3123
<210> 51
   <211> 1008
   <212> DNA
   <213> artificial sequence
<400> 51
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggagga ggagatgcag aattccgaca tgactcagga     540
   tatgaagttg gaggaggagg cagcaccact caccagaacg aaccccagca atgggtcctc     600
   cctgactact cgggcaggga ttcccacaac gttcacttgg ctccggccgt ggctccaacc     660
   ttccctggag agcagttgct gttcttcaga tccactatgc caggctgctc tggatacccg     720
   aacatgaacc tcgactgtct cttgcctcag gagtgggtgc aacacttcta ccaggaatct     780
   gccccagctc aaagcgacgt cgctctgctc cgtttcgtta accccgatac cggtcgcgtg     840
   ctcttcgagt gtaagttgca caagtctggt tacgtcactg ttgcccacac aggccagcac     900
   gacctggtca tccctcccaa cggctacttc cgcttcgata gctgggtcaa ccagttctac     960
   acactcgccc cgatgggaaa cggagccggt cgtcgcagag ccttgtaa                1008
<210> 52
   <211> 1413
   <212> DNA
   <213> artificial sequence
<400> 52
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggagga ggagatgcag aattccgaca tgactcagga     540
   tatgaagttg gaggaggaga tgcagaattc cgacatgact caggatatga agttggagga     600
   ggagatgcag aattccgaca tgactcagga tatgaagttg gaggaggaga tgcagaattc     660
   cgacatgact caggatatga agttggagga ggagatgcag aattccgaca tgactcagga     720
   tatgaagttg gaggaggaga tgcagaattc cgacatgact caggatatga agttggagga     780
   ggagatgcag aattccgaca tgactcagga tatgaagttg gaggaggaga tgcagaattc     840
   cgacatgact caggatatga agttggagga ggagatgcag aattccgaca tgactcagga     900
   tatgaagttg gaggaggaga tgcagaattc cgacatgact caggatatga agttggagga     960
   ggaggcagca ccactcacca gaacgaaccc cagcaatggg tcctccctga ctactcgggc    1020
   agggattccc acaacgttca cttggctccg gccgtggctc caaccttccc tggagagcag    1080
   ttgctgttct tcagatccac tatgccaggc tgctctggat acccgaacat gaacctcgac    1140
   tgtctcttgc ctcaggagtg ggtgcaacac ttctaccagg aatctgcccc agctcaaagc    1200
   gacgtcgctc tgctccgttt cgttaacccc gataccggtc gcgtgctctt cgagtgtaag    1260
   ttgcacaagt ctggttacgt cactgttgcc cacacaggcc agcacgacct ggtcatccct    1320
   cccaacggct acttccgctt cgatagctgg gtcaaccagt tctacacact cgccccgatg    1380
   ggaaacggag ccggtcgtcg cagagccttg taa                                 1413
<210> 53
   <211> 1863
   <212> DNA
   <213> artificial sequence
<400> 53
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggagga ggagatgcag aattccgaca tgactcagga     540
   tatgaagttg gaggaggaga tgcagaattc cgacatgact caggatatga agttggagga     600
   ggagatgcag aattccgaca tgactcagga tatgaagttg gaggaggaga tgcagaattc     660
   cgacatgact caggatatga agttggagga ggagatgcag aattccgaca tgactcagga     720
   tatgaagttg gaggaggaga tgcagaattc cgacatgact caggatatga agttggagga     780
   ggagatgcag aattccgaca tgactcagga tatgaagttg gaggaggaga tgcagaattc     840
   cgacatgact caggatatga agttggagga ggagatgcag aattccgaca tgactcagga     900
   tatgaagttg gaggaggaga tgcagaattc cgacatgact caggatatga agttggagga     960
   ggagatgcag aattccgaca tgactcagga tatgaagttg gaggaggaga tgcagaattc    1020
   cgacatgact caggatatga agttggagga ggagatgcag aattccgaca tgactcagga    1080
   tatgaagttg gaggaggaga tgcagaattc cgacatgact caggatatga agttggagga    1140
   ggagatgcag aattccgaca tgactcagga tatgaagttg gaggaggaga tgcagaattc    1200
   cgacatgact caggatatga agttggagga ggagatgcag aattccgaca tgactcagga    1260
   tatgaagttg gaggaggaga tgcagaattc cgacatgact caggatatga agttggagga    1320
   ggagatgcag aattccgaca tgactcagga tatgaagttg gaggaggaga tgcagaattc    1380
   cgacatgact caggatatga agttggagga ggaggcagca ccactcacca gaacgaaccc    1440
   cagcaatggg tcctccctga ctactcgggc agggattccc acaacgttca cttggctccg    1500
   gccgtggctc caaccttccc tggagagcag ttgctgttct tcagatccac tatgccaggc 1560
   tgctctggat acccgaacat gaacctcgac tgtctcttgc ctcaggagtg ggtgcaacac 1620
   ttctaccagg aatctgcccc agctcaaagc gacgtcgctc tgctccgttt cgttaacccc 1680
   gataccggtc gcgtgctctt cgagtgtaag ttgcacaagt ctggttacgt cactgttgcc 1740
   cacacaggcc agcacgacct ggtcatccct cccaacggct acttccgctt cgatagctgg 1800
   gtcaaccagt tctacacact cgccccgatg ggaaacggag ccggtcgtcg cagagccttg 1860
   taa 1863
<210> 54
   <211> 2763
   <212> DNA
   <213> artificial sequence
<400> 54
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga cacctcaaac gatttcgaga ctggccagaa caccaggttc    480
   actcccgtgg gtgtcgttca agacggagga ggagatgcag aattccgaca tgactcagga    540
   tatgaagttg gaggaggaga tgcagaattc cgacatgact caggatatga agttggagga    600
   ggagatgcag aattccgaca tgactcagga tatgaagttg gaggaggaga tgcagaattc    660
   cgacatgact caggatatga agttggagga ggagatgcag aattccgaca tgactcagga    720
   tatgaagttg gaggaggaga tgcagaattc cgacatgact caggatatga agttggagga    780
   ggagatgcag aattccgaca tgactcagga tatgaagttg gaggaggaga tgcagaattc    840
   cgacatgact caggatatga agttggagga ggagatgcag aattccgaca tgactcagga    900
   tatgaagttg gaggaggaga tgcagaattc cgacatgact caggatatga agttggagga    960
   ggagatgcag aattccgaca tgactcagga tatgaagttg gaggaggaga tgcagaattc   1020
   cgacatgact caggatatga agttggagga ggagatgcag aattccgaca tgactcagga   1080
   tatgaagttg gaggaggaga tgcagaattc cgacatgact caggatatga agttggagga   1140
   ggagatgcag aattccgaca tgactcagga tatgaagttg gaggaggaga tgcagaattc   1200
   cgacatgact caggatatga agttggagga ggagatgcag aattccgaca tgactcagga   1260
   tatgaagttg gaggaggaga tgcagaattc cgacatgact caggatatga agttggagga 1320
   ggagatgcag aattccgaca tgactcagga tatgaagttg gaggaggaga tgcagaattc 1380
   cgacatgact caggatatga agttggagga ggagatgcag aattccgaca tgactcagga 1440
   tatgaagttg gaggaggaga tgcagaattc cgacatgact caggatatga agttggagga 1500
   ggagatgcag aattccgaca tgactcagga tatgaagttg gaggaggaga tgcagaattc 1560
   cgacatgact caggatatga agttggagga ggagatgcag aattccgaca tgactcagga 1620
   tatgaagttg gaggaggaga tgcagaattc cgacatgact caggatatga agttggagga 1680
   ggagatgcag aattccgaca tgactcagga tatgaagttg gaggaggaga tgcagaattc 1740
   cgacatgact caggatatga agttggagga ggagatgcag aattccgaca tgactcagga 1800
   tatgaagttg gaggaggaga tgcagaattc cgacatgact caggatatga agttggagga 1860
   ggagatgcag aattccgaca tgactcagga tatgaagttg gaggaggaga tgcagaattc 1920
   cgacatgact caggatatga agttggagga ggagatgcag aattccgaca tgactcagga 1980
   tatgaagttg gaggaggaga tgcagaattc cgacatgact caggatatga agttggagga 2040
   ggagatgcag aattccgaca tgactcagga tatgaagttg gaggaggaga tgcagaattc 2100
   cgacatgact caggatatga agttggagga ggagatgcag aattccgaca tgactcagga 2160
   tatgaagttg gaggaggaga tgcagaattc cgacatgact caggatatga agttggagga 2220
   ggagatgcag aattccgaca tgactcagga tatgaagttg gaggaggaga tgcagaattc 2280
   cgacatgact caggatatga agttggagga ggaggcagca ccactcacca gaacgaaccc 2340
   cagcaatggg tcctccctga ctactcgggc agggattccc acaacgttca cttggctccg 2400
   gccgtggctc caaccttccc tggagagcag ttgctgttct tcagatccac tatgccaggc 2460
   tgctctggat acccgaacat gaacctcgac tgtctcttgc ctcaggagtg ggtgcaacac 2520
   ttctaccagg aatctgcccc agctcaaagc gacgtcgctc tgctccgttt cgttaacccc 2580
   gataccggtc gcgtgctctt cgagtgtaag ttgcacaagt ctggttacgt cactgttgcc 2640
   cacacaggcc agcacgacct ggtcatccct cccaacggct acttccgctt cgatagctgg 2700
   gtcaaccagt tctacacact cgccccgatg ggaaacggag ccggtcgtcg cagagccttg 2760
   taa 2763
<210> 55
   <211> 990
   <212> DNA
   <213> artificial sequence
<400> 55
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac gatttcgaga ctggccagaa caccaggttc 480
   actcccgtgg gtgtcgttca agacggcgga ggaggagatg cagaattccg acatggagga 540
   ggaagcacca ctcaccagaa cgaaccccag caatgggtcc tccctgacta ctcgggcagg 600
   gattcccaca acgttcactt ggctccggcc gtggctccaa ccttccctgg agagcagttg 660
   ctgttcttca gatccactat gccaggctgc tctggatacc cgaacatgaa cctcgactgt 720
   ctcttgcctc aggagtgggt gcaacacttc taccaggaat ctgccccagc tcaaagcgac 780
   gtcgctctgc tccgtttcgt taaccccgat accggtcgcg tgctcttcga gtgtaagttg 840
   cacaagtctg gttacgtcac tgttgcccac acaggccagc acgacctggt catccctccc 900
   aacggctact tccgcttcga tagctgggtc aaccagttct acacactcgc cccgatggga 960
   aacggagccg gtcgtcgcag agccttgtaa 990
<210> 56
   <211> 1233
   <212> DNA
   <213> artificial sequence
<400> 56
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac gatttcgaga ctggccagaa caccaggttc 480
   actcccgtgg gtgtcgttca agacggcgga ggaggagatg cagaattccg acatggagga 540
   ggagatgcag aattccgaca tggaggagga gatgcagaat tccgacatgg aggaggagat 600
   gcagaattcc gacatggagg aggagatgca gaattccgac atggaggagg agatgcagaa 660
   ttccgacatg gaggaggaga tgcagaattc cgacatggag gaggagatgc agaattccga 720
   catggaggag gagatgcaga attccgacat ggaggaggag atgcagaatt ccgacatgga 780
   ggaggaagca ccactcacca gaacgaaccc cagcaatggg tcctccctga ctactcgggc 840
   agggattccc acaacgttca cttggctccg gccgtggctc caaccttccc tggagagcag 900
   ttgctgttct tcagatccac tatgccaggc tgctctggat acccgaacat gaacctcgac 960
   tgtctcttgc ctcaggagtg ggtgcaacac ttctaccagg aatctgcccc agctcaaagc 1020
   gacgtcgctc tgctccgttt cgttaacccc gataccggtc gcgtgctctt cgagtgtaag 1080
   ttgcacaagt ctggttacgt cactgttgcc cacacaggcc agcacgacct ggtcatccct 1140
   cccaacggct acttccgctt cgatagctgg gtcaaccagt tctacacact cgccccgatg 1200
   ggaaacggag ccggtcgtcg cagagccttg taa 1233
<210> 57
   <211> 1503
   <212> DNA
   <213> artificial sequence
<400> 57
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcgga ggaggagatg cagaattccg acatggagga     540
   ggagatgcag aattccgaca tggaggagga gatgcagaat tccgacatgg aggaggagat     600
   gcagaattcc gacatggagg aggagatgca gaattccgac atggaggagg agatgcagaa     660
   ttccgacatg gaggaggaga tgcagaattc cgacatggag gaggagatgc agaattccga     720
   catggaggag gagatgcaga attccgacat ggaggaggag atgcagaatt ccgacatgga     780
   ggaggagatg cagaattccg acatggagga ggagatgcag aattccgaca tggaggagga     840
   gatgcagaat tccgacatgg aggaggagat gcagaattcc gacatggagg aggagatgca     900
   gaattccgac atggaggagg agatgcagaa ttccgacatg gaggaggaga tgcagaattc     960
   cgacatggag gaggagatgc agaattccga catggaggag gagatgcaga attccgacat    1020
   ggaggaggag atgcagaatt ccgacatgga ggaggaagca ccactcacca gaacgaaccc    1080
   cagcaatggg tcctccctga ctactcgggc agggattccc acaacgttca cttggctccg    1140
   gccgtggctc caaccttccc tggagagcag ttgctgttct tcagatccac tatgccaggc    1200
   tgctctggat acccgaacat gaacctcgac tgtctcttgc ctcaggagtg ggtgcaacac    1260
   ttctaccagg aatctgcccc agctcaaagc gacgtcgctc tgctccgttt cgttaacccc    1320
   gataccggtc gcgtgctctt cgagtgtaag ttgcacaagt ctggttacgt cactgttgcc    1380
   cacacaggcc agcacgacct ggtcatccct cccaacggct acttccgctt cgatagctgg    1440
   gtcaaccagt tctacacact cgccccgatg ggaaacggag ccggtcgtcg cagagccttg    1500
   taa                                                                  1503
<210> 58
   <211> 2043
   <212> DNA
   <213> artificial sequence
<400> 58
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga cacctcaaac gatttcgaga ctggccagaa caccaggttc    480
   actcccgtgg gtgtcgttca agacggcgga ggaggagatg cagaattccg acatggagga    540
   ggagatgcag aattccgaca tggaggagga gatgcagaat tccgacatgg aggaggagat    600
   gcagaattcc gacatggagg aggagatgca gaattccgac atggaggagg agatgcagaa    660
   ttccgacatg gaggaggaga tgcagaattc cgacatggag gaggagatgc agaattccga    720
   catggaggag gagatgcaga attccgacat ggaggaggag atgcagaatt ccgacatgga    780
   ggaggagatg cagaattccg acatggagga ggagatgcag aattccgaca tggaggagga    840
   gatgcagaat tccgacatgg aggaggagat gcagaattcc gacatggagg aggagatgca    900
   gaattccgac atggaggagg agatgcagaa ttccgacatg gaggaggaga tgcagaattc    960
   cgacatggag gaggagatgc agaattccga catggaggag gagatgcaga attccgacat   1020
   ggaggaggag atgcagaatt ccgacatgga ggaggagatg cagaattccg acatggagga   1080
   ggagatgcag aattccgaca tggaggagga gatgcagaat tccgacatgg aggaggagat   1140
   gcagaattcc gacatggagg aggagatgca gaattccgac atggaggagg agatgcagaa   1200
   ttccgacatg gaggaggaga tgcagaattc cgacatggag gaggagatgc agaattccga   1260
   catggaggag gagatgcaga attccgacat ggaggaggag atgcagaatt ccgacatgga   1320
   ggaggagatg cagaattccg acatggagga ggagatgcag aattccgaca tggaggagga   1380
   gatgcagaat tccgacatgg aggaggagat gcagaattcc gacatggagg aggagatgca   1440
   gaattccgac atggaggagg agatgcagaa ttccgacatg gaggaggaga tgcagaattc   1500
   cgacatggag gaggagatgc agaattccga catggaggag gagatgcaga attccgacat   1560
   ggaggaggag atgcagaatt ccgacatgga ggaggaagca ccactcacca gaacgaaccc   1620
   cagcaatggg tcctccctga ctactcgggc agggattccc acaacgttca cttggctccg   1680
   gccgtggctc caaccttccc tggagagcag ttgctgttct tcagatccac tatgccaggc   1740
   tgctctggat acccgaacat gaacctcgac tgtctcttgc ctcaggagtg ggtgcaacac   1800
   ttctaccagg aatctgcccc agctcaaagc gacgtcgctc tgctccgttt cgttaacccc   1860
   gataccggtc gcgtgctctt cgagtgtaag ttgcacaagt ctggttacgt cactgttgcc   1920
   cacacaggcc agcacgacct ggtcatccct cccaacggct acttccgctt cgatagctgg   1980
   gtcaaccagt tctacacact cgccccgatg ggaaacggag ccggtcgtcg cagagccttg   2040
   taa                                                                 2043
<210> 59
   <211> 1017
   <212> DNA
   <213> artificial sequence
<400> 59
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcagc ggaggaggag atgcagaatt ccgacatgac     540
   tcaggatatg aagttcatca tcaaggagga ggaaccactc accagaacga accccagcaa     600
   tgggtcctcc ctgactactc gggcagggat tcccacaacg ttcacttggc tccggccgtg     660
   gctccaacct tccctggaga gcagttgctg ttcttcagat ccactatgcc aggctgctct     720
   ggatacccga acatgaacct cgactgtctc ttgcctcagg agtgggtgca acacttctac     780
   caggaatctg ccccagctca aagcgacgtc gctctgctcc gtttcgttaa ccccgatacc     840
   ggtcgcgtgc tcttcgagtg taagttgcac aagtctggtt acgtcactgt tgcccacaca     900
   ggccagcacg acctggtcat ccctcccaac ggctacttcc gcttcgatag ctgggtcaac     960
   cagttctaca cactcgcccc gatgggaaac ggagccggtc gtcgcagagc cttgtaa       1017
<210> 60
   <211> 1503
   <212> DNA
   <213> artificial sequence
<400> 60
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcagc ggaggaggag atgcagaatt ccgacatgac     540
   tcaggatatg aagttcatca tcaaggagga ggagatgcag aattccgaca tgactcagga     600
   tatgaagttc atcatcaagg aggaggagat gcagaattcc gacatgactc aggatatgaa     660
   gttcatcatc aaggaggagg agatgcagaa ttccgacatg actcaggata tgaagttcat     720
   catcaaggag gaggagatgc agaattccga catgactcag gatatgaagt tcatcatcaa     780
   ggaggaggag atgcagaatt ccgacatgac tcaggatatg aagttcatca tcaaggagga     840
   ggagatgcag aattccgaca tgactcagga tatgaagttc atcatcaagg aggaggagat     900
   gcagaattcc gacatgactc aggatatgaa gttcatcatc aaggaggagg agatgcagaa     960
   ttccgacatg actcaggata tgaagttcat catcaaggag gaggagatgc agaattccga    1020
   catgactcag gatatgaagt tcatcatcaa ggaggaggaa ccactcacca gaacgaaccc    1080
   cagcaatggg tcctccctga ctactcgggc agggattccc acaacgttca cttggctccg    1140
   gccgtggctc caaccttccc tggagagcag ttgctgttct tcagatccac tatgccaggc    1200
   tgctctggat acccgaacat gaacctcgac tgtctcttgc ctcaggagtg ggtgcaacac    1260
   ttctaccagg aatctgcccc agctcaaagc gacgtcgctc tgctccgttt cgttaacccc    1320
   gataccggtc gcgtgctctt cgagtgtaag ttgcacaagt ctggttacgt cactgttgcc    1380
   cacacaggcc agcacgacct ggtcatccct cccaacggct acttccgctt cgatagctgg    1440
   gtcaaccagt tctacacact cgccccgatg ggaaacggag ccggtcgtcg cagagccttg    1500
   taa                                                                  1503
<210> 61
   <211> 2043
   <212> DNA
   <213> artificial sequence
<400> 61
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcagc ggaggaggag atgcagaatt ccgacatgac     540
   tcaggatatg aagttcatca tcaaggagga ggagatgcag aattccgaca tgactcagga     600
   tatgaagttc atcatcaagg aggaggagat gcagaattcc gacatgactc aggatatgaa     660
   gttcatcatc aaggaggagg agatgcagaa ttccgacatg actcaggata tgaagttcat     720
   catcaaggag gaggagatgc agaattccga catgactcag gatatgaagt tcatcatcaa     780
   ggaggaggag atgcagaatt ccgacatgac tcaggatatg aagttcatca tcaaggagga     840
   ggagatgcag aattccgaca tgactcagga tatgaagttc atcatcaagg aggaggagat     900
   gcagaattcc gacatgactc aggatatgaa gttcatcatc aaggaggagg agatgcagaa     960
   ttccgacatg actcaggata tgaagttcat catcaaggag gaggagatgc agaattccga    1020
   catgactcag gatatgaagt tcatcatcaa ggaggaggag atgcagaatt ccgacatgac    1080
   tcaggatatg aagttcatca tcaaggagga ggagatgcag aattccgaca tgactcagga    1140
   tatgaagttc atcatcaagg aggaggagat gcagaattcc gacatgactc aggatatgaa    1200
   gttcatcatc aaggaggagg agatgcagaa ttccgacatg actcaggata tgaagttcat    1260
   catcaaggag gaggagatgc agaattccga catgactcag gatatgaagt tcatcatcaa    1320
   ggaggaggag atgcagaatt ccgacatgac tcaggatatg aagttcatca tcaaggagga    1380
   ggagatgcag aattccgaca tgactcagga tatgaagttc atcatcaagg aggaggagat    1440
   gcagaattcc gacatgactc aggatatgaa gttcatcatc aaggaggagg agatgcagaa    1500
   ttccgacatg actcaggata tgaagttcat catcaaggag gaggagatgc agaattccga    1560
   catgactcag gatatgaagt tcatcatcaa ggaggaggaa ccactcacca gaacgaaccc    1620
   cagcaatggg tcctccctga ctactcgggc agggattccc acaacgttca cttggctccg    1680
   gccgtggctc caaccttccc tggagagcag ttgctgttct tcagatccac tatgccaggc   1740
   tgctctggat acccgaacat gaacctcgac tgtctcttgc ctcaggagtg ggtgcaacac   1800
   ttctaccagg aatctgcccc agctcaaagc gacgtcgctc tgctccgttt cgttaacccc   1860
   gataccggtc gcgtgctctt cgagtgtaag ttgcacaagt ctggttacgt cactgttgcc   1920
   cacacaggcc agcacgacct ggtcatccct cccaacggct acttccgctt cgatagctgg   1980
   gtcaaccagt tctacacact cgccccgatg ggaaacggag ccggtcgtcg cagagccttg   2040
   taa                                                                 2043
<210> 62
   <211> 3123
   <212> DNA
   <213> artificial sequence
<400> 62
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcagc ggaggaggag atgcagaatt ccgacatgac     540
   tcaggatatg aagttcatca tcaaggagga ggagatgcag aattccgaca tgactcagga     600
   tatgaagttc atcatcaagg aggaggagat gcagaattcc gacatgactc aggatatgaa     660
   gttcatcatc aaggaggagg agatgcagaa ttccgacatg actcaggata tgaagttcat     720
   catcaaggag gaggagatgc agaattccga catgactcag gatatgaagt tcatcatcaa     780
   ggaggaggag atgcagaatt ccgacatgac tcaggatatg aagttcatca tcaaggagga     840
   ggagatgcag aattccgaca tgactcagga tatgaagttc atcatcaagg aggaggagat     900
   gcagaattcc gacatgactc aggatatgaa gttcatcatc aaggaggagg agatgcagaa     960
   ttccgacatg actcaggata tgaagttcat catcaaggag gaggagatgc agaattccga    1020
   catgactcag gatatgaagt tcatcatcaa ggaggaggag atgcagaatt ccgacatgac    1080
   tcaggatatg aagttcatca tcaaggagga ggagatgcag aattccgaca tgactcagga    1140
   tatgaagttc atcatcaagg aggaggagat gcagaattcc gacatgactc aggatatgaa    1200
   gttcatcatc aaggaggagg agatgcagaa ttccgacatg actcaggata tgaagttcat    1260
   catcaaggag gaggagatgc agaattccga catgactcag gatatgaagt tcatcatcaa   1320
   ggaggaggag atgcagaatt ccgacatgac tcaggatatg aagttcatca tcaaggagga   1380
   ggagatgcag aattccgaca tgactcagga tatgaagttc atcatcaagg aggaggagat   1440
   gcagaattcc gacatgactc aggatatgaa gttcatcatc aaggaggagg agatgcagaa   1500
   ttccgacatg actcaggata tgaagttcat catcaaggag gaggagatgc agaattccga   1560
   catgactcag gatatgaagt tcatcatcaa ggaggaggag atgcagaatt ccgacatgac   1620
   tcaggatatg aagttcatca tcaaggagga ggagatgcag aattccgaca tgactcagga   1680
   tatgaagttc atcatcaagg aggaggagat gcagaattcc gacatgactc aggatatgaa   1740
   gttcatcatc aaggaggagg agatgcagaa ttccgacatg actcaggata tgaagttcat   1800
   catcaaggag gaggagatgc agaattccga catgactcag gatatgaagt tcatcatcaa   1860
   ggaggaggag atgcagaatt ccgacatgac tcaggatatg aagttcatca tcaaggagga   1920
   ggagatgcag aattccgaca tgactcagga tatgaagttc atcatcaagg aggaggagat   1980
   gcagaattcc gacatgactc aggatatgaa gttcatcatc aaggaggagg agatgcagaa   2040
   ttccgacatg actcaggata tgaagttcat catcaaggag gaggagatgc agaattccga   2100
   catgactcag gatatgaagt tcatcatcaa ggaggaggag atgcagaatt ccgacatgac   2160
   tcaggatatg aagttcatca tcaaggagga ggagatgcag aattccgaca tgactcagga   2220
   tatgaagttc atcatcaagg aggaggagat gcagaattcc gacatgactc aggatatgaa   2280
   gttcatcatc aaggaggagg agatgcagaa ttccgacatg actcaggata tgaagttcat   2340
   catcaaggag gaggagatgc agaattccga catgactcag gatatgaagt tcatcatcaa   2400
   ggaggaggag atgcagaatt ccgacatgac tcaggatatg aagttcatca tcaaggagga   2460
   ggagatgcag aattccgaca tgactcagga tatgaagttc atcatcaagg aggaggagat   2520
   gcagaattcc gacatgactc aggatatgaa gttcatcatc aaggaggagg agatgcagaa   2580
   ttccgacatg actcaggata tgaagttcat catcaaggag gaggagatgc agaattccga   2640
   catgactcag gatatgaagt tcatcatcaa ggaggaggaa ccactcacca gaacgaaccc   2700
   cagcaatggg tcctccctga ctactcgggc agggattccc acaacgttca cttggctccg   2760
   gccgtggctc caaccttccc tggagagcag ttgctgttct tcagatccac tatgccaggc   2820
   tgctctggat acccgaacat gaacctcgac tgtctcttgc ctcaggagtg ggtgcaacac   2880
   ttctaccagg aatctgcccc agctcaaagc gacgtcgctc tgctccgttt cgttaacccc   2940
   gataccggtc gcgtgctctt cgagtgtaag ttgcacaagt ctggttacgt cactgttgcc   3000
   cacacaggcc agcacgacct ggtcatccct cccaacggct acttccgctt cgatagctgg   3060
   gtcaaccagt tctacacact cgccccgatg ggaaacggag ccggtcgtcg cagagccttg   3120
   taa                                                                 3123
<210> 63
   <211> 999
   <212> DNA
   <213> artificial sequence
<400> 63
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcagc accggaggag gagatgcaga attccgacat     540
   gactcaggag gaggaggaac tcaccagaac gaaccccagc aatgggtcct ccctgactac     600
   tcgggcaggg attcccacaa cgttcacttg gctccggccg tggctccaac cttccctgga     660
   gagcagttgc tgttcttcag atccactatg ccaggctgct ctggataccc gaacatgaac     720
   ctcgactgtc tcttgcctca ggagtgggtg caacacttct accaggaatc tgccccagct     780
   caaagcgacg tcgctctgct ccgtttcgtt aaccccgata ccggtcgcgt gctcttcgag     840
   tgtaagttgc acaagtctgg ttacgtcact gttgcccaca caggccagca cgacctggtc     900
   atccctccca acggctactt ccgcttcgat agctgggtca accagttcta cacactcgcc     960
   ccgatgggaa acggagccgg tcgtcgcaga gccttgtaa                            999
<210> 64
   <211> 1323
   <212> DNA
   <213> artificial sequence
<400> 64
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcagc accggaggag gagatgcaga attccgacat     540
   gactcaggag gaggaggaga tgcagaattc cgacatgact caggaggagg aggagatgca     600
   gaattccgac atgactcagg aggaggagga gatgcagaat tccgacatga ctcaggagga     660
   ggaggagatg cagaattccg acatgactca ggaggaggag gagatgcaga attccgacat     720
   gactcaggag gaggaggaga tgcagaattc cgacatgact caggaggagg aggagatgca     780
   gaattccgac atgactcagg aggaggagga gatgcagaat tccgacatga ctcaggagga     840
   ggaggagatg cagaattccg acatgactca ggaggaggag gaactcacca gaacgaaccc     900
   cagcaatggg tcctccctga ctactcgggc agggattccc acaacgttca cttggctccg     960
   gccgtggctc caaccttccc tggagagcag ttgctgttct tcagatccac tatgccaggc    1020
   tgctctggat acccgaacat gaacctcgac tgtctcttgc ctcaggagtg ggtgcaacac    1080
   ttctaccagg aatctgcccc agctcaaagc gacgtcgctc tgctccgttt cgttaacccc    1140
   gataccggtc gcgtgctctt cgagtgtaag ttgcacaagt ctggttacgt cactgttgcc    1200
   cacacaggcc agcacgacct ggtcatccct cccaacggct acttccgctt cgatagctgg    1260
   gtcaaccagt tctacacact cgccccgatg ggaaacggag ccggtcgtcg cagagccttg    1320
   taa                                                                  1323
<210> 65
   <211> 1683
   <212> DNA
   <213> artificial sequence
<400> 65
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac gatttcgaga ctggccagaa caccaggttc 480
   actcccgtgg gtgtcgttca agacggcagc accggaggag gagatgcaga attccgacat 540
   gactcaggag gaggaggaga tgcagaattc cgacatgact caggaggagg aggagatgca 600
   gaattccgac atgactcagg aggaggagga gatgcagaat tccgacatga ctcaggagga 660
   ggaggagatg cagaattccg acatgactca ggaggaggag gagatgcaga attccgacat 720
   gactcaggag gaggaggaga tgcagaattc cgacatgact caggaggagg aggagatgca 780
   gaattccgac atgactcagg aggaggagga gatgcagaat tccgacatga ctcaggagga 840
   ggaggagatg cagaattccg acatgactca ggaggaggag gagatgcaga attccgacat 900
   gactcaggag gaggaggaga tgcagaattc cgacatgact caggaggagg aggagatgca 960
   gaattccgac atgactcagg aggaggagga gatgcagaat tccgacatga ctcaggagga 1020
   ggaggagatg cagaattccg acatgactca ggaggaggag gagatgcaga attccgacat 1080
   gactcaggag gaggaggaga tgcagaattc cgacatgact caggaggagg aggagatgca 1140
   gaattccgac atgactcagg aggaggagga gatgcagaat tccgacatga ctcaggagga 1200
   ggaggagatg cagaattccg acatgactca ggaggaggag gaactcacca gaacgaaccc 1260
   cagcaatggg tcctccctga ctactcgggc agggattccc acaacgttca cttggctccg 1320
   gccgtggctc caaccttccc tggagagcag ttgctgttct tcagatccac tatgccaggc 1380
   tgctctggat acccgaacat gaacctcgac tgtctcttgc ctcaggagtg ggtgcaacac 1440
   ttctaccagg aatctgcccc agctcaaagc gacgtcgctc tgctccgttt cgttaacccc 1500
   gataccggtc gcgtgctctt cgagtgtaag ttgcacaagt ctggttacgt cactgttgcc 1560
   cacacaggcc agcacgacct ggtcatccct cccaacggct acttccgctt cgatagctgg 1620
   gtcaaccagt tctacacact cgccccgatg ggaaacggag ccggtcgtcg cagagccttg 1680
   taa 1683
<210> 66
   <211> 2403
   <212> DNA
   <213> artificial sequence
<400> 66
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac gatttcgaga ctggccagaa caccaggttc     480
   actcccgtgg gtgtcgttca agacggcagc accggaggag gagatgcaga attccgacat     540
   gactcaggag gaggaggaga tgcagaattc cgacatgact caggaggagg aggagatgca     600
   gaattccgac atgactcagg aggaggagga gatgcagaat tccgacatga ctcaggagga     660
   ggaggagatg cagaattccg acatgactca ggaggaggag gagatgcaga attccgacat     720
   gactcaggag gaggaggaga tgcagaattc cgacatgact caggaggagg aggagatgca     780
   gaattccgac atgactcagg aggaggagga gatgcagaat tccgacatga ctcaggagga     840
   ggaggagatg cagaattccg acatgactca ggaggaggag gagatgcaga attccgacat     900
   gactcaggag gaggaggaga tgcagaattc cgacatgact caggaggagg aggagatgca     960
   gaattccgac atgactcagg aggaggagga gatgcagaat tccgacatga ctcaggagga    1020
   ggaggagatg cagaattccg acatgactca ggaggaggag gagatgcaga attccgacat    1080
   gactcaggag gaggaggaga tgcagaattc cgacatgact caggaggagg aggagatgca    1140
   gaattccgac atgactcagg aggaggagga gatgcagaat tccgacatga ctcaggagga    1200
   ggaggagatg cagaattccg acatgactca ggaggaggag gagatgcaga attccgacat    1260
   gactcaggag gaggaggaga tgcagaattc cgacatgact caggaggagg aggagatgca    1320
   gaattccgac atgactcagg aggaggagga gatgcagaat tccgacatga ctcaggagga    1380
   ggaggagatg cagaattccg acatgactca ggaggaggag gagatgcaga attccgacat    1440
   gactcaggag gaggaggaga tgcagaattc cgacatgact caggaggagg aggagatgca    1500
   gaattccgac atgactcagg aggaggagga gatgcagaat tccgacatga ctcaggagga    1560
   ggaggagatg cagaattccg acatgactca ggaggaggag gagatgcaga attccgacat    1620
   gactcaggag gaggaggaga tgcagaattc cgacatgact caggaggagg aggagatgca    1680
   gaattccgac atgactcagg aggaggagga gatgcagaat tccgacatga ctcaggagga    1740
   ggaggagatg cagaattccg acatgactca ggaggaggag gagatgcaga attccgacat    1800
   gactcaggag gaggaggaga tgcagaattc cgacatgact caggaggagg aggagatgca    1860
   gaattccgac atgactcagg aggaggagga gatgcagaat tccgacatga ctcaggagga    1920
   ggaggagatg cagaattccg acatgactca ggaggaggag gaactcacca gaacgaaccc    1980
   cagcaatggg tcctccctga ctactcgggc agggattccc acaacgttca cttggctccg    2040
   gccgtggctc caaccttccc tggagagcag ttgctgttct tcagatccac tatgccaggc    2100
   tgctctggat acccgaacat gaacctcgac tgtctcttgc ctcaggagtg ggtgcaacac    2160
   ttctaccagg aatctgcccc agctcaaagc gacgtcgctc tgctccgttt cgttaacccc    2220
   gataccggtc gcgtgctctt cgagtgtaag ttgcacaagt ctggttacgt cactgttgcc    2280
   cacacaggcc agcacgacct ggtcatccct cccaacggct acttccgctt cgatagctgg    2340
   gtcaaccagt tctacacact cgccccgatg ggaaacggag ccggtcgtcg cagagccttg    2400
   taa                                                                  2403
<210> 67
   <211> 1134
   <212> DNA
   <213> artificial sequence
<400> 67
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   ggaggaggag atgcagaatt ccgacatgga ggaggagatg cagaattccg acatggagga     300
   ggaggaacac aaaactacac catgaacttg gcctcgcaga actggaacaa ctacgatcca     360
   accgaggaaa tccccgctcc tttgggaact cccgacttcg tgggacgtat ccaaggtgtc     420
   ctgacacaga ctacacgtcg cgacggctct actcgcggac acaaggccac tgtgtcgaca     480
   ggttccgtcc acttcacccc taagctgggc tctgtgcaat tcagcacaga cacctcagga     540
   ggaggagatg cagaattccg acatggagga ggagatgcag aattccgaca tggaggagga     600
   gatgcagaat tccgacatgg aggaggaaac gatttcgaga ctggccagaa caccaggttc     660
   actcccgtgg gtgtcgttca agacggcagc accactcacc agaacgaacc ccagcaatgg     720
   gtcctccctg actactcggg cagggattcc cacaacgttc acttggctcc ggccgtggct     780
   ccaaccttcc ctggagagca gttgctgttc ttcagatcca ctatgccagg ctgctctgga     840
   tacccgaaca tgaacctcga ctgtctcttg cctcaggagt gggtgcaaca cttctaccag     900
   gaatctgccc cagctcaaag cgacgtcgct ctgctccgtt tcgttaaccc cgataccggt     960
   cgcgtgctct tcgagtgtaa gttgcacaag tctggttacg tcactgttgc ccacacaggc    1020
   cagcacgacc tggtcatccc tcccaacggc tacttccgct tcgatagctg ggtcaaccag    1080
   ttctacacac tcgccccgat gggaaacgga gccggtcgtc gcagagcctt gtaa          1134
<210> 68
   <211> 1692
   <212> DNA
   <213> artificial sequence
<400> 68
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggag gaggaggaga tgcagaattc cgacatgact caggaggagg aggagatgca     300
   gaattccgac atgactcagg aggaggagga gatgcagaat tccgacatga ctcaggagga     360
   ggaggagatg cagaattccg acatgactca ggaggaggag gagatgcaga attccgacat     420
   gactcaggag gaggaggaga tgcagaattc cgacatgact caggaggagg aggagatgca     480
   gaattccgac atgactcagg aggaggagga gatgcagaat tccgacatga ctcaggagga     540
   ggaggagatg cagaattccg acatgactca ggaggaggag gaggaacaca aaactacacc     600
   atgaacttgg cctcgcagaa ctggaacaac tacgatccaa ccgaggaaat ccccgctcct     660
   ttgggaactc ccgacttcgt gggacgtatc caaggtgtcc tgacacagac tacacgtcgc     720
   gacggctcta ctcgcggaca caaggccact gtgtcgacag gttccgtcca cttcacccct     780
   aagctgggct ctgtgcaatt cagcacagac acctcaggag gaggagatgc agaattccga     840
   catgactcag gaggaggagg agatgcagaa ttccgacatg actcaggagg aggaggagat     900
   gcagaattcc gacatgactc aggaggagga ggagatgcag aattccgaca tgactcagga     960
   ggaggaggag atgcagaatt ccgacatgac tcaggaggag gaggagatgc agaattccga    1020
   catgactcag gaggaggagg agatgcagaa ttccgacatg actcaggagg aggaggagat    1080
   gcagaattcc gacatgactc aggaggagga ggagatgcag aattccgaca tgactcagga    1140
   ggaggaggag atgcagaatt ccgacatgac tcaggaggag gaggaaacga tttcgagact    1200
   ggccagaaca ccaggttcac tcccgtgggt gtcgttcaag acggcagcac cactcaccag    1260
   aacgaacccc agcaatgggt cctccctgac tactcgggca gggattccca caacgttcac    1320
   ttggctccgg ccgtggctcc aaccttccct ggagagcagt tgctgttctt cagatccact    1380
   atgccaggct gctctggata cccgaacatg aacctcgact gtctcttgcc tcaggagtgg    1440
   gtgcaacact tctaccagga atctgcccca gctcaaagcg acgtcgctct gctccgtttc    1500
   gttaaccccg ataccggtcg cgtgctcttc gagtgtaagt tgcacaagtc tggttacgtc    1560
   actgttgccc acacaggcca gcacgacctg gtcatccctc ccaacggcta cttccgcttc    1620
   gatagctggg tcaaccagtt ctacacactc gccccgatgg gaaacggagc cggtcgtcgc    1680
   agagccttgt aa                                                        1692
<210> 69
   <211> 2772
   <212> DNA
   <213> artificial sequence
<400> 69
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggat atgaagttgg aggaggagat gcagaattcc gacatgactc aggatatgaa     300
   gttggaggag gagatgcaga attccgacat gactcaggat atgaagttgg aggaggagat     360
   gcagaattcc gacatgactc aggatatgaa gttggaggag gagatgcaga attccgacat 420
   gactcaggat atgaagttgg aggaggagat gcagaattcc gacatgactc aggatatgaa 480
   gttggaggag gagatgcaga attccgacat gactcaggat atgaagttgg aggaggagat 540
   gcagaattcc gacatgactc aggatatgaa gttggaggag gagatgcaga attccgacat 600
   gactcaggat atgaagttgg aggaggagat gcagaattcc gacatgactc aggatatgaa 660
   gttggaggag gagatgcaga attccgacat gactcaggat atgaagttgg aggaggagat 720
   gcagaattcc gacatgactc aggatatgaa gttggaggag gagatgcaga attccgacat 780
   gactcaggat atgaagttgg aggaggagat gcagaattcc gacatgactc aggatatgaa 840
   gttggaggag gagatgcaga attccgacat gactcaggat atgaagttgg aggaggagat 900
   gcagaattcc gacatgactc aggatatgaa gttggaggag gagatgcaga attccgacat 960
   gactcaggat atgaagttgg aggaggagat gcagaattcc gacatgactc aggatatgaa 1020
   gttggaggag gagatgcaga attccgacat gactcaggat atgaagttgg aggaggagat 1080
   gcagaattcc gacatgactc aggatatgaa gttggaggag gaggaacaca aaactacacc 1140
   atgaacttgg cctcgcagaa ctggaacaac tacgatccaa ccgaggaaat ccccgctcct 1200
   ttgggaactc ccgacttcgt gggacgtatc caaggtgtcc tgacacagac tacacgtcgc 1260
   gacggctcta ctcgcggaca caaggccact gtgtcgacag gttccgtcca cttcacccct 1320
   aagctgggct ctgtgcaatt cagcacagac acctcaggag gaggagatgc agaattccga 1380
   catgactcag gatatgaagt tggaggagga gatgcagaat tccgacatga ctcaggatat 1440
   gaagttggag gaggagatgc agaattccga catgactcag gatatgaagt tggaggagga 1500
   gatgcagaat tccgacatga ctcaggatat gaagttggag gaggagatgc agaattccga 1560
   catgactcag gatatgaagt tggaggagga gatgcagaat tccgacatga ctcaggatat 1620
   gaagttggag gaggagatgc agaattccga catgactcag gatatgaagt tggaggagga 1680
   gatgcagaat tccgacatga ctcaggatat gaagttggag gaggagatgc agaattccga 1740
   catgactcag gatatgaagt tggaggagga gatgcagaat tccgacatga ctcaggatat 1800
   gaagttggag gaggagatgc agaattccga catgactcag gatatgaagt tggaggagga 1860
   gatgcagaat tccgacatga ctcaggatat gaagttggag gaggagatgc agaattccga 1920
   catgactcag gatatgaagt tggaggagga gatgcagaat tccgacatga ctcaggatat 1980
   gaagttggag gaggagatgc agaattccga catgactcag gatatgaagt tggaggagga 2040
   gatgcagaat tccgacatga ctcaggatat gaagttggag gaggagatgc agaattccga 2100
   catgactcag gatatgaagt tggaggagga gatgcagaat tccgacatga ctcaggatat 2160
   gaagttggag gaggagatgc agaattccga catgactcag gatatgaagt tggaggagga 2220
   gatgcagaat tccgacatga ctcaggatat gaagttggag gaggaaacga tttcgagact 2280
   ggccagaaca ccaggttcac tcccgtgggt gtcgttcaag acggcagcac cactcaccag 2340
   aacgaacccc agcaatgggt cctccctgac tactcgggca gggattccca caacgttcac 2400
   ttggctccgg ccgtggctcc aaccttccct ggagagcagt tgctgttctt cagatccact 2460
   atgccaggct gctctggata cccgaacatg aacctcgact gtctcttgcc tcaggagtgg 2520
   gtgcaacact tctaccagga atctgcccca gctcaaagcg acgtcgctct gctccgtttc 2580
   gttaaccccg ataccggtcg cgtgctcttc gagtgtaagt tgcacaagtc tggttacgtc 2640
   actgttgccc acacaggcca gcacgacctg gtcatccctc ccaacggcta cttccgcttc 2700
   gatagctggg tcaaccagtt ctacacactc gccccgatgg gaaacggagc cggtcgtcgc 2760
   agagccttgt aa 2772
<210> 70
   <211> 5292
   <212> DNA
   <213> artificial sequence
<400> 70
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggat atgaagttca tcatcaagga ggaggagatg cagaattccg acatgactca     300
   ggatatgaag ttcatcatca aggaggagga gatgcagaat tccgacatga ctcaggatat     360
   gaagttcatc atcaaggagg aggagatgca gaattccgac atgactcagg atatgaagtt     420
   catcatcaag gaggaggaga tgcagaattc cgacatgact caggatatga agttcatcat     480
   caaggaggag gagatgcaga attccgacat gactcaggat atgaagttca tcatcaagga     540
   ggaggagatg cagaattccg acatgactca ggatatgaag ttcatcatca aggaggagga     600
   gatgcagaat tccgacatga ctcaggatat gaagttcatc atcaaggagg aggagatgca     660
   gaattccgac atgactcagg atatgaagtt catcatcaag gaggaggaga tgcagaattc     720
   cgacatgact caggatatga agttcatcat caaggaggag gagatgcaga attccgacat     780
   gactcaggat atgaagttca tcatcaagga ggaggagatg cagaattccg acatgactca     840
   ggatatgaag ttcatcatca aggaggagga gatgcagaat tccgacatga ctcaggatat     900
   gaagttcatc atcaaggagg aggagatgca gaattccgac atgactcagg atatgaagtt     960
   catcatcaag gaggaggaga tgcagaattc cgacatgact caggatatga agttcatcat    1020
   caaggaggag gagatgcaga attccgacat gactcaggat atgaagttca tcatcaagga    1080
   ggaggagatg cagaattccg acatgactca ggatatgaag ttcatcatca aggaggagga    1140
   gatgcagaat tccgacatga ctcaggatat gaagttcatc atcaaggagg aggagatgca 1200
   gaattccgac atgactcagg atatgaagtt catcatcaag gaggaggaga tgcagaattc 1260
   cgacatgact caggatatga agttcatcat caaggaggag gagatgcaga attccgacat 1320
   gactcaggat atgaagttca tcatcaagga ggaggagatg cagaattccg acatgactca 1380
   ggatatgaag ttcatcatca aggaggagga gatgcagaat tccgacatga ctcaggatat 1440
   gaagttcatc atcaaggagg aggagatgca gaattccgac atgactcagg atatgaagtt 1500
   catcatcaag gaggaggaga tgcagaattc cgacatgact caggatatga agttcatcat 1560
   caaggaggag gagatgcaga attccgacat gactcaggat atgaagttca tcatcaagga 1620
   ggaggagatg cagaattccg acatgactca ggatatgaag ttcatcatca aggaggagga 1680
   gatgcagaat tccgacatga ctcaggatat gaagttcatc atcaaggagg aggagatgca 1740
   gaattccgac atgactcagg atatgaagtt catcatcaag gaggaggaga tgcagaattc 1800
   cgacatgact caggatatga agttcatcat caaggaggag gagatgcaga attccgacat 1860
   gactcaggat atgaagttca tcatcaagga ggaggagatg cagaattccg acatgactca 1920
   ggatatgaag ttcatcatca aggaggagga gatgcagaat tccgacatga ctcaggatat 1980
   gaagttcatc atcaaggagg aggagatgca gaattccgac atgactcagg atatgaagtt 2040
   catcatcaag gaggaggaga tgcagaattc cgacatgact caggatatga agttcatcat 2100
   caaggaggag gagatgcaga attccgacat gactcaggat atgaagttca tcatcaagga 2160
   ggaggagatg cagaattccg acatgactca ggatatgaag ttcatcatca aggaggagga 2220
   gatgcagaat tccgacatga ctcaggatat gaagttcatc atcaaggagg aggagatgca 2280
   gaattccgac atgactcagg atatgaagtt catcatcaag gaggaggaga tgcagaattc 2340
   cgacatgact caggatatga agttcatcat caaggaggag gaggaacaca aaactacacc 2400
   atgaacttgg cctcgcagaa ctggaacaac tacgatccaa ccgaggaaat ccccgctcct 2460
   ttgggaactc ccgacttcgt gggacgtatc caaggtgtcc tgacacagac tacacgtcgc 2520
   gacggctcta ctcgcggaca caaggccact gtgtcgacag gttccgtcca cttcacccct 2580
   aagctgggct ctgtgcaatt cagcacagac acctcaggag gaggagatgc agaattccga 2640
   catgactcag gatatgaagt tcatcatcaa ggaggaggag atgcagaatt ccgacatgac 2700
   tcaggatatg aagttcatca tcaaggagga ggagatgcag aattccgaca tgactcagga 2760
   tatgaagttc atcatcaagg aggaggagat gcagaattcc gacatgactc aggatatgaa 2820
   gttcatcatc aaggaggagg agatgcagaa ttccgacatg actcaggata tgaagttcat 2880
   catcaaggag gaggagatgc agaattccga catgactcag gatatgaagt tcatcatcaa 2940
   ggaggaggag atgcagaatt ccgacatgac tcaggatatg aagttcatca tcaaggagga 3000
   ggagatgcag aattccgaca tgactcagga tatgaagttc atcatcaagg aggaggagat 3060
   gcagaattcc gacatgactc aggatatgaa gttcatcatc aaggaggagg agatgcagaa 3120
   ttccgacatg actcaggata tgaagttcat catcaaggag gaggagatgc agaattccga 3180
   catgactcag gatatgaagt tcatcatcaa ggaggaggag atgcagaatt ccgacatgac 3240
   tcaggatatg aagttcatca tcaaggagga ggagatgcag aattccgaca tgactcagga 3300
   tatgaagttc atcatcaagg aggaggagat gcagaattcc gacatgactc aggatatgaa 3360
   gttcatcatc aaggaggagg agatgcagaa ttccgacatg actcaggata tgaagttcat 3420
   catcaaggag gaggagatgc agaattccga catgactcag gatatgaagt tcatcatcaa 3480
   ggaggaggag atgcagaatt ccgacatgac tcaggatatg aagttcatca tcaaggagga 3540
   ggagatgcag aattccgaca tgactcagga tatgaagttc atcatcaagg aggaggagat 3600
   gcagaattcc gacatgactc aggatatgaa gttcatcatc aaggaggagg agatgcagaa 3660
   ttccgacatg actcaggata tgaagttcat catcaaggag gaggagatgc agaattccga 3720
   catgactcag gatatgaagt tcatcatcaa ggaggaggag atgcagaatt ccgacatgac 3780
   tcaggatatg aagttcatca tcaaggagga ggagatgcag aattccgaca tgactcagga 3840
   tatgaagttc atcatcaagg aggaggagat gcagaattcc gacatgactc aggatatgaa 3900
   gttcatcatc aaggaggagg agatgcagaa ttccgacatg actcaggata tgaagttcat 3960
   catcaaggag gaggagatgc agaattccga catgactcag gatatgaagt tcatcatcaa 4020
   ggaggaggag atgcagaatt ccgacatgac tcaggatatg aagttcatca tcaaggagga 4080
   ggagatgcag aattccgaca tgactcagga tatgaagttc atcatcaagg aggaggagat 4140
   gcagaattcc gacatgactc aggatatgaa gttcatcatc aaggaggagg agatgcagaa 4200
   ttccgacatg actcaggata tgaagttcat catcaaggag gaggagatgc agaattccga 4260
   catgactcag gatatgaagt tcatcatcaa ggaggaggag atgcagaatt ccgacatgac 4320
   tcaggatatg aagttcatca tcaaggagga ggagatgcag aattccgaca tgactcagga 4380
   tatgaagttc atcatcaagg aggaggagat gcagaattcc gacatgactc aggatatgaa 4440
   gttcatcatc aaggaggagg agatgcagaa ttccgacatg actcaggata tgaagttcat 4500
   catcaaggag gaggagatgc agaattccga catgactcag gatatgaagt tcatcatcaa 4560
   ggaggaggag atgcagaatt ccgacatgac tcaggatatg aagttcatca tcaaggagga 4620
   ggagatgcag aattccgaca tgactcagga tatgaagttc atcatcaagg aggaggagat 4680
   gcagaattcc gacatgactc aggatatgaa gttcatcatc aaggaggagg agatgcagaa 4740
   ttccgacatg actcaggata tgaagttcat catcaaggag gaggaaacga tttcgagact 4800
   ggccagaaca ccaggttcac tcccgtgggt gtcgttcaag acggcagcac cactcaccag 4860
   aacgaacccc agcaatgggt cctccctgac tactcgggca gggattccca caacgttcac 4920
   ttggctccgg ccgtggctcc aaccttccct ggagagcagt tgctgttctt cagatccact 4980
   atgccaggct gctctggata cccgaacatg aacctcgact gtctcttgcc tcaggagtgg 5040
   gtgcaacact tctaccagga atctgcccca gctcaaagcg acgtcgctct gctccgtttc 5100
   gttaaccccg ataccggtcg cgtgctcttc gagtgtaagt tgcacaagtc tggttacgtc 5160
   actgttgccc acacaggcca gcacgacctg gtcatccctc ccaacggcta cttccgcttc 5220
   gatagctggg tcaaccagtt ctacacactc gccccgatgg gaaacggagc cggtcgtcgc 5280
   agagccttgt aa 5292
<210> 71
   <211> 1044
   <212> DNA
   <213> artificial sequence
<400> 71
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc ggaggaggag atgcagaatt ccgacatgga 240
   ggaggagctg gaacacaaaa ctacaccatg aacttggcct cgcagaactg gaacaactac 300
   gatccaaccg aggaaatccc cgctcctttg ggaactcccg acttcgtggg acgtatccaa 360
   ggtgtcctga cacagactac acgtcgcgac ggctctactc gcggacacaa ggccactgtg 420
   tcgacaggtt ccgtccactt cacccctaag ctgggctctg tgcaattcag cacagacacc 480
   ggaggaggag atgcagaatt ccgacatgac tcaggatatg aagttggagg aggatcaaac 540
   gatttcgaga ctggccagaa caccaggttc actcccgtgg gtgtcgttca agacggcagc 600
   accactcacc agaacgaacc ccagcaatgg gtcctccctg actactcggg cagggattcc 660
   cacaacgttc acttggctcc ggccgtggct ccaaccttcc ctggagagca gttgctgttc 720
   ttcagatcca ctatgccagg ctgctctgga tacccgaaca tgaacctcga ctgtctcttg 780
   cctcaggagt gggtgcaaca cttctaccag gaatctgccc cagctcaaag cgacgtcgct 840
   ctgctccgtt tcgttaaccc cgataccggt cgcgtgctct tcgagtgtaa gttgcacaag 900
   tctggttacg tcactgttgc ccacacaggc cagcacgacc tggtcatccc tcccaacggc 960
   tacttccgct tcgatagctg ggtcaaccag ttctacacac tcgccccgat gggaaacgga 1020
   gccggtcgtc gcagagcctt gtaa 1044
<210> 72
   <211> 1161
   <212> DNA
   <213> artificial sequence
<400> 72
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggag gaggaggaga tgcagaattc cgacatgact caggaggagg aggagatgca     300
   gaattccgac atgactcagg aggaggagga ggaacacaaa actacaccat gaacttggcc     360
   tcgcagaact ggaacaacta cgatccaacc gaggaaatcc ccgctccttt gggaactccc     420
   gacttcgtgg gacgtatcca aggtgtcctg acacagacta cacgtcgcga cggctctact     480
   cgcggacaca aggccactgt gtcgacaggt tccgtccact tcacccctaa gctgggctct     540
   gtgcaattca gcacagacac ctcaggagga ggagatgcag aattccgaca tggaggagga     600
   gatgcagaat tccgacatgg aggaggagat gcagaattcc gacatggagg aggaaacgat     660
   ttcgagactg gccagaacac caggttcact cccgtgggtg tcgttcaaga cggcagcacc     720
   actcaccaga acgaacccca gcaatgggtc ctccctgact actcgggcag ggattcccac     780
   aacgttcact tggctccggc cgtggctcca accttccctg gagagcagtt gctgttcttc     840
   agatccacta tgccaggctg ctctggatac ccgaacatga acctcgactg tctcttgcct     900
   caggagtggg tgcaacactt ctaccaggaa tctgccccag ctcaaagcga cgtcgctctg     960
   ctccgtttcg ttaaccccga taccggtcgc gtgctcttcg agtgtaagtt gcacaagtct    1020
   ggttacgtca ctgttgccca cacaggccag cacgacctgg tcatccctcc caacggctac    1080
   ttccgcttcg atagctgggt caaccagttc tacacactcg ccccgatggg aaacggagcc    1140
   ggtcgtcgca gagccttgta a                                              1161
<210> 73
   <211> 1782
   <212> DNA
   <213> artificial sequence
<400> 73
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tcatcatcaa ggaggaggag atgcagaatt ccgacatgac     300
   tcaggatatg aagttcatca tcaaggagga ggagatgcag aattccgaca tgactcagga     360
   tatgaagttc atcatcaagg aggaggagat gcagaattcc gacatgactc aggatatgaa     420
   gttcatcatc aaggaggagg agatgcagaa ttccgacatg actcaggata tgaagttcat     480
   catcaaggag gaggagatgc agaattccga catgactcag gatatgaagt tcatcatcaa     540
   ggaggaggag atgcagaatt ccgacatgac tcaggatatg aagttcatca tcaaggagga     600
   ggagatgcag aattccgaca tgactcagga tatgaagttc atcatcaagg aggaggagat     660
   gcagaattcc gacatgactc aggatatgaa gttcatcatc aaggaggagg agatgcagaa     720
   ttccgacatg actcaggata tgaagttcat catcaaggag gaggaacaca aaactacacc     780
   atgaacttgg cctcgcagaa ctggaacaac tacgatccaa ccgaggaaat ccccgctcct     840
   ttgggaactc ccgacttcgt gggacgtatc caaggtgtcc tgacacagac tacacgtcgc     900
   gacggctcta ctcgcggaca caaggccact gtgtcgacag gttccgtcca cttcacccct     960
   aagctgggct ctgtgcaatt cagcacagac acctcaggag gaggagatgc agaattccga    1020
   catggaggag gagatgcaga attccgacat ggaggaggag atgcagaatt ccgacatgga    1080
   ggaggagatg cagaattccg acatggagga ggagatgcag aattccgaca tggaggagga    1140
   gatgcagaat tccgacatgg aggaggagat gcagaattcc gacatggagg aggagatgca    1200
   gaattccgac atggaggagg agatgcagaa ttccgacatg gaggaggaga tgcagaattc    1260
   cgacatggag gaggaaacga tttcgagact ggccagaaca ccaggttcac tcccgtgggt    1320
   gtcgttcaag acggcagcac cactcaccag aacgaacccc agcaatgggt cctccctgac    1380
   tactcgggca gggattccca caacgttcac ttggctccgg ccgtggctcc aaccttccct    1440
   ggagagcagt tgctgttctt cagatccact atgccaggct gctctggata cccgaacatg    1500
   aacctcgact gtctcttgcc tcaggagtgg gtgcaacact tctaccagga atctgcccca    1560
   gctcaaagcg acgtcgctct gctccgtttc gttaaccccg ataccggtcg cgtgctcttc    1620
   gagtgtaagt tgcacaagtc tggttacgtc actgttgccc acacaggcca gcacgacctg    1680
   gtcatccctc ccaacggcta cttccgcttc gatagctggg tcaaccagtt ctacacactc    1740
   gccccgatgg gaaacggagc cggtcgtcgc agagccttgt aa                        1782
<210> 74
   <211> 2772
   <212> DNA
   <213> artificial sequence
<400> 74
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gaggaggagg agatgcagaa ttccgacatg actcaggagg aggaggagat     300
   gcagaattcc gacatgactc aggaggagga ggagatgcag aattccgaca tgactcagga    360
   ggaggaggag atgcagaatt ccgacatgac tcaggaggag gaggagatgc agaattccga    420
   catgactcag gaggaggagg agatgcagaa ttccgacatg actcaggagg aggaggagat    480
   gcagaattcc gacatgactc aggaggagga ggagatgcag aattccgaca tgactcagga    540
   ggaggaggag atgcagaatt ccgacatgac tcaggaggag gaggagatgc agaattccga    600
   catgactcag gaggaggagg agatgcagaa ttccgacatg actcaggagg aggaggagat    660
   gcagaattcc gacatgactc aggaggagga ggagatgcag aattccgaca tgactcagga    720
   ggaggaggag atgcagaatt ccgacatgac tcaggaggag gaggagatgc agaattccga    780
   catgactcag gaggaggagg agatgcagaa ttccgacatg actcaggagg aggaggagat    840
   gcagaattcc gacatgactc aggaggagga ggagatgcag aattccgaca tgactcagga    900
   ggaggaggag atgcagaatt ccgacatgac tcaggaggag gaggaacaca aaactacacc    960
   atgaacttgg cctcgcagaa ctggaacaac tacgatccaa ccgaggaaat ccccgctcct   1020
   ttgggaactc ccgacttcgt gggacgtatc caaggtgtcc tgacacagac tacacgtcgc   1080
   gacggctcta ctcgcggaca caaggccact gtgtcgacag gttccgtcca cttcacccct   1140
   aagctgggct ctgtgcaatt cagcacagac acctcaaacg gaggaggaga tgcagaattc   1200
   cgacatgact caggatatga agttcatcat caaggaggag gagatgcaga attccgacat   1260
   gactcaggat atgaagttca tcatcaagga ggaggagatg cagaattccg acatgactca   1320
   ggatatgaag ttcatcatca aggaggagga gatgcagaat tccgacatga ctcaggatat   1380
   gaagttcatc atcaaggagg aggagatgca gaattccgac atgactcagg atatgaagtt   1440
   catcatcaag gaggaggaga tgcagaattc cgacatgact caggatatga agttcatcat   1500
   caaggaggag gagatgcaga attccgacat gactcaggat atgaagttca tcatcaagga   1560
   ggaggagatg cagaattccg acatgactca ggatatgaag ttcatcatca aggaggagga   1620
   gatgcagaat tccgacatga ctcaggatat gaagttcatc atcaaggagg aggagatgca   1680
   gaattccgac atgactcagg atatgaagtt catcatcaag gaggaggaga tgcagaattc   1740
   cgacatgact caggatatga agttcatcat caaggaggag gagatgcaga attccgacat   1800
   gactcaggat atgaagttca tcatcaagga ggaggagatg cagaattccg acatgactca   1860
   ggatatgaag ttcatcatca aggaggagga gatgcagaat tccgacatga ctcaggatat   1920
   gaagttcatc atcaaggagg aggagatgca gaattccgac atgactcagg atatgaagtt   1980
   catcatcaag gaggaggaga tgcagaattc cgacatgact caggatatga agttcatcat   2040
   caaggaggag gagatgcaga attccgacat gactcaggat atgaagttca tcatcaagga   2100
   ggaggagatg cagaattccg acatgactca ggatatgaag ttcatcatca aggaggagga   2160
   gatgcagaat tccgacatga ctcaggatat gaagttcatc atcaaggagg aggagatgca   2220
   gaattccgac atgactcagg atatgaagtt catcatcaag gaggaggaga tttcgagact   2280
   ggccagaaca ccaggttcac tcccgtgggt gtcgttcaag acggcagcac cactcaccag   2340
   aacgaacccc agcaatgggt cctccctgac tactcgggca gggattccca caacgttcac   2400
   ttggctccgg ccgtggctcc aaccttccct ggagagcagt tgctgttctt cagatccact   2460
   atgccaggct gctctggata cccgaacatg aacctcgact gtctcttgcc tcaggagtgg   2520
   gtgcaacact tctaccagga atctgcccca gctcaaagcg acgtcgctct gctccgtttc   2580
   gttaaccccg ataccggtcg cgtgctcttc gagtgtaagt tgcacaagtc tggttacgtc   2640
   actgttgccc acacaggcca gcacgacctg gtcatccctc ccaacggcta cttccgcttc   2700
   gatagctggg tcaaccagtt ctacacactc gccccgatgg gaaacggagc cggtcgtcgc   2760
   agagccttgt aa                                                        2772
<210> 75
   <211> 3942
   <212> DNA
   <213> artificial sequence
<400> 75
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacag gaggaggaga tgcagaattc     240
   cgacatgact caggatatga agttcatcat caaggaggag gagatgcaga attccgacat     300
   gactcaggat atgaagttca tcatcaagga ggaggagatg cagaattccg acatgactca     360
   ggatatgaag ttcatcatca aggaggagga gatgcagaat tccgacatga ctcaggatat     420
   gaagttcatc atcaaggagg aggagatgca gaattccgac atgactcagg atatgaagtt     480
   catcatcaag gaggaggaga tgcagaattc cgacatgact caggatatga agttcatcat     540
   caaggaggag gagatgcaga attccgacat gactcaggat atgaagttca tcatcaagga     600
   ggaggagatg cagaattccg acatgactca ggatatgaag ttcatcatca aggaggagga     660
   gatgcagaat tccgacatga ctcaggatat gaagttcatc atcaaggagg aggagatgca     720
   gaattccgac atgactcagg atatgaagtt catcatcaag gaggaggaga tgcagaattc     780
   cgacatgact caggatatga agttcatcat caaggaggag gagatgcaga attccgacat     840
   gactcaggat atgaagttca tcatcaagga ggaggagatg cagaattccg acatgactca     900
   ggatatgaag ttcatcatca aggaggagga gatgcagaat tccgacatga ctcaggatat     960
   gaagttcatc atcaaggagg aggagatgca gaattccgac atgactcagg atatgaagtt    1020
   catcatcaag gaggaggaga tgcagaattc cgacatgact caggatatga agttcatcat    1080
   caaggaggag gagatgcaga attccgacat gactcaggat atgaagttca tcatcaagga   1140
   ggaggagatg cagaattccg acatgactca ggatatgaag ttcatcatca aggaggagga   1200
   gatgcagaat tccgacatga ctcaggatat gaagttcatc atcaaggagg aggagatgca   1260
   gaattccgac atgactcagg atatgaagtt catcatcaag gaggaggaga tgcagaattc   1320
   cgacatgact caggatatga agttcatcat caaggaggag gagatgcaga attccgacat   1380
   gactcaggat atgaagttca tcatcaagga ggaggagatg cagaattccg acatgactca   1440
   ggatatgaag ttcatcatca aggaggagga gatgcagaat tccgacatga ctcaggatat   1500
   gaagttcatc atcaaggagg aggagatgca gaattccgac atgactcagg atatgaagtt   1560
   catcatcaag gaggaggaga tgcagaattc cgacatgact caggatatga agttcatcat   1620
   caaggaggag gagatgcaga attccgacat gactcaggat atgaagttca tcatcaagga   1680
   ggaggagatg cagaattccg acatgactca ggatatgaag ttcatcatca aggaggagga   1740
   gatgcagaat tccgacatga ctcaggatat gaagttcatc atcaaggagg aggagatgca   1800
   gaattccgac atgactcagg atatgaagtt catcatcaag gaggaggaca aaactacacc   1860
   atgaacttgg cctcgcagaa ctggaacaac tacgatccaa ccgaggaaat ccccgctcct   1920
   ttgggaactc ccgacttcgt gggacgtatc caaggtgtcc tgacacagac tacacgtcgc   1980
   gacggctcta ctcgcggaca caaggccact gtgtcgacag gttccgtcca cttcacccct   2040
   aagctgggct ctgtgcaatt cagcacagac acctcaaacg atggaggagg agatgcagaa   2100
   ttccgacatg actcaggata tgaagttgga ggaggagatg cagaattccg acatgactca   2160
   ggatatgaag ttggaggagg agatgcagaa ttccgacatg actcaggata tgaagttgga   2220
   ggaggagatg cagaattccg acatgactca ggatatgaag ttggaggagg agatgcagaa   2280
   ttccgacatg actcaggata tgaagttgga ggaggagatg cagaattccg acatgactca   2340
   ggatatgaag ttggaggagg agatgcagaa ttccgacatg actcaggata tgaagttgga   2400
   ggaggagatg cagaattccg acatgactca ggatatgaag ttggaggagg agatgcagaa   2460
   ttccgacatg actcaggata tgaagttgga ggaggagatg cagaattccg acatgactca   2520
   ggatatgaag ttggaggagg agatgcagaa ttccgacatg actcaggata tgaagttgga   2580
   ggaggagatg cagaattccg acatgactca ggatatgaag ttggaggagg agatgcagaa   2640
   ttccgacatg actcaggata tgaagttgga ggaggagatg cagaattccg acatgactca   2700
   ggatatgaag ttggaggagg agatgcagaa ttccgacatg actcaggata tgaagttgga   2760
   ggaggagatg cagaattccg acatgactca ggatatgaag ttggaggagg agatgcagaa   2820
   ttccgacatg actcaggata tgaagttgga ggaggagatg cagaattccg acatgactca   2880
   ggatatgaag ttggaggagg agatgcagaa ttccgacatg actcaggata tgaagttgga   2940
   ggaggagatg cagaattccg acatgactca ggatatgaag ttggaggagg agatgcagaa   3000
   ttccgacatg actcaggata tgaagttgga ggaggagatg cagaattccg acatgactca   3060
   ggatatgaag ttggaggagg agatgcagaa ttccgacatg actcaggata tgaagttgga   3120
   ggaggagatg cagaattccg acatgactca ggatatgaag ttggaggagg agatgcagaa   3180
   ttccgacatg actcaggata tgaagttgga ggaggagatg cagaattccg acatgactca   3240
   ggatatgaag ttggaggagg agatgcagaa ttccgacatg actcaggata tgaagttgga   3300
   ggaggagatg cagaattccg acatgactca ggatatgaag ttggaggagg agatgcagaa   3360
   ttccgacatg actcaggata tgaagttgga ggaggagatg cagaattccg acatgactca   3420
   ggatatgaag ttggaggagg attcgagact ggccagaaca ccaggttcac tcccgtgggt   3480
   gtcgttcaag acggcagcac cactcaccag aacgaacccc agcaatgggt cctccctgac   3540
   tactcgggca gggattccca caacgttcac ttggctccgg ccgtggctcc aaccttccct   3600
   ggagagcagt tgctgttctt cagatccact atgccaggct gctctggata cccgaacatg   3660
   aacctcgact gtctcttgcc tcaggagtgg gtgcaacact tctaccagga atctgcccca   3720
   gctcaaagcg acgtcgctct gctccgtttc gttaaccccg ataccggtcg cgtgctcttc   3780
   gagtgtaagt tgcacaagtc tggttacgtc actgttgccc acacaggcca gcacgacctg   3840
   gtcatccctc ccaacggcta cttccgcttc gatagctggg tcaaccagtt ctacacactc   3900
   gccccgatgg gaaacggagc cggtcgtcgc agagccttgt aa                      3942
<210> 76
   <211> 4212
   <212> DNA
   <213> artificial sequence
<400> 76
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaggaggagg agatgcagaa    240
   ttccgacatg actcaggata tgaagttgga ggaggagatg cagaattccg acatgactca    300
   ggatatgaag ttggaggagg agatgcagaa ttccgacatg actcaggata tgaagttgga    360
   ggaggagatg cagaattccg acatgactca ggatatgaag ttggaggagg agatgcagaa    420
   ttccgacatg actcaggata tgaagttgga ggaggagatg cagaattccg acatgactca    480
   ggatatgaag ttggaggagg agatgcagaa ttccgacatg actcaggata tgaagttgga    540
   ggaggagatg cagaattccg acatgactca ggatatgaag ttggaggagg agatgcagaa    600
   ttccgacatg actcaggata tgaagttgga ggaggagatg cagaattccg acatgactca    660
   ggatatgaag ttggaggagg agatgcagaa ttccgacatg actcaggata tgaagttgga    720
   ggaggagatg cagaattccg acatgactca ggatatgaag ttggaggagg agatgcagaa    780
   ttccgacatg actcaggata tgaagttgga ggaggagatg cagaattccg acatgactca    840
   ggatatgaag ttggaggagg agatgcagaa ttccgacatg actcaggata tgaagttgga    900
   ggaggagatg cagaattccg acatgactca ggatatgaag ttggaggagg agatgcagaa    960
   ttccgacatg actcaggata tgaagttgga ggaggagatg cagaattccg acatgactca   1020
   ggatatgaag ttggaggagg agatgcagaa ttccgacatg actcaggata tgaagttgga   1080
   ggaggagatg cagaattccg acatgactca ggatatgaag ttggaggagg agatgcagaa   1140
   ttccgacatg actcaggata tgaagttgga ggaggagatg cagaattccg acatgactca   1200
   ggatatgaag ttggaggagg agatgcagaa ttccgacatg actcaggata tgaagttgga   1260
   ggaggagatg cagaattccg acatgactca ggatatgaag ttggaggagg agatgcagaa   1320
   ttccgacatg actcaggata tgaagttgga ggaggagatg cagaattccg acatgactca   1380
   ggatatgaag ttggaggagg agatgcagaa ttccgacatg actcaggata tgaagttgga   1440
   ggaggagatg cagaattccg acatgactca ggatatgaag ttggaggagg agatgcagaa   1500
   ttccgacatg actcaggata tgaagttgga ggaggagatg cagaattccg acatgactca   1560
   ggatatgaag ttggaggagg agatgcagaa ttccgacatg actcaggata tgaagttgga   1620
   ggaggagatg cagaattccg acatgactca ggatatgaag ttggaggagg agatgcagaa   1680
   ttccgacatg actcaggata tgaagttgga ggaggagatg cagaattccg acatgactca   1740
   ggatatgaag ttggaggagg agatgcagaa ttccgacatg actcaggata tgaagttgga   1800
   ggaggagatg cagaattccg acatgactca ggatatgaag ttggaggagg agatgcagaa   1860
   ttccgacatg actcaggata tgaagttgga ggaggagatg cagaattccg acatgactca   1920
   ggatatgaag ttggaggagg agatgcagaa ttccgacatg actcaggata tgaagttgga   1980
   ggaggagatg cagaattccg acatgactca ggatatgaag ttggaggagg aaactacacc   2040
   atgaacttgg cctcgcagaa ctggaacaac tacgatccaa ccgaggaaat ccccgctcct   2100
   ttgggaactc ccgacttcgt gggacgtatc caaggtgtcc tgacacagac tacacgtcgc   2160
   gacggctcta ctcgcggaca caaggccact gtgtcgacag gttccgtcca cttcacccct   2220
   aagctgggct ctgtgcaatt cagcacagac acctcaggag gaggagatgc agaattccga   2280
   catgactcag gaggaggagg agatgcagaa ttccgacatg actcaggagg aggaggagat   2340
   gcagaattcc gacatgactc aggaggagga ggagatgcag aattccgaca tgactcagga   2400
   ggaggaggag atgcagaatt ccgacatgac tcaggaggag gaggagatgc agaattccga   2460
   catgactcag gaggaggagg agatgcagaa ttccgacatg actcaggagg aggaggagat   2520
   gcagaattcc gacatgactc aggaggagga ggagatgcag aattccgaca tgactcagga   2580
   ggaggaggag atgcagaatt ccgacatgac tcaggaggag gaggagatgc agaattccga 2640
   catgactcag gaggaggagg agatgcagaa ttccgacatg actcaggagg aggaggagat 2700
   gcagaattcc gacatgactc aggaggagga ggagatgcag aattccgaca tgactcagga 2760
   ggaggaggag atgcagaatt ccgacatgac tcaggaggag gaggagatgc agaattccga 2820
   catgactcag gaggaggagg agatgcagaa ttccgacatg actcaggagg aggaggagat 2880
   gcagaattcc gacatgactc aggaggagga ggagatgcag aattccgaca tgactcagga 2940
   ggaggaggag atgcagaatt ccgacatgac tcaggaggag gaggagatgc agaattccga 3000
   catgactcag gaggaggagg agatgcagaa ttccgacatg actcaggagg aggaggagat 3060
   gcagaattcc gacatgactc aggaggagga ggagatgcag aattccgaca tgactcagga 3120
   ggaggaggag atgcagaatt ccgacatgac tcaggaggag gaggagatgc agaattccga 3180
   catgactcag gaggaggagg agatgcagaa ttccgacatg actcaggagg aggaggagat 3240
   gcagaattcc gacatgactc aggaggagga ggagatgcag aattccgaca tgactcagga 3300
   ggaggaggag atgcagaatt ccgacatgac tcaggaggag gaggagatgc agaattccga 3360
   catgactcag gaggaggagg agatgcagaa ttccgacatg actcaggagg aggaggagat 3420
   gcagaattcc gacatgactc aggaggagga ggagatgcag aattccgaca tgactcagga 3480
   ggaggaggag atgcagaatt ccgacatgac tcaggaggag gaggagatgc agaattccga 3540
   catgactcag gaggaggagg agatgcagaa ttccgacatg actcaggagg aggaggagat 3600
   gcagaattcc gacatgactc aggaggagga ggagatgcag aattccgaca tgactcagga 3660
   ggaggaggag atgcagaatt ccgacatgac tcaggaggag gaggaaacga tttcgagact 3720
   ggccagaaca ccaggttcac tcccgtgggt gtcgttcaag acggcagcac cactcaccag 3780
   aacgaacccc agcaatgggt cctccctgac tactcgggca gggattccca caacgttcac 3840
   ttggctccgg ccgtggctcc aaccttccct ggagagcagt tgctgttctt cagatccact 3900
   atgccaggct gctctggata cccgaacatg aacctcgact gtctcttgcc tcaggagtgg 3960
   gtgcaacact tctaccagga atctgcccca gctcaaagcg acgtcgctct gctccgtttc 4020
   gttaaccccg ataccggtcg cgtgctcttc gagtgtaagt tgcacaagtc tggttacgtc 4080
   actgttgccc acacaggcca gcacgacctg gtcatccctc ccaacggcta cttccgcttc 4140
   gatagctggg tcaaccagtt ctacacactc gccccgatgg gaaacggagc cggtcgtcgc 4200
   agagccttgt aa 4212
<210> 77
   <211> 1269
   <212> DNA
   <213> artificial sequence
<400> 77
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaggag gaggagatgc agaattccga    240
   catggaggag gaacacaaaa ctacaccatg aacttggcct cgcagaactg gaacaactac    300
   gatccaaccg aggaaatccc cgctcctttg ggaactcccg acttcgtggg acgtatccaa    360
   ggtgtcctga cacagactac acgtcgcgac ggctctactc gcggacacaa ggccactgtg    420
   tcgacaggtt ccgtccactt cacccctaag ctgggctctg tgcaattcag cacagacacc    480
   tcaggaggag gagatgcaga attccgacat ggaggaggag atgcagaatt ccgacatgga    540
   ggaggagatg cagaattccg acatggagga ggagatgcag aattccgaca tggaggagga    600
   gatgcagaat tccgacatgg aggaggagat gcagaattcc gacatggagg aggagatgca    660
   gaattccgac atggaggagg agatgcagaa ttccgacatg gaggaggaga tgcagaattc    720
   cgacatggag gaggagatgc agaattccga catggaggag gaaacgattt cgagactggc    780
   cagaacacca ggttcactcc cgtgggtgtc gttcaagacg gcagcaccac tcaccagaac    840
   gaaccccagc aatgggtcct ccctgactac tcgggcaggg attcccacaa cgttcacttg    900
   gctccggccg tggctccaac cttccctgga gagcagttgc tgttcttcag atccactatg    960
   ccaggctgct ctggataccc gaacatgaac ctcgactgtc tcttgcctca ggagtgggtg   1020
   caacacttct accaggaatc tgccccagct caaagcgacg tcgctctgct ccgtttcgtt   1080
   aaccccgata ccggtcgcgt gctcttcgag tgtaagttgc acaagtctgg ttacgtcact   1140
   gttgcccaca caggccagca cgacctggtc atccctccca acggctactt ccgcttcgat   1200
   agctgggtca accagttcta cacactcgcc ccgatgggaa acggagccgg tcgtcgcaga   1260
   gccttgtaa                                                          1269
<210> 78
   <211> 2052
   <212> DNA
   <213> artificial sequence
<400> 78
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaggag gaggagatgc agaattccga    240
   catgactcag gaggaggagg agatgcagaa ttccgacatg actcaggagg aggaggagat    300
   gcagaattcc gacatgactc aggaggagga ggagatgcag aattccgaca tgactcagga    360
   ggaggaggag atgcagaatt ccgacatgac tcaggaggag gaggagatgc agaattccga     420
   catgactcag gaggaggagg agatgcagaa ttccgacatg actcaggagg aggaggagat     480
   gcagaattcc gacatgactc aggaggagga ggagatgcag aattccgaca tgactcagga     540
   ggaggaggag atgcagaatt ccgacatgac tcaggaggag gaggaacaca aaactacacc     600
   atgaacttgg cctcgcagaa ctggaacaac tacgatccaa ccgaggaaat ccccgctcct     660
   ttgggaactc ccgacttcgt gggacgtatc caaggtgtcc tgacacagac tacacgtcgc     720
   gacggctcta ctcgcggaca caaggccact gtgtcgacag gttccgtcca cttcacccct     780
   aagctgggct ctgtgcaatt cagcacagac acctcaggag gaggagatgc agaattccga     840
   catgactcag gaggaggagg agatgcagaa ttccgacatg actcaggagg aggaggagat     900
   gcagaattcc gacatgactc aggaggagga ggagatgcag aattccgaca tgactcagga     960
   ggaggaggag atgcagaatt ccgacatgac tcaggaggag gaggagatgc agaattccga    1020
   catgactcag gaggaggagg agatgcagaa ttccgacatg actcaggagg aggaggagat    1080
   gcagaattcc gacatgactc aggaggagga ggagatgcag aattccgaca tgactcagga    1140
   ggaggaggag atgcagaatt ccgacatgac tcaggaggag gaggagatgc agaattccga    1200
   catgactcag gaggaggagg agatgcagaa ttccgacatg actcaggagg aggaggagat    1260
   gcagaattcc gacatgactc aggaggagga ggagatgcag aattccgaca tgactcagga    1320
   ggaggaggag atgcagaatt ccgacatgac tcaggaggag gaggagatgc agaattccga    1380
   catgactcag gaggaggagg agatgcagaa ttccgacatg actcaggagg aggaggagat    1440
   gcagaattcc gacatgactc aggaggagga ggagatgcag aattccgaca tgactcagga    1500
   ggaggaggag atgcagaatt ccgacatgac tcaggaggag gaggaaacga tttcgagact    1560
   ggccagaaca ccaggttcac tcccgtgggt gtcgttcaag acggcagcac cactcaccag    1620
   aacgaacccc agcaatgggt cctccctgac tactcgggca gggattccca caacgttcac    1680
   ttggctccgg ccgtggctcc aaccttccct ggagagcagt tgctgttctt cagatccact    1740
   atgccaggct gctctggata cccgaacatg aacctcgact gtctcttgcc tcaggagtgg    1800
   gtgcaacact tctaccagga atctgcccca gctcaaagcg acgtcgctct gctccgtttc    1860
   gttaaccccg ataccggtcg cgtgctcttc gagtgtaagt tgcacaagtc tggttacgtc    1920
   actgttgccc acacaggcca gcacgacctg gtcatccctc ccaacggcta cttccgcttc    1980
   gatagctggg tcaaccagtt ctacacactc gccccgatgg gaaacggagc cggtcgtcgc    2040
   agagccttgt aa                                                        2052
<210> 79
   <211> 3672
   <212> DNA
   <213> artificial sequence
<400> 79
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catgactcag gatatgaagt tggaggagga gatgcagaat tccgacatga ctcaggatat 300
   gaagttggag gaggagatgc agaattccga catgactcag gatatgaagt tggaggagga 360
   gatgcagaat tccgacatga ctcaggatat gaagttggag gaggagatgc agaattccga 420
   catgactcag gatatgaagt tggaggagga gatgcagaat tccgacatga ctcaggatat 480
   gaagttggag gaggagatgc agaattccga catgactcag gatatgaagt tggaggagga 540
   gatgcagaat tccgacatga ctcaggatat gaagttggag gaggagatgc agaattccga 600
   catgactcag gatatgaagt tggaggagga gatgcagaat tccgacatga ctcaggatat 660
   gaagttggag gaggagatgc agaattccga catgactcag gatatgaagt tggaggagga 720
   gatgcagaat tccgacatga ctcaggatat gaagttggag gaggagatgc agaattccga 780
   catgactcag gatatgaagt tggaggagga gatgcagaat tccgacatga ctcaggatat 840
   gaagttggag gaggagatgc agaattccga catgactcag gatatgaagt tggaggagga 900
   gatgcagaat tccgacatga ctcaggatat gaagttggag gaggagatgc agaattccga 960
   catgactcag gatatgaagt tggaggagga gatgcagaat tccgacatga ctcaggatat 1020
   gaagttggag gaggagatgc agaattccga catgactcag gatatgaagt tggaggagga 1080
   gatgcagaat tccgacatga ctcaggatat gaagttggag gaggaacaca aaactacacc 1140
   atgaacttgg cctcgcagaa ctggaacaac tacgatccaa ccgaggaaat ccccgctcct 1200
   ttgggaactc ccgacttcgt gggacgtatc caaggtgtcc tgacacagac tacacgtcgc 1260
   gacggctcta ctcgcggaca caaggccact gtgtcgacag gttccgtcca cttcacccct 1320
   aagctgggct ctgtgcaatt cagcacagac acctcaggag gaggagatgc agaattccga 1380
   catgactcag gatatgaagt tggaggagga gatgcagaat tccgacatga ctcaggatat 1440
   gaagttggag gaggagatgc agaattccga catgactcag gatatgaagt tggaggagga 1500
   gatgcagaat tccgacatga ctcaggatat gaagttggag gaggagatgc agaattccga 1560
   catgactcag gatatgaagt tggaggagga gatgcagaat tccgacatga ctcaggatat 1620
   gaagttggag gaggagatgc agaattccga catgactcag gatatgaagt tggaggagga 1680
   gatgcagaat tccgacatga ctcaggatat gaagttggag gaggagatgc agaattccga 1740
   catgactcag gatatgaagt tggaggagga gatgcagaat tccgacatga ctcaggatat 1800
   gaagttggag gaggagatgc agaattccga catgactcag gatatgaagt tggaggagga 1860
   gatgcagaat tccgacatga ctcaggatat gaagttggag gaggagatgc agaattccga 1920
   catgactcag gatatgaagt tggaggagga gatgcagaat tccgacatga ctcaggatat 1980
   gaagttggag gaggagatgc agaattccga catgactcag gatatgaagt tggaggagga 2040
   gatgcagaat tccgacatga ctcaggatat gaagttggag gaggagatgc agaattccga 2100
   catgactcag gatatgaagt tggaggagga gatgcagaat tccgacatga ctcaggatat 2160
   gaagttggag gaggagatgc agaattccga catgactcag gatatgaagt tggaggagga 2220
   gatgcagaat tccgacatga ctcaggatat gaagttggag gaggagatgc agaattccga 2280
   catgactcag gatatgaagt tggaggagga gatgcagaat tccgacatga ctcaggatat 2340
   gaagttggag gaggagatgc agaattccga catgactcag gatatgaagt tggaggagga 2400
   gatgcagaat tccgacatga ctcaggatat gaagttggag gaggagatgc agaattccga 2460
   catgactcag gatatgaagt tggaggagga gatgcagaat tccgacatga ctcaggatat 2520
   gaagttggag gaggagatgc agaattccga catgactcag gatatgaagt tggaggagga 2580
   gatgcagaat tccgacatga ctcaggatat gaagttggag gaggagatgc agaattccga 2640
   catgactcag gatatgaagt tggaggagga gatgcagaat tccgacatga ctcaggatat 2700
   gaagttggag gaggagatgc agaattccga catgactcag gatatgaagt tggaggagga 2760
   gatgcagaat tccgacatga ctcaggatat gaagttggag gaggagatgc agaattccga 2820
   catgactcag gatatgaagt tggaggagga gatgcagaat tccgacatga ctcaggatat 2880
   gaagttggag gaggagatgc agaattccga catgactcag gatatgaagt tggaggagga 2940
   gatgcagaat tccgacatga ctcaggatat gaagttggag gaggagatgc agaattccga 3000
   catgactcag gatatgaagt tggaggagga gatgcagaat tccgacatga ctcaggatat 3060
   gaagttggag gaggagatgc agaattccga catgactcag gatatgaagt tggaggagga 3120
   gatgcagaat tccgacatga ctcaggatat gaagttggag gaggaaacga tttcgagact 3180
   ggccagaaca ccaggttcac tcccgtgggt gtcgttcaag acggcagcac cactcaccag 3240
   aacgaacccc agcaatgggt cctccctgac tactcgggca gggattccca caacgttcac 3300
   ttggctccgg ccgtggctcc aaccttccct ggagagcagt tgctgttctt cagatccact 3360
   atgccaggct gctctggata cccgaacatg aacctcgact gtctcttgcc tcaggagtgg 3420
   gtgcaacact tctaccagga atctgcccca gctcaaagcg acgtcgctct gctccgtttc 3480
   gttaaccccg ataccggtcg cgtgctcttc gagtgtaagt tgcacaagtc tggttacgtc 3540
   actgttgccc acacaggcca gcacgacctg gtcatccctc ccaacggcta cttccgcttc 3600
   gatagctggg tcaaccagtt ctacacactc gccccgatgg gaaacggagc cggtcgtcgc 3660
   agagccttgt aa 3672
<210> 80
   <211> 3672
   <212> DNA
   <213> artificial sequence
<400> 80
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tcatcatcaa ggaggaggag atgcagaatt ccgacatgac     300
   tcaggatatg aagttcatca tcaaggagga ggagatgcag aattccgaca tgactcagga     360
   tatgaagttc atcatcaagg aggaggagat gcagaattcc gacatgactc aggatatgaa     420
   gttcatcatc aaggaggagg agatgcagaa ttccgacatg actcaggata tgaagttcat     480
   catcaaggag gaggagatgc agaattccga catgactcag gatatgaagt tcatcatcaa     540
   ggaggaggag atgcagaatt ccgacatgac tcaggatatg aagttcatca tcaaggagga     600
   ggagatgcag aattccgaca tgactcagga tatgaagttc atcatcaagg aggaggagat     660
   gcagaattcc gacatgactc aggatatgaa gttcatcatc aaggaggagg agatgcagaa     720
   ttccgacatg actcaggata tgaagttcat catcaaggag gaggagatgc agaattccga     780
   catgactcag gatatgaagt tcatcatcaa ggaggaggag atgcagaatt ccgacatgac     840
   tcaggatatg aagttcatca tcaaggagga ggagatgcag aattccgaca tgactcagga     900
   tatgaagttc atcatcaagg aggaggagat gcagaattcc gacatgactc aggatatgaa     960
   gttcatcatc aaggaggagg agatgcagaa ttccgacatg actcaggata tgaagttcat    1020
   catcaaggag gaggagatgc agaattccga catgactcag gatatgaagt tcatcatcaa    1080
   ggaggaggag atgcagaatt ccgacatgac tcaggatatg aagttcatca tcaaggagga    1140
   ggagatgcag aattccgaca tgactcagga tatgaagttc atcatcaagg aggaggagat    1200
   gcagaattcc gacatgactc aggatatgaa gttcatcatc aaggaggagg agatgcagaa    1260
   ttccgacatg actcaggata tgaagttcat catcaaggag gaggagatgc agaattccga    1320
   catgactcag gatatgaagt tcatcatcaa ggaggaggag atgcagaatt ccgacatgac    1380
   tcaggatatg aagttcatca tcaaggagga ggagatgcag aattccgaca tgactcagga    1440
   tatgaagttc atcatcaagg aggaggagat gcagaattcc gacatgactc aggatatgaa    1500
   gttcatcatc aaggaggagg agatgcagaa ttccgacatg actcaggata tgaagttcat    1560
   catcaaggag gaggagatgc agaattccga catgactcag gatatgaagt tcatcatcaa    1620
   ggaggaggag atgcagaatt ccgacatgac tcaggatatg aagttcatca tcaaggagga    1680
   ggagatgcag aattccgaca tgactcagga tatgaagttc atcatcaagg aggaggagat 1740
   gcagaattcc gacatgactc aggatatgaa gttcatcatc aaggaggagg agatgcagaa 1800
   ttccgacatg actcaggata tgaagttcat catcaaggag gaggagatgc agaattccga 1860
   catgactcag gatatgaagt tcatcatcaa ggaggaggag atgcagaatt ccgacatgac 1920
   tcaggatatg aagttcatca tcaaggagga ggagatgcag aattccgaca tgactcagga 1980
   tatgaagttc atcatcaagg aggaggagat gcagaattcc gacatgactc aggatatgaa 2040
   gttcatcatc aaggaggagg agatgcagaa ttccgacatg actcaggata tgaagttcat 2100
   catcaaggag gaggagatgc agaattccga catgactcag gatatgaagt tcatcatcaa 2160
   ggaggaggag atgcagaatt ccgacatgac tcaggatatg aagttcatca tcaaggagga 2220
   ggagatgcag aattccgaca tgactcagga tatgaagttc atcatcaagg aggaggagat 2280
   gcagaattcc gacatgactc aggatatgaa gttcatcatc aaggaggagg agatgcagaa 2340
   ttccgacatg actcaggata tgaagttcat catcaaggag gaggaacaca aaactacacc 2400
   atgaacttgg cctcgcagaa ctggaacaac tacgatccaa ccgaggaaat ccccgctcct 2460
   ttgggaactc ccgacttcgt gggacgtatc caaggtgtcc tgacacagac tacacgtcgc 2520
   gacggctcta ctcgcggaca caaggccact gtgtcgacag gttccgtcca cttcacccct 2580
   aagctgggct ctgtgcaatt cagcacagac acctcaggag gaggagatgc agaattccga 2640
   catgactcag gatatgaagt tcatcatcaa ggaggaggag atgcagaatt ccgacatgac 2700
   tcaggatatg aagttcatca tcaaggagga ggagatgcag aattccgaca tgactcagga 2760
   tatgaagttc atcatcaagg aggaggagat gcagaattcc gacatgactc aggatatgaa 2820
   gttcatcatc aaggaggagg agatgcagaa ttccgacatg actcaggata tgaagttcat 2880
   catcaaggag gaggagatgc agaattccga catgactcag gatatgaagt tcatcatcaa 2940
   ggaggaggag atgcagaatt ccgacatgac tcaggatatg aagttcatca tcaaggagga 3000
   ggagatgcag aattccgaca tgactcagga tatgaagttc atcatcaagg aggaggagat 3060
   gcagaattcc gacatgactc aggatatgaa gttcatcatc aaggaggagg agatgcagaa 3120
   ttccgacatg actcaggata tgaagttcat catcaaggag gaggaaacga tttcgagact 3180
   ggccagaaca ccaggttcac tcccgtgggt gtcgttcaag acggcagcac cactcaccag 3240
   aacgaacccc agcaatgggt cctccctgac tactcgggca gggattccca caacgttcac 3300
   ttggctccgg ccgtggctcc aaccttccct ggagagcagt tgctgttctt cagatccact 3360
   atgccaggct gctctggata cccgaacatg aacctcgact gtctcttgcc tcaggagtgg 3420
   gtgcaacact tctaccagga atctgcccca gctcaaagcg acgtcgctct gctccgtttc 3480
   gttaaccccg ataccggtcg cgtgctcttc gagtgtaagt tgcacaagtc tggttacgtc 3540
   actgttgccc acacaggcca gcacgacctg gtcatccctc ccaacggcta cttccgcttc   3600
   gatagctggg tcaaccagtt ctacacactc gccccgatgg gaaacggagc cggtcgtcgc   3660
   agagccttgt aa                                                       3672
<210> 81
   <211> 1449
   <212> DNA
   <213> artificial sequence
<400> 81
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc ggaggaggag atgcagaatt ccgacatgga    240
   ggaggagctg gaacacaaaa ctacaccatg aacttggcct cgcagaactg gaacaactac    300
   gatccaaccg aggaaatccc cgctcctttg ggaactcccg acttcgtggg acgtatccaa    360
   ggtgtcctga cacagactac acgtcgcgac ggctctactc gcggacacaa ggccactgtg    420
   tcgacaggtt ccgtccactt cacccctaag ctgggctctg tgcaattcag cacagacacc    480
   ggaggaggag atgcagaatt ccgacatgac tcaggatatg aagttggagg aggagatgca    540
   gaattccgac atgactcagg atatgaagtt ggaggaggag atgcagaatt ccgacatgac    600
   tcaggatatg aagttggagg aggagatgca gaattccgac atgactcagg atatgaagtt    660
   ggaggaggag atgcagaatt ccgacatgac tcaggatatg aagttggagg aggagatgca    720
   gaattccgac atgactcagg atatgaagtt ggaggaggag atgcagaatt ccgacatgac    780
   tcaggatatg aagttggagg aggagatgca gaattccgac atgactcagg atatgaagtt    840
   ggaggaggag atgcagaatt ccgacatgac tcaggatatg aagttggagg aggagatgca    900
   gaattccgac atgactcagg atatgaagtt ggaggaggat caaacgattt cgagactggc    960
   cagaacacca ggttcactcc cgtgggtgtc gttcaagacg gcagcaccac tcaccagaac   1020
   gaaccccagc aatgggtcct ccctgactac tcgggcaggg attcccacaa cgttcacttg   1080
   gctccggccg tggctccaac cttccctgga gagcagttgc tgttcttcag atccactatg   1140
   ccaggctgct ctggataccc gaacatgaac ctcgactgtc tcttgcctca ggagtgggtg   1200
   caacacttct accaggaatc tgccccagct caaagcgacg tcgctctgct ccgtttcgtt   1260
   aaccccgata ccggtcgcgt gctcttcgag tgtaagttgc acaagtctgg ttacgtcact   1320
   gttgcccaca caggccagca cgacctggtc atccctccca acggctactt ccgcttcgat   1380
   agctgggtca accagttcta cacactcgcc ccgatgggaa acggagccgg tcgtcgcaga   1440
   gccttgtaa                                                            1449
<210> 82
   <211> 1872
   <212> DNA
   <213> artificial sequence
<400> 82
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggag gaggaggaga tgcagaattc cgacatgact caggaggagg aggagatgca     300
   gaattccgac atgactcagg aggaggagga gatgcagaat tccgacatga ctcaggagga     360
   ggaggagatg cagaattccg acatgactca ggaggaggag gagatgcaga attccgacat     420
   gactcaggag gaggaggaga tgcagaattc cgacatgact caggaggagg aggagatgca     480
   gaattccgac atgactcagg aggaggagga gatgcagaat tccgacatga ctcaggagga     540
   ggaggagatg cagaattccg acatgactca ggaggaggag gaggaacaca aaactacacc     600
   atgaacttgg cctcgcagaa ctggaacaac tacgatccaa ccgaggaaat ccccgctcct     660
   ttgggaactc ccgacttcgt gggacgtatc caaggtgtcc tgacacagac tacacgtcgc     720
   gacggctcta ctcgcggaca caaggccact gtgtcgacag gttccgtcca cttcacccct     780
   aagctgggct ctgtgcaatt cagcacagac acctcaggag gaggagatgc agaattccga     840
   catggaggag gagatgcaga attccgacat ggaggaggag atgcagaatt ccgacatgga     900
   ggaggagatg cagaattccg acatggagga ggagatgcag aattccgaca tggaggagga     960
   gatgcagaat tccgacatgg aggaggagat gcagaattcc gacatggagg aggagatgca    1020
   gaattccgac atggaggagg agatgcagaa ttccgacatg gaggaggaga tgcagaattc    1080
   cgacatggag gaggagatgc agaattccga catggaggag gagatgcaga attccgacat    1140
   ggaggaggag atgcagaatt ccgacatgga ggaggagatg cagaattccg acatggagga    1200
   ggagatgcag aattccgaca tggaggagga gatgcagaat tccgacatgg aggaggagat    1260
   gcagaattcc gacatggagg aggagatgca gaattccgac atggaggagg agatgcagaa    1320
   ttccgacatg gaggaggaga tgcagaattc cgacatggag gaggaaacga tttcgagact    1380
   ggccagaaca ccaggttcac tcccgtgggt gtcgttcaag acggcagcac cactcaccag    1440
   aacgaacccc agcaatgggt cctccctgac tactcgggca gggattccca caacgttcac    1500
   ttggctccgg ccgtggctcc aaccttccct ggagagcagt tgctgttctt cagatccact    1560
   atgccaggct gctctggata cccgaacatg aacctcgact gtctcttgcc tcaggagtgg    1620
   gtgcaacact tctaccagga atctgcccca gctcaaagcg acgtcgctct gctccgtttc    1680
   gttaaccccg ataccggtcg cgtgctcttc gagtgtaagt tgcacaagtc tggttacgtc 1740
   actgttgccc acacaggcca gcacgacctg gtcatccctc ccaacggcta cttccgcttc 1800
   gatagctggg tcaaccagtt ctacacactc gccccgatgg gaaacggagc cggtcgtcgc 1860
   agagccttgt aa 1872
<210> 83
   <211> 3132
   <212> DNA
   <213> artificial sequence
<400> 83
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tcatcatcaa ggaggaggag atgcagaatt ccgacatgac     300
   tcaggatatg aagttcatca tcaaggagga ggagatgcag aattccgaca tgactcagga     360
   tatgaagttc atcatcaagg aggaggagat gcagaattcc gacatgactc aggatatgaa     420
   gttcatcatc aaggaggagg agatgcagaa ttccgacatg actcaggata tgaagttcat     480
   catcaaggag gaggagatgc agaattccga catgactcag gatatgaagt tcatcatcaa     540
   ggaggaggag atgcagaatt ccgacatgac tcaggatatg aagttcatca tcaaggagga     600
   ggagatgcag aattccgaca tgactcagga tatgaagttc atcatcaagg aggaggagat     660
   gcagaattcc gacatgactc aggatatgaa gttcatcatc aaggaggagg agatgcagaa     720
   ttccgacatg actcaggata tgaagttcat catcaaggag gaggagatgc agaattccga     780
   catgactcag gatatgaagt tcatcatcaa ggaggaggag atgcagaatt ccgacatgac     840
   tcaggatatg aagttcatca tcaaggagga ggagatgcag aattccgaca tgactcagga     900
   tatgaagttc atcatcaagg aggaggagat gcagaattcc gacatgactc aggatatgaa     960
   gttcatcatc aaggaggagg agatgcagaa ttccgacatg actcaggata tgaagttcat    1020
   catcaaggag gaggagatgc agaattccga catgactcag gatatgaagt tcatcatcaa    1080
   ggaggaggag atgcagaatt ccgacatgac tcaggatatg aagttcatca tcaaggagga    1140
   ggagatgcag aattccgaca tgactcagga tatgaagttc atcatcaagg aggaggagat    1200
   gcagaattcc gacatgactc aggatatgaa gttcatcatc aaggaggagg agatgcagaa    1260
   ttccgacatg actcaggata tgaagttcat catcaaggag gaggaacaca aaactacacc    1320
   atgaacttgg cctcgcagaa ctggaacaac tacgatccaa ccgaggaaat ccccgctcct    1380
   ttgggaactc ccgacttcgt gggacgtatc caaggtgtcc tgacacagac tacacgtcgc    1440
   gacggctcta ctcgcggaca caaggccact gtgtcgacag gttccgtcca cttcacccct 1500
   aagctgggct ctgtgcaatt cagcacagac acctcaggag gaggagatgc agaattccga 1560
   catggaggag gagatgcaga attccgacat ggaggaggag atgcagaatt ccgacatgga 1620
   ggaggagatg cagaattccg acatggagga ggagatgcag aattccgaca tggaggagga 1680
   gatgcagaat tccgacatgg aggaggagat gcagaattcc gacatggagg aggagatgca 1740
   gaattccgac atggaggagg agatgcagaa ttccgacatg gaggaggaga tgcagaattc 1800
   cgacatggag gaggagatgc agaattccga catggaggag gagatgcaga attccgacat 1860
   ggaggaggag atgcagaatt ccgacatgga ggaggagatg cagaattccg acatggagga 1920
   ggagatgcag aattccgaca tggaggagga gatgcagaat tccgacatgg aggaggagat 1980
   gcagaattcc gacatggagg aggagatgca gaattccgac atggaggagg agatgcagaa 2040
   ttccgacatg gaggaggaga tgcagaattc cgacatggag gaggagatgc agaattccga 2100
   catggaggag gagatgcaga attccgacat ggaggaggag atgcagaatt ccgacatgga 2160
   ggaggagatg cagaattccg acatggagga ggagatgcag aattccgaca tggaggagga 2220
   gatgcagaat tccgacatgg aggaggagat gcagaattcc gacatggagg aggagatgca 2280
   gaattccgac atggaggagg agatgcagaa ttccgacatg gaggaggaga tgcagaattc 2340
   cgacatggag gaggagatgc agaattccga catggaggag gagatgcaga attccgacat 2400
   ggaggaggag atgcagaatt ccgacatgga ggaggagatg cagaattccg acatggagga 2460
   ggagatgcag aattccgaca tggaggagga gatgcagaat tccgacatgg aggaggagat 2520
   gcagaattcc gacatggagg aggagatgca gaattccgac atggaggagg agatgcagaa 2580
   ttccgacatg gaggaggaga tgcagaattc cgacatggag gaggaaacga tttcgagact 2640
   ggccagaaca ccaggttcac tcccgtgggt gtcgttcaag acggcagcac cactcaccag 2700
   aacgaacccc agcaatgggt cctccctgac tactcgggca gggattccca caacgttcac 2760
   ttggctccgg ccgtggctcc aaccttccct ggagagcagt tgctgttctt cagatccact 2820
   atgccaggct gctctggata cccgaacatg aacctcgact gtctcttgcc tcaggagtgg 2880
   gtgcaacact tctaccagga atctgcccca gctcaaagcg acgtcgctct gctccgtttc 2940
   gttaaccccg ataccggtcg cgtgctcttc gagtgtaagt tgcacaagtc tggttacgtc 3000
   actgttgccc acacaggcca gcacgacctg gtcatccctc ccaacggcta cttccgcttc 3060
   gatagctggg tcaaccagtt ctacacactc gccccgatgg gaaacggagc cggtcgtcgc 3120
   agagccttgt aa 3132
<210> 84
   <211> 3492
   <212> DNA
   <213> artificial sequence
<400> 84
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catgactcag gaggaggagg agatgcagaa ttccgacatg actcaggagg aggaggagat 300
   gcagaattcc gacatgactc aggaggagga ggagatgcag aattccgaca tgactcagga 360
   ggaggaggag atgcagaatt ccgacatgac tcaggaggag gaggagatgc agaattccga 420
   catgactcag gaggaggagg agatgcagaa ttccgacatg actcaggagg aggaggagat 480
   gcagaattcc gacatgactc aggaggagga ggagatgcag aattccgaca tgactcagga 540
   ggaggaggag atgcagaatt ccgacatgac tcaggaggag gaggagatgc agaattccga 600
   catgactcag gaggaggagg agatgcagaa ttccgacatg actcaggagg aggaggagat 660
   gcagaattcc gacatgactc aggaggagga ggagatgcag aattccgaca tgactcagga 720
   ggaggaggag atgcagaatt ccgacatgac tcaggaggag gaggagatgc agaattccga 780
   catgactcag gaggaggagg agatgcagaa ttccgacatg actcaggagg aggaggagat 840
   gcagaattcc gacatgactc aggaggagga ggagatgcag aattccgaca tgactcagga 900
   ggaggaggag atgcagaatt ccgacatgac tcaggaggag gaggagatgc agaattccga 960
   catgactcag gaggaggagg agatgcagaa ttccgacatg actcaggagg aggaggagat 1020
   gcagaattcc gacatgactc aggaggagga ggagatgcag aattccgaca tgactcagga 1080
   ggaggaggag atgcagaatt ccgacatgac tcaggaggag gaggagatgc agaattccga 1140
   catgactcag gaggaggagg agatgcagaa ttccgacatg actcaggagg aggaggagat 1200
   gcagaattcc gacatgactc aggaggagga ggagatgcag aattccgaca tgactcagga 1260
   ggaggaggag atgcagaatt ccgacatgac tcaggaggag gaggagatgc agaattccga 1320
   catgactcag gaggaggagg agatgcagaa ttccgacatg actcaggagg aggaggagat 1380
   gcagaattcc gacatgactc aggaggagga ggagatgcag aattccgaca tgactcagga 1440
   ggaggaggag atgcagaatt ccgacatgac tcaggaggag gaggagatgc agaattccga 1500
   catgactcag gaggaggagg agatgcagaa ttccgacatg actcaggagg aggaggagat 1560
   gcagaattcc gacatgactc aggaggagga ggagatgcag aattccgaca tgactcagga 1620
   ggaggaggag atgcagaatt ccgacatgac tcaggaggag gaggaacaca aaactacacc 1680
   atgaacttgg cctcgcagaa ctggaacaac tacgatccaa ccgaggaaat ccccgctcct 1740
   ttgggaactc ccgacttcgt gggacgtatc caaggtgtcc tgacacagac tacacgtcgc 1800
   gacggctcta ctcgcggaca caaggccact gtgtcgacag gttccgtcca cttcacccct 1860
   aagctgggct ctgtgcaatt cagcacagac acctcaaacg gaggaggaga tgcagaattc 1920
   cgacatgact caggatatga agttcatcat caaggaggag gagatgcaga attccgacat 1980
   gactcaggat atgaagttca tcatcaagga ggaggagatg cagaattccg acatgactca 2040
   ggatatgaag ttcatcatca aggaggagga gatgcagaat tccgacatga ctcaggatat 2100
   gaagttcatc atcaaggagg aggagatgca gaattccgac atgactcagg atatgaagtt 2160
   catcatcaag gaggaggaga tgcagaattc cgacatgact caggatatga agttcatcat 2220
   caaggaggag gagatgcaga attccgacat gactcaggat atgaagttca tcatcaagga 2280
   ggaggagatg cagaattccg acatgactca ggatatgaag ttcatcatca aggaggagga 2340
   gatgcagaat tccgacatga ctcaggatat gaagttcatc atcaaggagg aggagatgca 2400
   gaattccgac atgactcagg atatgaagtt catcatcaag gaggaggaga tgcagaattc 2460
   cgacatgact caggatatga agttcatcat caaggaggag gagatgcaga attccgacat 2520
   gactcaggat atgaagttca tcatcaagga ggaggagatg cagaattccg acatgactca 2580
   ggatatgaag ttcatcatca aggaggagga gatgcagaat tccgacatga ctcaggatat 2640
   gaagttcatc atcaaggagg aggagatgca gaattccgac atgactcagg atatgaagtt 2700
   catcatcaag gaggaggaga tgcagaattc cgacatgact caggatatga agttcatcat 2760
   caaggaggag gagatgcaga attccgacat gactcaggat atgaagttca tcatcaagga 2820
   ggaggagatg cagaattccg acatgactca ggatatgaag ttcatcatca aggaggagga 2880
   gatgcagaat tccgacatga ctcaggatat gaagttcatc atcaaggagg aggagatgca 2940
   gaattccgac atgactcagg atatgaagtt catcatcaag gaggaggaga tttcgagact 3000
   ggccagaaca ccaggttcac tcccgtgggt gtcgttcaag acggcagcac cactcaccag 3060
   aacgaacccc agcaatgggt cctccctgac tactcgggca gggattccca caacgttcac 3120
   ttggctccgg ccgtggctcc aaccttccct ggagagcagt tgctgttctt cagatccact 3180
   atgccaggct gctctggata cccgaacatg aacctcgact gtctcttgcc tcaggagtgg 3240
   gtgcaacact tctaccagga atctgcccca gctcaaagcg acgtcgctct gctccgtttc 3300
   gttaaccccg ataccggtcg cgtgctcttc gagtgtaagt tgcacaagtc tggttacgtc 3360
   actgttgccc acacaggcca gcacgacctg gtcatccctc ccaacggcta cttccgcttc 3420
   gatagctggg tcaaccagtt ctacacactc gccccgatgg gaaacggagc cggtcgtcgc 3480
   agagccttgt aa 3492
<210> 85
   <211> 2502
   <212> DNA
   <213> artificial sequence
<400> 85
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacag gaggaggaga tgcagaattc 240
   cgacatgact caggatatga agttcatcat caaggaggag gagatgcaga attccgacat 300
   gactcaggat atgaagttca tcatcaagga ggaggagatg cagaattccg acatgactca 360
   ggatatgaag ttcatcatca aggaggagga gatgcagaat tccgacatga ctcaggatat 420
   gaagttcatc atcaaggagg aggagatgca gaattccgac atgactcagg atatgaagtt 480
   catcatcaag gaggaggaga tgcagaattc cgacatgact caggatatga agttcatcat 540
   caaggaggag gagatgcaga attccgacat gactcaggat atgaagttca tcatcaagga 600
   ggaggagatg cagaattccg acatgactca ggatatgaag ttcatcatca aggaggagga 660
   gatgcagaat tccgacatga ctcaggatat gaagttcatc atcaaggagg aggagatgca 720
   gaattccgac atgactcagg atatgaagtt catcatcaag gaggaggaga tgcagaattc 780
   cgacatgact caggatatga agttcatcat caaggaggag gagatgcaga attccgacat 840
   gactcaggat atgaagttca tcatcaagga ggaggagatg cagaattccg acatgactca 900
   ggatatgaag ttcatcatca aggaggagga gatgcagaat tccgacatga ctcaggatat 960
   gaagttcatc atcaaggagg aggagatgca gaattccgac atgactcagg atatgaagtt 1020
   catcatcaag gaggaggaga tgcagaattc cgacatgact caggatatga agttcatcat 1080
   caaggaggag gagatgcaga attccgacat gactcaggat atgaagttca tcatcaagga 1140
   ggaggagatg cagaattccg acatgactca ggatatgaag ttcatcatca aggaggagga 1200
   gatgcagaat tccgacatga ctcaggatat gaagttcatc atcaaggagg aggagatgca 1260
   gaattccgac atgactcagg atatgaagtt catcatcaag gaggaggaca aaactacacc 1320
   atgaacttgg cctcgcagaa ctggaacaac tacgatccaa ccgaggaaat ccccgctcct 1380
   ttgggaactc ccgacttcgt gggacgtatc caaggtgtcc tgacacagac tacacgtcgc 1440
   gacggctcta ctcgcggaca caaggccact gtgtcgacag gttccgtcca cttcacccct 1500
   aagctgggct ctgtgcaatt cagcacagac acctcaaacg atggaggagg agatgcagaa 1560
   ttccgacatg actcaggata tgaagttgga ggaggagatg cagaattccg acatgactca 1620
   ggatatgaag ttggaggagg agatgcagaa ttccgacatg actcaggata tgaagttgga 1680
   ggaggagatg cagaattccg acatgactca ggatatgaag ttggaggagg agatgcagaa 1740
   ttccgacatg actcaggata tgaagttgga ggaggagatg cagaattccg acatgactca 1800
   ggatatgaag ttggaggagg agatgcagaa ttccgacatg actcaggata tgaagttgga 1860
   ggaggagatg cagaattccg acatgactca ggatatgaag ttggaggagg agatgcagaa   1920
   ttccgacatg actcaggata tgaagttgga ggaggagatg cagaattccg acatgactca   1980
   ggatatgaag ttggaggagg attcgagact ggccagaaca ccaggttcac tcccgtgggt   2040
   gtcgttcaag acggcagcac cactcaccag aacgaacccc agcaatgggt cctccctgac   2100
   tactcgggca gggattccca caacgttcac ttggctccgg ccgtggctcc aaccttccct   2160
   ggagagcagt tgctgttctt cagatccact atgccaggct gctctggata cccgaacatg   2220
   aacctcgact gtctcttgcc tcaggagtgg gtgcaacact tctaccagga atctgcccca   2280
   gctcaaagcg acgtcgctct gctccgtttc gttaaccccg ataccggtcg cgtgctcttc   2340
   gagtgtaagt tgcacaagtc tggttacgtc actgttgccc acacaggcca gcacgacctg   2400
   gtcatccctc ccaacggcta cttccgcttc gatagctggg tcaaccagtt ctacacactc   2460
   gccccgatgg gaaacggagc cggtcgtcgc agagccttgt aa                       2502
<210> 86
   <211> 1458
   <212> DNA
   <213> artificial sequence
<400> 86
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaggaggagg agatgcagaa    240
   ttccgacatg actcaggata tgaagttgga ggaggagatg cagaattccg acatgactca    300
   ggatatgaag ttggaggagg agatgcagaa ttccgacatg actcaggata tgaagttgga    360
   ggaggagatg cagaattccg acatgactca ggatatgaag ttggaggagg agatgcagaa    420
   ttccgacatg actcaggata tgaagttgga ggaggagatg cagaattccg acatgactca    480
   ggatatgaag ttggaggagg agatgcagaa ttccgacatg actcaggata tgaagttgga    540
   ggaggagatg cagaattccg acatgactca ggatatgaag ttggaggagg agatgcagaa    600
   ttccgacatg actcaggata tgaagttgga ggaggagatg cagaattccg acatgactca    660
   ggatatgaag ttggaggagg aaactacacc atgaacttgg cctcgcagaa ctggaacaac    720
   tacgatccaa ccgaggaaat ccccgctcct ttgggaactc ccgacttcgt gggacgtatc    780
   caaggtgtcc tgacacagac tacacgtcgc gacggctcta ctcgcggaca caaggccact    840
   gtgtcgacag gttccgtcca cttcacccct aagctgggct ctgtgcaatt cagcacagac    900
   acctcaggag gaggagatgc agaattccga catgactcag gaggaggagg aaacgatttc    960
   gagactggcc agaacaccag gttcactccc gtgggtgtcg ttcaagacgg cagcaccact   1020
   caccagaacg aaccccagca atgggtcctc cctgactact cgggcaggga ttcccacaac   1080
   gttcacttgg ctccggccgt ggctccaacc ttccctggag agcagttgct gttcttcaga   1140
   tccactatgc caggctgctc tggatacccg aacatgaacc tcgactgtct cttgcctcag   1200
   gagtgggtgc aacacttcta ccaggaatct gccccagctc aaagcgacgt cgctctgctc   1260
   cgtttcgtta accccgatac cggtcgcgtg ctcttcgagt gtaagttgca caagtctggt   1320
   tacgtcactg ttgcccacac aggccagcac gacctggtca tccctcccaa cggctacttc   1380
   cgcttcgata gctgggtcaa ccagttctac acactcgccc cgatgggaaa cggagccggt   1440
   cgtcgcagag ccttgtaa                                                 1458
<210> 87
   <211> 1080
   <212> DNA
   <213> artificial sequence
<400> 87
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatgactca 480
   ggatatgaag ttcatcatca aggaggagga aacgatttcg agactggcca gaacaccagg 540
   ttcactcccg tgggtgtcgt tcaagacggc ggaggaggag atgcagaatt ccgacatgac 600
   tcaggatatg aagttcatca tcaaggagga ggaagcacca ctcaccagaa cgaaccccag 660
   caatgggtcc tccctgacta ctcgggcagg gattcccaca acgttcactt ggctccggcc 720
   gtggctccaa ccttccctgg agagcagttg ctgttcttca gatccactat gccaggctgc 780
   tctggatacc cgaacatgaa cctcgactgt ctcttgcctc aggagtgggt gcaacacttc 840
   taccaggaat ctgccccagc tcaaagcgac gtcgctctgc tccgtttcgt taaccccgat 900
   accggtcgcg tgctcttcga gtgtaagttg cacaagtctg gttacgtcac tgttgcccac 960
   acaggccagc acgacctggt catccctccc aacggctact tccgcttcga tagctgggtc 1020
   aaccagttct acacactcgc cccgatggga aacggagccg gtcgtcgcag agccttgtaa 1080
<210> 88
   <211> 1872
   <212> DNA
   <213> artificial sequence
<400> 88
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatgactca 480
   ggatatgaag ttggaggagg agatgcagaa ttccgacatg actcaggata tgaagttgga 540
   ggaggagatg cagaattccg acatgactca ggatatgaag ttggaggagg agatgcagaa 600
   ttccgacatg actcaggata tgaagttgga ggaggagatg cagaattccg acatgactca 660
   ggatatgaag ttggaggagg agatgcagaa ttccgacatg actcaggata tgaagttgga 720
   ggaggagatg cagaattccg acatgactca ggatatgaag ttggaggagg agatgcagaa 780
   ttccgacatg actcaggata tgaagttgga ggaggagatg cagaattccg acatgactca 840
   ggatatgaag ttggaggagg agatgcagaa ttccgacatg actcaggata tgaagttgga 900
   ggaggaaacg atttcgagac tggccagaac accaggttca ctcccgtggg tgtcgttcaa 960
   gacggcggag gaggagatgc agaattccga catgactcag gatatgaagt tggaggagga 1020
   gatgcagaat tccgacatga ctcaggatat gaagttggag gaggagatgc agaattccga 1080
   catgactcag gatatgaagt tggaggagga gatgcagaat tccgacatga ctcaggatat 1140
   gaagttggag gaggagatgc agaattccga catgactcag gatatgaagt tggaggagga 1200
   gatgcagaat tccgacatga ctcaggatat gaagttggag gaggagatgc agaattccga 1260
   catgactcag gatatgaagt tggaggagga gatgcagaat tccgacatga ctcaggatat 1320
   gaagttggag gaggagatgc agaattccga catgactcag gatatgaagt tggaggagga 1380
   gatgcagaat tccgacatga ctcaggatat gaagttggag gaggaagcac cactcaccag 1440
   aacgaacccc agcaatgggt cctccctgac tactcgggca gggattccca caacgttcac 1500
   ttggctccgg ccgtggctcc aaccttccct ggagagcagt tgctgttctt cagatccact 1560
   atgccaggct gctctggata cccgaacatg aacctcgact gtctcttgcc tcaggagtgg 1620
   gtgcaacact tctaccagga atctgcccca gctcaaagcg acgtcgctct gctccgtttc 1680
   gttaaccccg ataccggtcg cgtgctcttc gagtgtaagt tgcacaagtc tggttacgtc 1740
   actgttgccc acacaggcca gcacgacctg gtcatccctc ccaacggcta cttccgcttc 1800
   gatagctggg tcaaccagtt ctacacactc gccccgatgg gaaacggagc cggtcgtcgc 1860
   agagccttgt aa 1872
<210> 89
   <211> 2052
   <212> DNA
   <213> artificial sequence
<400> 89
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatggagga 480
   ggagatgcag aattccgaca tggaggagga gatgcagaat tccgacatgg aggaggagat 540
   gcagaattcc gacatggagg aggagatgca gaattccgac atggaggagg agatgcagaa 600
   ttccgacatg gaggaggaga tgcagaattc cgacatggag gaggagatgc agaattccga 660
   catggaggag gagatgcaga attccgacat ggaggaggag atgcagaatt ccgacatgga 720
   ggaggagatg cagaattccg acatggagga ggagatgcag aattccgaca tggaggagga 780
   gatgcagaat tccgacatgg aggaggagat gcagaattcc gacatggagg aggagatgca 840
   gaattccgac atggaggagg agatgcagaa ttccgacatg gaggaggaga tgcagaattc 900
   cgacatggag gaggagatgc agaattccga catggaggag gagatgcaga attccgacat 960
   ggaggaggag atgcagaatt ccgacatgga ggaggaaacg atttcgagac tggccagaac 1020
   accaggttca ctcccgtggg tgtcgttcaa gacggcggag gaggagatgc agaattccga 1080
   catggaggag gagatgcaga attccgacat ggaggaggag atgcagaatt ccgacatgga 1140
   ggaggagatg cagaattccg acatggagga ggagatgcag aattccgaca tggaggagga 1200
   gatgcagaat tccgacatgg aggaggagat gcagaattcc gacatggagg aggagatgca 1260
   gaattccgac atggaggagg agatgcagaa ttccgacatg gaggaggaga tgcagaattc 1320
   cgacatggag gaggagatgc agaattccga catggaggag gagatgcaga attccgacat 1380
   ggaggaggag atgcagaatt ccgacatgga ggaggagatg cagaattccg acatggagga 1440
   ggagatgcag aattccgaca tggaggagga gatgcagaat tccgacatgg aggaggagat 1500
   gcagaattcc gacatggagg aggagatgca gaattccgac atggaggagg agatgcagaa 1560
   ttccgacatg gaggaggaga tgcagaattc cgacatggag gaggaagcac cactcaccag   1620
   aacgaacccc agcaatgggt cctccctgac tactcgggca gggattccca caacgttcac   1680
   ttggctccgg ccgtggctcc aaccttccct ggagagcagt tgctgttctt cagatccact   1740
   atgccaggct gctctggata cccgaacatg aacctcgact gtctcttgcc tcaggagtgg   1800
   gtgcaacact tctaccagga atctgcccca gctcaaagcg acgtcgctct gctccgtttc   1860
   gttaaccccg ataccggtcg cgtgctcttc gagtgtaagt tgcacaagtc tggttacgtc   1920
   actgttgccc acacaggcca gcacgacctg gtcatccctc ccaacggcta cttccgcttc   1980
   gatagctggg tcaaccagtt ctacacactc gccccgatgg gaaacggagc cggtcgtcgc   2040
   agagccttgt aa                                                       2052
<210> 90
   <211> 3852
   <212> DNA
   <213> artificial sequence
<400> 90
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga cacctcagga ggaggagatg cagaattccg acatgactca    480
   ggaggaggag gagatgcaga attccgacat gactcaggag gaggaggaga tgcagaattc    540
   cgacatgact caggaggagg aggagatgca gaattccgac atgactcagg aggaggagga    600
   gatgcagaat tccgacatga ctcaggagga ggaggagatg cagaattccg acatgactca    660
   ggaggaggag gagatgcaga attccgacat gactcaggag gaggaggaga tgcagaattc    720
   cgacatgact caggaggagg aggagatgca gaattccgac atgactcagg aggaggagga    780
   gatgcagaat tccgacatga ctcaggagga ggaggagatg cagaattccg acatgactca    840
   ggaggaggag gagatgcaga attccgacat gactcaggag gaggaggaga tgcagaattc    900
   cgacatgact caggaggagg aggagatgca gaattccgac atgactcagg aggaggagga    960
   gatgcagaat tccgacatga ctcaggagga ggaggagatg cagaattccg acatgactca   1020
   ggaggaggag gagatgcaga attccgacat gactcaggag gaggaggaga tgcagaattc   1080
   cgacatgact caggaggagg aggagatgca gaattccgac atgactcagg aggaggagga   1140
   gatgcagaat tccgacatga ctcaggagga ggaggagatg cagaattccg acatgactca 1200
   ggaggaggag gagatgcaga attccgacat gactcaggag gaggaggaga tgcagaattc 1260
   cgacatgact caggaggagg aggagatgca gaattccgac atgactcagg aggaggagga 1320
   gatgcagaat tccgacatga ctcaggagga ggaggagatg cagaattccg acatgactca 1380
   ggaggaggag gagatgcaga attccgacat gactcaggag gaggaggaga tgcagaattc 1440
   cgacatgact caggaggagg aggagatgca gaattccgac atgactcagg aggaggagga 1500
   gatgcagaat tccgacatga ctcaggagga ggaggagatg cagaattccg acatgactca 1560
   ggaggaggag gagatgcaga attccgacat gactcaggag gaggaggaga tgcagaattc 1620
   cgacatgact caggaggagg aggagatgca gaattccgac atgactcagg aggaggagga 1680
   gatgcagaat tccgacatga ctcaggagga ggaggagatg cagaattccg acatgactca 1740
   ggaggaggag gagatgcaga attccgacat gactcaggag gaggaggaga tgcagaattc 1800
   cgacatgact caggaggagg aggagatgca gaattccgac atgactcagg aggaggagga 1860
   gatgcagaat tccgacatga ctcaggagga ggaggaaacg atttcgagac tggccagaac 1920
   accaggttca ctcccgtggg tgtcgttcaa gacggcggag gaggagatgc agaattccga 1980
   catgactcag gaggaggagg agatgcagaa ttccgacatg actcaggagg aggaggagat 2040
   gcagaattcc gacatgactc aggaggagga ggagatgcag aattccgaca tgactcagga 2100
   ggaggaggag atgcagaatt ccgacatgac tcaggaggag gaggagatgc agaattccga 2160
   catgactcag gaggaggagg agatgcagaa ttccgacatg actcaggagg aggaggagat 2220
   gcagaattcc gacatgactc aggaggagga ggagatgcag aattccgaca tgactcagga 2280
   ggaggaggag atgcagaatt ccgacatgac tcaggaggag gaggagatgc agaattccga 2340
   catgactcag gaggaggagg agatgcagaa ttccgacatg actcaggagg aggaggagat 2400
   gcagaattcc gacatgactc aggaggagga ggagatgcag aattccgaca tgactcagga 2460
   ggaggaggag atgcagaatt ccgacatgac tcaggaggag gaggagatgc agaattccga 2520
   catgactcag gaggaggagg agatgcagaa ttccgacatg actcaggagg aggaggagat 2580
   gcagaattcc gacatgactc aggaggagga ggagatgcag aattccgaca tgactcagga 2640
   ggaggaggag atgcagaatt ccgacatgac tcaggaggag gaggagatgc agaattccga 2700
   catgactcag gaggaggagg agatgcagaa ttccgacatg actcaggagg aggaggagat 2760
   gcagaattcc gacatgactc aggaggagga ggagatgcag aattccgaca tgactcagga 2820
   ggaggaggag atgcagaatt ccgacatgac tcaggaggag gaggagatgc agaattccga 2880
   catgactcag gaggaggagg agatgcagaa ttccgacatg actcaggagg aggaggagat 2940
   gcagaattcc gacatgactc aggaggagga ggagatgcag aattccgaca tgactcagga 3000
   ggaggaggag atgcagaatt ccgacatgac tcaggaggag gaggagatgc agaattccga 3060
   catgactcag gaggaggagg agatgcagaa ttccgacatg actcaggagg aggaggagat   3120
   gcagaattcc gacatgactc aggaggagga ggagatgcag aattccgaca tgactcagga   3180
   ggaggaggag atgcagaatt ccgacatgac tcaggaggag gaggagatgc agaattccga   3240
   catgactcag gaggaggagg agatgcagaa ttccgacatg actcaggagg aggaggagat   3300
   gcagaattcc gacatgactc aggaggagga ggagatgcag aattccgaca tgactcagga   3360
   ggaggaggag atgcagaatt ccgacatgac tcaggaggag gaggaagcac cactcaccag   3420
   aacgaacccc agcaatgggt cctccctgac tactcgggca gggattccca caacgttcac   3480
   ttggctccgg ccgtggctcc aaccttccct ggagagcagt tgctgttctt cagatccact   3540
   atgccaggct gctctggata cccgaacatg aacctcgact gtctcttgcc tcaggagtgg   3600
   gtgcaacact tctaccagga atctgcccca gctcaaagcg acgtcgctct gctccgtttc   3660
   gttaaccccg ataccggtcg cgtgctcttc gagtgtaagt tgcacaagtc tggttacgtc   3720
   actgttgccc acacaggcca gcacgacctg gtcatccctc ccaacggcta cttccgcttc   3780
   gatagctggg tcaaccagtt ctacacactc gccccgatgg gaaacggagc cggtcgtcgc   3840
   agagccttgt aa                                                        3852
<210> 91
   <211> 1035
   <212> DNA
   <213> artificial sequence
<400> 91
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga caccggagga ggagatgcag aattccgaca tggaggagga 480
   tcaaacgatt tcgagactgg ccagaacacc aggttcactc ccgtgggtgt cgttcaagac 540
   ggaggaggag atgcagaatt ccgacatgac tcaggaggag gaggaggcag caccactcac 600
   cagaacgaac cccagcaatg ggtcctccct gactactcgg gcagggattc ccacaacgtt 660
   cacttggctc cggccgtggc tccaaccttc cctggagagc agttgctgtt cttcagatcc 720
   actatgccag gctgctctgg atacccgaac atgaacctcg actgtctctt gcctcaggag 780
   tgggtgcaac acttctacca ggaatctgcc ccagctcaaa gcgacgtcgc tctgctccgt 840
   ttcgttaacc ccgataccgg tcgcgtgctc ttcgagtgta agttgcacaa gtctggttac 900
   gtcactgttg cccacacagg ccagcacgac ctggtcatcc ctcccaacgg ctacttccgc 960
   ttcgatagct gggtcaacca gttctacaca ctcgccccga tgggaaacgg agccggtcgt 1020
   cgcagagcct tgtaa 1035
<210> 92
   <211> 1188
   <212> DNA
   <213> artificial sequence
<400> 92
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatgactca 480
   ggatatgaag ttggaggagg agatgcagaa ttccgacatg actcaggata tgaagttgga 540
   ggaggagatg cagaattccg acatgactca ggatatgaag ttggaggagg aaacgatttc 600
   gagactggcc agaacaccag gttcactccc gtgggtgtcg ttcaagacgg cggaggagga 660
   gatgcagaat tccgacatgg aggaggagat gcagaattcc gacatggagg aggagatgca 720
   gaattccgac atggaggagg aagcaccact caccagaacg aaccccagca atgggtcctc 780
   cctgactact cgggcaggga ttcccacaac gttcacttgg ctccggccgt ggctccaacc 840
   ttccctggag agcagttgct gttcttcaga tccactatgc caggctgctc tggatacccg 900
   aacatgaacc tcgactgtct cttgcctcag gagtgggtgc aacacttcta ccaggaatct 960
   gccccagctc aaagcgacgt cgctctgctc cgtttcgtta accccgatac cggtcgcgtg 1020
   ctcttcgagt gtaagttgca caagtctggt tacgtcactg ttgcccacac aggccagcac 1080
   gacctggtca tccctcccaa cggctacttc cgcttcgata gctgggtcaa ccagttctac 1140
   acactcgccc cgatgggaaa cggagccggt cgtcgcagag ccttgtaa 1188
<210> 93
   <211> 1872
   <212> DNA
   <213> artificial sequence
<400> 93
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga cacctcagga ggaggagatg cagaattccg acatgactca    480
   ggaggaggag gagatgcaga attccgacat gactcaggag gaggaggaga tgcagaattc    540
   cgacatgact caggaggagg aggagatgca gaattccgac atgactcagg aggaggagga    600
   gatgcagaat tccgacatga ctcaggagga ggaggagatg cagaattccg acatgactca    660
   ggaggaggag gagatgcaga attccgacat gactcaggag gaggaggaga tgcagaattc    720
   cgacatgact caggaggagg aggagatgca gaattccgac atgactcagg aggaggagga    780
   gatgcagaat tccgacatga ctcaggagga ggaggaaacg atttcgagac tggccagaac    840
   accaggttca ctcccgtggg tgtcgttcaa gacggcggag gaggagatgc agaattccga    900
   catgactcag gatatgaagt tcatcatcaa ggaggaggag atgcagaatt ccgacatgac    960
   tcaggatatg aagttcatca tcaaggagga ggagatgcag aattccgaca tgactcagga   1020
   tatgaagttc atcatcaagg aggaggagat gcagaattcc gacatgactc aggatatgaa   1080
   gttcatcatc aaggaggagg agatgcagaa ttccgacatg actcaggata tgaagttcat   1140
   catcaaggag gaggagatgc agaattccga catgactcag gatatgaagt tcatcatcaa   1200
   ggaggaggag atgcagaatt ccgacatgac tcaggatatg aagttcatca tcaaggagga   1260
   ggagatgcag aattccgaca tgactcagga tatgaagttc atcatcaagg aggaggagat   1320
   gcagaattcc gacatgactc aggatatgaa gttcatcatc aaggaggagg agatgcagaa   1380
   ttccgacatg actcaggata tgaagttcat catcaaggag gaggaagcac cactcaccag   1440
   aacgaacccc agcaatgggt cctccctgac tactcgggca gggattccca caacgttcac   1500
   ttggctccgg ccgtggctcc aaccttccct ggagagcagt tgctgttctt cagatccact   1560
   atgccaggct gctctggata cccgaacatg aacctcgact gtctcttgcc tcaggagtgg   1620
   gtgcaacact tctaccagga atctgcccca gctcaaagcg acgtcgctct gctccgtttc   1680
   gttaaccccg ataccggtcg cgtgctcttc gagtgtaagt tgcacaagtc tggttacgtc   1740
   actgttgccc acacaggcca gcacgacctg gtcatccctc ccaacggcta cttccgcttc   1800
   gatagctggg tcaaccagtt ctacacactc gccccgatgg gaaacggagc cggtcgtcgc   1860
   agagccttgt aa                                                       1872
<210> 94
   <211> 2592
   <212> DNA
   <213> artificial sequence
<400> 94
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatgactca 480
   ggatatgaag ttcatcatca aggaggagga gatgcagaat tccgacatga ctcaggatat 540
   gaagttcatc atcaaggagg aggagatgca gaattccgac atgactcagg atatgaagtt 600
   catcatcaag gaggaggaga tgcagaattc cgacatgact caggatatga agttcatcat 660
   caaggaggag gagatgcaga attccgacat gactcaggat atgaagttca tcatcaagga 720
   ggaggagatg cagaattccg acatgactca ggatatgaag ttcatcatca aggaggagga 780
   gatgcagaat tccgacatga ctcaggatat gaagttcatc atcaaggagg aggagatgca 840
   gaattccgac atgactcagg atatgaagtt catcatcaag gaggaggaga tgcagaattc 900
   cgacatgact caggatatga agttcatcat caaggaggag gagatgcaga attccgacat 960
   gactcaggat atgaagttca tcatcaagga ggaggagatg cagaattccg acatgactca 1020
   ggatatgaag ttcatcatca aggaggagga gatgcagaat tccgacatga ctcaggatat 1080
   gaagttcatc atcaaggagg aggagatgca gaattccgac atgactcagg atatgaagtt 1140
   catcatcaag gaggaggaga tgcagaattc cgacatgact caggatatga agttcatcat 1200
   caaggaggag gagatgcaga attccgacat gactcaggat atgaagttca tcatcaagga 1260
   ggaggagatg cagaattccg acatgactca ggatatgaag ttcatcatca aggaggagga 1320
   gatgcagaat tccgacatga ctcaggatat gaagttcatc atcaaggagg aggagatgca 1380
   gaattccgac atgactcagg atatgaagtt catcatcaag gaggaggaga tgcagaattc 1440
   cgacatgact caggatatga agttcatcat caaggaggag gagatgcaga attccgacat 1500
   gactcaggat atgaagttca tcatcaagga ggaggaaacg atttcgagac tggccagaac 1560
   accaggttca ctcccgtggg tgtcgttcaa gacggcggag gaggagatgc agaattccga 1620
   catggaggag gagatgcaga attccgacat ggaggaggag atgcagaatt ccgacatgga 1680
   ggaggagatg cagaattccg acatggagga ggagatgcag aattccgaca tggaggagga 1740
   gatgcagaat tccgacatgg aggaggagat gcagaattcc gacatggagg aggagatgca 1800
   gaattccgac atggaggagg agatgcagaa ttccgacatg gaggaggaga tgcagaattc 1860
   cgacatggag gaggagatgc agaattccga catggaggag gagatgcaga attccgacat 1920
   ggaggaggag atgcagaatt ccgacatgga ggaggagatg cagaattccg acatggagga 1980
   ggagatgcag aattccgaca tggaggagga gatgcagaat tccgacatgg aggaggagat 2040
   gcagaattcc gacatggagg aggagatgca gaattccgac atggaggagg agatgcagaa 2100
   ttccgacatg gaggaggaga tgcagaattc cgacatggag gaggaagcac cactcaccag 2160
   aacgaacccc agcaatgggt cctccctgac tactcgggca gggattccca caacgttcac 2220
   ttggctccgg ccgtggctcc aaccttccct ggagagcagt tgctgttctt cagatccact 2280
   atgccaggct gctctggata cccgaacatg aacctcgact gtctcttgcc tcaggagtgg 2340
   gtgcaacact tctaccagga atctgcccca gctcaaagcg acgtcgctct gctccgtttc 2400
   gttaaccccg ataccggtcg cgtgctcttc gagtgtaagt tgcacaagtc tggttacgtc 2460
   actgttgccc acacaggcca gcacgacctg gtcatccctc ccaacggcta cttccgcttc 2520
   gatagctggg tcaaccagtt ctacacactc gccccgatgg gaaacggagc cggtcgtcgc 2580
   agagccttgt aa 2592
<210> 95
   <211> 3402
   <212> **DNA**
   <213> artificial sequence
<400> 95
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcaaac ggaggaggag atgcagaatt ccgacatgac     480
   tcaggaggag gaggagatgc agaattccga catgactcag gaggaggagg agatgcagaa     540
   ttccgacatg actcaggagg aggaggagat gcagaattcc gacatgactc aggaggagga     600
   ggagatgcag aattccgaca tgactcagga ggaggaggag atgcagaatt ccgacatgac     660
   tcaggaggag gaggagatgc agaattccga catgactcag gaggaggagg agatgcagaa     720
   ttccgacatg actcaggagg aggaggagat gcagaattcc gacatgactc aggaggagga 780
   ggagatgcag aattccgaca tgactcagga ggaggaggag atgcagaatt ccgacatgac 840
   tcaggaggag gaggagatgc agaattccga catgactcag gaggaggagg agatgcagaa 900
   ttccgacatg actcaggagg aggaggagat gcagaattcc gacatgactc aggaggagga 960
   ggagatgcag aattccgaca tgactcagga ggaggaggag atgcagaatt ccgacatgac 1020
   tcaggaggag gaggagatgc agaattccga catgactcag gaggaggagg agatgcagaa 1080
   ttccgacatg actcaggagg aggaggagat gcagaattcc gacatgactc aggaggagga 1140
   ggagatgcag aattccgaca tgactcagga ggaggaggag atgcagaatt ccgacatgac 1200
   tcaggaggag gaggagatgc agaattccga catgactcag gaggaggagg agatgcagaa 1260
   ttccgacatg actcaggagg aggaggagat gcagaattcc gacatgactc aggaggagga 1320
   ggagatgcag aattccgaca tgactcagga ggaggaggag atgcagaatt ccgacatgac 1380
   tcaggaggag gaggagatgc agaattccga catgactcag gaggaggagg agatgcagaa 1440
   ttccgacatg actcaggagg aggaggagat gcagaattcc gacatgactc aggaggagga 1500
   ggagatgcag aattccgaca tgactcagga ggaggaggag atttcgagac tggccagaac 1560
   accaggttca ctcccgtggg tgtcgttcaa gacggcagcg gaggaggaga tgcagaattc 1620
   cgacatgact caggatatga agttggagga ggagatgcag aattccgaca tgactcagga 1680
   tatgaagttg gaggaggaga tgcagaattc cgacatgact caggatatga agttggagga 1740
   ggagatgcag aattccgaca tgactcagga tatgaagttg gaggaggaga tgcagaattc 1800
   cgacatgact caggatatga agttggagga ggagatgcag aattccgaca tgactcagga 1860
   tatgaagttg gaggaggaga tgcagaattc cgacatgact caggatatga agttggagga 1920
   ggagatgcag aattccgaca tgactcagga tatgaagttg gaggaggaga tgcagaattc 1980
   cgacatgact caggatatga agttggagga ggagatgcag aattccgaca tgactcagga 2040
   tatgaagttg gaggaggaga tgcagaattc cgacatgact caggatatga agttggagga 2100
   ggagatgcag aattccgaca tgactcagga tatgaagttg gaggaggaga tgcagaattc 2160
   cgacatgact caggatatga agttggagga ggagatgcag aattccgaca tgactcagga 2220
   tatgaagttg gaggaggaga tgcagaattc cgacatgact caggatatga agttggagga 2280
   ggagatgcag aattccgaca tgactcagga tatgaagttg gaggaggaga tgcagaattc 2340
   cgacatgact caggatatga agttggagga ggagatgcag aattccgaca tgactcagga 2400
   tatgaagttg gaggaggaga tgcagaattc cgacatgact caggatatga agttggagga 2460
   ggagatgcag aattccgaca tgactcagga tatgaagttg gaggaggaga tgcagaattc 2520
   cgacatgact caggatatga agttggagga ggagatgcag aattccgaca tgactcagga 2580
   tatgaagttg gaggaggaga tgcagaattc cgacatgact caggatatga agttggagga   2640
   ggagatgcag aattccgaca tgactcagga tatgaagttg gaggaggaga tgcagaattc   2700
   cgacatgact caggatatga agttggagga ggagatgcag aattccgaca tgactcagga   2760
   tatgaagttg gaggaggaga tgcagaattc cgacatgact caggatatga agttggagga   2820
   ggagatgcag aattccgaca tgactcagga tatgaagttg gaggaggaga tgcagaattc   2880
   cgacatgact caggatatga agttggagga ggagatgcag aattccgaca tgactcagga   2940
   tatgaagttg gaggaggaac cactcaccag aacgaacccc agcaatgggt cctccctgac   3000
   tactcgggca gggattccca caacgttcac ttggctccgg ccgtggctcc aaccttccct   3060
   ggagagcagt tgctgttctt cagatccact atgccaggct gctctggata cccgaacatg   3120
   aacctcgact gtctcttgcc tcaggagtgg gtgcaacact tctaccagga atctgcccca   3180
   gctcaaagcg acgtcgctct gctccgtttc gttaaccccg ataccggtcg cgtgctcttc   3240
   gagtgtaagt tgcacaagtc tggttacgtc actgttgccc acacaggcca gcacgacctg   3300
   gtcatccctc ccaacggcta cttccgcttc gatagctggg tcaaccagtt ctacacactc   3360
   gccccgatgg gaaacggagc cggtcgtcgc agagccttgt aa                      3402
<210> 96
   <211> 4932
   <212> **DNA**
   <213> artificial sequence
<400> 96
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaaactacac catgaacttg    240
   gcctcgcaga actggaacaa ctacgatcca accgaggaaa tccccgctcc tttgggaact    300
   cccgacttcg tgggacgtat ccaaggtgtc ctgacacaga ctacacgtcg cgacggctct    360
   actcgcggac acaaggccac tgtgtcgaca ggttccgtcc acttcacccc taagctgggc    420
   tctgtgcaat tcagcacaga cacctcaaac gatggaggag gagatgcaga attccgacat    480
   gactcaggat atgaagttca tcatcaagga ggaggagatg cagaattccg acatgactca    540
   ggatatgaag ttcatcatca aggaggagga gatgcagaat tccgacatga ctcaggatat    600
   gaagttcatc atcaaggagg aggagatgca gaattccgac atgactcagg atatgaagtt    660
   catcatcaag gaggaggaga tgcagaattc cgacatgact caggatatga agttcatcat    720
   caaggaggag gagatgcaga attccgacat gactcaggat atgaagttca tcatcaagga    780
   ggaggagatg cagaattccg acatgactca ggatatgaag ttcatcatca aggaggagga    840
   gatgcagaat tccgacatga ctcaggatat gaagttcatc atcaaggagg aggagatgca 900
   gaattccgac atgactcagg atatgaagtt catcatcaag gaggaggaga tgcagaattc 960
   cgacatgact caggatatga agttcatcat caaggaggag gagatgcaga attccgacat 1020
   gactcaggat atgaagttca tcatcaagga ggaggagatg cagaattccg acatgactca 1080
   ggatatgaag ttcatcatca aggaggagga gatgcagaat tccgacatga ctcaggatat 1140
   gaagttcatc atcaaggagg aggagatgca gaattccgac atgactcagg atatgaagtt 1200
   catcatcaag gaggaggaga tgcagaattc cgacatgact caggatatga agttcatcat 1260
   caaggaggag gagatgcaga attccgacat gactcaggat atgaagttca tcatcaagga 1320
   ggaggagatg cagaattccg acatgactca ggatatgaag ttcatcatca aggaggagga 1380
   gatgcagaat tccgacatga ctcaggatat gaagttcatc atcaaggagg aggagatgca 1440
   gaattccgac atgactcagg atatgaagtt catcatcaag gaggaggaga tgcagaattc 1500
   cgacatgact caggatatga agttcatcat caaggaggag gagatgcaga attccgacat 1560
   gactcaggat atgaagttca tcatcaagga ggaggagatg cagaattccg acatgactca 1620
   ggatatgaag ttcatcatca aggaggagga gatgcagaat tccgacatga ctcaggatat 1680
   gaagttcatc atcaaggagg aggagatgca gaattccgac atgactcagg atatgaagtt 1740
   catcatcaag gaggaggaga tgcagaattc cgacatgact caggatatga agttcatcat 1800
   caaggaggag gagatgcaga attccgacat gactcaggat atgaagttca tcatcaagga 1860
   ggaggagatg cagaattccg acatgactca ggatatgaag ttcatcatca aggaggagga 1920
   gatgcagaat tccgacatga ctcaggatat gaagttcatc atcaaggagg aggagatgca 1980
   gaattccgac atgactcagg atatgaagtt catcatcaag gaggaggaga tgcagaattc 2040
   cgacatgact caggatatga agttcatcat caaggaggag gagatgcaga attccgacat 2100
   gactcaggat atgaagttca tcatcaagga ggaggagatg cagaattccg acatgactca 2160
   ggatatgaag ttcatcatca aggaggagga gatgcagaat tccgacatga ctcaggatat 2220
   gaagttcatc atcaaggagg aggagatgca gaattccgac atgactcagg atatgaagtt 2280
   catcatcaag gaggaggaga tgcagaattc cgacatgact caggatatga agttcatcat 2340
   caaggaggag gagatgcaga attccgacat gactcaggat atgaagttca tcatcaagga 2400
   ggaggagatg cagaattccg acatgactca ggatatgaag ttcatcatca aggaggagga 2460
   gatgcagaat tccgacatga ctcaggatat gaagttcatc atcaaggagg aggagatgca 2520
   gaattccgac atgactcagg atatgaagtt catcatcaag gaggaggaga tgcagaattc 2580
   cgacatgact caggatatga agttcatcat caaggaggag gattcgagac tggccagaac 2640
   accaggttca ctcccgtggg tgtcgttcaa gacggcagca ccggaggagg agatgcagaa 2700
   ttccgacatg actcaggata tgaagttgga ggaggagatg cagaattccg acatgactca 2760
   ggatatgaag ttggaggagg agatgcagaa ttccgacatg actcaggata tgaagttgga 2820
   ggaggagatg cagaattccg acatgactca ggatatgaag ttggaggagg agatgcagaa 2880
   ttccgacatg actcaggata tgaagttgga ggaggagatg cagaattccg acatgactca 2940
   ggatatgaag ttggaggagg agatgcagaa ttccgacatg actcaggata tgaagttgga 3000
   ggaggagatg cagaattccg acatgactca ggatatgaag ttggaggagg agatgcagaa 3060
   ttccgacatg actcaggata tgaagttgga ggaggagatg cagaattccg acatgactca 3120
   ggatatgaag ttggaggagg agatgcagaa ttccgacatg actcaggata tgaagttgga 3180
   ggaggagatg cagaattccg acatgactca ggatatgaag ttggaggagg agatgcagaa 3240
   ttccgacatg actcaggata tgaagttgga ggaggagatg cagaattccg acatgactca 3300
   ggatatgaag ttggaggagg agatgcagaa ttccgacatg actcaggata tgaagttgga 3360
   ggaggagatg cagaattccg acatgactca ggatatgaag ttggaggagg agatgcagaa 3420
   ttccgacatg actcaggata tgaagttgga ggaggagatg cagaattccg acatgactca 3480
   ggatatgaag ttggaggagg agatgcagaa ttccgacatg actcaggata tgaagttgga 3540
   ggaggagatg cagaattccg acatgactca ggatatgaag ttggaggagg agatgcagaa 3600
   ttccgacatg actcaggata tgaagttgga ggaggagatg cagaattccg acatgactca 3660
   ggatatgaag ttggaggagg agatgcagaa ttccgacatg actcaggata tgaagttgga 3720
   ggaggagatg cagaattccg acatgactca ggatatgaag ttggaggagg agatgcagaa 3780
   ttccgacatg actcaggata tgaagttgga ggaggagatg cagaattccg acatgactca 3840
   ggatatgaag ttggaggagg agatgcagaa ttccgacatg actcaggata tgaagttgga 3900
   ggaggagatg cagaattccg acatgactca ggatatgaag ttggaggagg agatgcagaa 3960
   ttccgacatg actcaggata tgaagttgga ggaggagatg cagaattccg acatgactca 4020
   ggatatgaag ttggaggagg agatgcagaa ttccgacatg actcaggata tgaagttgga 4080
   ggaggagatg cagaattccg acatgactca ggatatgaag ttggaggagg agatgcagaa 4140
   ttccgacatg actcaggata tgaagttgga ggaggagatg cagaattccg acatgactca 4200
   ggatatgaag ttggaggagg agatgcagaa ttccgacatg actcaggata tgaagttgga 4260
   ggaggagatg cagaattccg acatgactca ggatatgaag ttggaggagg agatgcagaa 4320
   ttccgacatg actcaggata tgaagttgga ggaggagatg cagaattccg acatgactca 4380
   ggatatgaag ttggaggagg agatgcagaa ttccgacatg actcaggata tgaagttgga 4440
   ggaggagatg cagaattccg acatgactca ggatatgaag ttggaggagg aactcaccag 4500
   aacgaacccc agcaatgggt cctccctgac tactcgggca gggattccca caacgttcac 4560
   ttggctccgg ccgtggctcc aaccttccct ggagagcagt tgctgttctt cagatccact 4620
   atgccaggct gctctggata cccgaacatg aacctcgact gtctcttgcc tcaggagtgg 4680
   gtgcaacact tctaccagga atctgcccca gctcaaagcg acgtcgctct gctccgtttc 4740
   gttaaccccg ataccggtcg cgtgctcttc gagtgtaagt tgcacaagtc tggttacgtc 4800
   actgttgccc acacaggcca gcacgacctg gtcatccctc ccaacggcta cttccgcttc 4860
   gatagctggg tcaaccagtt ctacacactc gccccgatgg gaaacggagc cggtcgtcgc 4920
   agagccttgt aa 4932
<210> 97
   <211> 1269
   <212> DNA
   <213> artificial sequence
<400> 97
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagga ggaggagatg cagaattccg acatggagga     480
   ggaaacgatt tcgagactgg ccagaacacc aggttcactc ccgtgggtgt cgttcaagac     540
   ggcggaggag gagatgcaga attccgacat ggaggaggag atgcagaatt ccgacatgga     600
   ggaggagatg cagaattccg acatggagga ggagatgcag aattccgaca tggaggagga     660
   gatgcagaat tccgacatgg aggaggagat gcagaattcc gacatggagg aggagatgca     720
   gaattccgac atggaggagg agatgcagaa ttccgacatg gaggaggaga tgcagaattc     780
   cgacatggag gaggagatgc agaattccga catggaggag gaagcaccac tcaccagaac     840
   gaaccccagc aatgggtcct ccctgactac tcgggcaggg attcccacaa cgttcacttg     900
   gctccggccg tggctccaac cttccctgga gagcagttgc tgttcttcag atccactatg     960
   ccaggctgct ctggataccc gaacatgaac ctcgactgtc tcttgcctca ggagtgggtg    1020
   caacacttct accaggaatc tgccccagct caaagcgacg tcgctctgct ccgtttcgtt    1080
   aaccccgata ccggtcgcgt gctcttcgag tgtaagttgc acaagtctgg ttacgtcact    1140
   gttgcccaca caggccagca cgacctggtc atccctccca acggctactt ccgcttcgat    1200
   agctgggtca accagttcta cacactcgcc ccgatgggaa acggagccgg tcgtcgcaga    1260
   gccttgtaa                                                            1269
<210> 98
   <211> 2052
   <212> DNA
   <213> artificial sequence
<400> 98
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatgactca 480
   ggaggaggag gagatgcaga attccgacat gactcaggag gaggaggaga tgcagaattc 540
   cgacatgact caggaggagg aggagatgca gaattccgac atgactcagg aggaggagga 600
   gatgcagaat tccgacatga ctcaggagga ggaggagatg cagaattccg acatgactca 660
   ggaggaggag gagatgcaga attccgacat gactcaggag gaggaggaga tgcagaattc 720
   cgacatgact caggaggagg aggagatgca gaattccgac atgactcagg aggaggagga 780
   gatgcagaat tccgacatga ctcaggagga ggaggaaacg atttcgagac tggccagaac 840
   accaggttca ctcccgtggg tgtcgttcaa gacggcggag gaggagatgc agaattccga 900
   catgactcag gaggaggagg agatgcagaa ttccgacatg actcaggagg aggaggagat 960
   gcagaattcc gacatgactc aggaggagga ggagatgcag aattccgaca tgactcagga 1020
   ggaggaggag atgcagaatt ccgacatgac tcaggaggag gaggagatgc agaattccga 1080
   catgactcag gaggaggagg agatgcagaa ttccgacatg actcaggagg aggaggagat 1140
   gcagaattcc gacatgactc aggaggagga ggagatgcag aattccgaca tgactcagga 1200
   ggaggaggag atgcagaatt ccgacatgac tcaggaggag gaggagatgc agaattccga 1260
   catgactcag gaggaggagg agatgcagaa ttccgacatg actcaggagg aggaggagat 1320
   gcagaattcc gacatgactc aggaggagga ggagatgcag aattccgaca tgactcagga 1380
   ggaggaggag atgcagaatt ccgacatgac tcaggaggag gaggagatgc agaattccga 1440
   catgactcag gaggaggagg agatgcagaa ttccgacatg actcaggagg aggaggagat 1500
   gcagaattcc gacatgactc aggaggagga ggagatgcag aattccgaca tgactcagga 1560
   ggaggaggag atgcagaatt ccgacatgac tcaggaggag gaggaagcac cactcaccag 1620
   aacgaacccc agcaatgggt cctccctgac tactcgggca gggattccca caacgttcac 1680
   ttggctccgg ccgtggctcc aaccttccct ggagagcagt tgctgttctt cagatccact 1740
   atgccaggct gctctggata cccgaacatg aacctcgact gtctcttgcc tcaggagtgg 1800
   gtgcaacact tctaccagga atctgcccca gctcaaagcg acgtcgctct gctccgtttc 1860
   gttaaccccg ataccggtcg cgtgctcttc gagtgtaagt tgcacaagtc tggttacgtc 1920
   actgttgccc acacaggcca gcacgacctg gtcatccctc ccaacggcta cttccgcttc 1980
   gatagctggg tcaaccagtt ctacacactc gccccgatgg gaaacggagc cggtcgtcgc 2040
   agagccttgt aa 2052
<210> 99
   <211> 3672
   <212> DNA
   <213> artificial sequence
<400> 99
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagga ggaggagatg cagaattccg acatgactca     480
   ggatatgaag ttggaggagg agatgcagaa ttccgacatg actcaggata tgaagttgga     540
   ggaggagatg cagaattccg acatgactca ggatatgaag ttggaggagg agatgcagaa     600
   ttccgacatg actcaggata tgaagttgga ggaggagatg cagaattccg acatgactca     660
   ggatatgaag ttggaggagg agatgcagaa ttccgacatg actcaggata tgaagttgga     720
   ggaggagatg cagaattccg acatgactca ggatatgaag ttggaggagg agatgcagaa     780
   ttccgacatg actcaggata tgaagttgga ggaggagatg cagaattccg acatgactca     840
   ggatatgaag ttggaggagg agatgcagaa ttccgacatg actcaggata tgaagttgga     900
   ggaggagatg cagaattccg acatgactca ggatatgaag ttggaggagg agatgcagaa     960
   ttccgacatg actcaggata tgaagttgga ggaggagatg cagaattccg acatgactca    1020
   ggatatgaag ttggaggagg agatgcagaa ttccgacatg actcaggata tgaagttgga    1080
   ggaggagatg cagaattccg acatgactca ggatatgaag ttggaggagg agatgcagaa    1140
   ttccgacatg actcaggata tgaagttgga ggaggagatg cagaattccg acatgactca    1200
   ggatatgaag ttggaggagg agatgcagaa ttccgacatg actcaggata tgaagttgga    1260
   ggaggagatg cagaattccg acatgactca ggatatgaag ttggaggagg agatgcagaa 1320
   ttccgacatg actcaggata tgaagttgga ggaggaaacg atttcgagac tggccagaac 1380
   accaggttca ctcccgtggg tgtcgttcaa gacggcggag gaggagatgc agaattccga 1440
   catgactcag gatatgaagt tggaggagga gatgcagaat tccgacatga ctcaggatat 1500
   gaagttggag gaggagatgc agaattccga catgactcag gatatgaagt tggaggagga 1560
   gatgcagaat tccgacatga ctcaggatat gaagttggag gaggagatgc agaattccga 1620
   catgactcag gatatgaagt tggaggagga gatgcagaat tccgacatga ctcaggatat 1680
   gaagttggag gaggagatgc agaattccga catgactcag gatatgaagt tggaggagga 1740
   gatgcagaat tccgacatga ctcaggatat gaagttggag gaggagatgc agaattccga 1800
   catgactcag gatatgaagt tggaggagga gatgcagaat tccgacatga ctcaggatat 1860
   gaagttggag gaggagatgc agaattccga catgactcag gatatgaagt tggaggagga 1920
   gatgcagaat tccgacatga ctcaggatat gaagttggag gaggagatgc agaattccga 1980
   catgactcag gatatgaagt tggaggagga gatgcagaat tccgacatga ctcaggatat 2040
   gaagttggag gaggagatgc agaattccga catgactcag gatatgaagt tggaggagga 2100
   gatgcagaat tccgacatga ctcaggatat gaagttggag gaggagatgc agaattccga 2160
   catgactcag gatatgaagt tggaggagga gatgcagaat tccgacatga ctcaggatat 2220
   gaagttggag gaggagatgc agaattccga catgactcag gatatgaagt tggaggagga 2280
   gatgcagaat tccgacatga ctcaggatat gaagttggag gaggagatgc agaattccga 2340
   catgactcag gatatgaagt tggaggagga gatgcagaat tccgacatga ctcaggatat 2400
   gaagttggag gaggagatgc agaattccga catgactcag gatatgaagt tggaggagga 2460
   gatgcagaat tccgacatga ctcaggatat gaagttggag gaggagatgc agaattccga 2520
   catgactcag gatatgaagt tggaggagga gatgcagaat tccgacatga ctcaggatat 2580
   gaagttggag gaggagatgc agaattccga catgactcag gatatgaagt tggaggagga 2640
   gatgcagaat tccgacatga ctcaggatat gaagttggag gaggagatgc agaattccga 2700
   catgactcag gatatgaagt tggaggagga gatgcagaat tccgacatga ctcaggatat 2760
   gaagttggag gaggagatgc agaattccga catgactcag gatatgaagt tggaggagga 2820
   gatgcagaat tccgacatga ctcaggatat gaagttggag gaggagatgc agaattccga 2880
   catgactcag gatatgaagt tggaggagga gatgcagaat tccgacatga ctcaggatat 2940
   gaagttggag gaggagatgc agaattccga catgactcag gatatgaagt tggaggagga 3000
   gatgcagaat tccgacatga ctcaggatat gaagttggag gaggagatgc agaattccga 3060
   catgactcag gatatgaagt tggaggagga gatgcagaat tccgacatga ctcaggatat 3120
   gaagttggag gaggagatgc agaattccga catgactcag gatatgaagt tggaggagga 3180
   gatgcagaat tccgacatga ctcaggatat gaagttggag gaggaagcac cactcaccag 3240
   aacgaacccc agcaatgggt cctccctgac tactcgggca gggattccca caacgttcac 3300
   ttggctccgg ccgtggctcc aaccttccct ggagagcagt tgctgttctt cagatccact 3360
   atgccaggct gctctggata cccgaacatg aacctcgact gtctcttgcc tcaggagtgg 3420
   gtgcaacact tctaccagga atctgcccca gctcaaagcg acgtcgctct gctccgtttc 3480
   gttaaccccg ataccggtcg cgtgctcttc gagtgtaagt tgcacaagtc tggttacgtc 3540
   actgttgccc acacaggcca gcacgacctg gtcatccctc ccaacggcta cttccgcttc 3600
   gatagctggg tcaaccagtt ctacacactc gccccgatgg gaaacggagc cggtcgtcgc 3660
   agagccttgt aa 3672
<210> 100
   <211> 3672
   <212> DNA
   <213> artificial sequence
<400> 100
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatgactca 480
   ggatatgaag ttcatcatca aggaggagga gatgcagaat tccgacatga ctcaggatat 540
   gaagttcatc atcaaggagg aggagatgca gaattccgac atgactcagg atatgaagtt 600
   catcatcaag gaggaggaga tgcagaattc cgacatgact caggatatga agttcatcat 660
   caaggaggag gagatgcaga attccgacat gactcaggat atgaagttca tcatcaagga 720
   ggaggagatg cagaattccg acatgactca ggatatgaag ttcatcatca aggaggagga 780
   gatgcagaat tccgacatga ctcaggatat gaagttcatc atcaaggagg aggagatgca 840
   gaattccgac atgactcagg atatgaagtt catcatcaag gaggaggaga tgcagaattc 900
   cgacatgact caggatatga agttcatcat caaggaggag gagatgcaga attccgacat 960
   gactcaggat atgaagttca tcatcaagga ggaggagatg cagaattccg acatgactca 1020
   ggatatgaag ttcatcatca aggaggagga gatgcagaat tccgacatga ctcaggatat 1080
   gaagttcatc atcaaggagg aggagatgca gaattccgac atgactcagg atatgaagtt 1140
   catcatcaag gaggaggaga tgcagaattc cgacatgact caggatatga agttcatcat 1200
   caaggaggag gagatgcaga attccgacat gactcaggat atgaagttca tcatcaagga 1260
   ggaggagatg cagaattccg acatgactca ggatatgaag ttcatcatca aggaggagga 1320
   gatgcagaat tccgacatga ctcaggatat gaagttcatc atcaaggagg aggagatgca 1380
   gaattccgac atgactcagg atatgaagtt catcatcaag gaggaggaga tgcagaattc 1440
   cgacatgact caggatatga agttcatcat caaggaggag gagatgcaga attccgacat 1500
   gactcaggat atgaagttca tcatcaagga ggaggagatg cagaattccg acatgactca 1560
   ggatatgaag ttcatcatca aggaggagga gatgcagaat tccgacatga ctcaggatat 1620
   gaagttcatc atcaaggagg aggagatgca gaattccgac atgactcagg atatgaagtt 1680
   catcatcaag gaggaggaga tgcagaattc cgacatgact caggatatga agttcatcat 1740
   caaggaggag gagatgcaga attccgacat gactcaggat atgaagttca tcatcaagga 1800
   ggaggagatg cagaattccg acatgactca ggatatgaag ttcatcatca aggaggagga 1860
   gatgcagaat tccgacatga ctcaggatat gaagttcatc atcaaggagg aggagatgca 1920
   gaattccgac atgactcagg atatgaagtt catcatcaag gaggaggaga tgcagaattc 1980
   cgacatgact caggatatga agttcatcat caaggaggag gagatgcaga attccgacat 2040
   gactcaggat atgaagttca tcatcaagga ggaggagatg cagaattccg acatgactca 2100
   ggatatgaag ttcatcatca aggaggagga gatgcagaat tccgacatga ctcaggatat 2160
   gaagttcatc atcaaggagg aggagatgca gaattccgac atgactcagg atatgaagtt 2220
   catcatcaag gaggaggaga tgcagaattc cgacatgact caggatatga agttcatcat 2280
   caaggaggag gagatgcaga attccgacat gactcaggat atgaagttca tcatcaagga 2340
   ggaggagatg cagaattccg acatgactca ggatatgaag ttcatcatca aggaggagga 2400
   gatgcagaat tccgacatga ctcaggatat gaagttcatc atcaaggagg aggagatgca 2460
   gaattccgac atgactcagg atatgaagtt catcatcaag gaggaggaga tgcagaattc 2520
   cgacatgact caggatatga agttcatcat caaggaggag gagatgcaga attccgacat 2580
   gactcaggat atgaagttca tcatcaagga ggaggaaacg atttcgagac tggccagaac 2640
   accaggttca ctcccgtggg tgtcgttcaa gacggcggag gaggagatgc agaattccga 2700
   catgactcag gatatgaagt tcatcatcaa ggaggaggag atgcagaatt ccgacatgac 2760
   tcaggatatg aagttcatca tcaaggagga ggagatgcag aattccgaca tgactcagga 2820
   tatgaagttc atcatcaagg aggaggagat gcagaattcc gacatgactc aggatatgaa 2880
   gttcatcatc aaggaggagg agatgcagaa ttccgacatg actcaggata tgaagttcat 2940
   catcaaggag gaggagatgc agaattccga catgactcag gatatgaagt tcatcatcaa 3000
   ggaggaggag atgcagaatt ccgacatgac tcaggatatg aagttcatca tcaaggagga 3060
   ggagatgcag aattccgaca tgactcagga tatgaagttc atcatcaagg aggaggagat 3120
   gcagaattcc gacatgactc aggatatgaa gttcatcatc aaggaggagg agatgcagaa 3180
   ttccgacatg actcaggata tgaagttcat catcaaggag gaggaagcac cactcaccag 3240
   aacgaacccc agcaatgggt cctccctgac tactcgggca gggattccca caacgttcac 3300
   ttggctccgg ccgtggctcc aaccttccct ggagagcagt tgctgttctt cagatccact 3360
   atgccaggct gctctggata cccgaacatg aacctcgact gtctcttgcc tcaggagtgg 3420
   gtgcaacact tctaccagga atctgcccca gctcaaagcg acgtcgctct gctccgtttc 3480
   gttaaccccg ataccggtcg cgtgctcttc gagtgtaagt tgcacaagtc tggttacgtc 3540
   actgttgccc acacaggcca gcacgacctg gtcatccctc ccaacggcta cttccgcttc 3600
   gatagctggg tcaaccagtt ctacacactc gccccgatgg gaaacggagc cggtcgtcgc 3660
   agagccttgt aa 3672
<210> 101
   <211> 1359
   <212> DNA
   <213> artificial sequence
<400> 101
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga caccggagga ggagatgcag aattccgaca tggaggagga     480
   tcaaacgatt tcgagactgg ccagaacacc aggttcactc ccgtgggtgt cgttcaagac     540
   ggaggaggag atgcagaatt ccgacatgac tcaggaggag gaggagatgc agaattccga     600
   catgactcag gaggaggagg agatgcagaa ttccgacatg actcaggagg aggaggagat     660
   gcagaattcc gacatgactc aggaggagga ggagatgcag aattccgaca tgactcagga     720
   ggaggaggag atgcagaatt ccgacatgac tcaggaggag gaggagatgc agaattccga     780
   catgactcag gaggaggagg agatgcagaa ttccgacatg actcaggagg aggaggagat     840
   gcagaattcc gacatgactc aggaggagga ggagatgcag aattccgaca tgactcagga     900
   ggaggaggag gcagcaccac tcaccagaac gaaccccagc aatgggtcct ccctgactac     960
   tcgggcaggg attcccacaa cgttcacttg gctccggccg tggctccaac cttccctgga    1020
   gagcagttgc tgttcttcag atccactatg ccaggctgct ctggataccc gaacatgaac    1080
   ctcgactgtc tcttgcctca ggagtgggtg caacacttct accaggaatc tgccccagct    1140
   caaagcgacg tcgctctgct ccgtttcgtt aaccccgata ccggtcgcgt gctcttcgag    1200
   tgtaagttgc acaagtctgg ttacgtcact gttgcccaca caggccagca cgacctggtc    1260
   atccctccca acggctactt ccgcttcgat agctgggtca accagttcta cacactcgcc    1320
   ccgatgggaa acggagccgg tcgtcgcaga gccttgtaa                           1359
<210> 102
   <211> 2052
   <212> DNA
   <213> artificial sequence
<400> 102
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaaac ggaggaggag atgcagaatt ccgacatgac 480
   tcaggatatg aagttcatca tcaaggagga ggagatgcag aattccgaca tgactcagga 540
   tatgaagttc atcatcaagg aggaggagat gcagaattcc gacatgactc aggatatgaa 600
   gttcatcatc aaggaggagg agatgcagaa ttccgacatg actcaggata tgaagttcat 660
   catcaaggag gaggagatgc agaattccga catgactcag gatatgaagt tcatcatcaa 720
   ggaggaggag atgcagaatt ccgacatgac tcaggatatg aagttcatca tcaaggagga 780
   ggagatgcag aattccgaca tgactcagga tatgaagttc atcatcaagg aggaggagat 840
   gcagaattcc gacatgactc aggatatgaa gttcatcatc aaggaggagg agatgcagaa 900
   ttccgacatg actcaggata tgaagttcat catcaaggag gaggagatgc agaattccga 960
   catgactcag gatatgaagt tcatcatcaa ggaggaggag atttcgagac tggccagaac 1020
   accaggttca ctcccgtggg tgtcgttcaa gacggcagcg gaggaggaga tgcagaattc 1080
   cgacatggag gaggagatgc agaattccga catggaggag gagatgcaga attccgacat 1140
   ggaggaggag atgcagaatt ccgacatgga ggaggagatg cagaattccg acatggagga 1200
   ggagatgcag aattccgaca tggaggagga gatgcagaat tccgacatgg aggaggagat 1260
   gcagaattcc gacatggagg aggagatgca gaattccgac atggaggagg agatgcagaa   1320
   ttccgacatg gaggaggaga tgcagaattc cgacatggag gaggagatgc agaattccga   1380
   catggaggag gagatgcaga attccgacat ggaggaggag atgcagaatt ccgacatgga   1440
   ggaggagatg cagaattccg acatggagga ggagatgcag aattccgaca tggaggagga   1500
   gatgcagaat tccgacatgg aggaggagat gcagaattcc gacatggagg aggagatgca   1560
   gaattccgac atggaggagg agatgcagaa ttccgacatg gaggaggaac cactcaccag   1620
   aacgaacccc agcaatgggt cctccctgac tactcgggca gggattccca caacgttcac   1680
   ttggctccgg ccgtggctcc aaccttccct ggagagcagt tgctgttctt cagatccact   1740
   atgccaggct gctctggata cccgaacatg aacctcgact gtctcttgcc tcaggagtgg   1800
   gtgcaacact tctaccagga atctgcccca gctcaaagcg acgtcgctct gctccgtttc   1860
   gttaaccccg ataccggtcg cgtgctcttc gagtgtaagt tgcacaagtc tggttacgtc   1920
   actgttgccc acacaggcca gcacgacctg gtcatccctc ccaacggcta cttccgcttc   1980
   gatagctggg tcaaccagtt ctacacactc gccccgatgg gaaacggagc cggtcgtcgc   2040
   agagccttgt aa                                                       2052
<210> 103
   <211> 4032
   <212> DNA
   <213> artificial sequence
<400> 103
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagga ggaggagatg cagaattccg acatgactca     480
   ggatatgaag ttggaggagg agatgcagaa ttccgacatg actcaggata tgaagttgga     540
   ggaggagatg cagaattccg acatgactca ggatatgaag ttggaggagg agatgcagaa     600
   ttccgacatg actcaggata tgaagttgga ggaggagatg cagaattccg acatgactca     660
   ggatatgaag ttggaggagg agatgcagaa ttccgacatg actcaggata tgaagttgga     720
   ggaggagatg cagaattccg acatgactca ggatatgaag ttggaggagg agatgcagaa     780
   ttccgacatg actcaggata tgaagttgga ggaggagatg cagaattccg acatgactca     840
   ggatatgaag ttggaggagg agatgcagaa ttccgacatg actcaggata tgaagttgga 900
   ggaggagatg cagaattccg acatgactca ggatatgaag ttggaggagg agatgcagaa 960
   ttccgacatg actcaggata tgaagttgga ggaggagatg cagaattccg acatgactca 1020
   ggatatgaag ttggaggagg agatgcagaa ttccgacatg actcaggata tgaagttgga 1080
   ggaggagatg cagaattccg acatgactca ggatatgaag ttggaggagg agatgcagaa 1140
   ttccgacatg actcaggata tgaagttgga ggaggagatg cagaattccg acatgactca 1200
   ggatatgaag ttggaggagg agatgcagaa ttccgacatg actcaggata tgaagttgga 1260
   ggaggagatg cagaattccg acatgactca ggatatgaag ttggaggagg agatgcagaa 1320
   ttccgacatg actcaggata tgaagttgga ggaggaaacg atttcgagac tggccagaac 1380
   accaggttca ctcccgtggg tgtcgttcaa gacggcggag gaggagatgc agaattccga 1440
   catgactcag gatatgaagt tcatcatcaa ggaggaggag atgcagaatt ccgacatgac 1500
   tcaggatatg aagttcatca tcaaggagga ggagatgcag aattccgaca tgactcagga 1560
   tatgaagttc atcatcaagg aggaggagat gcagaattcc gacatgactc aggatatgaa 1620
   gttcatcatc aaggaggagg agatgcagaa ttccgacatg actcaggata tgaagttcat 1680
   catcaaggag gaggagatgc agaattccga catgactcag gatatgaagt tcatcatcaa 1740
   ggaggaggag atgcagaatt ccgacatgac tcaggatatg aagttcatca tcaaggagga 1800
   ggagatgcag aattccgaca tgactcagga tatgaagttc atcatcaagg aggaggagat 1860
   gcagaattcc gacatgactc aggatatgaa gttcatcatc aaggaggagg agatgcagaa 1920
   ttccgacatg actcaggata tgaagttcat catcaaggag gaggagatgc agaattccga 1980
   catgactcag gatatgaagt tcatcatcaa ggaggaggag atgcagaatt ccgacatgac 2040
   tcaggatatg aagttcatca tcaaggagga ggagatgcag aattccgaca tgactcagga 2100
   tatgaagttc atcatcaagg aggaggagat gcagaattcc gacatgactc aggatatgaa 2160
   gttcatcatc aaggaggagg agatgcagaa ttccgacatg actcaggata tgaagttcat 2220
   catcaaggag gaggagatgc agaattccga catgactcag gatatgaagt tcatcatcaa 2280
   ggaggaggag atgcagaatt ccgacatgac tcaggatatg aagttcatca tcaaggagga 2340
   ggagatgcag aattccgaca tgactcagga tatgaagttc atcatcaagg aggaggagat 2400
   gcagaattcc gacatgactc aggatatgaa gttcatcatc aaggaggagg agatgcagaa 2460
   ttccgacatg actcaggata tgaagttcat catcaaggag gaggagatgc agaattccga 2520
   catgactcag gatatgaagt tcatcatcaa ggaggaggag atgcagaatt ccgacatgac 2580
   tcaggatatg aagttcatca tcaaggagga ggagatgcag aattccgaca tgactcagga 2640
   tatgaagttc atcatcaagg aggaggagat gcagaattcc gacatgactc aggatatgaa 2700
   gttcatcatc aaggaggagg agatgcagaa ttccgacatg actcaggata tgaagttcat 2760
   catcaaggag gaggagatgc agaattccga catgactcag gatatgaagt tcatcatcaa 2820
   ggaggaggag atgcagaatt ccgacatgac tcaggatatg aagttcatca tcaaggagga 2880
   ggagatgcag aattccgaca tgactcagga tatgaagttc atcatcaagg aggaggagat 2940
   gcagaattcc gacatgactc aggatatgaa gttcatcatc aaggaggagg agatgcagaa 3000
   ttccgacatg actcaggata tgaagttcat catcaaggag gaggagatgc agaattccga 3060
   catgactcag gatatgaagt tcatcatcaa ggaggaggag atgcagaatt ccgacatgac 3120
   tcaggatatg aagttcatca tcaaggagga ggagatgcag aattccgaca tgactcagga 3180
   tatgaagttc atcatcaagg aggaggagat gcagaattcc gacatgactc aggatatgaa 3240
   gttcatcatc aaggaggagg agatgcagaa ttccgacatg actcaggata tgaagttcat 3300
   catcaaggag gaggagatgc agaattccga catgactcag gatatgaagt tcatcatcaa 3360
   ggaggaggag atgcagaatt ccgacatgac tcaggatatg aagttcatca tcaaggagga 3420
   ggagatgcag aattccgaca tgactcagga tatgaagttc atcatcaagg aggaggagat 3480
   gcagaattcc gacatgactc aggatatgaa gttcatcatc aaggaggagg agatgcagaa 3540
   ttccgacatg actcaggata tgaagttcat catcaaggag gaggaagcac cactcaccag 3600
   aacgaacccc agcaatgggt cctccctgac tactcgggca gggattccca caacgttcac 3660
   ttggctccgg ccgtggctcc aaccttccct ggagagcagt tgctgttctt cagatccact 3720
   atgccaggct gctctggata cccgaacatg aacctcgact gtctcttgcc tcaggagtgg 3780
   gtgcaacact tctaccagga atctgcccca gctcaaagcg acgtcgctct gctccgtttc 3840
   gttaaccccg ataccggtcg cgtgctcttc gagtgtaagt tgcacaagtc tggttacgtc 3900
   actgttgccc acacaggcca gcacgacctg gtcatccctc ccaacggcta cttccgcttc 3960
   gatagctggg tcaaccagtt ctacacactc gccccgatgg gaaacggagc cggtcgtcgc 4020
   agagccttgt aa 4032
<210> 104
   <211> 4032
   <212> DNA
   <213> artificial sequence
<400> 104
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc 420
   tctgtgcaat tcagcacaga cacctcaaac gatggaggag gagatgcaga attccgacat 480
   gactcaggat atgaagttca tcatcaagga ggaggagatg cagaattccg acatgactca 540
   ggatatgaag ttcatcatca aggaggagga gatgcagaat tccgacatga ctcaggatat 600
   gaagttcatc atcaaggagg aggagatgca gaattccgac atgactcagg atatgaagtt 660
   catcatcaag gaggaggaga tgcagaattc cgacatgact caggatatga agttcatcat 720
   caaggaggag gagatgcaga attccgacat gactcaggat atgaagttca tcatcaagga 780
   ggaggagatg cagaattccg acatgactca ggatatgaag ttcatcatca aggaggagga 840
   gatgcagaat tccgacatga ctcaggatat gaagttcatc atcaaggagg aggagatgca 900
   gaattccgac atgactcagg atatgaagtt catcatcaag gaggaggaga tgcagaattc 960
   cgacatgact caggatatga agttcatcat caaggaggag gagatgcaga attccgacat 1020
   gactcaggat atgaagttca tcatcaagga ggaggagatg cagaattccg acatgactca 1080
   ggatatgaag ttcatcatca aggaggagga gatgcagaat tccgacatga ctcaggatat 1140
   gaagttcatc atcaaggagg aggagatgca gaattccgac atgactcagg atatgaagtt 1200
   catcatcaag gaggaggaga tgcagaattc cgacatgact caggatatga agttcatcat 1260
   caaggaggag gagatgcaga attccgacat gactcaggat atgaagttca tcatcaagga 1320
   ggaggagatg cagaattccg acatgactca ggatatgaag ttcatcatca aggaggagga 1380
   gatgcagaat tccgacatga ctcaggatat gaagttcatc atcaaggagg aggagatgca 1440
   gaattccgac atgactcagg atatgaagtt catcatcaag gaggaggaga tgcagaattc 1500
   cgacatgact caggatatga agttcatcat caaggaggag gagatgcaga attccgacat 1560
   gactcaggat atgaagttca tcatcaagga ggaggagatg cagaattccg acatgactca 1620
   ggatatgaag ttcatcatca aggaggagga gatgcagaat tccgacatga ctcaggatat 1680
   gaagttcatc atcaaggagg aggagatgca gaattccgac atgactcagg atatgaagtt 1740
   catcatcaag gaggaggaga tgcagaattc cgacatgact caggatatga agttcatcat 1800
   caaggaggag gagatgcaga attccgacat gactcaggat atgaagttca tcatcaagga 1860
   ggaggagatg cagaattccg acatgactca ggatatgaag ttcatcatca aggaggagga 1920
   gatgcagaat tccgacatga ctcaggatat gaagttcatc atcaaggagg aggagatgca 1980
   gaattccgac atgactcagg atatgaagtt catcatcaag gaggaggaga tgcagaattc 2040
   cgacatgact caggatatga agttcatcat caaggaggag gagatgcaga attccgacat 2100
   gactcaggat atgaagttca tcatcaagga ggaggagatg cagaattccg acatgactca 2160
   ggatatgaag ttcatcatca aggaggagga gatgcagaat tccgacatga ctcaggatat 2220
   gaagttcatc atcaaggagg aggagatgca gaattccgac atgactcagg atatgaagtt 2280
   catcatcaag gaggaggaga tgcagaattc cgacatgact caggatatga agttcatcat 2340
   caaggaggag gagatgcaga attccgacat gactcaggat atgaagttca tcatcaagga 2400
   ggaggagatg cagaattccg acatgactca ggatatgaag ttcatcatca aggaggagga 2460
   gatgcagaat tccgacatga ctcaggatat gaagttcatc atcaaggagg aggagatgca 2520
   gaattccgac atgactcagg atatgaagtt catcatcaag gaggaggaga tgcagaattc 2580
   cgacatgact caggatatga agttcatcat caaggaggag gattcgagac tggccagaac 2640
   accaggttca ctcccgtggg tgtcgttcaa gacggcagca ccggaggagg agatgcagaa 2700
   ttccgacatg actcaggata tgaagttgga ggaggagatg cagaattccg acatgactca 2760
   ggatatgaag ttggaggagg agatgcagaa ttccgacatg actcaggata tgaagttgga 2820
   ggaggagatg cagaattccg acatgactca ggatatgaag ttggaggagg agatgcagaa 2880
   ttccgacatg actcaggata tgaagttgga ggaggagatg cagaattccg acatgactca 2940
   ggatatgaag ttggaggagg agatgcagaa ttccgacatg actcaggata tgaagttgga 3000
   ggaggagatg cagaattccg acatgactca ggatatgaag ttggaggagg agatgcagaa 3060
   ttccgacatg actcaggata tgaagttgga ggaggagatg cagaattccg acatgactca 3120
   ggatatgaag ttggaggagg agatgcagaa ttccgacatg actcaggata tgaagttgga 3180
   ggaggagatg cagaattccg acatgactca ggatatgaag ttggaggagg agatgcagaa 3240
   ttccgacatg actcaggata tgaagttgga ggaggagatg cagaattccg acatgactca 3300
   ggatatgaag ttggaggagg agatgcagaa ttccgacatg actcaggata tgaagttgga 3360
   ggaggagatg cagaattccg acatgactca ggatatgaag ttggaggagg agatgcagaa 3420
   ttccgacatg actcaggata tgaagttgga ggaggagatg cagaattccg acatgactca 3480
   ggatatgaag ttggaggagg agatgcagaa ttccgacatg actcaggata tgaagttgga 3540
   ggaggagatg cagaattccg acatgactca ggatatgaag ttggaggagg aactcaccag 3600
   aacgaacccc agcaatgggt cctccctgac tactcgggca gggattccca caacgttcac 3660
   ttggctccgg ccgtggctcc aaccttccct ggagagcagt tgctgttctt cagatccact 3720
   atgccaggct gctctggata cccgaacatg aacctcgact gtctcttgcc tcaggagtgg 3780
   gtgcaacact tctaccagga atctgcccca gctcaaagcg acgtcgctct gctccgtttc 3840
   gttaaccccg ataccggtcg cgtgctcttc gagtgtaagt tgcacaagtc tggttacgtc 3900
   actgttgccc acacaggcca gcacgacctg gtcatccctc ccaacggcta cttccgcttc 3960
   gatagctggg tcaaccagtt ctacacactc gccccgatgg gaaacggagc cggtcgtcgc 4020
   agagccttgt aa 4032
<210> 105
   <211> 2142
   <212> DNA
   <213> artificial sequence
<400> 105
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggagatg cagaattccg acatgactca 480
   ggatatgaag ttggaggagg agatgcagaa ttccgacatg actcaggata tgaagttgga 540
   ggaggagatg cagaattccg acatgactca ggatatgaag ttggaggagg agatgcagaa 600
   ttccgacatg actcaggata tgaagttgga ggaggagatg cagaattccg acatgactca 660
   ggatatgaag ttggaggagg agatgcagaa ttccgacatg actcaggata tgaagttgga 720
   ggaggagatg cagaattccg acatgactca ggatatgaag ttggaggagg agatgcagaa 780
   ttccgacatg actcaggata tgaagttgga ggaggagatg cagaattccg acatgactca 840
   ggatatgaag ttggaggagg agatgcagaa ttccgacatg actcaggata tgaagttgga 900
   ggaggagatg cagaattccg acatgactca ggatatgaag ttggaggagg agatgcagaa 960
   ttccgacatg actcaggata tgaagttgga ggaggagatg cagaattccg acatgactca 1020
   ggatatgaag ttggaggagg agatgcagaa ttccgacatg actcaggata tgaagttgga 1080
   ggaggagatg cagaattccg acatgactca ggatatgaag ttggaggagg agatgcagaa 1140
   ttccgacatg actcaggata tgaagttgga ggaggagatg cagaattccg acatgactca 1200
   ggatatgaag ttggaggagg agatgcagaa ttccgacatg actcaggata tgaagttgga 1260
   ggaggagatg cagaattccg acatgactca ggatatgaag ttggaggagg agatgcagaa 1320
   ttccgacatg actcaggata tgaagttgga ggaggaaacg atttcgagac tggccagaac 1380
   accaggttca ctcccgtggg tgtcgttcaa gacggcggag gaggagatgc agaattccga 1440
   catggaggag gagatgcaga attccgacat ggaggaggag atgcagaatt ccgacatgga 1500
   ggaggagatg cagaattccg acatggagga ggagatgcag aattccgaca tggaggagga 1560
   gatgcagaat tccgacatgg aggaggagat gcagaattcc gacatggagg aggagatgca 1620
   gaattccgac atggaggagg agatgcagaa ttccgacatg gaggaggaga tgcagaattc 1680
   cgacatggag gaggaagcac cactcaccag aacgaacccc agcaatgggt cctccctgac 1740
   tactcgggca gggattccca caacgttcac ttggctccgg ccgtggctcc aaccttccct   1800
   ggagagcagt tgctgttctt cagatccact atgccaggct gctctggata cccgaacatg   1860
   aacctcgact gtctcttgcc tcaggagtgg gtgcaacact tctaccagga atctgcccca   1920
   gctcaaagcg acgtcgctct gctccgtttc gttaaccccg ataccggtcg cgtgctcttc   1980
   gagtgtaagt tgcacaagtc tggttacgtc actgttgccc acacaggcca gcacgacctg   2040
   gtcatccctc ccaacggcta cttccgcttc gatagctggg tcaaccagtt ctacacactc   2100
   gccccgatgg gaaacggagc cggtcgtcgc agagccttgt aa                      2142
<210> 106
   <211> 1386
   <212> DNA
   <213> artificial sequence
<400> 106
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagga ggaggagatg cagaattccg acatgactca     480
   ggaggaggag gagatgcaga attccgacat gactcaggag gaggaggaga tgcagaattc     540
   cgacatgact caggaggagg aggagatgca gaattccgac atgactcagg aggaggagga     600
   gatgcagaat tccgacatga ctcaggagga ggaggagatg cagaattccg acatgactca     660
   ggaggaggag gagatgcaga attccgacat gactcaggag gaggaggaga tgcagaattc     720
   cgacatgact caggaggagg aggagatgca gaattccgac atgactcagg aggaggagga     780
   gatgcagaat tccgacatga ctcaggagga ggaggaaacg atttcgagac tggccagaac     840
   accaggttca ctcccgtggg tgtcgttcaa gacggcggag gaggagatgc agaattccga     900
   catgactcag gatatgaagt tcatcatcaa ggaggaggaa gcaccactca ccagaacgaa     960
   ccccagcaat gggtcctccc tgactactcg ggcagggatt cccacaacgt tcacttggct    1020
   ccggccgtgg ctccaacctt ccctggagag cagttgctgt tcttcagatc cactatgcca    1080
   ggctgctctg gatacccgaa catgaacctc gactgtctct tgcctcagga gtgggtgcaa    1140
   cacttctacc aggaatctgc cccagctcaa agcgacgtcg ctctgctccg tttcgttaac    1200
   cccgataccg gtcgcgtgct cttcgagtgt aagttgcaca agtctggtta cgtcactgtt    1260
   gcccacacag gccagcacga cctggtcatc cctcccaacg gctacttccg cttcgatagc    1320
   tgggtcaacc agttctacac actcgccccg atgggaaacg gagccggtcg tcgcagagcc    1380
   ttgtaa                                                               1386
<210> 107
   <211> 1080
   <212> DNA
   <213> artificial sequence
<400> 107
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tcatcatcaa ggaggaggaa cacaaaacta caccatgaac     300
   ttggcctcgc agaactggaa caactacgat ccaaccgagg aaatccccgc tcctttggga     360
   actcccgact tcgtgggacg tatccaaggt gtcctgacac agactacacg tcgcgacggc     420
   tctactcgcg gacacaaggc cactgtgtcg acaggttccg tccacttcac ccctaagctg     480
   ggctctgtgc aattcagcac agacacctca aacgatttcg agactggcca gaacaccagg     540
   ttcactcccg tgggtgtcgt tcaagacggc ggaggaggag atgcagaatt ccgacatgac     600
   tcaggatatg aagttcatca tcaaggagga ggaagcacca ctcaccagaa cgaaccccag     660
   caatgggtcc tccctgacta ctcgggcagg gattcccaca acgttcactt ggctccggcc     720
   gtggctccaa ccttccctgg agagcagttg ctgttcttca gatccactat gccaggctgc     780
   tctggatacc cgaacatgaa cctcgactgt ctcttgcctc aggagtgggt gcaacacttc     840
   taccaggaat ctgccccagc tcaaagcgac gtcgctctgc tccgtttcgt taaccccgat     900
   accggtcgcg tgctcttcga gtgtaagttg cacaagtctg gttacgtcac tgttgcccac     960
   acaggccagc acgacctggt catccctccc aacggctact tccgcttcga tagctgggtc    1020
   aaccagttct acacactcgc cccgatggga aacggagccg gtcgtcgcag agccttgtaa    1080
<210> 108
   <211> 1692
   <212> DNA
   <213> artificial sequence
<400> 108
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga    240
   catgactcag gaggaggagg agatgcagaa ttccgacatg actcaggagg aggaggagat    300
   gcagaattcc gacatgactc aggaggagga ggagatgcag aattccgaca tgactcagga    360
   ggaggaggag atgcagaatt ccgacatgac tcaggaggag gaggagatgc agaattccga    420
   catgactcag gaggaggagg agatgcagaa ttccgacatg actcaggagg aggaggagat    480
   gcagaattcc gacatgactc aggaggagga ggagatgcag aattccgaca tgactcagga    540
   ggaggaggag atgcagaatt ccgacatgac tcaggaggag gaggaacaca aaactacacc    600
   atgaacttgg cctcgcagaa ctggaacaac tacgatccaa ccgaggaaat ccccgctcct    660
   ttgggaactc ccgacttcgt gggacgtatc caaggtgtcc tgacacagac tacacgtcgc    720
   gacggctcta ctcgcggaca caaggccact gtgtcgacag gttccgtcca cttcacccct    780
   aagctgggct ctgtgcaatt cagcacagac acctcaaacg atttcgagac tggccagaac    840
   accaggttca ctcccgtggg tgtcgttcaa gacggcggag gaggagatgc agaattccga    900
   catgactcag gaggaggagg agatgcagaa ttccgacatg actcaggagg aggaggagat    960
   gcagaattcc gacatgactc aggaggagga ggagatgcag aattccgaca tgactcagga   1020
   ggaggaggag atgcagaatt ccgacatgac tcaggaggag gaggagatgc agaattccga   1080
   catgactcag gaggaggagg agatgcagaa ttccgacatg actcaggagg aggaggagat   1140
   gcagaattcc gacatgactc aggaggagga ggagatgcag aattccgaca tgactcagga   1200
   ggaggaggag atgcagaatt ccgacatgac tcaggaggag gaggaagcac cactcaccag   1260
   aacgaacccc agcaatgggt cctccctgac tactcgggca gggattccca caacgttcac   1320
   ttggctccgg ccgtggctcc aaccttccct ggagagcagt tgctgttctt cagatccact   1380
   atgccaggct gctctggata cccgaacatg aacctcgact gtctcttgcc tcaggagtgg   1440
   gtgcaacact tctaccagga atctgcccca gctcaaagcg acgtcgctct gctccgtttc   1500
   gttaaccccg ataccggtcg cgtgctcttc gagtgtaagt tgcacaagtc tggttacgtc   1560
   actgttgccc acacaggcca gcacgacctg gtcatccctc ccaacggcta cttccgcttc   1620
   gatagctggg tcaaccagtt ctacacactc gccccgatgg gaaacggagc cggtcgtcgc   1680
   agagccttgt aa                                                       1692
<210> 109
   <211> 2052
   <212> DNA
   <213> artificial sequence
<400> 109
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc    180
   tgtactttcc gcggcgacgt cacacacatc gctggaggag gaggagatgc agaattccga    240
   catggaggag gagatgcaga attccgacat ggaggaggag atgcagaatt ccgacatgga    300
   ggaggagatg cagaattccg acatggagga ggagatgcag aattccgaca tggaggagga    360
   gatgcagaat tccgacatgg aggaggagat gcagaattcc gacatggagg aggagatgca    420
   gaattccgac atggaggagg agatgcagaa ttccgacatg gaggaggaga tgcagaattc    480
   cgacatggag gaggagatgc agaattccga catggaggag gagatgcaga attccgacat    540
   ggaggaggag atgcagaatt ccgacatgga ggaggagatg cagaattccg acatggagga    600
   ggagatgcag aattccgaca tggaggagga gatgcagaat tccgacatgg aggaggagat    660
   gcagaattcc gacatggagg aggagatgca gaattccgac atggaggagg agatgcagaa    720
   ttccgacatg gaggaggaga tgcagaattc cgacatggag gaggaacaca aaactacacc    780
   atgaacttgg cctcgcagaa ctggaacaac tacgatccaa ccgaggaaat ccccgctcct    840
   ttgggaactc ccgacttcgt gggacgtatc caaggtgtcc tgacacagac tacacgtcgc    900
   gacggctcta ctcgcggaca caaggccact gtgtcgacag gttccgtcca cttcacccct    960
   aagctgggct ctgtgcaatt cagcacagac acctcaaacg atttcgagac tggccagaac   1020
   accaggttca ctcccgtggg tgtcgttcaa gacggcggag gaggagatgc agaattccga   1080
   catggaggag gagatgcaga attccgacat ggaggaggag atgcagaatt ccgacatgga   1140
   ggaggagatg cagaattccg acatggagga ggagatgcag aattccgaca tggaggagga   1200
   gatgcagaat tccgacatgg aggaggagat gcagaattcc gacatggagg aggagatgca   1260
   gaattccgac atggaggagg agatgcagaa ttccgacatg gaggaggaga tgcagaattc   1320
   cgacatggag gaggagatgc agaattccga catggaggag gagatgcaga attccgacat   1380
   ggaggaggag atgcagaatt ccgacatgga ggaggagatg cagaattccg acatggagga   1440
   ggagatgcag aattccgaca tggaggagga gatgcagaat tccgacatgg aggaggagat   1500
   gcagaattcc gacatggagg aggagatgca gaattccgac atggaggagg agatgcagaa   1560
   ttccgacatg gaggaggaga tgcagaattc cgacatggag gaggaagcac cactcaccag   1620
   aacgaacccc agcaatgggt cctccctgac tactcgggca gggattccca caacgttcac   1680
   ttggctccgg ccgtggctcc aaccttccct ggagagcagt tgctgttctt cagatccact   1740
   atgccaggct gctctggata cccgaacatg aacctcgact gtctcttgcc tcaggagtgg   1800
   gtgcaacact tctaccagga atctgcccca gctcaaagcg acgtcgctct gctccgtttc   1860
   gttaaccccg ataccggtcg cgtgctcttc gagtgtaagt tgcacaagtc tggttacgtc   1920
   actgttgccc acacaggcca gcacgacctg gtcatccctc ccaacggcta cttccgcttc   1980
   gatagctggg tcaaccagtt ctacacactc gccccgatgg gaaacggagc cggtcgtcgc   2040
   agagccttgt aa 2052
<210> 110
   <211> 4572
   <212> DNA
   <213> artificial sequence
<400> 110
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tggaggagga gatgcagaat tccgacatga ctcaggatat     300
   gaagttggag gaggagatgc agaattccga catgactcag gatatgaagt tggaggagga     360
   gatgcagaat tccgacatga ctcaggatat gaagttggag gaggagatgc agaattccga     420
   catgactcag gatatgaagt tggaggagga gatgcagaat tccgacatga ctcaggatat     480
   gaagttggag gaggagatgc agaattccga catgactcag gatatgaagt tggaggagga     540
   gatgcagaat tccgacatga ctcaggatat gaagttggag gaggagatgc agaattccga     600
   catgactcag gatatgaagt tggaggagga gatgcagaat tccgacatga ctcaggatat     660
   gaagttggag gaggagatgc agaattccga catgactcag gatatgaagt tggaggagga     720
   gatgcagaat tccgacatga ctcaggatat gaagttggag gaggagatgc agaattccga     780
   catgactcag gatatgaagt tggaggagga gatgcagaat tccgacatga ctcaggatat     840
   gaagttggag gaggagatgc agaattccga catgactcag gatatgaagt tggaggagga     900
   gatgcagaat tccgacatga ctcaggatat gaagttggag gaggagatgc agaattccga     960
   catgactcag gatatgaagt tggaggagga gatgcagaat tccgacatga ctcaggatat    1020
   gaagttggag gaggagatgc agaattccga catgactcag gatatgaagt tggaggagga    1080
   gatgcagaat tccgacatga ctcaggatat gaagttggag gaggagatgc agaattccga    1140
   catgactcag gatatgaagt tggaggagga gatgcagaat tccgacatga ctcaggatat    1200
   gaagttggag gaggagatgc agaattccga catgactcag gatatgaagt tggaggagga    1260
   gatgcagaat tccgacatga ctcaggatat gaagttggag gaggagatgc agaattccga    1320
   catgactcag gatatgaagt tggaggagga gatgcagaat tccgacatga ctcaggatat    1380
   gaagttggag gaggagatgc agaattccga catgactcag gatatgaagt tggaggagga    1440
   gatgcagaat tccgacatga ctcaggatat gaagttggag gaggagatgc agaattccga    1500
   catgactcag gatatgaagt tggaggagga gatgcagaat tccgacatga ctcaggatat    1560
   gaagttggag gaggagatgc agaattccga catgactcag gatatgaagt tggaggagga    1620
   gatgcagaat tccgacatga ctcaggatat gaagttggag gaggagatgc agaattccga 1680
   catgactcag gatatgaagt tggaggagga gatgcagaat tccgacatga ctcaggatat 1740
   gaagttggag gaggagatgc agaattccga catgactcag gatatgaagt tggaggagga 1800
   gatgcagaat tccgacatga ctcaggatat gaagttggag gaggagatgc agaattccga 1860
   catgactcag gatatgaagt tggaggagga gatgcagaat tccgacatga ctcaggatat 1920
   gaagttggag gaggagatgc agaattccga catgactcag gatatgaagt tggaggagga 1980
   gatgcagaat tccgacatga ctcaggatat gaagttggag gaggaacaca aaactacacc 2040
   atgaacttgg cctcgcagaa ctggaacaac tacgatccaa ccgaggaaat ccccgctcct 2100
   ttgggaactc ccgacttcgt gggacgtatc caaggtgtcc tgacacagac tacacgtcgc 2160
   gacggctcta ctcgcggaca caaggccact gtgtcgacag gttccgtcca cttcacccct 2220
   aagctgggct ctgtgcaatt cagcacagac acctcaaacg atttcgagac tggccagaac 2280
   accaggttca ctcccgtggg tgtcgttcaa gacggcggag gaggagatgc agaattccga 2340
   catgactcag gatatgaagt tggaggagga gatgcagaat tccgacatga ctcaggatat 2400
   gaagttggag gaggagatgc agaattccga catgactcag gatatgaagt tggaggagga 2460
   gatgcagaat tccgacatga ctcaggatat gaagttggag gaggagatgc agaattccga 2520
   catgactcag gatatgaagt tggaggagga gatgcagaat tccgacatga ctcaggatat 2580
   gaagttggag gaggagatgc agaattccga catgactcag gatatgaagt tggaggagga 2640
   gatgcagaat tccgacatga ctcaggatat gaagttggag gaggagatgc agaattccga 2700
   catgactcag gatatgaagt tggaggagga gatgcagaat tccgacatga ctcaggatat 2760
   gaagttggag gaggagatgc agaattccga catgactcag gatatgaagt tggaggagga 2820
   gatgcagaat tccgacatga ctcaggatat gaagttggag gaggagatgc agaattccga 2880
   catgactcag gatatgaagt tggaggagga gatgcagaat tccgacatga ctcaggatat 2940
   gaagttggag gaggagatgc agaattccga catgactcag gatatgaagt tggaggagga 3000
   gatgcagaat tccgacatga ctcaggatat gaagttggag gaggagatgc agaattccga 3060
   catgactcag gatatgaagt tggaggagga gatgcagaat tccgacatga ctcaggatat 3120
   gaagttggag gaggagatgc agaattccga catgactcag gatatgaagt tggaggagga 3180
   gatgcagaat tccgacatga ctcaggatat gaagttggag gaggagatgc agaattccga 3240
   catgactcag gatatgaagt tggaggagga gatgcagaat tccgacatga ctcaggatat 3300
   gaagttggag gaggagatgc agaattccga catgactcag gatatgaagt tggaggagga 3360
   gatgcagaat tccgacatga ctcaggatat gaagttggag gaggagatgc agaattccga 3420
   catgactcag gatatgaagt tggaggagga gatgcagaat tccgacatga ctcaggatat 3480
   gaagttggag gaggagatgc agaattccga catgactcag gatatgaagt tggaggagga 3540
   gatgcagaat tccgacatga ctcaggatat gaagttggag gaggagatgc agaattccga 3600
   catgactcag gatatgaagt tggaggagga gatgcagaat tccgacatga ctcaggatat 3660
   gaagttggag gaggagatgc agaattccga catgactcag gatatgaagt tggaggagga 3720
   gatgcagaat tccgacatga ctcaggatat gaagttggag gaggagatgc agaattccga 3780
   catgactcag gatatgaagt tggaggagga gatgcagaat tccgacatga ctcaggatat 3840
   gaagttggag gaggagatgc agaattccga catgactcag gatatgaagt tggaggagga 3900
   gatgcagaat tccgacatga ctcaggatat gaagttggag gaggagatgc agaattccga 3960
   catgactcag gatatgaagt tggaggagga gatgcagaat tccgacatga ctcaggatat 4020
   gaagttggag gaggagatgc agaattccga catgactcag gatatgaagt tggaggagga 4080
   gatgcagaat tccgacatga ctcaggatat gaagttggag gaggaagcac cactcaccag 4140
   aacgaacccc agcaatgggt cctccctgac tactcgggca gggattccca caacgttcac 4200
   ttggctccgg ccgtggctcc aaccttccct ggagagcagt tgctgttctt cagatccact 4260
   atgccaggct gctctggata cccgaacatg aacctcgact gtctcttgcc tcaggagtgg 4320
   gtgcaacact tctaccagga atctgcccca gctcaaagcg acgtcgctct gctccgtttc 4380
   gttaaccccg ataccggtcg cgtgctcttc gagtgtaagt tgcacaagtc tggttacgtc 4440
   actgttgccc acacaggcca gcacgacctg gtcatccctc ccaacggcta cttccgcttc 4500
   gatagctggg tcaaccagtt ctacacactc gccccgatgg gaaacggagc cggtcgtcgc 4560
   agagccttgt aa 4572
<210> 111
   <211> 1035
   <212> DNA
   <213> artificial sequence
<400> 111
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc ggaggaggag atgcagaatt ccgacatgga     240
   ggaggagctg gaacacaaaa ctacaccatg aacttggcct cgcagaactg gaacaactac     300
   gatccaaccg aggaaatccc cgctcctttg ggaactcccg acttcgtggg acgtatccaa     360
   ggtgtcctga cacagactac acgtcgcgac ggctctactc gcggacacaa ggccactgtg     420
   tcgacaggtt ccgtccactt cacccctaag ctgggctctg tgcaattcag cacagacacc     480
   tcaaacgatt tcgagactgg ccagaacacc aggttcactc ccgtgggtgt cgttcaagac     540
   ggaggaggag atgcagaatt ccgacatgac tcaggaggag gaggaggcag caccactcac     600
   cagaacgaac cccagcaatg ggtcctccct gactactcgg gcagggattc ccacaacgtt     660
   cacttggctc cggccgtggc tccaaccttc cctggagagc agttgctgtt cttcagatcc     720
   actatgccag gctgctctgg atacccgaac atgaacctcg actgtctctt gcctcaggag     780
   tgggtgcaac acttctacca ggaatctgcc ccagctcaaa gcgacgtcgc tctgctccgt     840
   ttcgttaacc ccgataccgg tcgcgtgctc ttcgagtgta agttgcacaa gtctggttac     900
   gtcactgttg cccacacagg ccagcacgac ctggtcatcc ctcccaacgg ctacttccgc     960
   ttcgatagct gggtcaacca gttctacaca ctcgccccga tgggaaacgg agccggtcgt    1020
   cgcagagcct tgtaa                                                     1035
<210> 112
   <211> 1188
   <212> DNA
   <213> artificial sequence
<400> 112
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catggaggag gagatgcaga attccgacat ggaggaggag atgcagaatt ccgacatgga     300
   ggaggaacac aaaactacac catgaacttg gcctcgcaga actggaacaa ctacgatcca     360
   accgaggaaa tccccgctcc tttgggaact cccgacttcg tgggacgtat ccaaggtgtc     420
   ctgacacaga ctacacgtcg cgacggctct actcgcggac acaaggccac tgtgtcgaca     480
   ggttccgtcc acttcacccc taagctgggc tctgtgcaat tcagcacaga cacctcaaac     540
   gatttcgaga ctggccagaa caccaggttc actcccgtgg gtgtcgttca agacggcgga     600
   ggaggagatg cagaattccg acatgactca ggatatgaag ttggaggagg agatgcagaa     660
   ttccgacatg actcaggata tgaagttgga ggaggagatg cagaattccg acatgactca     720
   ggatatgaag ttggaggagg aagcaccact caccagaacg aaccccagca atgggtcctc     780
   cctgactact cgggcaggga ttcccacaac gttcacttgg ctccggccgt ggctccaacc     840
   ttccctggag agcagttgct gttcttcaga tccactatgc caggctgctc tggatacccg     900
   aacatgaacc tcgactgtct cttgcctcag gagtgggtgc aacacttcta ccaggaatct     960
   gccccagctc aaagcgacgt cgctctgctc cgtttcgtta accccgatac cggtcgcgtg    1020
   ctcttcgagt gtaagttgca caagtctggt tacgtcactg ttgcccacac aggccagcac    1080
   gacctggtca tccctcccaa cggctacttc cgcttcgata gctgggtcaa ccagttctac    1140
   acactcgccc cgatgggaaa cggagccggt cgtcgcagag ccttgtaa                 1188
<210> 113
   <211> 1872
   <212> DNA
   <213> artificial sequence
<400> 113
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaggag gagatgcaga attccgacat    240
   gactcaggag gaggaggaga tgcagaattc cgacatgact caggaggagg aggagatgca    300
   gaattccgac atgactcagg aggaggagga gatgcagaat tccgacatga ctcaggagga    360
   ggaggagatg cagaattccg acatgactca ggaggaggag gagatgcaga attccgacat    420
   gactcaggag gaggaggaga tgcagaattc cgacatgact caggaggagg aggagatgca    480
   gaattccgac atgactcagg aggaggagga gatgcagaat tccgacatga ctcaggagga    540
   ggaggagatg cagaattccg acatgactca ggaggaggag gaggaacaca aaactacacc    600
   atgaacttgg cctcgcagaa ctggaacaac tacgatccaa ccgaggaaat ccccgctcct    660
   ttgggaactc ccgacttcgt gggacgtatc caaggtgtcc tgacacagac tacacgtcgc    720
   gacggctcta ctcgcggaca caaggccact gtgtcgacag gttccgtcca cttcacccct    780
   aagctgggct ctgtgcaatt cagcacagac acctcaaacg atttcgagac tggccagaac    840
   accaggttca ctcccgtggg tgtcgttcaa gacggcggag gaggagatgc agaattccga    900
   catgactcag gatatgaagt tcatcatcaa ggaggaggag atgcagaatt ccgacatgac    960
   tcaggatatg aagttcatca tcaaggagga ggagatgcag aattccgaca tgactcagga   1020
   tatgaagttc atcatcaagg aggaggagat gcagaattcc gacatgactc aggatatgaa   1080
   gttcatcatc aaggaggagg agatgcagaa ttccgacatg actcaggata tgaagttcat   1140
   catcaaggag gaggagatgc agaattccga catgactcag gatatgaagt tcatcatcaa   1200
   ggaggaggag atgcagaatt ccgacatgac tcaggatatg aagttcatca tcaaggagga   1260
   ggagatgcag aattccgaca tgactcagga tatgaagttc atcatcaagg aggaggagat   1320
   gcagaattcc gacatgactc aggatatgaa gttcatcatc aaggaggagg agatgcagaa   1380
   ttccgacatg actcaggata tgaagttcat catcaaggag gaggaagcac cactcaccag   1440
   aacgaacccc agcaatgggt cctccctgac tactcgggca gggattccca caacgttcac   1500
   ttggctccgg ccgtggctcc aaccttccct ggagagcagt tgctgttctt cagatccact   1560
   atgccaggct gctctggata cccgaacatg aacctcgact gtctcttgcc tcaggagtgg   1620
   gtgcaacact tctaccagga atctgcccca gctcaaagcg acgtcgctct gctccgtttc 1680
   gttaaccccg ataccggtcg cgtgctcttc gagtgtaagt tgcacaagtc tggttacgtc 1740
   actgttgccc acacaggcca gcacgacctg gtcatccctc ccaacggcta cttccgcttc 1800
   gatagctggg tcaaccagtt ctacacactc gccccgatgg gaaacggagc cggtcgtcgc 1860
   agagccttgt aa 1872
<210> 114
   <211> 2592
   <212> DNA
   <213> artificial sequence
<400> 114
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaggag gaggagatgc agaattccga    240
   catgactcag gatatgaagt tggaggagga gatgcagaat tccgacatga ctcaggatat    300
   gaagttggag gaggagatgc agaattccga catgactcag gatatgaagt tggaggagga    360
   gatgcagaat tccgacatga ctcaggatat gaagttggag gaggagatgc agaattccga    420
   catgactcag gatatgaagt tggaggagga gatgcagaat tccgacatga ctcaggatat    480
   gaagttggag gaggagatgc agaattccga catgactcag gatatgaagt tggaggagga    540
   gatgcagaat tccgacatga ctcaggatat gaagttggag gaggagatgc agaattccga    600
   catgactcag gatatgaagt tggaggagga gatgcagaat tccgacatga ctcaggatat    660
   gaagttggag gaggagatgc agaattccga catgactcag gatatgaagt tggaggagga    720
   gatgcagaat tccgacatga ctcaggatat gaagttggag gaggagatgc agaattccga    780
   catgactcag gatatgaagt tggaggagga gatgcagaat tccgacatga ctcaggatat    840
   gaagttggag gaggagatgc agaattccga catgactcag gatatgaagt tggaggagga    900
   gatgcagaat tccgacatga ctcaggatat gaagttggag gaggagatgc agaattccga    960
   catgactcag gatatgaagt tggaggagga gatgcagaat tccgacatga ctcaggatat   1020
   gaagttggag gaggagatgc agaattccga catgactcag gatatgaagt tggaggagga   1080
   gatgcagaat tccgacatga ctcaggatat gaagttggag gaggaacaca aaactacacc   1140
   atgaacttgg cctcgcagaa ctggaacaac tacgatccaa ccgaggaaat ccccgctcct   1200
   ttgggaactc ccgacttcgt gggacgtatc caaggtgtcc tgacacagac tacacgtcgc   1260
   gacggctcta ctcgcggaca caaggccact gtgtcgacag gttccgtcca cttcacccct   1320
   aagctgggct ctgtgcaatt cagcacagac acctcaaacg atttcgagac tggccagaac   1380
   accaggttca ctcccgtggg tgtcgttcaa gacggcagcg gaggaggaga tgcagaattc   1440
   cgacatgact caggaggagg aggagatgca gaattccgac atgactcagg aggaggagga   1500
   gatgcagaat tccgacatga ctcaggagga ggaggagatg cagaattccg acatgactca   1560
   ggaggaggag gagatgcaga attccgacat gactcaggag gaggaggaga tgcagaattc   1620
   cgacatgact caggaggagg aggagatgca gaattccgac atgactcagg aggaggagga   1680
   gatgcagaat tccgacatga ctcaggagga ggaggagatg cagaattccg acatgactca   1740
   ggaggaggag gagatgcaga attccgacat gactcaggag gaggaggaga tgcagaattc   1800
   cgacatgact caggaggagg aggagatgca gaattccgac atgactcagg aggaggagga   1860
   gatgcagaat tccgacatga ctcaggagga ggaggagatg cagaattccg acatgactca   1920
   ggaggaggag gagatgcaga attccgacat gactcaggag gaggaggaga tgcagaattc   1980
   cgacatgact caggaggagg aggagatgca gaattccgac atgactcagg aggaggagga   2040
   gatgcagaat tccgacatga ctcaggagga ggaggagatg cagaattccg acatgactca   2100
   ggaggaggag gagatgcaga attccgacat gactcaggag gaggaggaac cactcaccag   2160
   aacgaacccc agcaatgggt cctccctgac tactcgggca gggattccca caacgttcac   2220
   ttggctccgg ccgtggctcc aaccttccct ggagagcagt tgctgttctt cagatccact   2280
   atgccaggct gctctggata cccgaacatg aacctcgact gtctcttgcc tcaggagtgg   2340
   gtgcaacact tctaccagga atctgcccca gctcaaagcg acgtcgctct gctccgtttc   2400
   gttaaccccg ataccggtcg cgtgctcttc gagtgtaagt tgcacaagtc tggttacgtc   2460
   actgttgccc acacaggcca gcacgacctg gtcatccctc ccaacggcta cttccgcttc   2520
   gatagctggg tcaaccagtt ctacacactc gccccgatgg gaaacggagc cggtcgtcgc   2580
   agagccttgt aa                                                        2592
<210> 115
   <211> 3942
   <212> DNA
   <213> artificial sequence
<400> 115
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaacac aaggaggagg agatgcagaa    240
   ttccgacatg actcaggata tgaagttgga ggaggagatg cagaattccg acatgactca    300
   ggatatgaag ttggaggagg agatgcagaa ttccgacatg actcaggata tgaagttgga    360
   ggaggagatg cagaattccg acatgactca ggatatgaag ttggaggagg agatgcagaa    420
   ttccgacatg actcaggata tgaagttgga ggaggagatg cagaattccg acatgactca 480
   ggatatgaag ttggaggagg agatgcagaa ttccgacatg actcaggata tgaagttgga 540
   ggaggagatg cagaattccg acatgactca ggatatgaag ttggaggagg agatgcagaa 600
   ttccgacatg actcaggata tgaagttgga ggaggagatg cagaattccg acatgactca 660
   ggatatgaag ttggaggagg agatgcagaa ttccgacatg actcaggata tgaagttgga 720
   ggaggagatg cagaattccg acatgactca ggatatgaag ttggaggagg agatgcagaa 780
   ttccgacatg actcaggata tgaagttgga ggaggagatg cagaattccg acatgactca 840
   ggatatgaag ttggaggagg agatgcagaa ttccgacatg actcaggata tgaagttgga 900
   ggaggagatg cagaattccg acatgactca ggatatgaag ttggaggagg agatgcagaa 960
   ttccgacatg actcaggata tgaagttgga ggaggagatg cagaattccg acatgactca 1020
   ggatatgaag ttggaggagg agatgcagaa ttccgacatg actcaggata tgaagttgga 1080
   ggaggagatg cagaattccg acatgactca ggatatgaag ttggaggagg agatgcagaa 1140
   ttccgacatg actcaggata tgaagttgga ggaggagatg cagaattccg acatgactca 1200
   ggatatgaag ttggaggagg agatgcagaa ttccgacatg actcaggata tgaagttgga 1260
   ggaggagatg cagaattccg acatgactca ggatatgaag ttggaggagg agatgcagaa 1320
   ttccgacatg actcaggata tgaagttgga ggaggagatg cagaattccg acatgactca 1380
   ggatatgaag ttggaggagg agatgcagaa ttccgacatg actcaggata tgaagttgga 1440
   ggaggagatg cagaattccg acatgactca ggatatgaag ttggaggagg agatgcagaa 1500
   ttccgacatg actcaggata tgaagttgga ggaggagatg cagaattccg acatgactca 1560
   ggatatgaag ttggaggagg aaactacacc atgaacttgg cctcgcagaa ctggaacaac 1620
   tacgatccaa ccgaggaaat ccccgctcct ttgggaactc ccgacttcgt gggacgtatc 1680
   caaggtgtcc tgacacagac tacacgtcgc gacggctcta ctcgcggaca caaggccact 1740
   gtgtcgacag gttccgtcca cttcacccct aagctgggct ctgtgcaatt cagcacagac 1800
   acctcaaacg atttcgagac tggccagaac accaggttca ctcccgtggg tgtcgttcaa 1860
   gacggcagca ccggaggagg agatgcagaa ttccgacatg actcaggata tgaagttcat 1920
   catcaaggag gaggagatgc agaattccga catgactcag gatatgaagt tcatcatcaa 1980
   ggaggaggag atgcagaatt ccgacatgac tcaggatatg aagttcatca tcaaggagga 2040
   ggagatgcag aattccgaca tgactcagga tatgaagttc atcatcaagg aggaggagat 2100
   gcagaattcc gacatgactc aggatatgaa gttcatcatc aaggaggagg agatgcagaa 2160
   ttccgacatg actcaggata tgaagttcat catcaaggag gaggagatgc agaattccga 2220
   catgactcag gatatgaagt tcatcatcaa ggaggaggag atgcagaatt ccgacatgac 2280
   tcaggatatg aagttcatca tcaaggagga ggagatgcag aattccgaca tgactcagga 2340
   tatgaagttc atcatcaagg aggaggagat gcagaattcc gacatgactc aggatatgaa 2400
   gttcatcatc aaggaggagg agatgcagaa ttccgacatg actcaggata tgaagttcat 2460
   catcaaggag gaggagatgc agaattccga catgactcag gatatgaagt tcatcatcaa 2520
   ggaggaggag atgcagaatt ccgacatgac tcaggatatg aagttcatca tcaaggagga 2580
   ggagatgcag aattccgaca tgactcagga tatgaagttc atcatcaagg aggaggagat 2640
   gcagaattcc gacatgactc aggatatgaa gttcatcatc aaggaggagg agatgcagaa 2700
   ttccgacatg actcaggata tgaagttcat catcaaggag gaggagatgc agaattccga 2760
   catgactcag gatatgaagt tcatcatcaa ggaggaggag atgcagaatt ccgacatgac 2820
   tcaggatatg aagttcatca tcaaggagga ggagatgcag aattccgaca tgactcagga 2880
   tatgaagttc atcatcaagg aggaggagat gcagaattcc gacatgactc aggatatgaa 2940
   gttcatcatc aaggaggagg agatgcagaa ttccgacatg actcaggata tgaagttcat 3000
   catcaaggag gaggagatgc agaattccga catgactcag gatatgaagt tcatcatcaa 3060
   ggaggaggag atgcagaatt ccgacatgac tcaggatatg aagttcatca tcaaggagga 3120
   ggagatgcag aattccgaca tgactcagga tatgaagttc atcatcaagg aggaggagat 3180
   gcagaattcc gacatgactc aggatatgaa gttcatcatc aaggaggagg agatgcagaa 3240
   ttccgacatg actcaggata tgaagttcat catcaaggag gaggagatgc agaattccga 3300
   catgactcag gatatgaagt tcatcatcaa ggaggaggag atgcagaatt ccgacatgac 3360
   tcaggatatg aagttcatca tcaaggagga ggagatgcag aattccgaca tgactcagga 3420
   tatgaagttc atcatcaagg aggaggagat gcagaattcc gacatgactc aggatatgaa 3480
   gttcatcatc aaggaggagg aactcaccag aacgaacccc agcaatgggt cctccctgac 3540
   tactcgggca gggattccca caacgttcac ttggctccgg ccgtggctcc aaccttccct 3600
   ggagagcagt tgctgttctt cagatccact atgccaggct gctctggata cccgaacatg 3660
   aacctcgact gtctcttgcc tcaggagtgg gtgcaacact tctaccagga atctgcccca 3720
   gctcaaagcg acgtcgctct gctccgtttc gttaaccccg ataccggtcg cgtgctcttc 3780
   gagtgtaagt tgcacaagtc tggttacgtc actgttgccc acacaggcca gcacgacctg 3840
   gtcatccctc ccaacggcta cttccgcttc gatagctggg tcaaccagtt ctacacactc 3900
   gccccgatgg gaaacggagc cggtcgtcgc agagccttgt aa 3942
<210> 116
   <211> 4212
   <212> DNA
   <213> artificial sequence
<400> 116
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacag gaggaggaga tgcagaattc 240
   cgacatgact caggatatga agttcatcat caaggaggag gagatgcaga attccgacat 300
   gactcaggat atgaagttca tcatcaagga ggaggagatg cagaattccg acatgactca 360
   ggatatgaag ttcatcatca aggaggagga gatgcagaat tccgacatga ctcaggatat 420
   gaagttcatc atcaaggagg aggagatgca gaattccgac atgactcagg atatgaagtt 480
   catcatcaag gaggaggaga tgcagaattc cgacatgact caggatatga agttcatcat 540
   caaggaggag gagatgcaga attccgacat gactcaggat atgaagttca tcatcaagga 600
   ggaggagatg cagaattccg acatgactca ggatatgaag ttcatcatca aggaggagga 660
   gatgcagaat tccgacatga ctcaggatat gaagttcatc atcaaggagg aggagatgca 720
   gaattccgac atgactcagg atatgaagtt catcatcaag gaggaggaga tgcagaattc 780
   cgacatgact caggatatga agttcatcat caaggaggag gagatgcaga attccgacat 840
   gactcaggat atgaagttca tcatcaagga ggaggagatg cagaattccg acatgactca 900
   ggatatgaag ttcatcatca aggaggagga gatgcagaat tccgacatga ctcaggatat 960
   gaagttcatc atcaaggagg aggagatgca gaattccgac atgactcagg atatgaagtt 1020
   catcatcaag gaggaggaga tgcagaattc cgacatgact caggatatga agttcatcat 1080
   caaggaggag gagatgcaga attccgacat gactcaggat atgaagttca tcatcaagga 1140
   ggaggagatg cagaattccg acatgactca ggatatgaag ttcatcatca aggaggagga 1200
   gatgcagaat tccgacatga ctcaggatat gaagttcatc atcaaggagg aggagatgca 1260
   gaattccgac atgactcagg atatgaagtt catcatcaag gaggaggaga tgcagaattc 1320
   cgacatgact caggatatga agttcatcat caaggaggag gagatgcaga attccgacat 1380
   gactcaggat atgaagttca tcatcaagga ggaggagatg cagaattccg acatgactca 1440
   ggatatgaag ttcatcatca aggaggagga gatgcagaat tccgacatga ctcaggatat 1500
   gaagttcatc atcaaggagg aggagatgca gaattccgac atgactcagg atatgaagtt 1560
   catcatcaag gaggaggaga tgcagaattc cgacatgact caggatatga agttcatcat 1620
   caaggaggag gagatgcaga attccgacat gactcaggat atgaagttca tcatcaagga 1680
   ggaggagatg cagaattccg acatgactca ggatatgaag ttcatcatca aggaggagga 1740
   gatgcagaat tccgacatga ctcaggatat gaagttcatc atcaaggagg aggagatgca 1800
   gaattccgac atgactcagg atatgaagtt catcatcaag gaggaggaga tgcagaattc 1860
   cgacatgact caggatatga agttcatcat caaggaggag gagatgcaga attccgacat 1920
   gactcaggat atgaagttca tcatcaagga ggaggagatg cagaattccg acatgactca 1980
   ggatatgaag ttcatcatca aggaggagga gatgcagaat tccgacatga ctcaggatat 2040
   gaagttcatc atcaaggagg aggagatgca gaattccgac atgactcagg atatgaagtt 2100
   catcatcaag gaggaggaga tgcagaattc cgacatgact caggatatga agttcatcat 2160
   caaggaggag gagatgcaga attccgacat gactcaggat atgaagttca tcatcaagga 2220
   ggaggagatg cagaattccg acatgactca ggatatgaag ttcatcatca aggaggagga 2280
   gatgcagaat tccgacatga ctcaggatat gaagttcatc atcaaggagg aggagatgca 2340
   gaattccgac atgactcagg atatgaagtt catcatcaag gaggaggaca aaactacacc 2400
   atgaacttgg cctcgcagaa ctggaacaac tacgatccaa ccgaggaaat ccccgctcct 2460
   ttgggaactc ccgacttcgt gggacgtatc caaggtgtcc tgacacagac tacacgtcgc 2520
   gacggctcta ctcgcggaca caaggccact gtgtcgacag gttccgtcca cttcacccct 2580
   aagctgggct ctgtgcaatt cagcacagac acctcaaacg atttcgagac tggccagaac 2640
   accaggttca ctcccgtggg tgtcgttcaa gacggcggag gaggagatgc agaattccga 2700
   catggaggag gagatgcaga attccgacat ggaggaggag atgcagaatt ccgacatgga 2760
   ggaggagatg cagaattccg acatggagga ggagatgcag aattccgaca tggaggagga 2820
   gatgcagaat tccgacatgg aggaggagat gcagaattcc gacatggagg aggagatgca 2880
   gaattccgac atggaggagg agatgcagaa ttccgacatg gaggaggaga tgcagaattc 2940
   cgacatggag gaggagatgc agaattccga catggaggag gagatgcaga attccgacat 3000
   ggaggaggag atgcagaatt ccgacatgga ggaggagatg cagaattccg acatggagga 3060
   ggagatgcag aattccgaca tggaggagga gatgcagaat tccgacatgg aggaggagat 3120
   gcagaattcc gacatggagg aggagatgca gaattccgac atggaggagg agatgcagaa 3180
   ttccgacatg gaggaggaga tgcagaattc cgacatggag gaggagatgc agaattccga 3240
   catggaggag gagatgcaga attccgacat ggaggaggag atgcagaatt ccgacatgga 3300
   ggaggagatg cagaattccg acatggagga ggagatgcag aattccgaca tggaggagga 3360
   gatgcagaat tccgacatgg aggaggagat gcagaattcc gacatggagg aggagatgca 3420
   gaattccgac atggaggagg agatgcagaa ttccgacatg gaggaggaga tgcagaattc 3480
   cgacatggag gaggagatgc agaattccga catggaggag gagatgcaga attccgacat 3540
   ggaggaggag atgcagaatt ccgacatgga ggaggagatg cagaattccg acatggagga 3600
   ggagatgcag aattccgaca tggaggagga gatgcagaat tccgacatgg aggaggagat 3660
   gcagaattcc gacatggagg aggagatgca gaattccgac atggaggagg agatgcagaa 3720
   ttccgacatg gaggaggaga tgcagaattc cgacatggag gaggaagcac cactcaccag 3780
   aacgaacccc agcaatgggt cctccctgac tactcgggca gggattccca caacgttcac 3840
   ttggctccgg ccgtggctcc aaccttccct ggagagcagt tgctgttctt cagatccact   3900
   atgccaggct gctctggata cccgaacatg aacctcgact gtctcttgcc tcaggagtgg   3960
   gtgcaacact tctaccagga atctgcccca gctcaaagcg acgtcgctct gctccgtttc   4020
   gttaaccccg ataccggtcg cgtgctcttc gagtgtaagt tgcacaagtc tggttacgtc   4080
   actgttgccc acacaggcca gcacgacctg gtcatccctc ccaacggcta cttccgcttc   4140
   gatagctggg tcaaccagtt ctacacactc gccccgatgg gaaacggagc cggtcgtcgc   4200
   agagccttgt aa                                                       4212
<210> 117
   <211> 1269
   <212> DNA
   <213> artificial sequence
<400> 117
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catggaggag gaacacaaaa ctacaccatg aacttggcct cgcagaactg gaacaactac     300
   gatccaaccg aggaaatccc cgctcctttg ggaactcccg acttcgtggg acgtatccaa     360
   ggtgtcctga cacagactac acgtcgcgac ggctctactc gcggacacaa ggccactgtg     420
   tcgacaggtt ccgtccactt cacccctaag ctgggctctg tgcaattcag cacagacacc     480
   tcaaacgatt tcgagactgg ccagaacacc aggttcactc ccgtgggtgt cgttcaagac     540
   ggcggaggag gagatgcaga attccgacat ggaggaggag atgcagaatt ccgacatgga     600
   ggaggagatg cagaattccg acatggagga ggagatgcag aattccgaca tggaggagga     660
   gatgcagaat tccgacatgg aggaggagat gcagaattcc gacatggagg aggagatgca     720
   gaattccgac atggaggagg agatgcagaa ttccgacatg gaggaggaga tgcagaattc     780
   cgacatggag gaggagatgc agaattccga catggaggag gaagcaccac tcaccagaac     840
   gaaccccagc aatgggtcct ccctgactac tcgggcaggg attcccacaa cgttcacttg     900
   gctccggccg tggctccaac cttccctgga gagcagttgc tgttcttcag atccactatg     960
   ccaggctgct ctggataccc gaacatgaac ctcgactgtc tcttgcctca ggagtgggtg    1020
   caacacttct accaggaatc tgccccagct caaagcgacg tcgctctgct ccgtttcgtt    1080
   aaccccgata ccggtcgcgt gctcttcgag tgtaagttgc acaagtctgg ttacgtcact    1140
   gttgcccaca caggccagca cgacctggtc atccctccca acggctactt ccgcttcgat    1200
   agctgggtca accagttcta cacactcgcc ccgatgggaa acggagccgg tcgtcgcaga    1260
   gccttgtaa                                                            1269
<210> 118
   <211> 2052
   <212> DNA
   <213> artificial sequence
<400> 118
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catgactcag gaggaggagg agatgcagaa ttccgacatg actcaggagg aggaggagat 300
   gcagaattcc gacatgactc aggaggagga ggagatgcag aattccgaca tgactcagga 360
   ggaggaggag atgcagaatt ccgacatgac tcaggaggag gaggagatgc agaattccga 420
   catgactcag gaggaggagg agatgcagaa ttccgacatg actcaggagg aggaggagat 480
   gcagaattcc gacatgactc aggaggagga ggagatgcag aattccgaca tgactcagga 540
   ggaggaggag atgcagaatt ccgacatgac tcaggaggag gaggaacaca aaactacacc 600
   atgaacttgg cctcgcagaa ctggaacaac tacgatccaa ccgaggaaat ccccgctcct 660
   ttgggaactc ccgacttcgt gggacgtatc caaggtgtcc tgacacagac tacacgtcgc 720
   gacggctcta ctcgcggaca caaggccact gtgtcgacag gttccgtcca cttcacccct 780
   aagctgggct ctgtgcaatt cagcacagac acctcaaacg atttcgagac tggccagaac 840
   accaggttca ctcccgtggg tgtcgttcaa gacggcggag gaggagatgc agaattccga 900
   catgactcag gaggaggagg agatgcagaa ttccgacatg actcaggagg aggaggagat 960
   gcagaattcc gacatgactc aggaggagga ggagatgcag aattccgaca tgactcagga 1020
   ggaggaggag atgcagaatt ccgacatgac tcaggaggag gaggagatgc agaattccga 1080
   catgactcag gaggaggagg agatgcagaa ttccgacatg actcaggagg aggaggagat 1140
   gcagaattcc gacatgactc aggaggagga ggagatgcag aattccgaca tgactcagga 1200
   ggaggaggag atgcagaatt ccgacatgac tcaggaggag gaggagatgc agaattccga 1260
   catgactcag gaggaggagg agatgcagaa ttccgacatg actcaggagg aggaggagat 1320
   gcagaattcc gacatgactc aggaggagga ggagatgcag aattccgaca tgactcagga 1380
   ggaggaggag atgcagaatt ccgacatgac tcaggaggag gaggagatgc agaattccga 1440
   catgactcag gaggaggagg agatgcagaa ttccgacatg actcaggagg aggaggagat 1500
   gcagaattcc gacatgactc aggaggagga ggagatgcag aattccgaca tgactcagga 1560
   ggaggaggag atgcagaatt ccgacatgac tcaggaggag gaggaagcac cactcaccag 1620
   aacgaacccc agcaatgggt cctccctgac tactcgggca gggattccca caacgttcac 1680
   ttggctccgg ccgtggctcc aaccttccct ggagagcagt tgctgttctt cagatccact 1740
   atgccaggct gctctggata cccgaacatg aacctcgact gtctcttgcc tcaggagtgg 1800
   gtgcaacact tctaccagga atctgcccca gctcaaagcg acgtcgctct gctccgtttc 1860
   gttaaccccg ataccggtcg cgtgctcttc gagtgtaagt tgcacaagtc tggttacgtc 1920
   actgttgccc acacaggcca gcacgacctg gtcatccctc ccaacggcta cttccgcttc 1980
   gatagctggg tcaaccagtt ctacacactc gccccgatgg gaaacggagc cggtcgtcgc 2040
   agagccttgt aa 2052
<210> 119
   <211> 3672
   <212> DNA
   <213> artificial sequence
<400> 119
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tggaggagga gatgcagaat tccgacatga ctcaggatat     300
   gaagttggag gaggagatgc agaattccga catgactcag gatatgaagt tggaggagga     360
   gatgcagaat tccgacatga ctcaggatat gaagttggag gaggagatgc agaattccga     420
   catgactcag gatatgaagt tggaggagga gatgcagaat tccgacatga ctcaggatat     480
   gaagttggag gaggagatgc agaattccga catgactcag gatatgaagt tggaggagga     540
   gatgcagaat tccgacatga ctcaggatat gaagttggag gaggagatgc agaattccga     600
   catgactcag gatatgaagt tggaggagga gatgcagaat tccgacatga ctcaggatat     660
   gaagttggag gaggagatgc agaattccga catgactcag gatatgaagt tggaggagga     720
   gatgcagaat tccgacatga ctcaggatat gaagttggag gaggagatgc agaattccga     780
   catgactcag gatatgaagt tggaggagga gatgcagaat tccgacatga ctcaggatat     840
   gaagttggag gaggagatgc agaattccga catgactcag gatatgaagt tggaggagga     900
   gatgcagaat tccgacatga ctcaggatat gaagttggag gaggagatgc agaattccga     960
   catgactcag gatatgaagt tggaggagga gatgcagaat tccgacatga ctcaggatat    1020
   gaagttggag gaggagatgc agaattccga catgactcag gatatgaagt tggaggagga    1080
   gatgcagaat tccgacatga ctcaggatat gaagttggag gaggaacaca aaactacacc    1140
   atgaacttgg cctcgcagaa ctggaacaac tacgatccaa ccgaggaaat ccccgctcct    1200
   ttgggaactc ccgacttcgt gggacgtatc caaggtgtcc tgacacagac tacacgtcgc 1260
   gacggctcta ctcgcggaca caaggccact gtgtcgacag gttccgtcca cttcacccct 1320
   aagctgggct ctgtgcaatt cagcacagac acctcaaacg atttcgagac tggccagaac 1380
   accaggttca ctcccgtggg tgtcgttcaa gacggcggag gaggagatgc agaattccga 1440
   catgactcag gatatgaagt tggaggagga gatgcagaat tccgacatga ctcaggatat 1500
   gaagttggag gaggagatgc agaattccga catgactcag gatatgaagt tggaggagga 1560
   gatgcagaat tccgacatga ctcaggatat gaagttggag gaggagatgc agaattccga 1620
   catgactcag gatatgaagt tggaggagga gatgcagaat tccgacatga ctcaggatat 1680
   gaagttggag gaggagatgc agaattccga catgactcag gatatgaagt tggaggagga 1740
   gatgcagaat tccgacatga ctcaggatat gaagttggag gaggagatgc agaattccga 1800
   catgactcag gatatgaagt tggaggagga gatgcagaat tccgacatga ctcaggatat 1860
   gaagttggag gaggagatgc agaattccga catgactcag gatatgaagt tggaggagga 1920
   gatgcagaat tccgacatga ctcaggatat gaagttggag gaggagatgc agaattccga 1980
   catgactcag gatatgaagt tggaggagga gatgcagaat tccgacatga ctcaggatat 2040
   gaagttggag gaggagatgc agaattccga catgactcag gatatgaagt tggaggagga 2100
   gatgcagaat tccgacatga ctcaggatat gaagttggag gaggagatgc agaattccga 2160
   catgactcag gatatgaagt tggaggagga gatgcagaat tccgacatga ctcaggatat 2220
   gaagttggag gaggagatgc agaattccga catgactcag gatatgaagt tggaggagga 2280
   gatgcagaat tccgacatga ctcaggatat gaagttggag gaggagatgc agaattccga 2340
   catgactcag gatatgaagt tggaggagga gatgcagaat tccgacatga ctcaggatat 2400
   gaagttggag gaggagatgc agaattccga catgactcag gatatgaagt tggaggagga 2460
   gatgcagaat tccgacatga ctcaggatat gaagttggag gaggagatgc agaattccga 2520
   catgactcag gatatgaagt tggaggagga gatgcagaat tccgacatga ctcaggatat 2580
   gaagttggag gaggagatgc agaattccga catgactcag gatatgaagt tggaggagga 2640
   gatgcagaat tccgacatga ctcaggatat gaagttggag gaggagatgc agaattccga 2700
   catgactcag gatatgaagt tggaggagga gatgcagaat tccgacatga ctcaggatat 2760
   gaagttggag gaggagatgc agaattccga catgactcag gatatgaagt tggaggagga 2820
   gatgcagaat tccgacatga ctcaggatat gaagttggag gaggagatgc agaattccga 2880
   catgactcag gatatgaagt tggaggagga gatgcagaat tccgacatga ctcaggatat 2940
   gaagttggag gaggagatgc agaattccga catgactcag gatatgaagt tggaggagga 3000
   gatgcagaat tccgacatga ctcaggatat gaagttggag gaggagatgc agaattccga 3060
   catgactcag gatatgaagt tggaggagga gatgcagaat tccgacatga ctcaggatat 3120
   gaagttggag gaggagatgc agaattccga catgactcag gatatgaagt tggaggagga 3180
   gatgcagaat tccgacatga ctcaggatat gaagttggag gaggaagcac cactcaccag 3240
   aacgaacccc agcaatgggt cctccctgac tactcgggca gggattccca caacgttcac 3300
   ttggctccgg ccgtggctcc aaccttccct ggagagcagt tgctgttctt cagatccact 3360
   atgccaggct gctctggata cccgaacatg aacctcgact gtctcttgcc tcaggagtgg 3420
   gtgcaacact tctaccagga atctgcccca gctcaaagcg acgtcgctct gctccgtttc 3480
   gttaaccccg ataccggtcg cgtgctcttc gagtgtaagt tgcacaagtc tggttacgtc 3540
   actgttgccc acacaggcca gcacgacctg gtcatccctc ccaacggcta cttccgcttc 3600
   gatagctggg tcaaccagtt ctacacactc gccccgatgg gaaacggagc cggtcgtcgc 3660
   agagccttgt aa 3672
<210> 120
   <211> 3672
   <212> DNA
   <213> artificial sequence
<400> 120
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tcatcatcaa ggaggaggag atgcagaatt ccgacatgac     300
   tcaggatatg aagttcatca tcaaggagga ggagatgcag aattccgaca tgactcagga     360
   tatgaagttc atcatcaagg aggaggagat gcagaattcc gacatgactc aggatatgaa     420
   gttcatcatc aaggaggagg agatgcagaa ttccgacatg actcaggata tgaagttcat     480
   catcaaggag gaggagatgc agaattccga catgactcag gatatgaagt tcatcatcaa     540
   ggaggaggag atgcagaatt ccgacatgac tcaggatatg aagttcatca tcaaggagga     600
   ggagatgcag aattccgaca tgactcagga tatgaagttc atcatcaagg aggaggagat     660
   gcagaattcc gacatgactc aggatatgaa gttcatcatc aaggaggagg agatgcagaa     720
   ttccgacatg actcaggata tgaagttcat catcaaggag gaggagatgc agaattccga     780
   catgactcag gatatgaagt tcatcatcaa ggaggaggag atgcagaatt ccgacatgac     840
   tcaggatatg aagttcatca tcaaggagga ggagatgcag aattccgaca tgactcagga     900
   tatgaagttc atcatcaagg aggaggagat gcagaattcc gacatgactc aggatatgaa     960
   gttcatcatc aaggaggagg agatgcagaa ttccgacatg actcaggata tgaagttcat    1020
   catcaaggag gaggagatgc agaattccga catgactcag gatatgaagt tcatcatcaa 1080
   ggaggaggag atgcagaatt ccgacatgac tcaggatatg aagttcatca tcaaggagga 1140
   ggagatgcag aattccgaca tgactcagga tatgaagttc atcatcaagg aggaggagat 1200
   gcagaattcc gacatgactc aggatatgaa gttcatcatc aaggaggagg agatgcagaa 1260
   ttccgacatg actcaggata tgaagttcat catcaaggag gaggagatgc agaattccga 1320
   catgactcag gatatgaagt tcatcatcaa ggaggaggag atgcagaatt ccgacatgac 1380
   tcaggatatg aagttcatca tcaaggagga ggagatgcag aattccgaca tgactcagga 1440
   tatgaagttc atcatcaagg aggaggagat gcagaattcc gacatgactc aggatatgaa 1500
   gttcatcatc aaggaggagg agatgcagaa ttccgacatg actcaggata tgaagttcat 1560
   catcaaggag gaggagatgc agaattccga catgactcag gatatgaagt tcatcatcaa 1620
   ggaggaggag atgcagaatt ccgacatgac tcaggatatg aagttcatca tcaaggagga 1680
   ggagatgcag aattccgaca tgactcagga tatgaagttc atcatcaagg aggaggagat 1740
   gcagaattcc gacatgactc aggatatgaa gttcatcatc aaggaggagg agatgcagaa 1800
   ttccgacatg actcaggata tgaagttcat catcaaggag gaggagatgc agaattccga 1860
   catgactcag gatatgaagt tcatcatcaa ggaggaggag atgcagaatt ccgacatgac 1920
   tcaggatatg aagttcatca tcaaggagga ggagatgcag aattccgaca tgactcagga 1980
   tatgaagttc atcatcaagg aggaggagat gcagaattcc gacatgactc aggatatgaa 2040
   gttcatcatc aaggaggagg agatgcagaa ttccgacatg actcaggata tgaagttcat 2100
   catcaaggag gaggagatgc agaattccga catgactcag gatatgaagt tcatcatcaa 2160
   ggaggaggag atgcagaatt ccgacatgac tcaggatatg aagttcatca tcaaggagga 2220
   ggagatgcag aattccgaca tgactcagga tatgaagttc atcatcaagg aggaggagat 2280
   gcagaattcc gacatgactc aggatatgaa gttcatcatc aaggaggagg agatgcagaa 2340
   ttccgacatg actcaggata tgaagttcat catcaaggag gaggaacaca aaactacacc 2400
   atgaacttgg cctcgcagaa ctggaacaac tacgatccaa ccgaggaaat ccccgctcct 2460
   ttgggaactc ccgacttcgt gggacgtatc caaggtgtcc tgacacagac tacacgtcgc 2520
   gacggctcta ctcgcggaca caaggccact gtgtcgacag gttccgtcca cttcacccct 2580
   aagctgggct ctgtgcaatt cagcacagac acctcaaacg atttcgagac tggccagaac 2640
   accaggttca ctcccgtggg tgtcgttcaa gacggcggag gaggagatgc agaattccga 2700
   catgactcag gatatgaagt tcatcatcaa ggaggaggag atgcagaatt ccgacatgac 2760
   tcaggatatg aagttcatca tcaaggagga ggagatgcag aattccgaca tgactcagga 2820
   tatgaagttc atcatcaagg aggaggagat gcagaattcc gacatgactc aggatatgaa 2880
   gttcatcatc aaggaggagg agatgcagaa ttccgacatg actcaggata tgaagttcat 2940
   catcaaggag gaggagatgc agaattccga catgactcag gatatgaagt tcatcatcaa 3000
   ggaggaggag atgcagaatt ccgacatgac tcaggatatg aagttcatca tcaaggagga 3060
   ggagatgcag aattccgaca tgactcagga tatgaagttc atcatcaagg aggaggagat 3120
   gcagaattcc gacatgactc aggatatgaa gttcatcatc aaggaggagg agatgcagaa 3180
   ttccgacatg actcaggata tgaagttcat catcaaggag gaggaagcac cactcaccag 3240
   aacgaacccc agcaatgggt cctccctgac tactcgggca gggattccca caacgttcac 3300
   ttggctccgg ccgtggctcc aaccttccct ggagagcagt tgctgttctt cagatccact 3360
   atgccaggct gctctggata cccgaacatg aacctcgact gtctcttgcc tcaggagtgg 3420
   gtgcaacact tctaccagga atctgcccca gctcaaagcg acgtcgctct gctccgtttc 3480
   gttaaccccg ataccggtcg cgtgctcttc gagtgtaagt tgcacaagtc tggttacgtc 3540
   actgttgccc acacaggcca gcacgacctg gtcatccctc ccaacggcta cttccgcttc 3600
   gatagctggg tcaaccagtt ctacacactc gccccgatgg gaaacggagc cggtcgtcgc 3660
   agagccttgt aa 3672
<210> 121
   <211> 1359
   <212> DNA
   <213> artificial sequence
<400> 121
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc ggaggaggag atgcagaatt ccgacatgga 240
   ggaggagctg gaacacaaaa ctacaccatg aacttggcct cgcagaactg gaacaactac 300
   gatccaaccg aggaaatccc cgctcctttg ggaactcccg acttcgtggg acgtatccaa 360
   ggtgtcctga cacagactac acgtcgcgac ggctctactc gcggacacaa ggccactgtg 420
   tcgacaggtt ccgtccactt cacccctaag ctgggctctg tgcaattcag cacagacacc 480
   tcaaacgatt tcgagactgg ccagaacacc aggttcactc ccgtgggtgt cgttcaagac 540
   ggaggaggag atgcagaatt ccgacatgac tcaggaggag gaggagatgc agaattccga 600
   catgactcag gaggaggagg agatgcagaa ttccgacatg actcaggagg aggaggagat 660
   gcagaattcc gacatgactc aggaggagga ggagatgcag aattccgaca tgactcagga 720
   ggaggaggag atgcagaatt ccgacatgac tcaggaggag gaggagatgc agaattccga 780
   catgactcag gaggaggagg agatgcagaa ttccgacatg actcaggagg aggaggagat 840
   gcagaattcc gacatgactc aggaggagga ggagatgcag aattccgaca tgactcagga 900
   ggaggaggag gcagcaccac tcaccagaac gaaccccagc aatgggtcct ccctgactac 960
   tcgggcaggg attcccacaa cgttcacttg gctccggccg tggctccaac cttccctgga 1020
   gagcagttgc tgttcttcag atccactatg ccaggctgct ctggataccc gaacatgaac 1080
   ctcgactgtc tcttgcctca ggagtgggtg caacacttct accaggaatc tgccccagct 1140
   caaagcgacg tcgctctgct ccgtttcgtt aaccccgata ccggtcgcgt gctcttcgag 1200
   tgtaagttgc acaagtctgg ttacgtcact gttgcccaca caggccagca cgacctggtc 1260
   atccctccca acggctactt ccgcttcgat agctgggtca accagttcta cacactcgcc 1320
   ccgatgggaa acggagccgg tcgtcgcaga gccttgtaa 1359
<210> 122
   <211> 1503
   <212> DNA
   <213> artificial sequence
<400> 122
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catggaggag gagatgcaga attccgacat ggaggaggag atgcagaatt ccgacatgga 300
   ggaggaacac aaaactacac catgaacttg gcctcgcaga actggaacaa ctacgatcca 360
   accgaggaaa tccccgctcc tttgggaact cccgacttcg tgggacgtat ccaaggtgtc 420
   ctgacacaga ctacacgtcg cgacggctct actcgcggac acaaggccac tgtgtcgaca 480
   ggttccgtcc acttcacccc taagctgggc tctgtgcaat tcagcacaga cacctcaaac 540
   gatttcgaga ctggccagaa caccaggttc actcccgtgg gtgtcgttca agacggcgga 600
   ggaggagatg cagaattccg acatgactca ggatatgaag ttggaggagg agatgcagaa 660
   ttccgacatg actcaggata tgaagttgga ggaggagatg cagaattccg acatgactca 720
   ggatatgaag ttggaggagg agatgcagaa ttccgacatg actcaggata tgaagttgga 780
   ggaggagatg cagaattccg acatgactca ggatatgaag ttggaggagg agatgcagaa 840
   ttccgacatg actcaggata tgaagttgga ggaggagatg cagaattccg acatgactca 900
   ggatatgaag ttggaggagg agatgcagaa ttccgacatg actcaggata tgaagttgga 960
   ggaggagatg cagaattccg acatgactca ggatatgaag ttggaggagg agatgcagaa 1020
   ttccgacatg actcaggata tgaagttgga ggaggaagca ccactcacca gaacgaaccc 1080
   cagcaatggg tcctccctga ctactcgggc agggattccc acaacgttca cttggctccg 1140
   gccgtggctc caaccttccc tggagagcag ttgctgttct tcagatccac tatgccaggc 1200
   tgctctggat acccgaacat gaacctcgac tgtctcttgc ctcaggagtg ggtgcaacac 1260
   ttctaccagg aatctgcccc agctcaaagc gacgtcgctc tgctccgttt cgttaacccc 1320
   gataccggtc gcgtgctctt cgagtgtaag ttgcacaagt ctggttacgt cactgttgcc 1380
   cacacaggcc agcacgacct ggtcatccct cccaacggct acttccgctt cgatagctgg 1440
   gtcaaccagt tctacacact cgccccgatg ggaaacggag ccggtcgtcg cagagccttg 1500
   taa 1503
<210> 123
   <211> 1476
   <212> DNA
   <213> artificial sequence
<400> 123
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catgactcag gatatgaagt tggaggagga gatgcagaat tccgacatga ctcaggatat     300
   gaagttggag gaggagatgc agaattccga catgactcag gatatgaagt tggaggagga     360
   gatgcagaat tccgacatga ctcaggatat gaagttggag gaggagatgc agaattccga     420
   catgactcag gatatgaagt tggaggagga gatgcagaat tccgacatga ctcaggatat     480
   gaagttggag gaggagatgc agaattccga catgactcag gatatgaagt tggaggagga     540
   gatgcagaat tccgacatga ctcaggatat gaagttggag gaggagatgc agaattccga     600
   catgactcag gatatgaagt tggaggagga gatgcagaat tccgacatga ctcaggatat     660
   gaagttggag gaggaacaca aaactacacc atgaacttgg cctcgcagaa ctggaacaac     720
   tacgatccaa ccgaggaaat ccccgctcct ttgggaactc ccgacttcgt gggacgtatc     780
   caaggtgtcc tgacacagac tacacgtcgc gacggctcta ctcgcggaca caaggccact     840
   gtgtcgacag gttccgtcca cttcacccct aagctgggct ctgtgcaatt cagcacagac     900
   acctcaaacg atttcgagac tggccagaac accaggttca ctcccgtggg tgtcgttcaa     960
   gacggcagcg gaggaggaga tgcagaattc cgacatgact caggatatga agttcatcat    1020
   caaggaggag gaaccactca ccagaacgaa ccccagcaat gggtcctccc tgactactcg    1080
   ggcagggatt cccacaacgt tcacttggct ccggccgtgg ctccaacctt ccctggagag    1140
   cagttgctgt tcttcagatc cactatgcca ggctgctctg gatacccgaa catgaacctc    1200
   gactgtctct tgcctcagga gtgggtgcaa cacttctacc aggaatctgc cccagctcaa    1260
   agcgacgtcg ctctgctccg tttcgttaac cccgataccg gtcgcgtgct cttcgagtgt    1320
   aagttgcaca agtctggtta cgtcactgtt gcccacacag gccagcacga cctggtcatc    1380
   cctcccaacg gctacttccg cttcgatagc tgggtcaacc agttctacac actcgccccg    1440
   atgggaaacg gagccggtcg tcgcagagcc ttgtaa                               1476
<210> 124
   <211> 3852
   <212> DNA
   <213> artificial sequence
<400> 124
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggag gaggaggaga tgcagaattc cgacatgact caggaggagg aggagatgca     300
   gaattccgac atgactcagg aggaggagga gatgcagaat tccgacatga ctcaggagga     360
   ggaggagatg cagaattccg acatgactca ggaggaggag gagatgcaga attccgacat     420
   gactcaggag gaggaggaga tgcagaattc cgacatgact caggaggagg aggagatgca     480
   gaattccgac atgactcagg aggaggagga gatgcagaat tccgacatga ctcaggagga     540
   ggaggagatg cagaattccg acatgactca ggaggaggag gagatgcaga attccgacat     600
   gactcaggag gaggaggaga tgcagaattc cgacatgact caggaggagg aggagatgca     660
   gaattccgac atgactcagg aggaggagga gatgcagaat tccgacatga ctcaggagga     720
   ggaggagatg cagaattccg acatgactca ggaggaggag gagatgcaga attccgacat     780
   gactcaggag gaggaggaga tgcagaattc cgacatgact caggaggagg aggagatgca     840
   gaattccgac atgactcagg aggaggagga gatgcagaat tccgacatga ctcaggagga     900
   ggaggagatg cagaattccg acatgactca ggaggaggag gaggaacaca aaactacacc     960
   atgaacttgg cctcgcagaa ctggaacaac tacgatccaa ccgaggaaat ccccgctcct    1020
   ttgggaactc ccgacttcgt gggacgtatc caaggtgtcc tgacacagac tacacgtcgc    1080
   gacggctcta ctcgcggaca caaggccact gtgtcgacag gttccgtcca cttcacccct    1140
   aagctgggct ctgtgcaatt cagcacagac acctcaaacg atttcgagac tggccagaac    1200
   accaggttca ctcccgtggg tgtcgttcaa gacggcggag gaggagatgc agaattccga    1260
   catgactcag gatatgaagt tcatcatcaa ggaggaggag atgcagaatt ccgacatgac    1320
   tcaggatatg aagttcatca tcaaggagga ggagatgcag aattccgaca tgactcagga    1380
   tatgaagttc atcatcaagg aggaggagat gcagaattcc gacatgactc aggatatgaa    1440
   gttcatcatc aaggaggagg agatgcagaa ttccgacatg actcaggata tgaagttcat    1500
   catcaaggag gaggagatgc agaattccga catgactcag gatatgaagt tcatcatcaa 1560
   ggaggaggag atgcagaatt ccgacatgac tcaggatatg aagttcatca tcaaggagga 1620
   ggagatgcag aattccgaca tgactcagga tatgaagttc atcatcaagg aggaggagat 1680
   gcagaattcc gacatgactc aggatatgaa gttcatcatc aaggaggagg agatgcagaa 1740
   ttccgacatg actcaggata tgaagttcat catcaaggag gaggagatgc agaattccga 1800
   catgactcag gatatgaagt tcatcatcaa ggaggaggag atgcagaatt ccgacatgac 1860
   tcaggatatg aagttcatca tcaaggagga ggagatgcag aattccgaca tgactcagga 1920
   tatgaagttc atcatcaagg aggaggagat gcagaattcc gacatgactc aggatatgaa 1980
   gttcatcatc aaggaggagg agatgcagaa ttccgacatg actcaggata tgaagttcat 2040
   catcaaggag gaggagatgc agaattccga catgactcag gatatgaagt tcatcatcaa 2100
   ggaggaggag atgcagaatt ccgacatgac tcaggatatg aagttcatca tcaaggagga 2160
   ggagatgcag aattccgaca tgactcagga tatgaagttc atcatcaagg aggaggagat 2220
   gcagaattcc gacatgactc aggatatgaa gttcatcatc aaggaggagg agatgcagaa 2280
   ttccgacatg actcaggata tgaagttcat catcaaggag gaggagatgc agaattccga 2340
   catgactcag gatatgaagt tcatcatcaa ggaggaggag atgcagaatt ccgacatgac 2400
   tcaggatatg aagttcatca tcaaggagga ggagatgcag aattccgaca tgactcagga 2460
   tatgaagttc atcatcaagg aggaggagat gcagaattcc gacatgactc aggatatgaa 2520
   gttcatcatc aaggaggagg agatgcagaa ttccgacatg actcaggata tgaagttcat 2580
   catcaaggag gaggagatgc agaattccga catgactcag gatatgaagt tcatcatcaa 2640
   ggaggaggag atgcagaatt ccgacatgac tcaggatatg aagttcatca tcaaggagga 2700
   ggagatgcag aattccgaca tgactcagga tatgaagttc atcatcaagg aggaggagat 2760
   gcagaattcc gacatgactc aggatatgaa gttcatcatc aaggaggagg agatgcagaa 2820
   ttccgacatg actcaggata tgaagttcat catcaaggag gaggagatgc agaattccga 2880
   catgactcag gatatgaagt tcatcatcaa ggaggaggag atgcagaatt ccgacatgac 2940
   tcaggatatg aagttcatca tcaaggagga ggagatgcag aattccgaca tgactcagga 3000
   tatgaagttc atcatcaagg aggaggagat gcagaattcc gacatgactc aggatatgaa 3060
   gttcatcatc aaggaggagg agatgcagaa ttccgacatg actcaggata tgaagttcat 3120
   catcaaggag gaggagatgc agaattccga catgactcag gatatgaagt tcatcatcaa 3180
   ggaggaggag atgcagaatt ccgacatgac tcaggatatg aagttcatca tcaaggagga 3240
   ggagatgcag aattccgaca tgactcagga tatgaagttc atcatcaagg aggaggagat 3300
   gcagaattcc gacatgactc aggatatgaa gttcatcatc aaggaggagg agatgcagaa 3360
   ttccgacatg actcaggata tgaagttcat catcaaggag gaggaagcac cactcaccag 3420
   aacgaacccc agcaatgggt cctccctgac tactcgggca gggattccca caacgttcac 3480
   ttggctccgg ccgtggctcc aaccttccct ggagagcagt tgctgttctt cagatccact 3540
   atgccaggct gctctggata cccgaacatg aacctcgact gtctcttgcc tcaggagtgg 3600
   gtgcaacact tctaccagga atctgcccca gctcaaagcg acgtcgctct gctccgtttc 3660
   gttaaccccg ataccggtcg cgtgctcttc gagtgtaagt tgcacaagtc tggttacgtc 3720
   actgttgccc acacaggcca gcacgacctg gtcatccctc ccaacggcta cttccgcttc 3780
   gatagctggg tcaaccagtt ctacacactc gccccgatgg gaaacggagc cggtcgtcgc 3840
   agagccttgt aa 3852
<210> 125
   <211> 3312
   <212> DNA
   <213> artificial sequence
<400> 125
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaggaggagg agatgcagaa     240
   ttccgacatg actcaggagg aggaggagat gcagaattcc gacatgactc aggaggagga     300
   ggagatgcag aattccgaca tgactcagga ggaggaggag atgcagaatt ccgacatgac     360
   tcaggaggag gaggagatgc agaattccga catgactcag gaggaggagg agatgcagaa     420
   ttccgacatg actcaggagg aggaggagat gcagaattcc gacatgactc aggaggagga     480
   ggagatgcag aattccgaca tgactcagga ggaggaggag atgcagaatt ccgacatgac     540
   tcaggaggag gaggagatgc agaattccga catgactcag gaggaggagg agatgcagaa     600
   ttccgacatg actcaggagg aggaggagat gcagaattcc gacatgactc aggaggagga     660
   ggagatgcag aattccgaca tgactcagga ggaggaggag atgcagaatt ccgacatgac     720
   tcaggaggag gaggagatgc agaattccga catgactcag gaggaggagg agatgcagaa     780
   ttccgacatg actcaggagg aggaggagat gcagaattcc gacatgactc aggaggagga     840
   ggagatgcag aattccgaca tgactcagga ggaggaggag atgcagaatt ccgacatgac     900
   tcaggaggag gaggagatgc agaattccga catgactcag gaggaggagg agatgcagaa     960
   ttccgacatg actcaggagg aggaggagat gcagaattcc gacatgactc aggaggagga    1020
   ggagatgcag aattccgaca tgactcagga ggaggaggag atgcagaatt ccgacatgac    1080
   tcaggaggag gaggagatgc agaattccga catgactcag gaggaggagg agatgcagaa    1140
   ttccgacatg actcaggagg aggaggagat gcagaattcc gacatgactc aggaggagga   1200
   ggagatgcag aattccgaca tgactcagga ggaggaggag atgcagaatt ccgacatgac   1260
   tcaggaggag gaggagatgc agaattccga catgactcag gaggaggagg agatgcagaa   1320
   ttccgacatg actcaggagg aggaggagat gcagaattcc gacatgactc aggaggagga   1380
   ggagatgcag aattccgaca tgactcagga ggaggaggag atgcagaatt ccgacatgac   1440
   tcaggaggag gaggagatgc agaattccga catgactcag gaggaggagg agatgcagaa   1500
   ttccgacatg actcaggagg aggaggagat gcagaattcc gacatgactc aggaggagga   1560
   ggagatgcag aattccgaca tgactcagga ggaggaggag atgcagaatt ccgacatgac   1620
   tcaggaggag gaggagatgc agaattccga catgactcag gaggaggagg aaactacacc   1680
   atgaacttgg cctcgcagaa ctggaacaac tacgatccaa ccgaggaaat ccccgctcct   1740
   ttgggaactc ccgacttcgt gggacgtatc caaggtgtcc tgacacagac tacacgtcgc   1800
   gacggctcta ctcgcggaca caaggccact gtgtcgacag gttccgtcca cttcacccct   1860
   aagctgggct ctgtgcaatt cagcacagac acctcaaacg atttcgagac tggccagaac   1920
   accaggttca ctcccgtggg tgtcgttcaa gacggcagca ccggaggagg agatgcagaa   1980
   ttccgacatg actcaggata tgaagttgga ggaggagatg cagaattccg acatgactca   2040
   ggatatgaag ttggaggagg agatgcagaa ttccgacatg actcaggata tgaagttgga   2100
   ggaggagatg cagaattccg acatgactca ggatatgaag ttggaggagg agatgcagaa   2160
   ttccgacatg actcaggata tgaagttgga ggaggagatg cagaattccg acatgactca   2220
   ggatatgaag ttggaggagg agatgcagaa ttccgacatg actcaggata tgaagttgga   2280
   ggaggagatg cagaattccg acatgactca ggatatgaag ttggaggagg agatgcagaa   2340
   ttccgacatg actcaggata tgaagttgga ggaggagatg cagaattccg acatgactca   2400
   ggatatgaag ttggaggagg agatgcagaa ttccgacatg actcaggata tgaagttgga   2460
   ggaggagatg cagaattccg acatgactca ggatatgaag ttggaggagg agatgcagaa   2520
   ttccgacatg actcaggata tgaagttgga ggaggagatg cagaattccg acatgactca   2580
   ggatatgaag ttggaggagg agatgcagaa ttccgacatg actcaggata tgaagttgga   2640
   ggaggagatg cagaattccg acatgactca ggatatgaag ttggaggagg agatgcagaa   2700
   ttccgacatg actcaggata tgaagttgga ggaggagatg cagaattccg acatgactca   2760
   ggatatgaag ttggaggagg agatgcagaa ttccgacatg actcaggata tgaagttgga   2820
   ggaggagatg cagaattccg acatgactca ggatatgaag ttggaggagg aactcaccag   2880
   aacgaacccc agcaatgggt cctccctgac tactcgggca gggattccca caacgttcac   2940
   ttggctccgg ccgtggctcc aaccttccct ggagagcagt tgctgttctt cagatccact   3000
   atgccaggct gctctggata cccgaacatg aacctcgact gtctcttgcc tcaggagtgg   3060
   gtgcaacact tctaccagga atctgcccca gctcaaagcg acgtcgctct gctccgtttc   3120
   gttaaccccg ataccggtcg cgtgctcttc gagtgtaagt tgcacaagtc tggttacgtc   3180
   actgttgccc acacaggcca gcacgacctg gtcatccctc ccaacggcta cttccgcttc   3240
   gatagctggg tcaaccagtt ctacacactc gccccgatgg gaaacggagc cggtcgtcgc   3300
   agagccttgt aa                                                       3312
<210> 126
   <211> 2997
   <212> DNA
   <213> artificial sequence
<400> 126
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacag gaggaggaga tgcagaattc     240
   cgacatgact caggatatga agttcatcat caaggaggag gagatgcaga attccgacat     300
   gactcaggat atgaagttca tcatcaagga ggaggagatg cagaattccg acatgactca     360
   ggatatgaag ttcatcatca aggaggagga gatgcagaat tccgacatga ctcaggatat     420
   gaagttcatc atcaaggagg aggagatgca gaattccgac atgactcagg atatgaagtt     480
   catcatcaag gaggaggaga tgcagaattc cgacatgact caggatatga agttcatcat     540
   caaggaggag gagatgcaga attccgacat gactcaggat atgaagttca tcatcaagga     600
   ggaggagatg cagaattccg acatgactca ggatatgaag ttcatcatca aggaggagga     660
   gatgcagaat tccgacatga ctcaggatat gaagttcatc atcaaggagg aggagatgca     720
   gaattccgac atgactcagg atatgaagtt catcatcaag gaggaggaga tgcagaattc     780
   cgacatgact caggatatga agttcatcat caaggaggag gagatgcaga attccgacat     840
   gactcaggat atgaagttca tcatcaagga ggaggagatg cagaattccg acatgactca     900
   ggatatgaag ttcatcatca aggaggagga gatgcagaat tccgacatga ctcaggatat     960
   gaagttcatc atcaaggagg aggagatgca gaattccgac atgactcagg atatgaagtt    1020
   catcatcaag gaggaggaga tgcagaattc cgacatgact caggatatga agttcatcat    1080
   caaggaggag gagatgcaga attccgacat gactcaggat atgaagttca tcatcaagga    1140
   ggaggagatg cagaattccg acatgactca ggatatgaag ttcatcatca aggaggagga    1200
   gatgcagaat tccgacatga ctcaggatat gaagttcatc atcaaggagg aggagatgca    1260
   gaattccgac atgactcagg atatgaagtt catcatcaag gaggaggaga tgcagaattc    1320
   cgacatgact caggatatga agttcatcat caaggaggag gagatgcaga attccgacat    1380
   gactcaggat atgaagttca tcatcaagga ggaggagatg cagaattccg acatgactca 1440
   ggatatgaag ttcatcatca aggaggagga gatgcagaat tccgacatga ctcaggatat 1500
   gaagttcatc atcaaggagg aggagatgca gaattccgac atgactcagg atatgaagtt 1560
   catcatcaag gaggaggaga tgcagaattc cgacatgact caggatatga agttcatcat 1620
   caaggaggag gagatgcaga attccgacat gactcaggat atgaagttca tcatcaagga 1680
   ggaggagatg cagaattccg acatgactca ggatatgaag ttcatcatca aggaggagga 1740
   gatgcagaat tccgacatga ctcaggatat gaagttcatc atcaaggagg aggagatgca 1800
   gaattccgac atgactcagg atatgaagtt catcatcaag gaggaggaca aaactacacc 1860
   atgaacttgg cctcgcagaa ctggaacaac tacgatccaa ccgaggaaat ccccgctcct 1920
   ttgggaactc ccgacttcgt gggacgtatc caaggtgtcc tgacacagac tacacgtcgc 1980
   gacggctcta ctcgcggaca caaggccact gtgtcgacag gttccgtcca cttcacccct 2040
   aagctgggct ctgtgcaatt cagcacagac acctcaaacg atttcgagac tggccagaac 2100
   accaggttca ctcccgtggg tgtcgttcaa gacggcggag gaggagatgc agaattccga 2160
   catggaggag gagatgcaga attccgacat ggaggaggag atgcagaatt ccgacatgga 2220
   ggaggagatg cagaattccg acatggagga ggagatgcag aattccgaca tggaggagga 2280
   gatgcagaat tccgacatgg aggaggagat gcagaattcc gacatggagg aggagatgca 2340
   gaattccgac atggaggagg agatgcagaa ttccgacatg gaggaggaga tgcagaattc 2400
   cgacatggag gaggagatgc agaattccga catggaggag gagatgcaga attccgacat 2460
   ggaggaggag atgcagaatt ccgacatgga ggaggagatg cagaattccg acatggagga 2520
   ggagatgcag aattccgaca tggaggagga agcaccactc accagaacga accccagcaa 2580
   tgggtcctcc ctgactactc gggcagggat tcccacaacg ttcacttggc tccggccgtg 2640
   gctccaacct tccctggaga gcagttgctg ttcttcagat ccactatgcc aggctgctct 2700
   ggatacccga acatgaacct cgactgtctc ttgcctcagg agtgggtgca acacttctac 2760
   caggaatctg ccccagctca aagcgacgtc gctctgctcc gtttcgttaa ccccgatacc 2820
   ggtcgcgtgc tcttcgagtg taagttgcac aagtctggtt acgtcactgt tgcccacaca 2880
   ggccagcacg acctggtcat ccctcccaac ggctacttcc gcttcgatag ctgggtcaac 2940
   cagttctaca cactcgcccc gatgggaaac ggagccggtc gtcgcagagc cttgtaa 2997
<210> 127
   <211> 2268
   <212> DNA
   <213> artificial sequence
<400> 127
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc ggaggaggag atgcagaatt ccgacatgga 240
   ggaggagctg gaacacaaaa ctacaccatg aacttggcct cgcagaactg gaacaactac 300
   gatccaaccg aggaaatccc cgctcctttg ggaactcccg acttcgtggg acgtatccaa 360
   ggtgtcctga cacagactac acgtcgcgac ggctctactc gcggacacaa ggccactgtg 420
   tcgacaggtt ccgtccactt cacccctaag ctgggctctg tgcaattcag cacagacacc 480
   ggaggaggag atgcagaatt ccgacatgac tcaggaggag gaggagatgc agaattccga 540
   catgactcag gaggaggagg agatgcagaa ttccgacatg actcaggagg aggaggagat 600
   gcagaattcc gacatgactc aggaggagga ggagatgcag aattccgaca tgactcagga 660
   ggaggaggag atgcagaatt ccgacatgac tcaggaggag gaggagatgc agaattccga 720
   catgactcag gaggaggagg agatgcagaa ttccgacatg actcaggagg aggaggagat 780
   gcagaattcc gacatgactc aggaggagga ggagatgcag aattccgaca tgactcagga 840
   ggaggaggat caaacgattt cgagactggc cagaacacca ggttcactcc cgtgggtgtc 900
   gttcaagacg gaggaggaga tgcagaattc cgacatgact caggatatga agttggagga 960
   ggagatgcag aattccgaca tgactcagga tatgaagttg gaggaggaga tgcagaattc 1020
   cgacatgact caggatatga agttggagga ggagatgcag aattccgaca tgactcagga 1080
   tatgaagttg gaggaggaga tgcagaattc cgacatgact caggatatga agttggagga 1140
   ggagatgcag aattccgaca tgactcagga tatgaagttg gaggaggaga tgcagaattc 1200
   cgacatgact caggatatga agttggagga ggagatgcag aattccgaca tgactcagga 1260
   tatgaagttg gaggaggaga tgcagaattc cgacatgact caggatatga agttggagga 1320
   ggagatgcag aattccgaca tgactcagga tatgaagttg gaggaggaga tgcagaattc 1380
   cgacatgact caggatatga agttggagga ggagatgcag aattccgaca tgactcagga 1440
   tatgaagttg gaggaggaga tgcagaattc cgacatgact caggatatga agttggagga 1500
   ggagatgcag aattccgaca tgactcagga tatgaagttg gaggaggaga tgcagaattc 1560
   cgacatgact caggatatga agttggagga ggagatgcag aattccgaca tgactcagga 1620
   tatgaagttg gaggaggaga tgcagaattc cgacatgact caggatatga agttggagga 1680
   ggagatgcag aattccgaca tgactcagga tatgaagttg gaggaggaga tgcagaattc 1740
   cgacatgact caggatatga agttggagga ggagatgcag aattccgaca tgactcagga 1800
   tatgaagttg gaggaggagg cagcaccact caccagaacg aaccccagca atgggtcctc 1860
   cctgactact cgggcaggga ttcccacaac gttcacttgg ctccggccgt ggctccaacc 1920
   ttccctggag agcagttgct gttcttcaga tccactatgc caggctgctc tggatacccg   1980
   aacatgaacc tcgactgtct cttgcctcag gagtgggtgc aacacttcta ccaggaatct   2040
   gccccagctc aaagcgacgt cgctctgctc cgtttcgtta accccgatac cggtcgcgtg   2100
   ctcttcgagt gtaagttgca caagtctggt tacgtcactg ttgcccacac aggccagcac   2160
   gacctggtca tccctcccaa cggctacttc cgcttcgata gctgggtcaa ccagttctac   2220
   acactcgccc cgatgggaaa cggagccggt cgtcgcagag ccttgtaa                2268
<210> 128
   <211> 3861
   <212> DNA
   <213> artificial sequence
<400> 128
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gagatgcaga attccgacat     240
   gactcaggat atgaagttca tcatcaagga ggaggagatg cagaattccg acatgactca     300
   ggatatgaag ttcatcatca aggaggagga gatgcagaat tccgacatga ctcaggatat     360
   gaagttcatc atcaaggagg aggagatgca gaattccgac atgactcagg atatgaagtt     420
   catcatcaag gaggaggaga tgcagaattc cgacatgact caggatatga agttcatcat     480
   caaggaggag gagatgcaga attccgacat gactcaggat atgaagttca tcatcaagga     540
   ggaggagatg cagaattccg acatgactca ggatatgaag ttcatcatca aggaggagga     600
   gatgcagaat tccgacatga ctcaggatat gaagttcatc atcaaggagg aggagatgca     660
   gaattccgac atgactcagg atatgaagtt catcatcaag gaggaggaga tgcagaattc     720
   cgacatgact caggatatga agttcatcat caaggaggag gaggaacaca aaactacacc     780
   atgaacttgg cctcgcagaa ctggaacaac tacgatccaa ccgaggaaat ccccgctcct     840
   ttgggaactc ccgacttcgt gggacgtatc caaggtgtcc tgacacagac tacacgtcgc     900
   gacggctcta ctcgcggaca caaggccact gtgtcgacag gttccgtcca cttcacccct     960
   aagctgggct ctgtgcaatt cagcacagac acctcaggag gaggagatgc agaattccga    1020
   catgactcag gatatgaagt tggaggagga gatgcagaat tccgacatga ctcaggatat    1080
   gaagttggag gaggagatgc agaattccga catgactcag gatatgaagt tggaggagga    1140
   gatgcagaat tccgacatga ctcaggatat gaagttggag gaggagatgc agaattccga    1200
   catgactcag gatatgaagt tggaggagga gatgcagaat tccgacatga ctcaggatat    1260
   gaagttggag gaggagatgc agaattccga catgactcag gatatgaagt tggaggagga    1320
   gatgcagaat tccgacatga ctcaggatat gaagttggag gaggagatgc agaattccga 1380
   catgactcag gatatgaagt tggaggagga gatgcagaat tccgacatga ctcaggatat 1440
   gaagttggag gaggagatgc agaattccga catgactcag gatatgaagt tggaggagga 1500
   gatgcagaat tccgacatga ctcaggatat gaagttggag gaggagatgc agaattccga 1560
   catgactcag gatatgaagt tggaggagga gatgcagaat tccgacatga ctcaggatat 1620
   gaagttggag gaggagatgc agaattccga catgactcag gatatgaagt tggaggagga 1680
   gatgcagaat tccgacatga ctcaggatat gaagttggag gaggagatgc agaattccga 1740
   catgactcag gatatgaagt tggaggagga gatgcagaat tccgacatga ctcaggatat 1800
   gaagttggag gaggagatgc agaattccga catgactcag gatatgaagt tggaggagga 1860
   gatgcagaat tccgacatga ctcaggatat gaagttggag gaggaaacga tttcgagact 1920
   ggccagaaca ccaggttcac tcccgtgggt gtcgttcaag acggcggagg aggagatgca 1980
   gaattccgac atgactcagg aggaggagga gatgcagaat tccgacatga ctcaggagga 2040
   ggaggagatg cagaattccg acatgactca ggaggaggag gagatgcaga attccgacat 2100
   gactcaggag gaggaggaga tgcagaattc cgacatgact caggaggagg aggagatgca 2160
   gaattccgac atgactcagg aggaggagga gatgcagaat tccgacatga ctcaggagga 2220
   ggaggagatg cagaattccg acatgactca ggaggaggag gagatgcaga attccgacat 2280
   gactcaggag gaggaggaga tgcagaattc cgacatgact caggaggagg aggagatgca 2340
   gaattccgac atgactcagg aggaggagga gatgcagaat tccgacatga ctcaggagga 2400
   ggaggagatg cagaattccg acatgactca ggaggaggag gagatgcaga attccgacat 2460
   gactcaggag gaggaggaga tgcagaattc cgacatgact caggaggagg aggagatgca 2520
   gaattccgac atgactcagg aggaggagga gatgcagaat tccgacatga ctcaggagga 2580
   ggaggagatg cagaattccg acatgactca ggaggaggag gagatgcaga attccgacat 2640
   gactcaggag gaggaggaga tgcagaattc cgacatgact caggaggagg aggagatgca 2700
   gaattccgac atgactcagg aggaggagga gatgcagaat tccgacatga ctcaggagga 2760
   ggaggagatg cagaattccg acatgactca ggaggaggag gagatgcaga attccgacat 2820
   gactcaggag gaggaggaga tgcagaattc cgacatgact caggaggagg aggagatgca 2880
   gaattccgac atgactcagg aggaggagga gatgcagaat tccgacatga ctcaggagga 2940
   ggaggagatg cagaattccg acatgactca ggaggaggag gagatgcaga attccgacat 3000
   gactcaggag gaggaggaga tgcagaattc cgacatgact caggaggagg aggagatgca 3060
   gaattccgac atgactcagg aggaggagga gatgcagaat tccgacatga ctcaggagga 3120
   ggaggagatg cagaattccg acatgactca ggaggaggag gagatgcaga attccgacat 3180
   gactcaggag gaggaggaga tgcagaattc cgacatgact caggaggagg aggagatgca 3240
   gaattccgac atgactcagg aggaggagga gatgcagaat tccgacatga ctcaggagga   3300
   ggaggagatg cagaattccg acatgactca ggaggaggag gagatgcaga attccgacat   3360
   gactcaggag gaggaggaga tgcagaattc cgacatgact caggaggagg aggaagcacc   3420
   actcaccaga acgaacccca gcaatgggtc ctccctgact actcgggcag ggattcccac   3480
   aacgttcact tggctccggc cgtggctcca accttccctg gagagcagtt gctgttcttc   3540
   agatccacta tgccaggctg ctctggatac ccgaacatga acctcgactg tctcttgcct   3600
   caggagtggg tgcaacactt ctaccaggaa tctgccccag ctcaaagcga cgtcgctctg   3660
   ctccgtttcg ttaaccccga taccggtcgc gtgctcttcg agtgtaagtt gcacaagtct   3720
   ggttacgtca ctgttgccca cacaggccag cacgacctgg tcatccctcc caacggctac   3780
   ttccgcttcg atagctgggt caaccagttc tacacactcg ccccgatggg aaacggagcc   3840
   ggtcgtcgca gagccttgta a                                             3861
<210> 129
   <211> 1656
   <212> DNA
   <213> artificial sequence
<400> 129
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catggaggag gaacacaaaa ctacaccatg aacttggcct cgcagaactg gaacaactac 300
   gatccaaccg aggaaatccc cgctcctttg ggaactcccg acttcgtggg acgtatccaa 360
   ggtgtcctga cacagactac acgtcgcgac ggctctactc gcggacacaa ggccactgtg 420
   tcgacaggtt ccgtccactt cacccctaag ctgggctctg tgcaattcag cacagacacc 480
   tcaaacggag gaggagatgc agaattccga catgactcag gaggaggagg agatgcagaa 540
   ttccgacatg actcaggagg aggaggagat gcagaattcc gacatgactc aggaggagga 600
   ggagatttcg agactggcca gaacaccagg ttcactcccg tgggtgtcgt tcaagacggc 660
   agcggaggag gagatgcaga attccgacat gactcaggat atgaagttca tcatcaagga 720
   ggaggagatg cagaattccg acatgactca ggatatgaag ttcatcatca aggaggagga 780
   gatgcagaat tccgacatga ctcaggatat gaagttcatc atcaaggagg aggagatgca 840
   gaattccgac atgactcagg atatgaagtt catcatcaag gaggaggaga tgcagaattc 900
   cgacatgact caggatatga agttcatcat caaggaggag gagatgcaga attccgacat 960
   gactcaggat atgaagttca tcatcaagga ggaggagatg cagaattccg acatgactca 1020
   ggatatgaag ttcatcatca aggaggagga gatgcagaat tccgacatga ctcaggatat 1080
   gaagttcatc atcaaggagg aggagatgca gaattccgac atgactcagg atatgaagtt 1140
   catcatcaag gaggaggaga tgcagaattc cgacatgact caggatatga agttcatcat 1200
   caaggaggag gaaccactca ccagaacgaa ccccagcaat gggtcctccc tgactactcg 1260
   ggcagggatt cccacaacgt tcacttggct ccggccgtgg ctccaacctt ccctggagag 1320
   cagttgctgt tcttcagatc cactatgcca ggctgctctg gatacccgaa catgaacctc 1380
   gactgtctct tgcctcagga gtgggtgcaa cacttctacc aggaatctgc cccagctcaa 1440
   agcgacgtcg ctctgctccg tttcgttaac cccgataccg gtcgcgtgct cttcgagtgt 1500
   aagttgcaca agtctggtta cgtcactgtt gcccacacag gccagcacga cctggtcatc 1560
   cctcccaacg gctacttccg cttcgatagc tgggtcaacc agttctacac actcgccccg 1620
   atgggaaacg gagccggtcg tcgcagagcc ttgtaa 1656
<210> 130
   <211> 3681
   <212> DNA
   <213> artificial sequence
<400> 130
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaggaggagg agatgcagaa     240
   ttccgacatg gaggaggaga tgcagaattc cgacatggag gaggagatgc agaattccga     300
   catggaggag gagatgcaga attccgacat ggaggaggag atgcagaatt ccgacatgga     360
   ggaggagatg cagaattccg acatggagga ggagatgcag aattccgaca tggaggagga     420
   gatgcagaat tccgacatgg aggaggagat gcagaattcc gacatggagg aggagatgca     480
   gaattccgac atggaggagg aaactacacc atgaacttgg cctcgcagaa ctggaacaac     540
   tacgatccaa ccgaggaaat ccccgctcct ttgggaactc ccgacttcgt gggacgtatc     600
   caaggtgtcc tgacacagac tacacgtcgc gacggctcta ctcgcggaca caaggccact     660
   gtgtcgacag gttccgtcca cttcacccct aagctgggct ctgtgcaatt cagcacagac     720
   acctcaggag gaggagatgc agaattccga catgactcag gatatgaagt tggaggagga     780
   gatgcagaat tccgacatga ctcaggatat gaagttggag gaggagatgc agaattccga     840
   catgactcag gatatgaagt tggaggagga gatgcagaat tccgacatga ctcaggatat     900
   gaagttggag gaggagatgc agaattccga catgactcag gatatgaagt tggaggagga     960
   gatgcagaat tccgacatga ctcaggatat gaagttggag gaggagatgc agaattccga    1020
   catgactcag gatatgaagt tggaggagga gatgcagaat tccgacatga ctcaggatat 1080
   gaagttggag gaggagatgc agaattccga catgactcag gatatgaagt tggaggagga 1140
   gatgcagaat tccgacatga ctcaggatat gaagttggag gaggagatgc agaattccga 1200
   catgactcag gatatgaagt tggaggagga gatgcagaat tccgacatga ctcaggatat 1260
   gaagttggag gaggagatgc agaattccga catgactcag gatatgaagt tggaggagga 1320
   gatgcagaat tccgacatga ctcaggatat gaagttggag gaggagatgc agaattccga 1380
   catgactcag gatatgaagt tggaggagga gatgcagaat tccgacatga ctcaggatat 1440
   gaagttggag gaggagatgc agaattccga catgactcag gatatgaagt tggaggagga 1500
   gatgcagaat tccgacatga ctcaggatat gaagttggag gaggagatgc agaattccga 1560
   catgactcag gatatgaagt tggaggagga gatgcagaat tccgacatga ctcaggatat 1620
   gaagttggag gaggagatgc agaattccga catgactcag gatatgaagt tggaggagga 1680
   gatgcagaat tccgacatga ctcaggatat gaagttggag gaggagatgc agaattccga 1740
   catgactcag gatatgaagt tggaggagga gatgcagaat tccgacatga ctcaggatat 1800
   gaagttggag gaggagatgc agaattccga catgactcag gatatgaagt tggaggagga 1860
   gatgcagaat tccgacatga ctcaggatat gaagttggag gaggagatgc agaattccga 1920
   catgactcag gatatgaagt tggaggagga gatgcagaat tccgacatga ctcaggatat 1980
   gaagttggag gaggagatgc agaattccga catgactcag gatatgaagt tggaggagga 2040
   gatgcagaat tccgacatga ctcaggatat gaagttggag gaggaaacga tttcgagact 2100
   ggccagaaca ccaggttcac tcccgtgggt gtcgttcaag acggcagcac cggaggagga 2160
   gatgcagaat tccgacatga ctcaggatat gaagttcatc atcaaggagg aggagatgca 2220
   gaattccgac atgactcagg atatgaagtt catcatcaag gaggaggaga tgcagaattc 2280
   cgacatgact caggatatga agttcatcat caaggaggag gagatgcaga attccgacat 2340
   gactcaggat atgaagttca tcatcaagga ggaggagatg cagaattccg acatgactca 2400
   ggatatgaag ttcatcatca aggaggagga gatgcagaat tccgacatga ctcaggatat 2460
   gaagttcatc atcaaggagg aggagatgca gaattccgac atgactcagg atatgaagtt 2520
   catcatcaag gaggaggaga tgcagaattc cgacatgact caggatatga agttcatcat 2580
   caaggaggag gagatgcaga attccgacat gactcaggat atgaagttca tcatcaagga 2640
   ggaggagatg cagaattccg acatgactca ggatatgaag ttcatcatca aggaggagga 2700
   gatgcagaat tccgacatga ctcaggatat gaagttcatc atcaaggagg aggagatgca 2760
   gaattccgac atgactcagg atatgaagtt catcatcaag gaggaggaga tgcagaattc 2820
   cgacatgact caggatatga agttcatcat caaggaggag gagatgcaga attccgacat 2880
   gactcaggat atgaagttca tcatcaagga ggaggagatg cagaattccg acatgactca 2940
   ggatatgaag ttcatcatca aggaggagga gatgcagaat tccgacatga ctcaggatat 3000
   gaagttcatc atcaaggagg aggagatgca gaattccgac atgactcagg atatgaagtt 3060
   catcatcaag gaggaggaga tgcagaattc cgacatgact caggatatga agttcatcat 3120
   caaggaggag gagatgcaga attccgacat gactcaggat atgaagttca tcatcaagga 3180
   ggaggagatg cagaattccg acatgactca ggatatgaag ttcatcatca aggaggagga 3240
   actcaccaga acgaacccca gcaatgggtc ctccctgact actcgggcag ggattcccac 3300
   aacgttcact tggctccggc cgtggctcca accttccctg gagagcagtt gctgttcttc 3360
   agatccacta tgccaggctg ctctggatac ccgaacatga acctcgactg tctcttgcct 3420
   caggagtggg tgcaacactt ctaccaggaa tctgccccag ctcaaagcga cgtcgctctg 3480
   ctccgtttcg ttaaccccga taccggtcgc gtgctcttcg agtgtaagtt gcacaagtct 3540
   ggttacgtca ctgttgccca cacaggccag cacgacctgg tcatccctcc caacggctac 3600
   ttccgcttcg atagctgggt caaccagttc tacacactcg ccccgatggg aaacggagcc 3660
   ggtcgtcgca gagccttgta a 3681
<210> 131
   <211> 1791
   <212> DNA
   <213> artificial sequence
<400> 131
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacag gaggaggaga tgcagaattc 240
   cgacatggag gaggagatgc agaattccga catggaggag gagatgcaga attccgacat 300
   ggaggaggag atgcagaatt ccgacatgga ggaggagatg cagaattccg acatggagga 360
   ggagatgcag aattccgaca tggaggagga gatgcagaat tccgacatgg aggaggagat 420
   gcagaattcc gacatggagg aggagatgca gaattccgac atggaggagg agatgcagaa 480
   ttccgacatg gaggaggaca aaactacacc atgaacttgg cctcgcagaa ctggaacaac 540
   tacgatccaa ccgaggaaat ccccgctcct ttgggaactc ccgacttcgt gggacgtatc 600
   caaggtgtcc tgacacagac tacacgtcgc gacggctcta ctcgcggaca caaggccact 660
   gtgtcgacag gttccgtcca cttcacccct aagctgggct ctgtgcaatt cagcacagac 720
   acctcaaacg atggaggagg agatgcagaa ttccgacatg gaggaggaga tgcagaattc 780
   cgacatggag gaggagatgc agaattccga catggaggag gagatgcaga attccgacat 840
   ggaggaggag atgcagaatt ccgacatgga ggaggagatg cagaattccg acatggagga 900
   ggagatgcag aattccgaca tggaggagga gatgcagaat tccgacatgg aggaggagat 960
   gcagaattcc gacatggagg aggagatgca gaattccgac atggaggagg attcgagact 1020
   ggccagaaca ccaggttcac tcccgtgggt gtcgttcaag acggcggagg aggagatgca 1080
   gaattccgac atggaggagg agatgcagaa ttccgacatg gaggaggaga tgcagaattc 1140
   cgacatggag gaggagatgc agaattccga catggaggag gagatgcaga attccgacat 1200
   ggaggaggag atgcagaatt ccgacatgga ggaggagatg cagaattccg acatggagga 1260
   ggagatgcag aattccgaca tggaggagga gatgcagaat tccgacatgg aggaggagat 1320
   gcagaattcc gacatggagg aggaagcacc actcaccaga acgaacccca gcaatgggtc 1380
   ctccctgact actcgggcag ggattcccac aacgttcact tggctccggc cgtggctcca 1440
   accttccctg gagagcagtt gctgttcttc agatccacta tgccaggctg ctctggatac 1500
   ccgaacatga acctcgactg tctcttgcct caggagtggg tgcaacactt ctaccaggaa 1560
   tctgccccag ctcaaagcga cgtcgctctg ctccgtttcg ttaaccccga taccggtcgc 1620
   gtgctcttcg agtgtaagtt gcacaagtct ggttacgtca ctgttgccca cacaggccag 1680
   cacgacctgg tcatccctcc caacggctac ttccgcttcg atagctgggt caaccagttc 1740
   tacacactcg ccccgatggg aaacggagcc ggtcgtcgca gagccttgta a 1791
<210> 132
   <211> 1062
   <212> DNA
   <213> artificial sequence
<400> 132
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catggaggag gaacacaaaa ctacaccatg aacttggcct cgcagaactg gaacaactac     300
   gatccaaccg aggaaatccc cgctcctttg ggaactcccg acttcgtggg acgtatccaa     360
   ggtgtcctga cacagactac acgtcgcgac ggctctactc gcggacacaa ggccactgtg     420
   tcgacaggtt ccgtccactt cacccctaag ctgggctctg tgcaattcag cacagacacc     480
   tcaggaggag gagatgcaga attccgacat ggaggaggaa acgatttcga gactggccag     540
   aacaccaggt tcactcccgt gggtgtcgtt caagacggcg gaggaggaga tgcagaattc     600
   cgacatggag gaggaagcac cactcaccag aacgaacccc agcaatgggt cctccctgac     660
   tactcgggca gggattccca caacgttcac ttggctccgg ccgtggctcc aaccttccct     720
   ggagagcagt tgctgttctt cagatccact atgccaggct gctctggata cccgaacatg     780
   aacctcgact gtctcttgcc tcaggagtgg gtgcaacact tctaccagga atctgcccca     840
   gctcaaagcg acgtcgctct gctccgtttc gttaaccccg ataccggtcg cgtgctcttc     900
   gagtgtaagt tgcacaagtc tggttacgtc actgttgccc acacaggcca gcacgacctg     960
   gtcatccctc ccaacggcta cttccgcttc gatagctggg tcaaccagtt ctacacactc    1020
   gccccgatgg gaaacggagc cggtcgtcgc agagccttgt aa                       1062
<210> 133
   <211> 1224
   <212> DNA
   <213> artificial sequence
<400> 133
   atgaagccct tctcggtccc tatcctgaca gttgaggaaa tgaccaactc tagattccca     60
   atcccgctgg aaaagctctt caccggaccc tcctctgctt tcgttgtgca gcctcaaaac    120
   ggtcgttgca ccactgatgg tgtcctgctc ggtaccaccc agctctcacc tgtgaacatc    180
   tgtactttcc gcggcgacgt cacacacatc gctggaggag gaggagatgc agaattccga    240
   catggaggag gagatgcaga attccgacat ggaggaggag atgcagaatt ccgacatgga    300
   ggaggaacac aaaactacac catgaacttg gcctcgcaga actggaacaa ctacgatcca    360
   accgaggaaa tccccgctcc tttgggaact cccgacttcg tgggacgtat ccaaggtgtc    420
   ctgacacaga ctacacgtcg cgacggctct actcgcggac acaaggccac tgtgtcgaca    480
   ggttccgtcc acttcacccc taagctgggc tctgtgcaat tcagcacaga cacctcagga    540
   ggaggagatg cagaattccg acatggagga ggagatgcag aattccgaca tggaggagga    600
   gatgcagaat tccgacatgg aggaggaaac gatttcgaga ctggccagaa caccaggttc    660
   actcccgtgg gtgtcgttca agacggcgga ggaggagatg cagaattccg acatggagga    720
   ggagatgcag aattccgaca tggaggagga gatgcagaat tccgacatgg aggaggaagc    780
   accactcacc agaacgaacc ccagcaatgg gtcctccctg actactcggg cagggattcc    840
   cacaacgttc acttggctcc ggccgtggct ccaaccttcc ctggagagca gttgctgttc    900
   ttcagatcca ctatgccagg ctgctctgga tacccgaaca tgaacctcga ctgtctcttg    960
   cctcaggagt gggtgcaaca cttctaccag gaatctgccc cagctcaaag cgacgtcgct   1020
   ctgctccgtt tcgttaaccc cgataccggt cgcgtgctct tcgagtgtaa gttgcacaag   1080
   tctggttacg tcactgttgc ccacacaggc cagcacgacc tggtcatccc tcccaacggc   1140
   tacttccgct tcgatagctg ggtcaaccag ttctacacac tcgccccgat gggaaacgga   1200
   gccggtcgtc gcagagcctt gtaa                                          1224
<210> 134
   <211> 1791
   <212> DNA
   <213> artificial sequence
<400> 134
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaggag gaggagatgc agaattccga     240
   catggaggag gagatgcaga attccgacat ggaggaggag atgcagaatt ccgacatgga     300
   ggaggagatg cagaattccg acatggagga ggagatgcag aattccgaca tggaggagga     360
   gatgcagaat tccgacatgg aggaggagat gcagaattcc gacatggagg aggagatgca     420
   gaattccgac atggaggagg agatgcagaa ttccgacatg gaggaggaga tgcagaattc     480
   cgacatggag gaggaacaca aaactacacc atgaacttgg cctcgcagaa ctggaacaac     540
   tacgatccaa ccgaggaaat ccccgctcct ttgggaactc ccgacttcgt gggacgtatc     600
   caaggtgtcc tgacacagac tacacgtcgc gacggctcta ctcgcggaca caaggccact     660
   gtgtcgacag gttccgtcca cttcacccct aagctgggct ctgtgcaatt cagcacagac     720
   acctcaggag gaggagatgc agaattccga catggaggag gagatgcaga attccgacat     780
   ggaggaggag atgcagaatt ccgacatgga ggaggagatg cagaattccg acatggagga     840
   ggagatgcag aattccgaca tggaggagga gatgcagaat tccgacatgg aggaggagat     900
   gcagaattcc gacatggagg aggagatgca gaattccgac atggaggagg agatgcagaa     960
   ttccgacatg gaggaggaga tgcagaattc cgacatggag gaggaaacga tttcgagact    1020
   ggccagaaca ccaggttcac tcccgtgggt gtcgttcaag acggcggagg aggagatgca    1080
   gaattccgac atggaggagg agatgcagaa ttccgacatg gaggaggaga tgcagaattc    1140
   cgacatggag gaggagatgc agaattccga catggaggag gagatgcaga attccgacat    1200
   ggaggaggag atgcagaatt ccgacatgga ggaggagatg cagaattccg acatggagga    1260
   ggagatgcag aattccgaca tggaggagga gatgcagaat tccgacatgg aggaggagat    1320
   gcagaattcc gacatggagg aggaagcacc actcaccaga acgaacccca gcaatgggtc    1380
   ctccctgact actcgggcag ggattcccac aacgttcact tggctccggc cgtggctcca    1440
   accttccctg gagagcagtt gctgttcttc agatccacta tgccaggctg ctctggatac    1500
   ccgaacatga acctcgactg tctcttgcct caggagtggg tgcaacactt ctaccaggaa    1560
   tctgccccag ctcaaagcga cgtcgctctg ctccgtttcg ttaaccccga taccggtcgc    1620
   gtgctcttcg agtgtaagtt gcacaagtct ggttacgtca ctgttgccca cacaggccag    1680
   cacgacctgg tcatccctcc caacggctac ttccgcttcg atagctgggt caaccagttc    1740
   tacacactcg ccccgatggg aaacggagcc ggtcgtcgca gagccttgta a             1791
<210> 135
   <211> 4221
   <212> DNA
   <213> artificial sequence
<400> 135
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaggag gaggagatgc agaattccga 240
   catggaggag gagatgcaga attccgacat ggaggaggag atgcagaatt ccgacatgga 300
   ggaggagatg cagaattccg acatggagga ggagatgcag aattccgaca tggaggagga 360
   gatgcagaat tccgacatgg aggaggagat gcagaattcc gacatggagg aggagatgca 420
   gaattccgac atggaggagg agatgcagaa ttccgacatg gaggaggaga tgcagaattc 480
   cgacatggag gaggagatgc agaattccga catggaggag gagatgcaga attccgacat 540
   ggaggaggag atgcagaatt ccgacatgga ggaggagatg cagaattccg acatggagga 600
   ggagatgcag aattccgaca tggaggagga gatgcagaat tccgacatgg aggaggagat 660
   gcagaattcc gacatggagg aggagatgca gaattccgac atggaggagg agatgcagaa 720
   ttccgacatg gaggaggaga tgcagaattc cgacatggag gaggagatgc agaattccga 780
   catggaggag gagatgcaga attccgacat ggaggaggag atgcagaatt ccgacatgga 840
   ggaggagatg cagaattccg acatggagga ggagatgcag aattccgaca tggaggagga 900
   gatgcagaat tccgacatgg aggaggagat gcagaattcc gacatggagg aggagatgca 960
   gaattccgac atggaggagg agatgcagaa ttccgacatg gaggaggaga tgcagaattc 1020
   cgacatggag gaggagatgc agaattccga catggaggag gagatgcaga attccgacat 1080
   ggaggaggag atgcagaatt ccgacatgga ggaggagatg cagaattccg acatggagga 1140
   ggagatgcag aattccgaca tggaggagga gatgcagaat tccgacatgg aggaggagat 1200
   gcagaattcc gacatggagg aggagatgca gaattccgac atggaggagg agatgcagaa 1260
   ttccgacatg gaggaggaga tgcagaattc cgacatggag gaggaacaca aaactacacc 1320
   atgaacttgg cctcgcagaa ctggaacaac tacgatccaa ccgaggaaat ccccgctcct 1380
   ttgggaactc ccgacttcgt gggacgtatc caaggtgtcc tgacacagac tacacgtcgc 1440
   gacggctcta ctcgcggaca caaggccact gtgtcgacag gttccgtcca cttcacccct 1500
   aagctgggct ctgtgcaatt cagcacagac acctcaggag gaggagatgc agaattccga 1560
   catggaggag gagatgcaga attccgacat ggaggaggag atgcagaatt ccgacatgga 1620
   ggaggagatg cagaattccg acatggagga ggagatgcag aattccgaca tggaggagga 1680
   gatgcagaat tccgacatgg aggaggagat gcagaattcc gacatggagg aggagatgca 1740
   gaattccgac atggaggagg agatgcagaa ttccgacatg gaggaggaga tgcagaattc 1800
   cgacatggag gaggagatgc agaattccga catggaggag gagatgcaga attccgacat 1860
   ggaggaggag atgcagaatt ccgacatgga ggaggagatg cagaattccg acatggagga 1920
   ggagatgcag aattccgaca tggaggagga gatgcagaat tccgacatgg aggaggagat 1980
   gcagaattcc gacatggagg aggagatgca gaattccgac atggaggagg agatgcagaa 2040
   ttccgacatg gaggaggaga tgcagaattc cgacatggag gaggagatgc agaattccga 2100
   catggaggag gagatgcaga attccgacat ggaggaggag atgcagaatt ccgacatgga 2160
   ggaggagatg cagaattccg acatggagga ggagatgcag aattccgaca tggaggagga 2220
   gatgcagaat tccgacatgg aggaggagat gcagaattcc gacatggagg aggagatgca 2280
   gaattccgac atggaggagg agatgcagaa ttccgacatg gaggaggaga tgcagaattc 2340
   cgacatggag gaggagatgc agaattccga catggaggag gagatgcaga attccgacat 2400
   ggaggaggag atgcagaatt ccgacatgga ggaggagatg cagaattccg acatggagga 2460
   ggagatgcag aattccgaca tggaggagga gatgcagaat tccgacatgg aggaggagat 2520
   gcagaattcc gacatggagg aggagatgca gaattccgac atggaggagg agatgcagaa 2580
   ttccgacatg gaggaggaga tgcagaattc cgacatggag gaggaaacga tttcgagact 2640
   ggccagaaca ccaggttcac tcccgtgggt gtcgttcaag acggcggagg aggagatgca 2700
   gaattccgac atggaggagg agatgcagaa ttccgacatg gaggaggaga tgcagaattc 2760
   cgacatggag gaggagatgc agaattccga catggaggag gagatgcaga attccgacat 2820
   ggaggaggag atgcagaatt ccgacatgga ggaggagatg cagaattccg acatggagga 2880
   ggagatgcag aattccgaca tggaggagga gatgcagaat tccgacatgg aggaggagat 2940
   gcagaattcc gacatggagg aggagatgca gaattccgac atggaggagg agatgcagaa 3000
   ttccgacatg gaggaggaga tgcagaattc cgacatggag gaggagatgc agaattccga 3060
   catggaggag gagatgcaga attccgacat ggaggaggag atgcagaatt ccgacatgga 3120
   ggaggagatg cagaattccg acatggagga ggagatgcag aattccgaca tggaggagga 3180
   gatgcagaat tccgacatgg aggaggagat gcagaattcc gacatggagg aggagatgca 3240
   gaattccgac atggaggagg agatgcagaa ttccgacatg gaggaggaga tgcagaattc 3300
   cgacatggag gaggagatgc agaattccga catggaggag gagatgcaga attccgacat 3360
   ggaggaggag atgcagaatt ccgacatgga ggaggagatg cagaattccg acatggagga 3420
   ggagatgcag aattccgaca tggaggagga gatgcagaat tccgacatgg aggaggagat 3480
   gcagaattcc gacatggagg aggagatgca gaattccgac atggaggagg agatgcagaa 3540
   ttccgacatg gaggaggaga tgcagaattc cgacatggag gaggagatgc agaattccga 3600
   catggaggag gagatgcaga attccgacat ggaggaggag atgcagaatt ccgacatgga 3660
   ggaggagatg cagaattccg acatggagga ggagatgcag aattccgaca tggaggagga 3720
   gatgcagaat tccgacatgg aggaggagat gcagaattcc gacatggagg aggaagcacc 3780
   actcaccaga acgaacccca gcaatgggtc ctccctgact actcgggcag ggattcccac 3840
   aacgttcact tggctccggc cgtggctcca accttccctg gagagcagtt gctgttcttc 3900
   agatccacta tgccaggctg ctctggatac ccgaacatga acctcgactg tctcttgcct 3960
   caggagtggg tgcaacactt ctaccaggaa tctgccccag ctcaaagcga cgtcgctctg 4020
   ctccgtttcg ttaaccccga taccggtcgc gtgctcttcg agtgtaagtt gcacaagtct 4080
   ggttacgtca ctgttgccca cacaggccag cacgacctgg tcatccctcc caacggctac 4140
   ttccgcttcg atagctgggt caaccagttc tacacactcg ccccgatggg aaacggagcc 4200
   ggtcgtcgca gagccttgta a 4221
<210> 136
   <211> 1134
   <212> DNA
   <213> artificial sequence
<400> 136
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagat gcagaattcc gacatgatgc agaattccga     480
   catgatgcag aattccgaca tgatgcagaa ttccgacatg atgcagaatt ccgacatgat     540
   gcagaattcc gacatgatgc agaattccga catgatgcag aattccgaca tgatgcagaa     600
   ttccgacatg atgcagaatt ccgacataac gatttcgaga ctggccagaa caccaggttc     660
   actcccgtgg gtgtcgttca agacggcagc accactcacc agaacgaacc ccagcaatgg     720
   gtcctccctg actactcggg cagggattcc cacaacgttc acttggctcc ggccgtggct     780
   ccaaccttcc ctggagagca gttgctgttc ttcagatcca ctatgccagg ctgctctgga     840
   tacccgaaca tgaacctcga ctgtctcttg cctcaggagt gggtgcaaca cttctaccag     900
   gaatctgccc cagctcaaag cgacgtcgct ctgctccgtt tcgttaaccc cgataccggt     960
   cgcgtgctct tcgagtgtaa gttgcacaag tctggttacg tcactgttgc ccacacaggc    1020
   cagcacgacc tggtcatccc tcccaacggc tacttccgct tcgatagctg ggtcaaccag    1080
   ttctacacac tcgccccgat gggaaacgga gccggtcgtc gcagagcctt gtaa          1134
<210> 137
   <211> 1167
   <212> DNA
   <213> artificial sequence
<400> 137
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagga gatgcagaat tccgacatgg agatgcagaa     480
   ttccgacatg gagatgcaga attccgacat ggagatgcag aattccgaca tggagatgca     540
   gaattccgac atggagatgc agaattccga catggagatg cagaattccg acatggagat     600
   gcagaattcc gacatggaga tgcagaattc cgacatggag atgcagaatt ccgacatgga     660
   aacgatttcg agactggcca gaacaccagg ttcactcccg tgggtgtcgt tcaagacggc     720
   agcaccactc accagaacga accccagcaa tgggtcctcc ctgactactc gggcagggat     780
   tcccacaacg ttcacttggc tccggccgtg gctccaacct tccctggaga gcagttgctg     840
   ttcttcagat ccactatgcc aggctgctct ggatacccga acatgaacct cgactgtctc     900
   ttgcctcagg agtgggtgca acacttctac caggaatctg ccccagctca aagcgacgtc     960
   gctctgctcc gtttcgttaa ccccgatacc ggtcgcgtgc tcttcgagtg taagttgcac    1020
   aagtctggtt acgtcactgt tgcccacaca ggccagcacg acctggtcat ccctcccaac    1080
   ggctacttcc gcttcgatag ctgggtcaac cagttctaca cactcgcccc gatgggaaac    1140
   ggagccggtc gtcgcagagc cttgtaa                                        1167
<210> 138
   <211> 1299
   <212> DNA
   <213> artificial sequence
<400> 138
   atgaagccct tctcggtccc tatcctgaca gttgaggaaa tgaccaactc tagattccca      60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcagga ggaggaggag gagatgcaga attccgacat 480
   ggaggaggag gaggagatgc agaattccga catggaggag gaggaggaga tgcagaattc 540
   cgacatggag gaggaggagg agatgcagaa ttccgacatg gaggaggagg aggagatgca 600
   gaattccgac atggaggagg aggaggagat gcagaattcc gacatggagg aggaggagga 660
   gatgcagaat tccgacatgg aggaggagga ggagatgcag aattccgaca tggaggagga 720
   ggaggagatg cagaattccg acatggagga ggaggaggag atgcagaatt ccgacatgga 780
   ggaggaggag gaaacgattt cgagactggc cagaacacca ggttcactcc cgtgggtgtc 840
   gttcaagacg gcagcaccac tcaccagaac gaaccccagc aatgggtcct ccctgactac 900
   tcgggcaggg attcccacaa cgttcacttg gctccggccg tggctccaac cttccctgga 960
   gagcagttgc tgttcttcag atccactatg ccaggctgct ctggataccc gaacatgaac 1020
   ctcgactgtc tcttgcctca ggagtgggtg caacacttct accaggaatc tgccccagct 1080
   caaagcgacg tcgctctgct ccgtttcgtt aaccccgata ccggtcgcgt gctcttcgag 1140
   tgtaagttgc acaagtctgg ttacgtcact gttgcccaca caggccagca cgacctggtc 1200
   atccctccca acggctactt ccgcttcgat agctgggtca accagttcta cacactcgcc 1260
   ccgatgggaa acggagccgg tcgtcgcaga gccttgtaa 1299
<210> 139
   <211> 1464
   <212> DNA
   <213> artificial sequence
<400> 139
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga cacctcagga ggaggaggag gaggaggagg aggaggagat     480
   gcagaattcc gacatggagg aggaggagga ggaggaggag gaggagatgc agaattccga     540
   catggaggag gaggaggagg aggaggagga ggagatgcag aattccgaca tggaggagga     600
   ggaggaggag gaggaggagg agatgcagaa ttccgacatg gaggaggagg aggaggagga     660
   ggaggaggag atgcagaatt ccgacatgga ggaggaggag gaggaggagg aggaggagat     720
   gcagaattcc gacatggagg aggaggagga ggaggaggag gaggagatgc agaattccga     780
   catggaggag gaggaggagg aggaggagga ggagatgcag aattccgaca tggaggagga     840
   ggaggaggag gaggaggagg agatgcagaa ttccgacatg gaggaggagg aggaggagga     900
   ggaggaggag atgcagaatt ccgacatgga ggaggaggag gaggaggagg aggaggaaac     960
   gatttcgaga ctggccagaa caccaggttc actcccgtgg gtgtcgttca agacggcagc    1020
   accactcacc agaacgaacc ccagcaatgg gtcctccctg actactcggg cagggattcc    1080
   cacaacgttc acttggctcc ggccgtggct ccaaccttcc ctggagagca gttgctgttc    1140
   ttcagatcca ctatgccagg ctgctctgga tacccgaaca tgaacctcga ctgtctcttg    1200
   cctcaggagt gggtgcaaca cttctaccag gaatctgccc cagctcaaag cgacgtcgct    1260
   ctgctccgtt tcgttaaccc cgataccggt cgcgtgctct tcgagtgtaa gttgcacaag    1320
   tctggttacg tcactgttgc ccacacaggc cagcacgacc tggtcatccc tcccaacggc    1380
   tacttccgct tcgatagctg ggtcaaccag ttctacacac tcgccccgat gggaaacgga    1440
   gccggtcgtc gcagagcctt gtaa                                           1464
<210> 140
   <211> 1233
   <212> DNA
   <213> artificial sequence
<400> 140
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggtaccaccc agctctcacc tgtgaacatc 180
   tgtactttcc gcggcgacgt cacacacatc gctggaacac aaaactacac catgaacttg 240
   gcctcgcaga actggaacaa ctacgatcca accgaggaaa tccccgctcc tttgggaact 300
   cccgacttcg tgggacgtat ccaaggtgtc ctgacacaga ctacacgtcg cgacggctct 360
   actcgcggac acaaggccac tgtgtcgaca ggttccgtcc acttcacccc taagctgggc 420
   tctgtgcaat tcagcacaga cacctcaggc ggcggcgatg cagaattccg acatggcggc 480
   ggcgatgcag aattccgaca tggcggcggc gatgcagaat tccgacatgg cggcggcgat 540
   gcagaattcc gacatggcgg cggcgatgca gaattccgac atggcggcgg cgatgcagaa 600
   ttccgacatg gcggcggcga tgcagaattc cgacatggcg gcggcgatgc agaattccga 660
   catggcggcg gcgatgcaga attccgacat ggcggcggcg atgcagaatt ccgacatggc 720
   ggcggcaacg atttcgagac tggccagaac accaggttca ctcccgtggg tgtcgttcaa 780
   gacggcagca ccactcacca gaacgaaccc cagcaatggg tcctccctga ctactcgggc 840
   agggattccc acaacgttca cttggctccg gccgtggctc caaccttccc tggagagcag 900
   ttgctgttct tcagatccac tatgccaggc tgctctggat acccgaacat gaacctcgac 960
   tgtctcttgc ctcaggagtg ggtgcaacac ttctaccagg aatctgcccc agctcaaagc 1020
   gacgtcgctc tgctccgttt cgttaacccc gataccggtc gcgtgctctt cgagtgtaag 1080
   ttgcacaagt ctggttacgt cactgttgcc cacacaggcc agcacgacct ggtcatccct 1140
   cccaacggct acttccgctt cgatagctgg gtcaaccagt tctacacact cgccccgatg 1200
   ggaaacggag ccggtcgtcg cagagccttg taa 1233
<210> 141
   <211> 1062
   <212> DNA
   <213> artificial sequence
<400> 141
   atgaagccct tctcggtccc tatcctgaca gttgaggaaa tgaccaactc tagattccca      60
   atcccgctgg aaaagctctt caccggaccc tcctctgctt tcgttgtgca gcctcaaaac     120
   ggtcgttgca ccactgatgg tgtcctgctc ggtaccaccc agctctcacc tgtgaacatc     180
   tgtactttcc gcggcgacgt cacacacatc gctggaacac aaaactacac catgaacttg     240
   gcctcgcaga actggaacaa ctacgatcca accgaggaaa tccccgctcc tttgggaact     300
   cccgacttcg tgggacgtat ccaaggtgtc ctgacacaga ctacacgtcg cgacggctct     360
   actcgcggac acaaggccac tgtgtcgaca ggttccgtcc acttcacccc taagctgggc     420
   tctgtgcaat tcagcacaga caccggagga ggagatgcag aattccgaca tggaggagga     480
   tcaggaggag gagatgcaga attccgacat ggaggaggaa acggaggagg agatgcagaa     540
   ttccgacatg gaggaggaga tttcgagact ggccagaaca ccaggttcac tcccgtgggt     600
   gtcgttcaag acggcagcac cactcaccag aacgaacccc agcaatgggt cctccctgac     660
   tactcgggca gggattccca caacgttcac ttggctccgg ccgtggctcc aaccttccct     720
   ggagagcagt tgctgttctt cagatccact atgccaggct gctctggata cccgaacatg     780
   aacctcgact gtctcttgcc tcaggagtgg gtgcaacact tctaccagga atctgcccca     840
   gctcaaagcg acgtcgctct gctccgtttc gttaaccccg ataccggtcg cgtgctcttc     900
   gagtgtaagt tgcacaagtc tggttacgtc actgttgccc acacaggcca gcacgacctg     960
   gtcatccctc ccaacggcta cttccgcttc gatagctggg tcaaccagtt ctacacactc    1020
   gccccgatgg gaaacggagc cggtcgtcgc agagccttgt aa                       1062
<210> 142
   <211> 1098
   <212> DNA
   <213> artificial sequence
<400> 142
   atgaagccct tctcggtccc tatcctgaca gttgaggaaa tgaccaactc tagattccca 60
   atcccgctgg aaaagctctt caccggaccc tcctctgctt tcgttgtgca gcctcaaaac 120
   ggtcgttgca ccactgatgg tgtcctgctc ggcacaaccc agctctcacc tgtgaacatc 180
   tgtactttcc gcggcgacgt cacacacatc ggaggaggag atgcagaatt ccgacatgga 240
   ggaggagctg gaggaggaga tgcagaattc cgacatggag gaggaggaac acaaaactac 300
   accatgaact tggcctcgca gaactggaac aactacgatc caaccgagga aatccccgct 360
   cctttgggaa ctcccgactt cgtgggacgt atccaaggtg tcctgacaca gactacacgt 420
   cgcgacggct ctactcgcgg acacaaggcc actgtgtcga caggttccgt ccacttcacc 480
   cctaagctgg gctctgtgca attcagcaca gacacctcag gaggaggaga tgcagaattc 540
   cgacatggag gaggaaacgg aggaggagat gcagaattcc gacatggagg aggagatttc 600
   gagactggcc agaacaccag gttcactccc gtgggtgtcg ttcaagacgg cagcaccact 660
   caccagaacg aaccccagca atgggtcctc cctgactact cgggcaggga ttcccacaac 720
   gttcacttgg ctcccgccgt ggctccaacc ttccctggag agcagttgct gttcttcaga 780
   tccactatgc caggctgctc tggatacccg aacatgaacc tcgactgtct cttgcctcag 840
   gagtgggtgc aacacttcta ccaggaatct gccccagctc aaagcgacgt cgctctgctc 900
   cgtttcgtta accccgatac cggtcgcgtg ctcttcgagt gtaagttgca caagtctggt 960
   tacgtcactg ttgcccacac aggccagcac gacctggtca tccctcccaa cggctacttc 1020
   cgcttcgata gctgggtcaa ccagttctac acactcgccc cgatgggaaa cggagccggt 1080
   cgtcgcagag ccttgtaa 1098
<210> 143
   <211> 518
   <212> DNA
   <213> artificial sequence
<400> 143
   ggtaccaccc agctctcacc tgtgaacatc tgtactttcc gcggcgacgt cacacacatc 60
   gctggaggag gaggagatgc agaattccga catggaggag gagatgcaga attccgacat 120
   ggaggaggag atgcagaatt ccgacatgga ggaggagatg cagaattccg acatggagga 180
   ggagatgcag aattccgaca tggaggagga gatgcagaat tccgacatgg aggaggagat 240
   gcagaattcc gacatggagg aggagatgca gaattccgac atggaggagg agatgcagaa 300
   ttccgacatg gaggaggaga tgcagaattc cgacatggag gaggaacaca aaactacacc 360
   atgaacttgg cctcgcagaa ctggaacaac tacgatccaa ccgaggaaat ccccgctcct 420
   ttgggaactc ccgacttcgt gggacgtatc caaggtgtcc tgacacagac tacacgtcgc 480
   gacggctcta ctcgcggaca caaggccact gtgtcgac 518
<210> 144
   <211> 248
   <212> DNA
   <213> artificial sequence
<400> 144
   gtcgacaggt tccgtccact tcacccctaa gctgggctct gtgcaattca gcacagacac 60
   ctcaggagga ggagatgcag aattccgaca tggaggagga aacgatttcg agactggcca 120
   gaacaccagg ttcactcccg tgggtgtcgt tcaagacggc agcaccactc accagaacga 180
   accccagcaa tgggtcctcc ctgactactc gggcagggat tcccacaacg ttcacttggc 240
   tccggccg 248
<210> 145
   <211> 491
   <212> DNA
   <213> artificial sequence
<400> 145
   gtcgacaggt tccgtccact tcacccctaa gctgggctct gtgcaattca gcacagacac 60
   ctcaggagga ggagatgcag aattccgaca tggaggagga gatgcagaat tccgacatgg 120
   aggaggagat gcagaattcc gacatggagg aggagatgca gaattccgac atggaggagg 180
   agatgcagaa ttccgacatg gaggaggaga tgcagaattc cgacatggag gaggagatgc 240
   agaattccga catggaggag gagatgcaga attccgacat ggaggaggag atgcagaatt 300
   ccgacatgga ggaggagatg cagaattccg acatggagga ggaaacgatt tcgagactgg 360
   ccagaacacc aggttcactc ccgtgggtgt cgttcaagac ggcagcacca ctcaccagaa 420
   cgaaccccag caatgggtcc tccctgacta ctcgggcagg gattcccaca acgttcactt 480
   ggctccggcc g 491
<210> 146
   <211> 761
   <212> DNA
   <213> artificial sequence
<400> 146
   gtcgacaggt tccgtccact tcacccctaa gctgggctct gtgcaattca gcacagacac     60
   ctcaaacgat ttcgagactg gccagaacac caggttcact cccgtgggtg tcgttcaaga    120
   cggcggagga ggagatgcag aattccgaca tggaggagga gatgcagaat tccgacatgg    180
   aggaggagat gcagaattcc gacatggagg aggagatgca gaattccgac atggaggagg    240
   agatgcagaa ttccgacatg gaggaggaga tgcagaattc cgacatggag gaggagatgc    300
   agaattccga catggaggag gagatgcaga attccgacat ggaggaggag atgcagaatt    360
   ccgacatgga ggaggagatg cagaattccg acatggagga ggagatgcag aattccgaca    420
   tggaggagga gatgcagaat tccgacatgg aggaggagat gcagaattcc gacatggagg    480
   aggagatgca gaattccgac atggaggagg agatgcagaa ttccgacatg gaggaggaga    540
   tgcagaattc cgacatggag gaggagatgc agaattccga catggaggag gagatgcaga    600
   attccgacat ggaggaggag atgcagaatt ccgacatgga ggaggagatg cagaattccg    660
   acatggagga ggaagcacca ctcaccagaa cgaaccccag caatgggtcc tccctgacta    720
   ctcgggcagg gattcccaca acgttcactt ggctccggcc g                        761
<210> 147
   <211> 446
   <212> DNA
   <213> artificial sequence
<400> 147
   gtcgacaggt tccgtccact tcacccctaa gctgggctct gtgcaattca gcacagacac     60
   ctcaggagga ggagatgcag aattccgaca tgactcagga tatgaagttg gaggaggaga    120
   tgcagaattc cgacatgact caggatatga agttggagga ggagatgcag aattccgaca    180
   tgactcagga tatgaagttg gaggaggaaa cgatttcgag actggccaga acaccaggtt    240
   cactcccgtg ggtgtcgttc aagacggcgg aggaggagat gcagaattcc gacatggagg    300
   aggagatgca gaattccgac atggaggagg agatgcagaa ttccgacatg gaggaggaag    360
   caccactcac cagaacgaac cccagcaatg ggtcctccct gactactcgg gcagggattc    420
   ccacaacgtt cacttggctc cggccg                                         446
<210> 148
   <211> 1273
   <212> DNA
   <213> artificial sequence
<400> 148
   ggtaccaccc agctctcacc tgtgaacatc tgtactttcc gcggcgacgt cacacacatc      60
   gctggaggag gaggagatgc agaattccga catgactcag gatatgaagt tcatcatcaa     120
   ggaggaggag atgcagaatt ccgacatgac tcaggatatg aagttcatca tcaaggagga     180
   ggagatgcag aattccgaca tgactcagga tatgaagttc atcatcaagg aggaggagat     240
   gcagaattcc gacatgactc aggatatgaa gttcatcatc aaggaggagg agatgcagaa     300
   ttccgacatg actcaggata tgaagttcat catcaaggag gaggagatgc agaattccga     360
   catgactcag gatatgaagt tcatcatcaa ggaggaggag atgcagaatt ccgacatgac     420
   tcaggatatg aagttcatca tcaaggagga ggagatgcag aattccgaca tgactcagga     480
   tatgaagttc atcatcaagg aggaggagat gcagaattcc gacatgactc aggatatgaa     540
   gttcatcatc aaggaggagg agatgcagaa ttccgacatg actcaggata tgaagttcat     600
   catcaaggag gaggaacaca aaactacacc atgaacttgg cctcgcagaa ctggaacaac     660
   tacgatccaa ccgaggaaat ccccgctcct ttgggaactc ccgacttcgt gggacgtatc     720
   caaggtgtcc tgacacagac tacacgtcgc gacggctcta ctcgcggaca caaggccact     780
   gtgtcgacag gttccgtcca cttcacccct aagctgggct ctgtgcaatt cagcacagac     840
   acctcaggag gaggagatgc agaattccga catggaggag gagatgcaga attccgacat     900
   ggaggaggag atgcagaatt ccgacatgga ggaggagatg cagaattccg acatggagga     960
   ggagatgcag aattccgaca tggaggagga gatgcagaat tccgacatgg aggaggagat    1020
   gcagaattcc gacatggagg aggagatgca gaattccgac atggaggagg agatgcagaa    1080
   ttccgacatg gaggaggaga tgcagaattc cgacatggag gaggaaacga tttcgagact    1140
   ggccagaaca ccaggttcac tcccgtgggt gtcgttcaag acggcagcac cactcaccag    1200
   aacgaacccc agcaatgggt cctccctgac tactcgggca gggattccca caacgttcac    1260
   ttggctccgg ccg                                                       1273
<210> 149
   <211> 760
   <212> DNA
   <213> artificial sequence
<400> 149
   ggtaccaccc agctctcacc tgtgaacatc tgtactttcc gcggcgacgt cacacacatc 60
   gctggaggag gaggagatgc agaattccga catggaggag gaacacaaaa ctacaccatg 120
   aacttggcct cgcagaactg gaacaactac gatccaaccg aggaaatccc cgctcctttg 180
   ggaactcccg acttcgtggg acgtatccaa ggtgtcctga cacagactac acgtcgcgac 240
   ggctctactc gcggacacaa ggccactgtg tcgacaggtt ccgtccactt cacccctaag 300
   ctgggctctg tgcaattcag cacagacacc tcaaacgatt tcgagactgg ccagaacacc 360
   aggttcactc ccgtgggtgt cgttcaagac ggcggaggag gagatgcaga attccgacat 420
   ggaggaggag atgcagaatt ccgacatgga ggaggagatg cagaattccg acatggagga 480
   ggagatgcag aattccgaca tggaggagga gatgcagaat tccgacatgg aggaggagat 540
   gcagaattcc gacatggagg aggagatgca gaattccgac atggaggagg agatgcagaa 600
   ttccgacatg gaggaggaga tgcagaattc cgacatggag gaggagatgc agaattccga 660
   catggaggag gaagcaccac tcaccagaac gaaccccagc aatgggtcct ccctgactac 720
   tcgggcaggg attcccacaa cgttcacttg gctccggccg 760
<210> 150
   <211> 553
   <212> DNA
   <213> artificial sequence
<400> 150
   ggtaccaccc agctctcacc tgtgaacatc tgtactttcc gcggcgacgt cacacacatc 60
   gctggaggag gaggagatgc agaattccga catggaggag gaacacaaaa ctacaccatg 120
   aacttggcct cgcagaactg gaacaactac gatccaaccg aggaaatccc cgctcctttg 180
   ggaactcccg acttcgtggg acgtatccaa ggtgtcctga cacagactac acgtcgcgac 240
   ggctctactc gcggacacaa ggccactgtg tcgacaggtt ccgtccactt cacccctaag 300
   ctgggctctg tgcaattcag cacagacacc tcaggaggag gagatgcaga attccgacat 360
   ggaggaggaa acgatttcga gactggccag aacaccaggt tcactcccgt gggtgtcgtt 420
   caagacggcg gaggaggaga tgcagaattc cgacatggag gaggaagcac cactcaccag 480
   aacgaacccc agcaatgggt cctccctgac tactcgggca gggattccca caacgttcac 540
   ttggctccgg ccg 553
<210> 151
   <211> 715
   <212> DNA
   <213> artificial sequence
<400> 151
   ggtaccaccc agctctcacc tgtgaacatc tgtactttcc gcggcgacgt cacacacatc      60
   gctggaggag gaggagatgc agaattccga catggaggag gagatgcaga attccgacat     120
   ggaggaggag atgcagaatt ccgacatgga ggaggaacac aaaactacac catgaacttg     180
   gcctcgcaga actggaacaa ctacgatcca accgaggaaa tccccgctcc tttgggaact     240
   cccgacttcg tgggacgtat ccaaggtgtc ctgacacaga ctacacgtcg cgacggctct     300
   actcgcggac acaaggccac tgtgtcgaca ggttccgtcc acttcacccc taagctgggc     360
   tctgtgcaat tcagcacaga cacctcagga ggaggagatg cagaattccg acatggagga     420
   ggagatgcag aattccgaca tggaggagga gatgcagaat tccgacatgg aggaggaaac     480
   gatttcgaga ctggccagaa caccaggttc actcccgtgg gtgtcgttca agacggcgga     540
   ggaggagatg cagaattccg acatggagga ggagatgcag aattccgaca tggaggagga     600
   gatgcagaat tccgacatgg aggaggaagc accactcacc agaacgaacc ccagcaatgg     660
   gtcctccctg actactcggg cagggattcc cacaacgttc acttggctcc ggccg          715
<210> 152
   <211> 1282
   <212> DNA
   <213> artificial sequence
<400> 152
   ggtaccaccc agctctcacc tgtgaacatc tgtactttcc gcggcgacgt cacacacatc      60
   gctggaggag gaggagatgc agaattccga catggaggag gagatgcaga attccgacat     120
   ggaggaggag atgcagaatt ccgacatgga ggaggagatg cagaattccg acatggagga     180
   ggagatgcag aattccgaca tggaggagga gatgcagaat tccgacatgg aggaggagat     240
   gcagaattcc gacatggagg aggagatgca gaattccgac atggaggagg agatgcagaa     300
   ttccgacatg gaggaggaga tgcagaattc cgacatggag gaggaacaca aaactacacc     360
   atgaacttgg cctcgcagaa ctggaacaac tacgatccaa ccgaggaaat ccccgctcct     420
   ttgggaactc ccgacttcgt gggacgtatc caaggtgtcc tgacacagac tacacgtcgc     480
   gacggctcta ctcgcggaca caaggccact gtgtcgacag gttccgtcca cttcacccct     540
   aagctgggct ctgtgcaatt cagcacagac acctcaggag gaggagatgc agaattccga     600
   catggaggag gagatgcaga attccgacat ggaggaggag atgcagaatt ccgacatgga     660
   ggaggagatg cagaattccg acatggagga ggagatgcag aattccgaca tggaggagga     720
   gatgcagaat tccgacatgg aggaggagat gcagaattcc gacatggagg aggagatgca     780
   gaattccgac atggaggagg agatgcagaa ttccgacatg gaggaggaga tgcagaattc     840
   cgacatggag gaggaaacga tttcgagact ggccagaaca ccaggttcac tcccgtgggt     900
   gtcgttcaag acggcggagg aggagatgca gaattccgac atggaggagg agatgcagaa     960
   ttccgacatg gaggaggaga tgcagaattc cgacatggag gaggagatgc agaattccga    1020
   catggaggag gagatgcaga attccgacat ggaggaggag atgcagaatt ccgacatgga    1080
   ggaggagatg cagaattccg acatggagga ggagatgcag aattccgaca tggaggagga    1140
   gatgcagaat tccgacatgg aggaggagat gcagaattcc gacatggagg aggaagcacc    1200
   actcaccaga acgaacccca gcaatgggtc ctccctgact actcgggcag ggattcccac    1260
   aacgttcact tggctccggc cg                                             1282

## Description

### Technical Field

The present invention relates to the fields of molecular biology and immunology. In particular, the present invention relates to a recombinant P particle formed from a norovirus capsid P protein of a chimeric Aβ1-m peptide, wherein the recombinant P particles form an ordered and repetitive antigen array, and wherein m is an integer ranging from 6 to 15.

### Background Art

Alzheimer's disease (AD), also named senile dementia, is a progressive neurodegenerative disease. Its main clinical characteristics include gradual occurrences of memory deterioration, cognitive function disorders and abnormal behaviors, and ending up with a loss of the ability to live independently. AD leads to death from the complications 10-20 years after the onset thereof. Currently, this most common neurodegenerative disease cannot be cured or effectively delayed, and severely jeopardizes the physical and mental health and life quality of the elderly.

Researchers generally believe that the amyloid-β protein (Aβ) in the brain tissue is a primary pathogenic cause of neuronal damage and cognitive function disorders. Aβ is derived from its precursor β-amyloid precursor protein (abbreviated as βAPP). βAPP, as a transmembrane protein, is ubiquitously present in various tissues of the body and expressed most highly in the brain tissue. In the amyloid cleaving pathway, βAPP can be cleaved into Aβ proteins which are 38-43 amino acids in length, wherein Aβ1-42 protein is the main type that forms amyloid deposits.

Aβ active immune vaccine, which is intended to stimulate the body to produce Aβ antibodies and thus clear up Aβ1-42 deposits in the brain, is an important method for treating Alzheimer's disease. As a pioneer of AD vaccines, Aβ1-42 polypeptide vaccine shows a good therapeutic effect of delaying memory decline in the AD transgenic mouse model. Nevertheless, in the clinical trials of Aβ1-42 polypeptide vaccine, 6% of the AD patients showed the side effect of cephalomeningitis, and they had a low level of Aβ1-42 antibody. The research showed that although Aβ1-42 polypeptide vaccine could elicit antibody reactions, it may promote vaccinated patients to produce autoimmune T cell reactions in the brain and thus induce meningoencephalitis as it carries a large number of T cell epitopes. In comparison with Aβ1-42, polypeptide Aβ1-15 comprising only the first 15 amino acids at the N-terminal of Aβ polypeptide, comprises only B cell epitopes instead of T cell epitopes, and this polypeptide vaccine could stimulate the human body to produce specific immunological reactions against Aβ1-42 protein and show good safety in human trials. Therefore, Aβ1-15 has become an antigenic peptide with great research potentials. However, as a hapten, Aβ1-15 does not have a good immunological effect and sustainability of inducing the production of antibodies. Therefore, there are urgent needs for a vaccine against Alzheimer's disease with high safety and good immunological effect.

In the prior art, it is often preferred to synthesize the virus-like particle and Aβ polypeptide respectively, and then couple them in order to obtain vaccines with high immunogenicity. However, as to the vaccines obtained by methods such as coupling, it is difficult to control the number of the inserted epitopes, and to purify the vaccines effectively.

Wiessner et al., (2011) (The Journal of Neuroscience) discloses a second-generation active Aβ immunotherapy, CAD106, that can reduce amyloid accumulation in APP transgenic mice while minimizing potential side effects. Tan et al., (2012) (Nanomedicine) discloses a subviral nanoparticle for vaccine development against norovirus, rotavirus and influenza virus. Li et al., (2016) (Protein Expression and Purification) disclosed the establishment of a novel method without sequence modification for developing NoV P particle-based chimeric vaccines. Fu et al., (2017) (Scientific Reports) discloses a norovirus P particle-based active Aβ immunotherapy that elicits sufficient immunogenicity and improves cognitive capacity in a mouse model of Alzheimer's disease. Fu et al., (2015) (Immunology Letters) discloses a norovirus P particle as a vaccine platform for antibody production against Alzheimer's disease. Tan et al., (2011) (Journal of Virology) discloses a norovirus P particle as a novel platform for vaccine development and antibody production. Feng et al., (2012) (Journal of Central South University (Medical Sciences)) discloses prokaryotic expression and immunogenicity of the chimeric HBcAg containing Aβ1-15. CN 101 538 558 A discloses an Aβ1-40 recombinant adenovirus and method for preparing an Alzheimer's disease model. CN 1 504 240 A discloses a recombinated adeno-associated virus gene vaccine for the prevention and cure of Alzheimer's disease. CN 102 272 301 A discloses a method for inducing anti-amyloid beta antibodies and prevention and treatment of Alzheimer's disease. WO 2006/112553 A2 discloses an intranasally administrable gene vaccine for treating Alzheimer's disease.

### Summary of the Invention

The inventors found that the capsid P protein of norovirus is a very ideal antigen exhibition platform for an Aβ1-m (m is an integer ranging from 6 to 15) peptide. When different copies of Aβ1-m (m is an integer ranging from 6 to 15) peptide encoding sequences with various lengths are inserted into the loop of the DNA sequence encoding the capsid P protein of norovirus, Aβ1-m can be exhibited on the utmost surface of the recombinant P particle formed from the recombinant P protein, thereby helping to stimulate the body to the greatest extent to produce specific immunological reactions against Aβ1-42 proteins. The recombinant P particle of the present application can be prepared easily, and are controllable in terms of the number of the inserted epitopes; they can be purified easily and manufactured with a low cost; they have good safety and high immunogenicity, and can induce the production of high-titer antibodies.

In the present invention, multiple copies of DNA encoding antigen peptide Aβ1-m (m is an integer ranging from 6 to 15) are inserted into the DNA encoding loop1, loop2 and/or loop3 of the P protein by means of genetic engineering. By *in vitro* expression and self-assembly of recombinant proteins, a recombinant P particle capable of sufficiently exhibiting the antigen epitopes of Aβ1-m (m is an integer ranging from 6 to 15) is formed, thereby helping to enhance the efficacy, sustainability and safety of vaccination.

In a first aspect, the present invention provides a recombinant P particle formed from a norovirus capsid P protein, chimerized with Aβ1-m peptide(s), wherein an Aβ1-m peptide means the polypeptide consisting of the first m amino acids at the N terminal of the entire Aβ1-42 peptide, wherein m is an integer ranging from 6 to 15, the recombinant P particle forms an ordered and repetitive antigen array, wherein the amino acid sequence of at least one of the Aβ1-m peptides is embedded into loop1, loop2 and/or loop3 of the norovirus capsid P protein.

In a preferred embodiment of the present invention, the amino acid sequence of a norovirus capsid P protein is shown as SEQ ID NO: 1. In the recombinant P particle, N1 Aβ1-m peptide sequences are embedded behind one or more amino acid sites selected from the group consisting of amino acids 70-74 of SEQ ID NO:1, i.e. 170, A71, G72, T73 and Q74; N2 Aβ1-m peptide sequences are embedded after one or more amino acid sites selected from the group consisting of amino acids 148-151 of SEQ ID NO:1, i.e. T148, S149, N150 and D151; and N3 Aβ1-m peptide sequences are embedded after one or more amino acid sites selected from the group consisting of amino acids 168-171 of SEQ ID NO:1, i.e. D168, G169, S170 and T171; wherein N1, N2 and N3 each are respectively the numbers of the Aβ1-m peptide-encoding nucleotide sequences embedded, and N1, N2 and N3 are each independently selected from an integer ranging from 0 to 40, and N1+N2+N3≥1. In particular, N1, N2 and N3 each are independently selected from 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 and 40, and N1+N2+N3≥1.

That "Aβ1-m peptide sequences are embedded after one amino acid site" means that the N terminal of the Aβ1-m peptide sequences is directly linked to the C terminal of the amino acid residue via a peptide bond or is linked to the amino acid via an amino acid linker. In a preferred embodiment, the amino acid linker is (Gly)ₙ, wherein preferably n is 1-10, and more preferably n=3.

The multiple consecutive Aβ1-m peptide sequences inserted into SEQ ID NO: 1 can be directly linked or linked via an amino acid linker. In a preferred embodiment, the amino acid linker is (Gly)ₙ, wherein preferably n is 1-10, and more preferably n=3.

In the present invention, an Aβ1-m peptide sequence means the polypeptide consisting of the first m amino acids at the N terminal of the entire Aβ1-42 protein, wherein m is an integer ranging from 6 to 15. For example, the Aβ1-m peptide according to the present invention can be a polypeptide consisting of amino acids 1-6, 1-7, 1-8, 1-9, 1-10, 1-11, 1-12, 1-13, 1-14 or 1-15 at the N terminal of the entire Aβ1-42 protein.

The Aβ1-m peptide sequence according to the present invention can be an Aβ1-m peptide from human, mouse, primate, rabbit, African clawed frog (*Xenopuslaevis*), rat and guinea pig. The amino acid sequences of Aβ1-15 peptides from human, mouse, primate, rabbit, African clawed frog, rat and guinea pig are shown as SEQ ID NO:2 to SEQ ID NO:8, respectively. According to these sequences, those skilled in the art could easily determine the Aβ1-m peptide (m is an integer ranging from 6 to 15) of the present invention. Preferably, the Aβ1-m peptide is a human Aβ1-m peptide.

In a second aspect, the present invention further provides a nucleotide sequence encoding a norovirus capsid P protein chimerized with Aβ1-m peptide from which the recombinant P particle of the first aspect is formed, wherein m is an integer ranging from 6 to 15. In an embodiment of the invention, m may be 6, 7, 8, 9, 10, 11, 12, 13, 14 or 15. The nucleotide sequence comprises a nucleotide sequence encoding a norovirus capsid P protein and at least one copy of a nucleotide sequence encoding a Aβ1-m peptide, wherein the at least one copy of a nucleotide sequence encoding a Aβ1-m peptide is inserted into the nucleotide sequence encoding a loop of a norovirus capsid P protein, and wherein the inserted nucleotide sequence encoding a Aβ1-m peptide would not result in a frameshift mutation of the norovirus capsid P protein.

In a specific embodiment, the at least one copy of a nucleotide sequence encoding a Aβ1-m peptide can be inserted into the nucleotide sequence encoding the same loop of a norovirus capsid P protein, or into the nucleotide sequences encoding different loops of a norovirus capsid P protein. For example, where the nucleotide sequence encoding a norovirus capsid P protein is SEQ ID NO: 9, loop1 corresponds to nucleotides 208-222, loop2 corresponds to nucleotides 442-453, and loop3 corresponds to nucleotides 502-513.

In a specific embodiment of the invention, the numbers of the Aβ1-m peptide-encoding nucleotide sequences inserted into loop1, loop2 and loop3 are N1, N2 and N3, respectively; N1, N2 and N3 each are independently selected from 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 and 40, and N1+N2+N3≥1.

In a specific embodiment of the invention, the 5' end nucleotide of the nucleotide sequence encoding a Aβ1-m peptide is linked directly to the nucleotide sequence encoding a loop of a norovirus capsid P protein via 3',5'-phosphodiester bond or via a DNA linker. The insertion would not result in frame shift during translation of the nucleotide sequence, whether it is a direct insertion or an insertion via a DNA linker. In a preferred embodiment, the DNA linker is (GGA)n, (GGG)n or (GGC)n, wherein preferably n is 1-10, and more preferably n is 3.

In a specific embodiment of the invention, the multiple consecutive Aβ1-m peptide-encoding nucleotide sequences inserted into the nucleotide sequence encoding a norovirus capsid P protein are linked directly to each other by 3',5'-phosphodiester bond or by a DNA linker. The insertion would not result in frame shift during translation of the nucleotide sequence, whether it is a direct insertion or an insertion by a DNA linker. In a preferred embodiment, the DNA linker is (GGA)n, (GGG)n or (GGC)n, wherein preferably n is 1-10, and more preferably n is 3.

Preferably, the Aβ1-m peptide nucleotide sequence is a nucleotide sequence encoding an Aβ1-m peptide from human, mouse, primate, rabbit, African clawed frog (*Xenopuslaevis*), rat and guinea pig. More preferably, the Aβ1-m peptide nucleotide sequence is a nucleotide sequence encoding an Aβ1-m peptide from human.

In a preferred embodiment of the invention, the nucleotide sequences encoding norovirus capsid P proteins chimerized with Aβ1-m peptides are SEQ ID NOs: 15-142. More preferably, the nucleotide sequence encoding a norovirus capsid P protein of a chimeric Aβ1-m peptide is selected from SEQ ID NO:24, SEQ ID NO:39, SEQ ID NO:40, SEQ ID NO:57, SEQ ID NO:73, SEQ ID NO:92, SEQ ID NO:117, SEQ ID NO:132, SEQ ID NO:133 and SEQ ID NO:134.

In a third aspect, the present invention also provides a pharmaceutical composition comprising the recombinant P particle in the first aspect of the invention and a pharmaceutically acceptable carrier. The pharmaceutical composition is preferably used in mammals, and more preferably used in human. Preferably, the pharmaceutical composition is a vaccine composition, and more preferably, the vaccine composition also comprises an adjuvant. In a specific embodiment of the invention, the adjuvant is CpG. In another specific embodiment of the invention, the adjuvant is an aluminum adjuvant. The vaccine composition is preferably administered subcutaneously or nasally, and more preferably administered subcutaneously.

In a fourth aspect, the present invention also provides the recombinant P particle in the first aspect of the invention for use in the treatment or prevention of Alzheimer's disease. Preferably, the medicament is a vaccine. The medicament is preferably used in mammals, and more preferably used in human.

In a fifth aspect, the present invention also provides a method for preparing the recombinant P particle in the first aspect of the invention, comprising the following steps:
i) obtaining an expression vector comprising a nucleic acid encoding a norovirus capsid P protein chimerized with Aβ1-m peptide, wherein m is an integer ranging from 6 to 15;
ii) transferring the expression vector into a receptor cell;
iii) expressing the norovirus capsid P protein chimerized with Aβ1-m peptide, and allowing it to self-assemble into a recombinant P particle;
and preferably, the method also comprises isolation and purification steps.
In a specific embodiment, cation exchange chromatography and/or hydrophobic chromatography can be used for purification.

### Brief Description of drawings

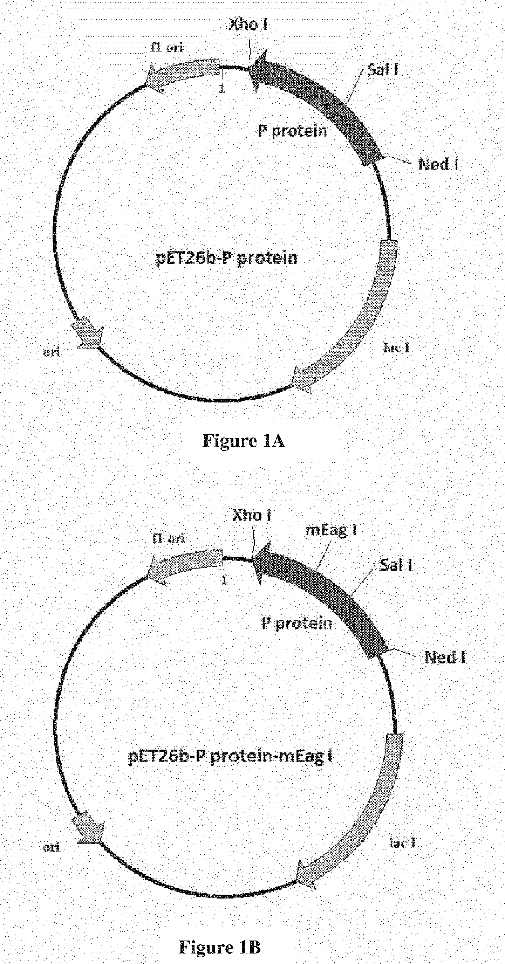

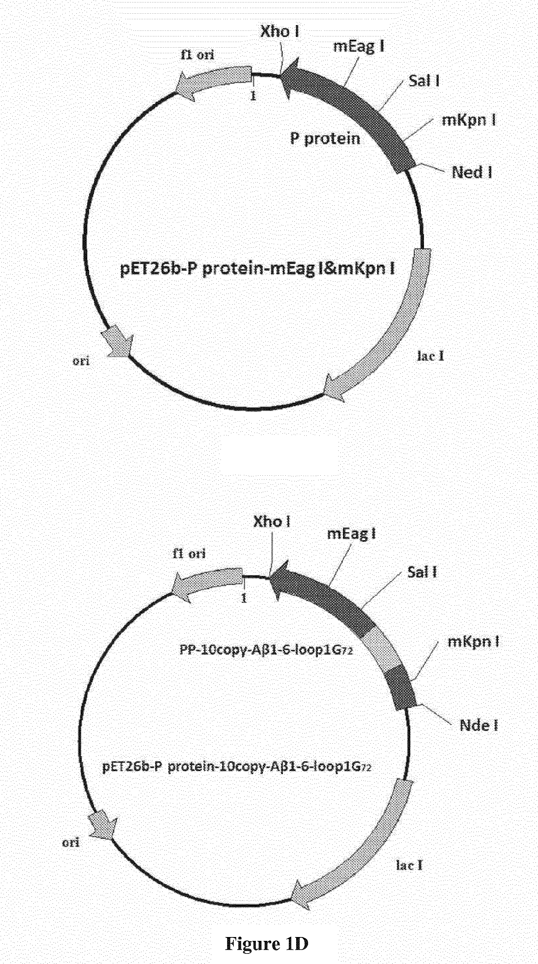

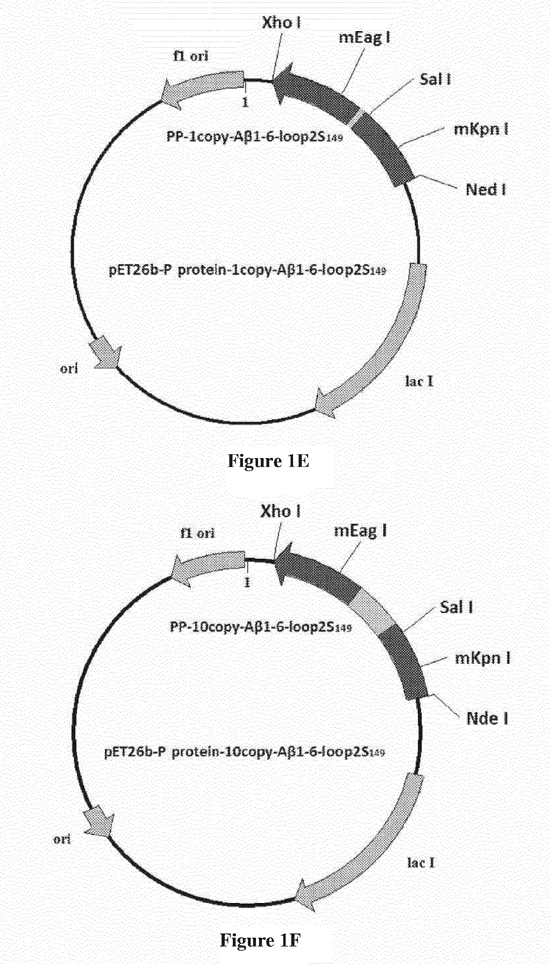

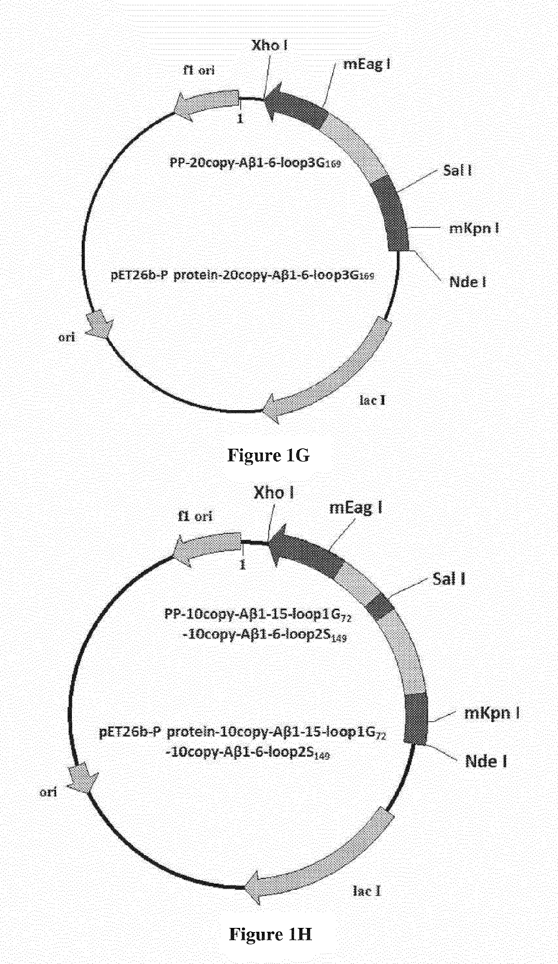

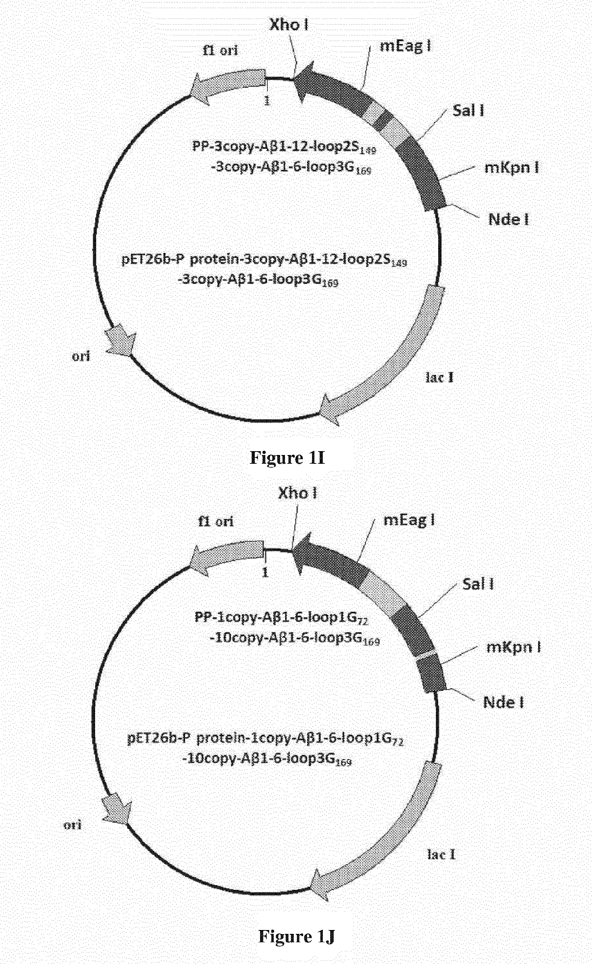

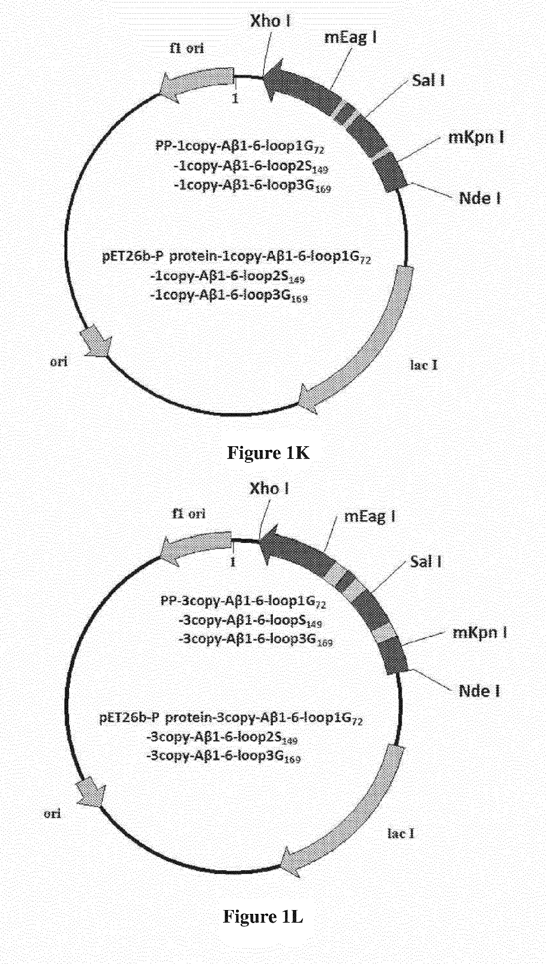

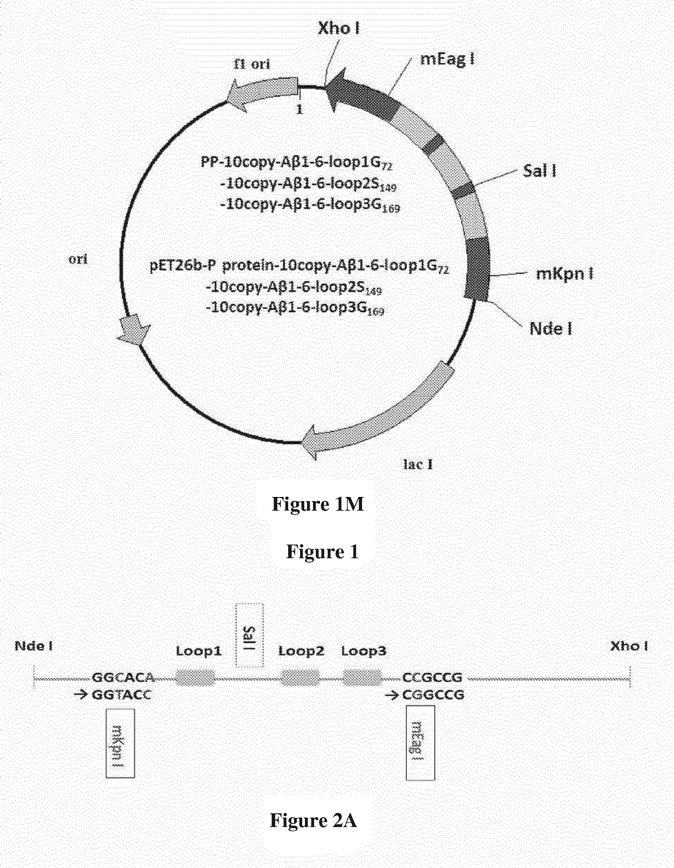

Figure 1 shows the maps of the various recombinant pET26b plasmid constructs.
   A is the schematic diagram of pET26b-P protein plasmid that has been constructed.
   B is the schematic diagram of pET26b-P protein-mEagI plasmid with a mEagI restriction enzyme site obtained by mutation.
   C is the schematic diagram of pET26b-P protein-mEagI&mKpnI plasmid with a mEagI restriction enzyme site and a mKpnI restriction enzyme site obtained by mutation.
   D is the schematic diagram of pET26b-P protein-10copy-Aβ1-6-loop1G₇₂ plasmid with the gene fragment of interest P protein-10copy-Aβ1-6-loop1G₇₂ loaded between a mKpnI restriction enzyme site and a SalI restriction enzyme site.
   E is the schematic diagram of pET26b-P protein-1copy-Aβ1-6-loop2S₁₄₉ plasmid with the gene fragment of interest P protein-1copy-Aβ1-6-loop2S₁₄₉ loaded between a mEagI restriction enzyme site and a SalI restriction enzyme site.
   F is a schematic diagram of pET26b-Pprotein-10copy-Aβ1-6-loop2S₁₄₉ plasmid with the gene fragment of interest P protein-10copy-Aβ1-6-loop2S₁₄₉ loaded between a mEagI restriction enzyme site and a SalI restriction enzyme site.
   G is the schematic diagram of pET26b-P protein-20copy-Aβ1-6-loop3G₁₆₉ plasmid with the gene fragment of interest P protein-20copy-Aβ1-6-loop3G₁₆₉ loaded between a mEagI restriction enzyme site and a SalI restriction enzyme site.
   H is the schematic diagram of pET26b-Pprotein-10copy-Aβ1-15-loop1G₇₂-10copy-Aβ1-6-loop2S₁₄₉ plasmid with the gene fragment of interest Pprotein-10copy-Aβ1-15-loop1G₇₂-10copy-Aβi-6-loop2S₁₄₉ loaded between a mEagI restriction enzyme site and a mKpnI restriction enzyme site.
   I is the schematic diagram of pET26b-P protein-3copy-Aβ1-12-loop2S₁₄₉-3copy-Aβ1-6-loop3G₁₆₉ plasmid with the gene fragment of interest Pprotein-3copy-Aβ1-12-loop2S₁₄₉-3copy-Aβ1-6-loop3G₁₆₉ loaded between a mEagI restriction enzyme site and a mKpnI restriction enzyme site.
   J is the schematic diagram of pET26b-P protein-1copy-Aβ1-6-loop1G₇₂-10copy-Aβ1-6-loop3G₁₆₉ plasmid with the gene fragment of interest P protein-1copy-Aβ1-6-loop1G₇₂-10copy-Aβ1-6-loop3G₁₆₉ loaded between a mEagI restriction enzyme site and a mKpnI restriction enzyme site.
   K is the schematic diagram of pET26b-P protein-1copy-Aβ1-6-loop1G₇₂-1copy-Aβ1-6-loop2S₁₄₉-1copy-Aβ1-6-loop3G₁₆₉ plasmid with the gene fragment of interest Pprotein-1copy-Aβ1-6-loop1G₇₂-1copy-Aβ1-6-loop2S ₁₄₉-1copy-Aβ1-6-loop3 G₁₆₉ loaded between a mEagI restriction enzyme site and a mKpnI restriction enzyme site.
   L is the schematic diagram of pET26b-P protein-3 copy-Aβ1-6-loop1G₇₂-3copy-Aβ1-6-loop2S₁₄₉-3copy-Aβ1-6-loop3 G₁₆₉ plasmid with the gene fragment of interest P protein-3 copy-Aβ1-6-loop 1G₇₂-3copy-Aβ1-6-loop2S ₁₄₉-3copy-Aβ1-6-loop3 G₁₆₉ loaded between a mEagI restriction enzyme site and a mKpnI restriction enzyme site.
   M is the schematic diagram of pET26b-P protein-10copy-Aβ1-6-loop 1G₇₂-10copy-Aβ1 -6-loop2S₁₄₉-10copy-Aβ1 -6-loop3G1₆₉ plasmid with the gene fragment of interest Pprotein-10copy-Aβ1-6-loop1G₇₂-10copy-Aβ1-6-loop2S₁₄₉-10copy-Aβ1-6-loop3G₁₆₉ loaded between a mEagI restriction enzyme site and a mKpnI restriction enzyme site.

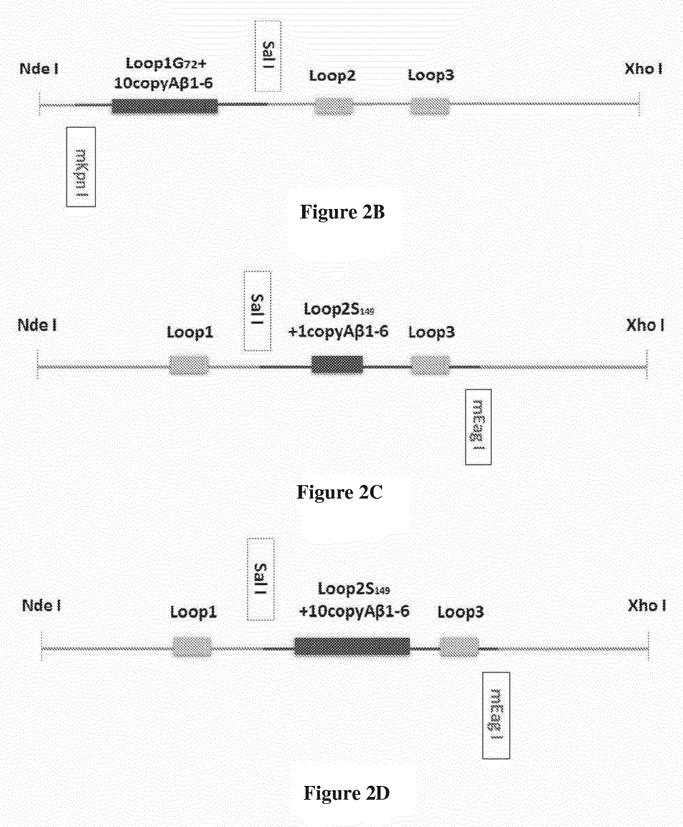

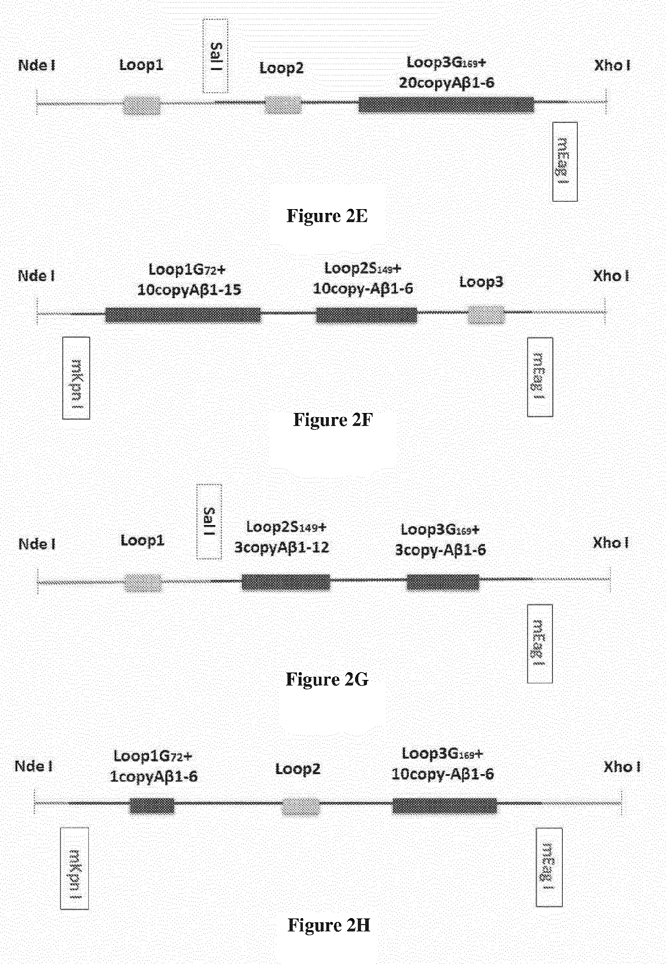

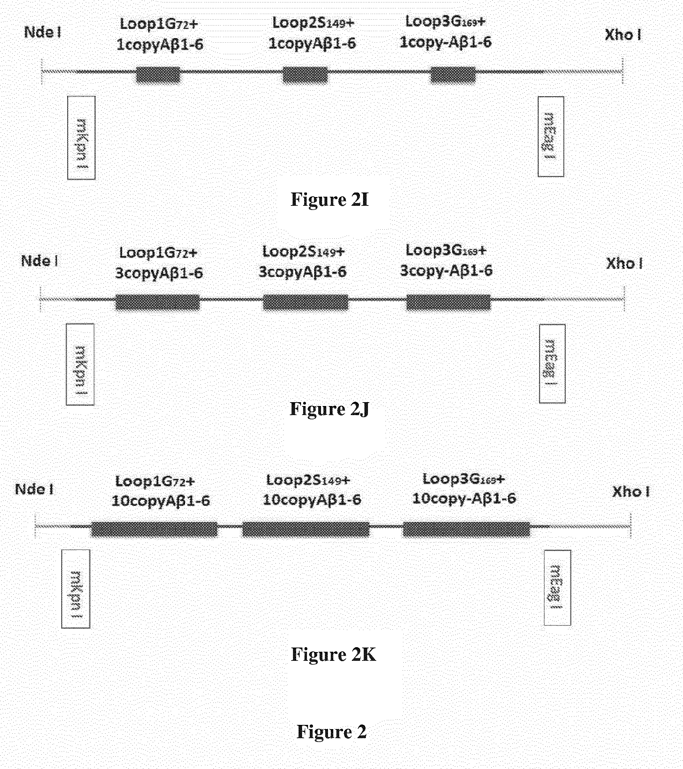

Figure 2 shows the schematic diagrams of the structures of gene fragments of interest.
   A is the schematic diagram of the locations of three loops of P protein (loop1, loop2 and loop3), together with site-directed mutation sites and restriction enzyme sites carried by P protein gene;
   B is the schematic diagram of a P particle gene fragment of interest with 10 copies of Aβ1-6 gene embedded behind site G₇₂ into loop1, abbreviated as P protein-10copy-Aβ1-6-loop1G₇₂;
   C is the schematic diagram of a P particle gene fragment of interest with one copy of Aβ1-6 gene embedded behind site S₁₄₉ into loop2, abbreviated as P protein-1copy-Aβ1-6-loop2S₁₄₉;
   D is the schematic diagram of a P particle gene fragment of interest with 10 copies of Aβ1-6 gene embedded behind site S₁₄₉ into loop2, abbreviated as P protein-10copy-Aβ1-6-loop2S₁₄₉;
   E is the schematic diagram of a P particle gene fragment of interest with 20 copies of Aβ1-6 gene embedded behind site G₁₆₉ into loop3, abbreviated as P protein-20copy-Aβ1-6-loop3G₁₆₉;
   F is the schematic diagram of a P particle gene fragment of interest with 10 copies of Aβ1-15 gene embedded behind site G₇₂ into loop1 and 10 copies of Aβ1-6 gene embedded behind site S₁₄₉ into loop2, abbreviated as P protein-10copy-Aβ1-15-loop1G₇₂-10copy-Aβ1-6-loop2S₁₄₉;
   G is the schematic diagram of a P particle gene fragment of interest with 3 copies of Aβ1-12 gene embedded behind site S₁₄₉ into loop2 and 3 copies of Aβ1-6 gene embedded behind site G₁₆₉ into loop3, abbreviated as P protein-3copy-Aβ1-12-loop2S₁₄₉-3copy-Aβ1-6-loop3G₁₆₉;
   H is the schematic diagram of a P particle gene fragment of interest with one copy of Aβ1-6 gene embedded behind site G₇₂ into loop1 and 10 copies of Aβ1-6 gene embedded behind site G₁₆₉ into loop3, abbreviated as P protein-1copy-Aβ1-6-loop1G₇₂-10copy-Aβ1-6-loop3G₁₆₉;
   I is the schematic diagram of a P particle gene fragment of interest with one copy of Aβ1-6 gene embedded behind site G₇₂ into loop1, one copy of Aβ1-6 gene embedded behind site S₁₄₉ into loop2 and one copy of Aβ1-6 gene embedded behind site G₁₆₉ into loop3, abbreviated as P protein-1copy-Aβ1-6-loop1G₇₂-1copy-Aβ1-6-loop2S₁₄₉-1copy-Aβ1-6-loop3G₁₆₉;
   J is the schematic diagram of a P particle gene fragment of interest with 3 copies of Aβ1-6 gene embedded behind site G₇₂ into loop1, 3 copies of Aβ1-6 gene embedded behind site S₁₄₉ into loop2 and 3 copies of Aβ1-6 gene embedded behind site G₁₆₉ into loop3, abbreviated as P protein-3copy-Aβ1-6-loop1G₇₂-3copy-Aβ1-6-loop2S₁₄₉-3copy-Aβ1-6-loop3G₁₆₉; and
   K is the schematic diagram of a P particle gene fragment of interest with 10 copies of Aβ1-6 gene embedded behind site G₇₂ into loop1, 10 copies of Aβ1-6 gene embedded behind site S₁₄₉ into loop2 and 10 copies of Aβ1-6 gene embedded behind site G₁₆₉ into loop3, abbreviated as P protein-10copy-Aβ1-6-loop1G₇₂-10copy-Aβ1-6-loop2S₁₄₉-10copy-Aβ1-6-100p3G₁₆₉.

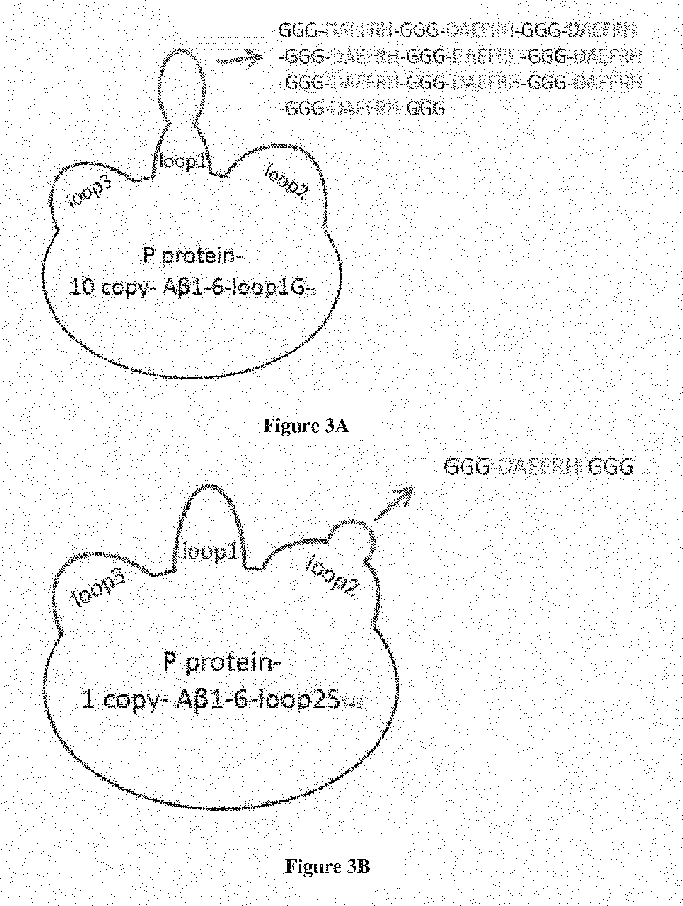

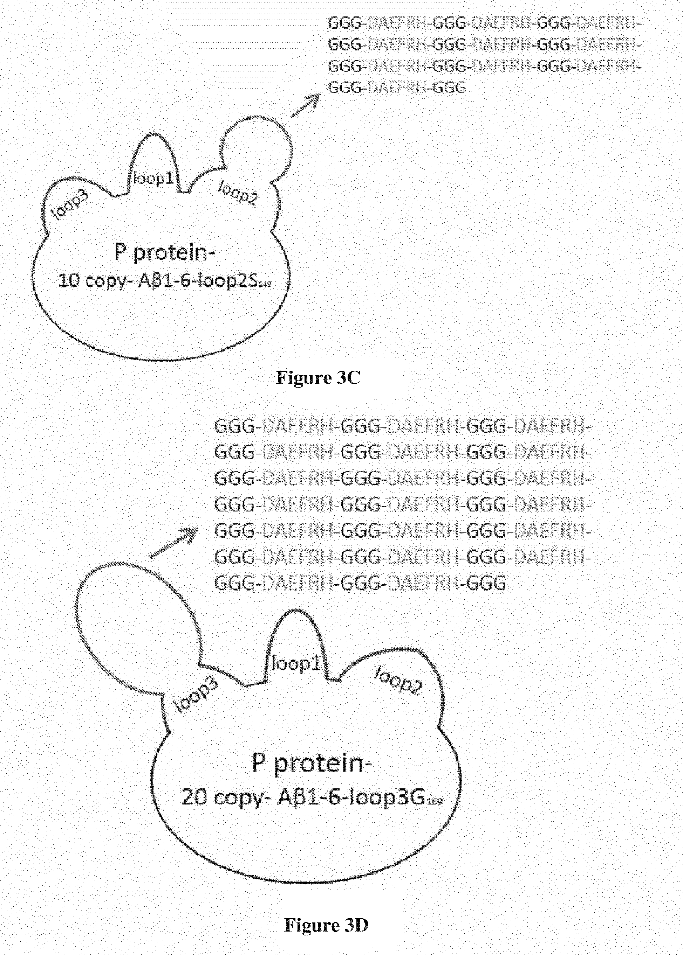

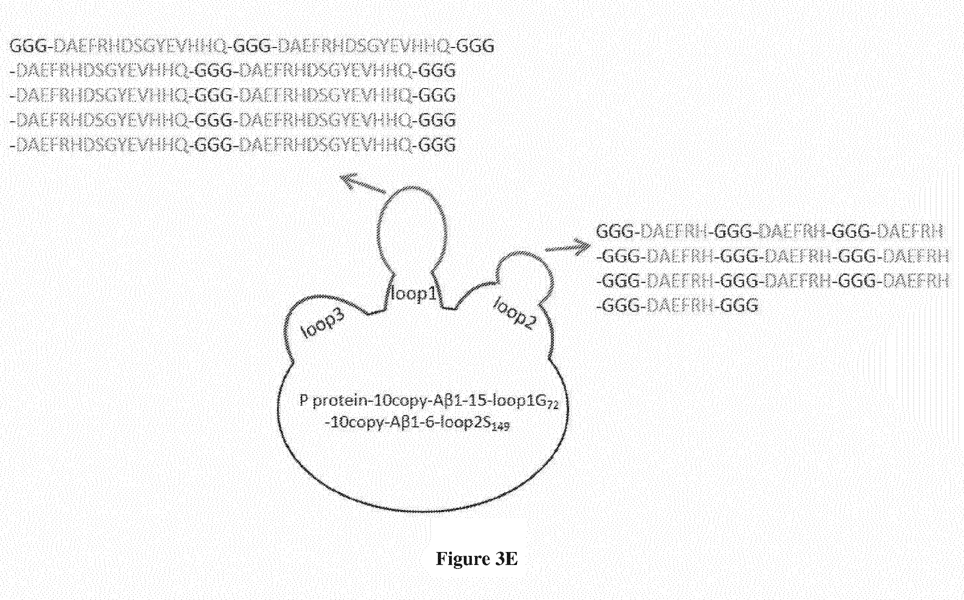

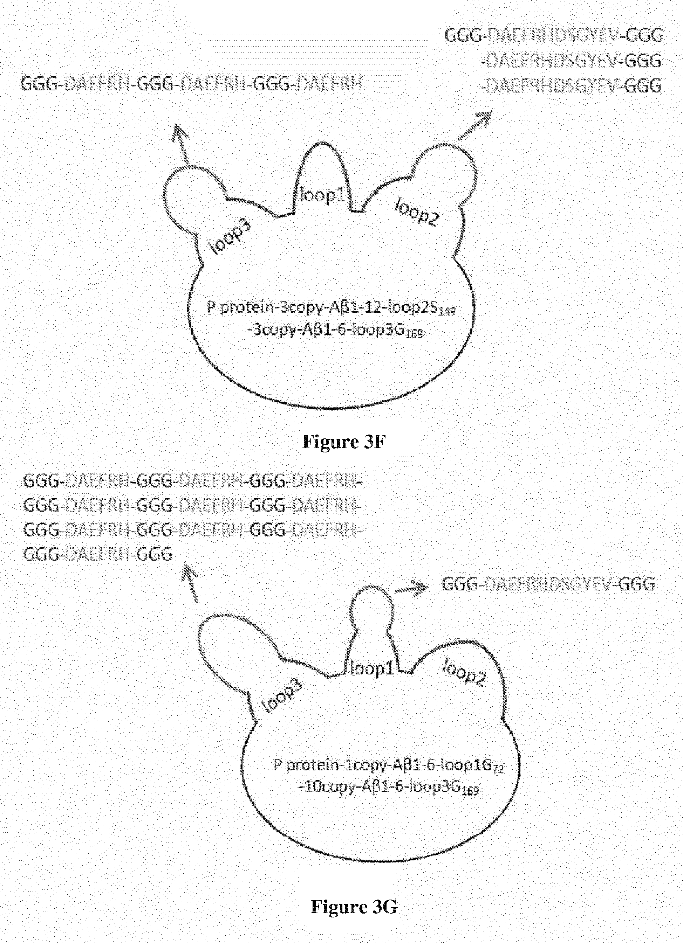

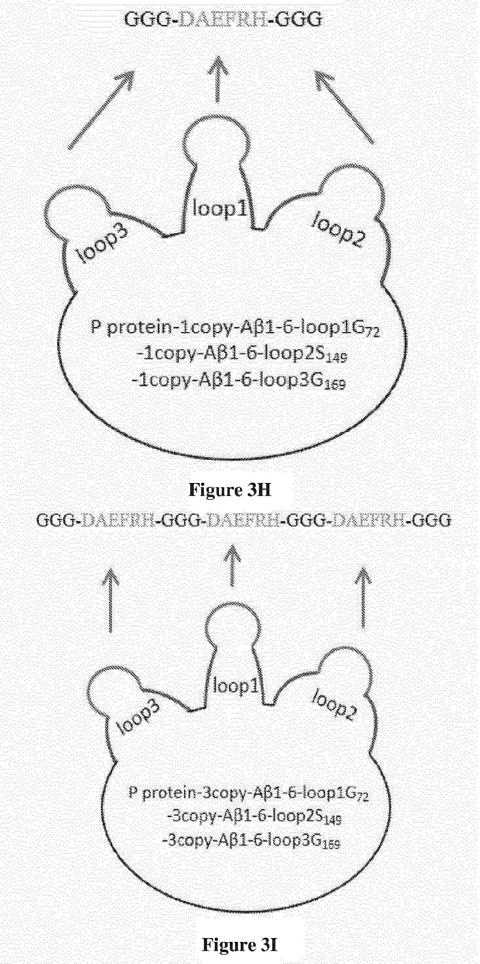

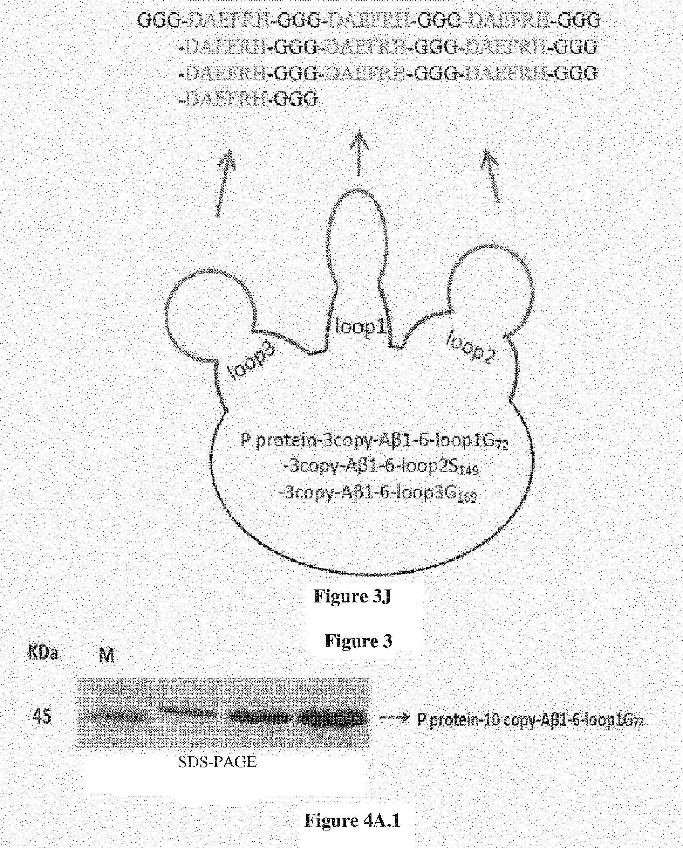

Figure 3 shows the schematic diagram of 10 recombinant P particles. A is the schematic diagram of P protein-10copy-Aβ1-6-loop1G₇₂; B is the schematic diagram of Pprotein-1copy-Aβ1-6-loop2S₁₄₉; C is the schematic diagram of P protein-10copy-Aβ1-6-loop2S₁₄₉; D is the schematic diagram of P protein-20copy-Aβ1-6-loop3G₁₆₉; E is the schematic diagram of P protein-10copy-Aβ1-15-loop1G₇₂-10copy-Aβ1-6-loop2S₁₄₉; F is the schematic diagram of P protein-3copy-Aβ1-12-loop2S₁₄₉-3copy-Aβ1-6-loop3G₁₆₉; G is the schematic diagram of P protein-1copy-Aβ1-6-loop1G₇₂-10copy-Aβ1-6-loop3G₁₆₉; H is the schematic diagram of P protein-1copy-Aβ1-6-loop1G₇₂-1copy-Aβ1-6-loop2S₁₄₉-1copy-Aβ1-6-loop3G₁₆₉; I is the schematic diagram of P protein-3copy-Aβ1-6-loop1G₇₂-3copy-Aβ1-6-loop2S₁₄₉-3copy-Aβ1-6-loop3G₁₆₉; and J is the schematic diagram of P protein-10copy-Aβ1-6-loop1G₇₂-10copy-Aβ1-6-loop2S₁₄₉-10copy-Aβ1-6-loop3G₁₆₉.

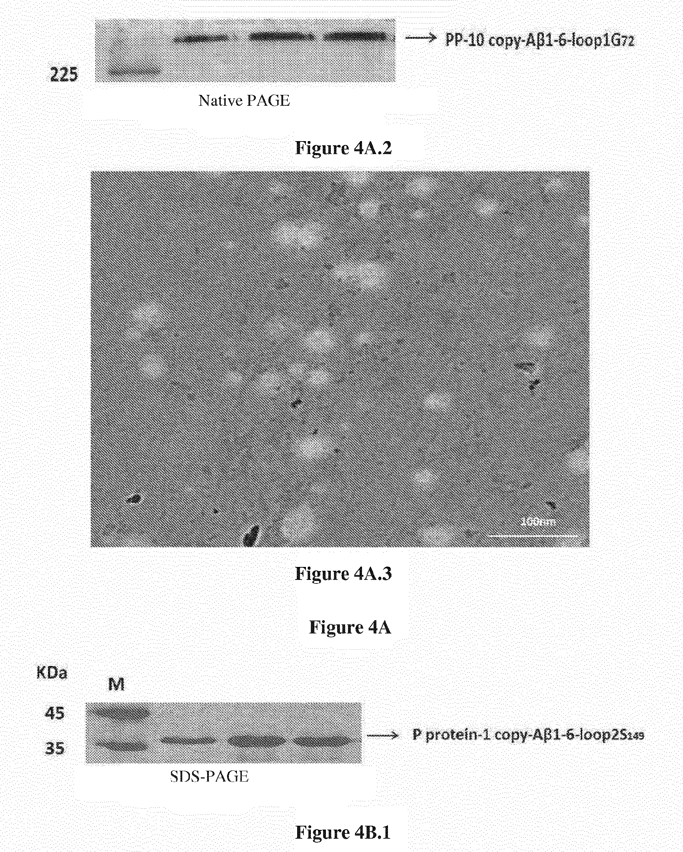

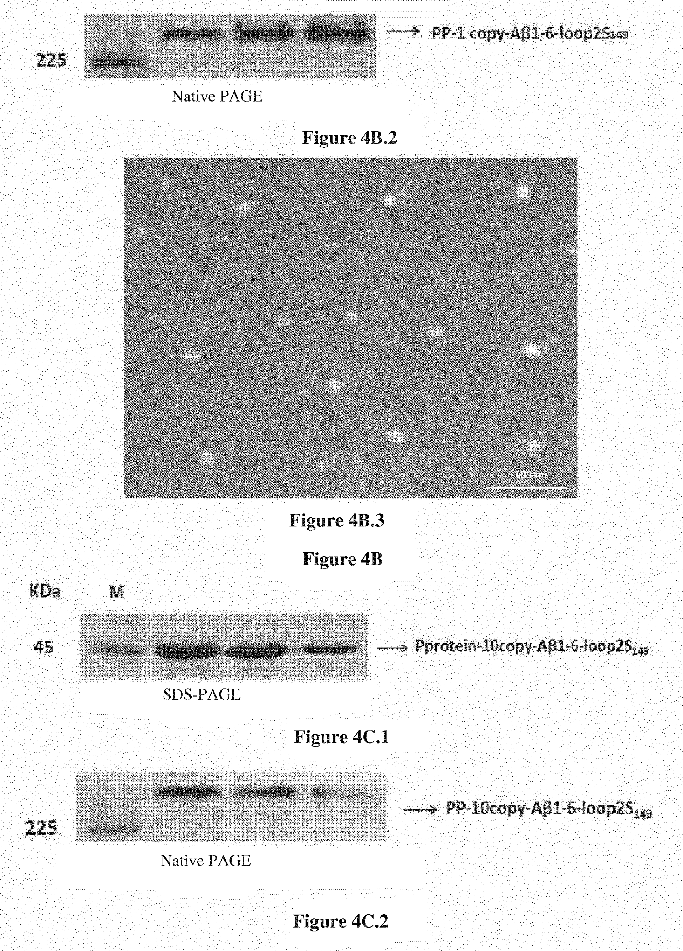

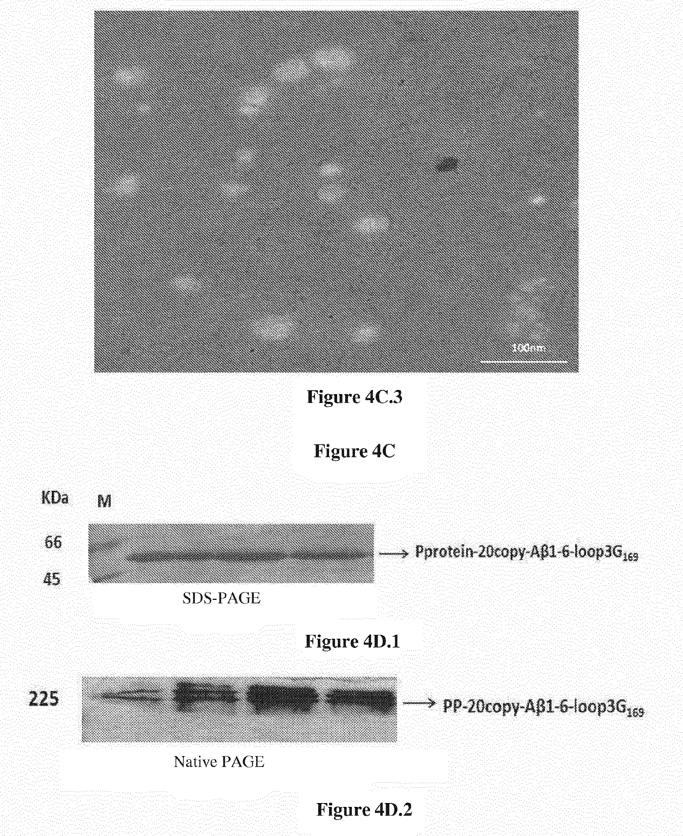

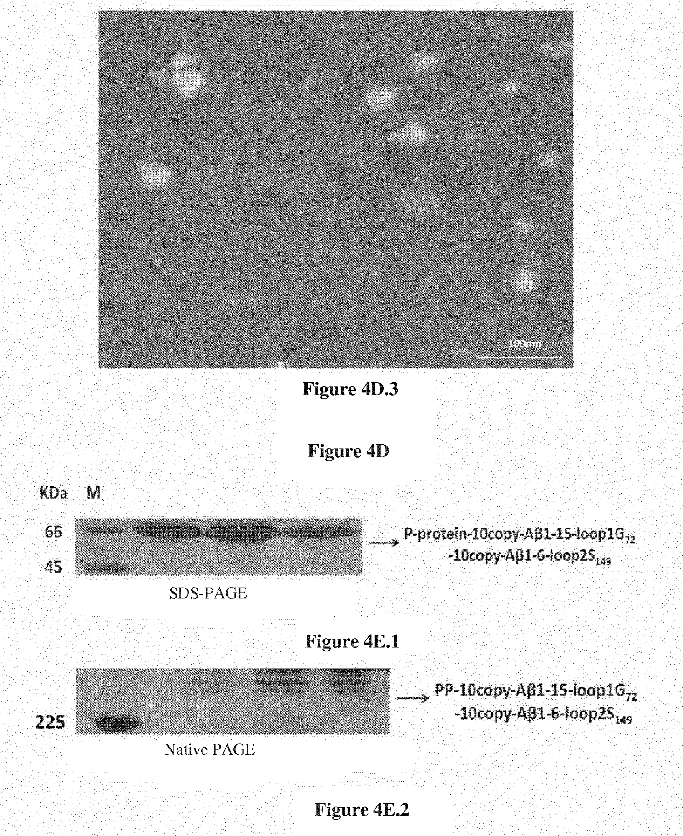

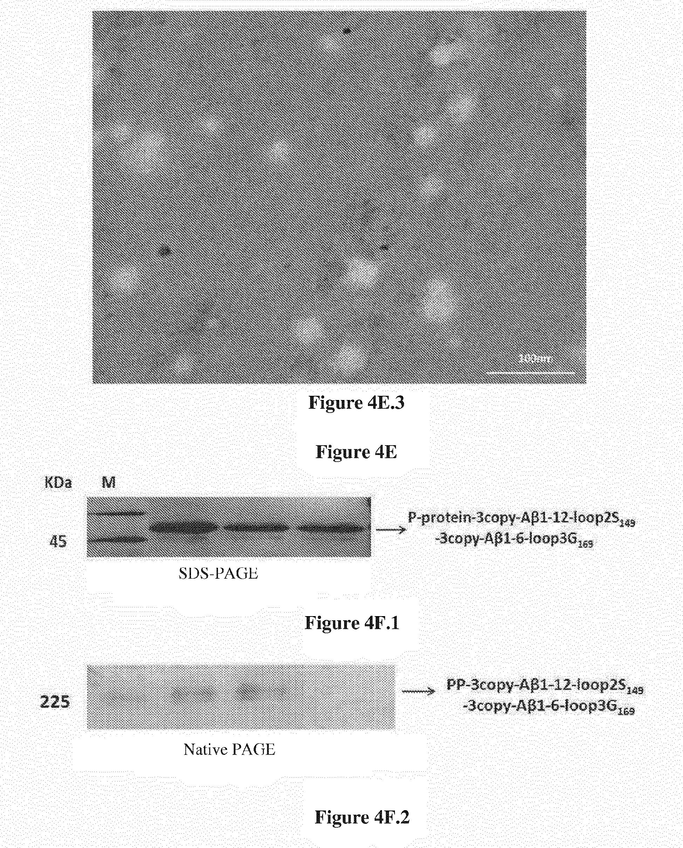

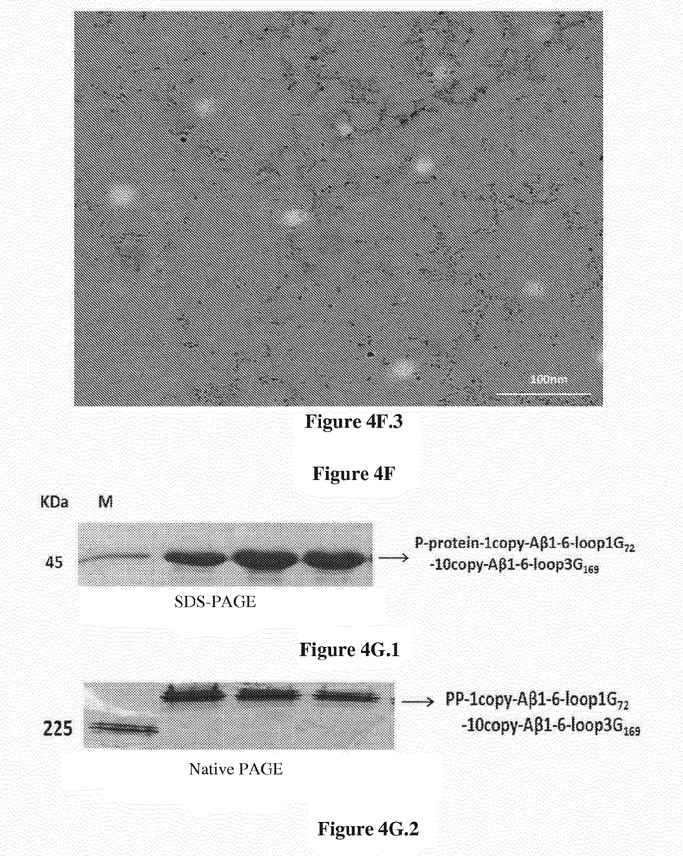

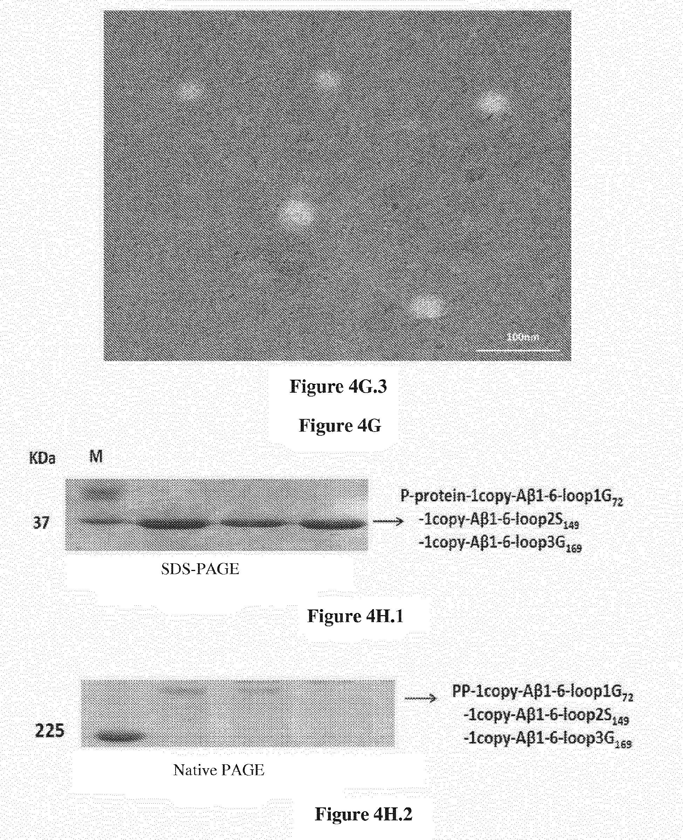

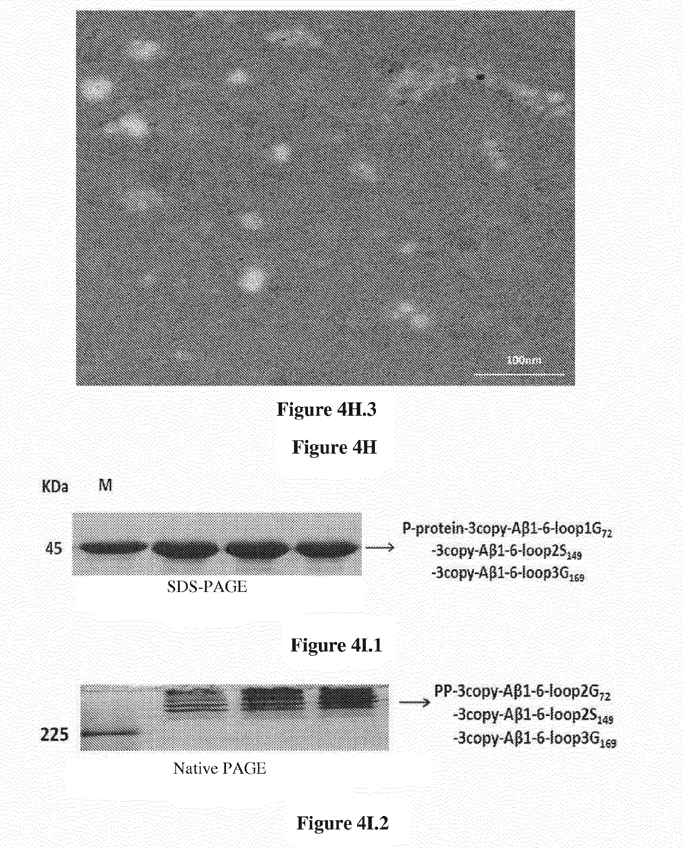

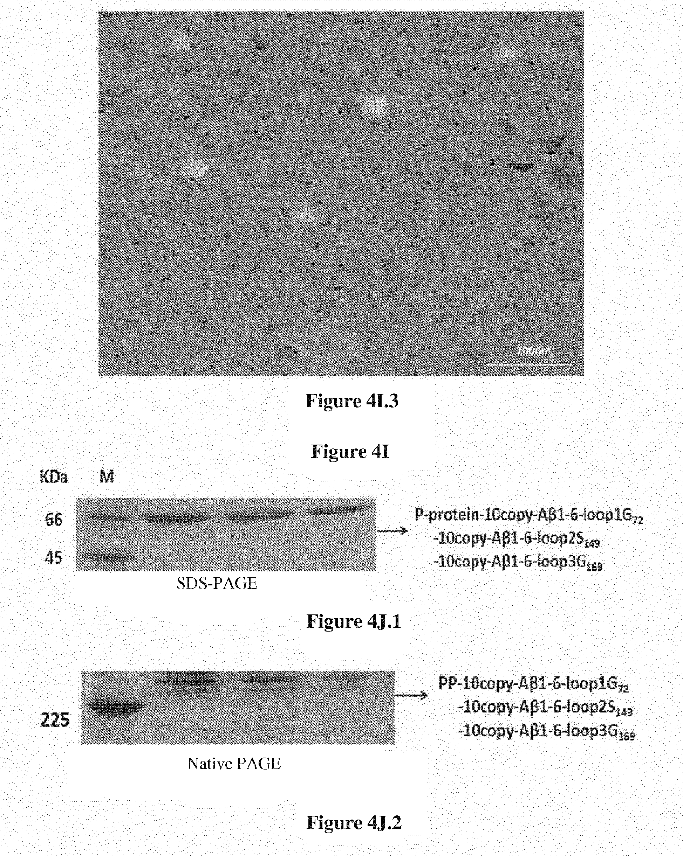

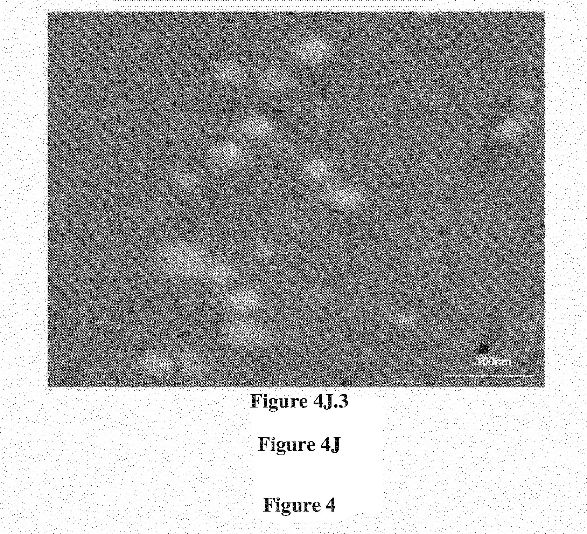

Figure 4 shows the purification and characterization of recombinant P particles. A is the SDS-PAGE and Native-PAGE graphs, as well as the electron microscope image of PP-10copy-Aβ1-6-loop1G₇₂ protein; B is the SDS-PAGE and Native-PAGE graphs, as well as the electron microscope image of PP-1copy-Aβ1-6-loop2S₁₄₉ protein; C is the SDS-PAGE and Native-PAGE graphs, as well as the electron microscope image of PP-10copy-Aβ1-6-loop2S₁₄₉ protein; D is the SDS-PAGE and Native-PAGE graphs, as well as the electron microscope image of PP-20copy-Aβ1-6-loop3G₁₆₉ protein; E is the SDS-PAGE and Native-PAGE graphs, as well as the electron microscope image of PP-10copy-Aβ1-15-loop1G₇₂-10copy-Aβ1-6-loop2S₁₄₉ protein; F is the SDS-PAGE and Native-PAGE graphs, as well as the electron microscope image of PP-3copy-Aβ1-12-loop2S₁₄₉-3copy-Aβ1-6-loop3G₁₆₉ protein; G is the SDS-PAGE and Native-PAGE graphs, as well as the electron microscope image of PP-1copy-Aβ1-6-loop1G₇₂-10copy-Aβ1-6-loop3G₁₆₉ protein; H is the SDS-PAGE and Native-PAGE graphs, as well as the electron microscope image of PP-1copy-Aβ1-6-loop1G₇₂-1copy-Aβ1-6-loop2S₁₄₉-1copy-Aβ1-6-loop3G₁₆₉ protein; I is the SDS-PAGE and Native-PAGE graphs, as well as the electron microscope image of PP-3copy-Aβ1-6-loop1G₇₂-3copy-Aβ1-6-loop2S₁₄₉-3copy-Aβ1-6-loop3G₁₆₉ protein; J is the SDS-PAGE and Native-PAGE graphs, as well as the electron microscope image of PP-10copy-Aβ1-6-loop1G₇₂-10copy-Aβ1-6-loop2S₁₄₉-10copy-Aβ1-6-loop3G₁₆₉ protein.

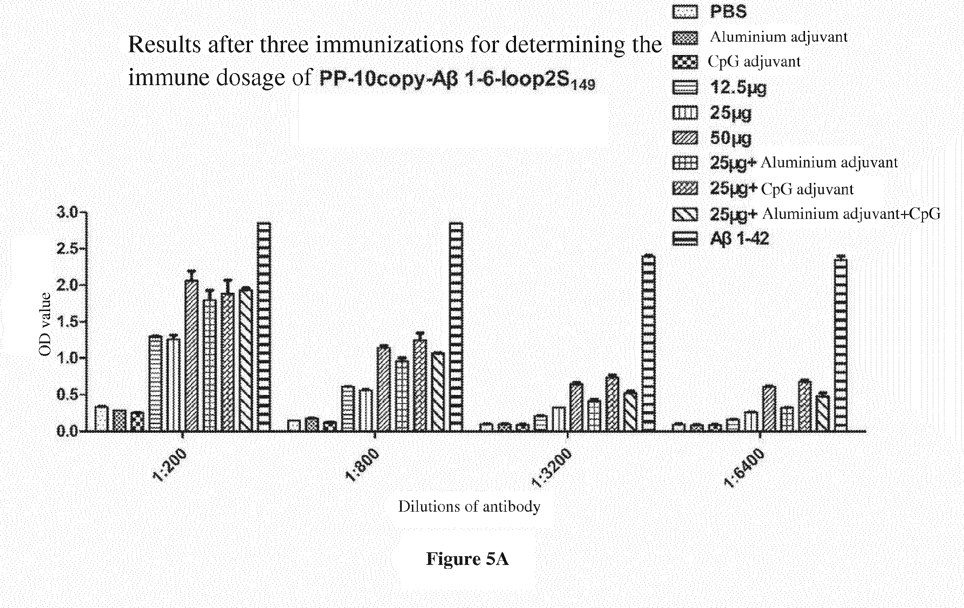

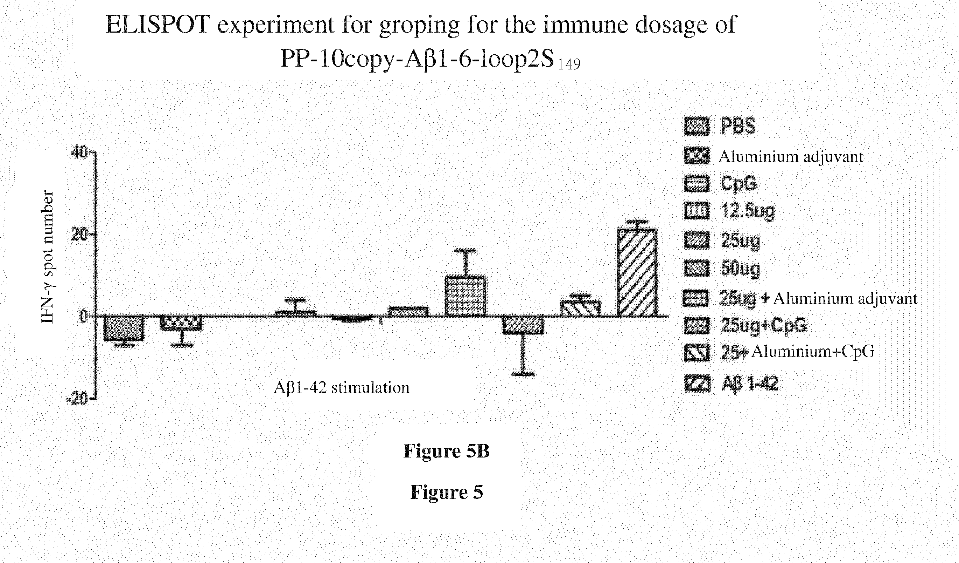

Figure 5 shows the determination of optimal immune dosage and optimal immune adjuvant of PP-10copy-Aβ1-6-loop2S₁₄₉ protein vaccine in a C57BL/6J mouse model. A is the detection of anti-Aβ42 antibody in mouse immune serum after immunization with different dosages of PP-10copy-Aβ1-6-loop2S₁₄₉ protein vaccine stimulated by different immune adjuvants. B is the detection of T cell responses produced by mouse spleen lymphocytes after immunization with different forms of PP-10copy-Aβ1-6-loop2S₁₄₉ protein vaccine.

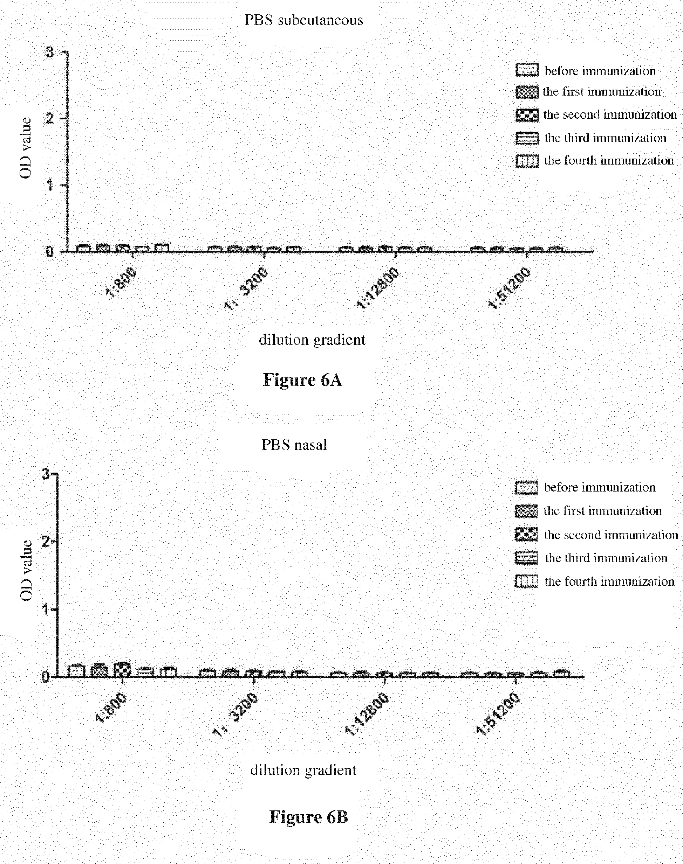

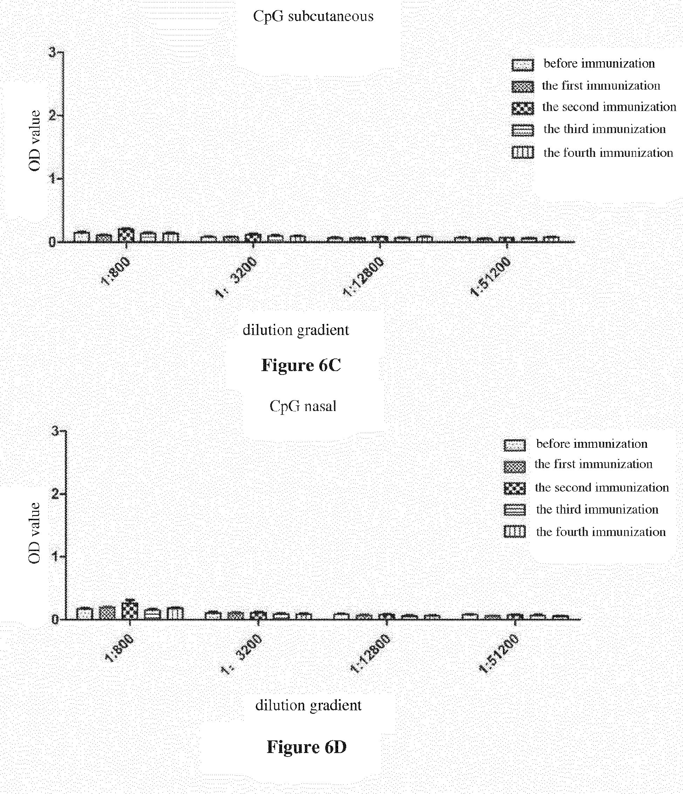

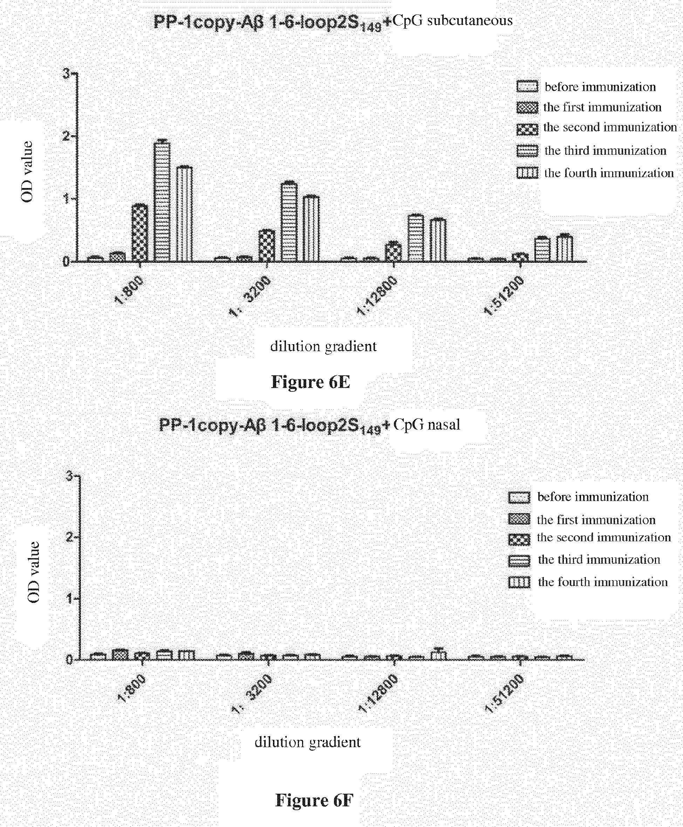

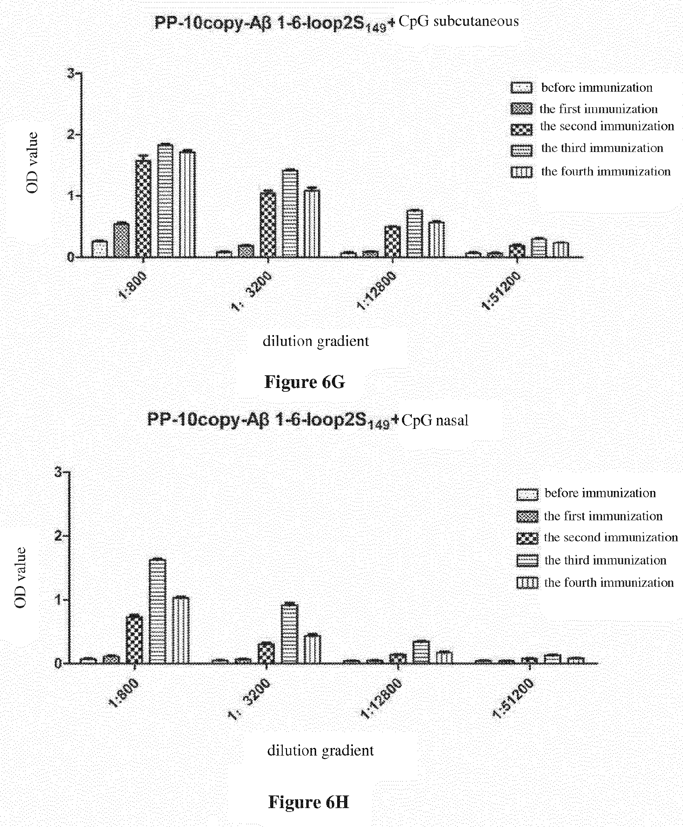

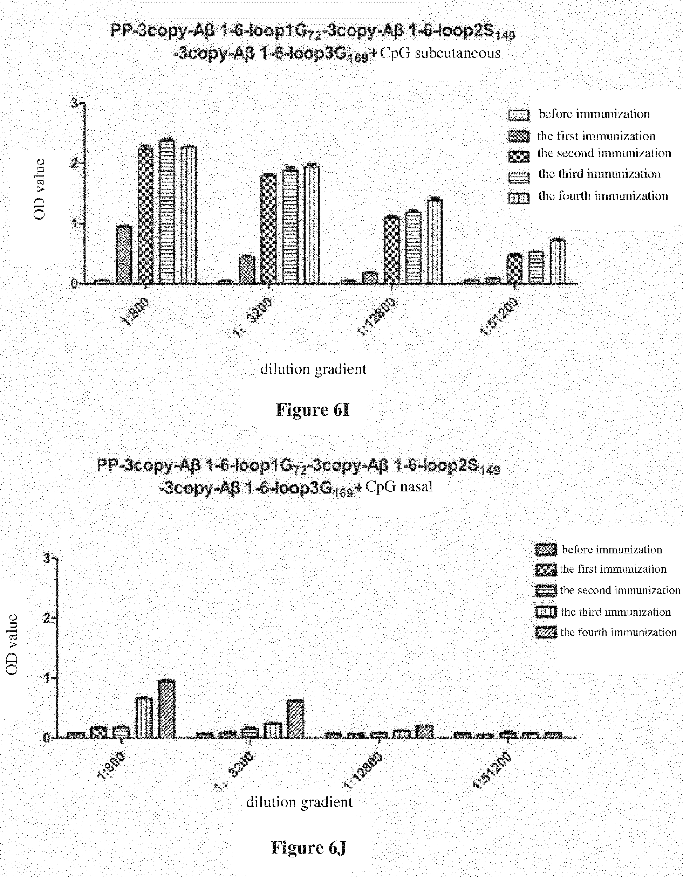

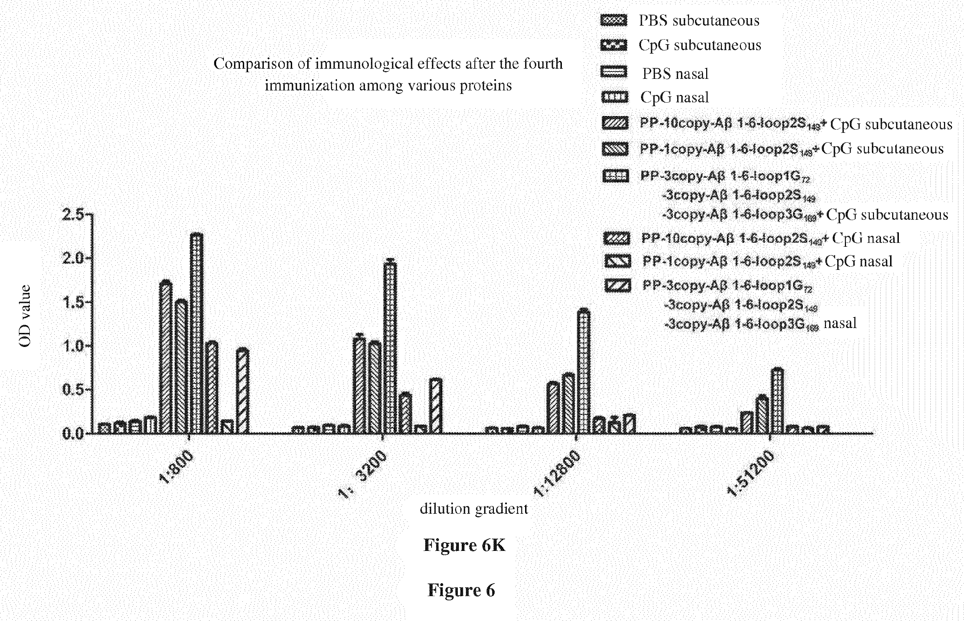

Figure 6 shows the ELISA detection of anti-Aβ42 antibody in mouse immune serum and the comparison of Aβ42 antibody levels from different immune groups after the mice are immunized with three different forms of protein vaccine. A is the statistical graph of four immunization results for the group of subcutaneous immunization with PBS. B is the statistical graph of four immunization results for the group of nasal immunization with PBS. C is the statistical graph of four immunization results for the group of subcutaneous immunization with CpG. D is the statistical graph of four immunization results for the group of nasal immunization with CpG. E is the statistical graph of four immunization results for the group of subcutaneous immunization with PP-1copy-Aβ1-6-loop2S₁₄₉ and the adjuvant CpG. F is the statistical graph of four immunization results for the group of nasal immunization with PP-1copy-Aβ1-6-loop2S₁₄₉ and the adjuvant CpG. G is the statistical graph of four immunization results for the group of subcutaneous immunization with PP-10copy-Aβ1-6-loop2S₁₄₉ and the adjuvant CpG. H is the statistical graph of four immunization results for the group of nasal immunization with PP-10copy-Aβ1-6-loop2S₁₄₉ and the adjuvant CpG. I is the statistical graph of four immunization results for the group of subcutaneous immunization with PP-3copy-Aβ1-6-loop1G₇₂-3copy-Aβ1-6-loop2S₁₄₉-3copy-Aβ1-6-loop3G₁₆₉ and the adjuvant CpG. J is the statistical graph of four immunization results for the group of nasal immunization with PP-3copy-Aβ1-6-loop1G₇₂-3copy-Aβ1-6-loop2S₁₄₉-3copy-Aβ1-6-loop3G₁₆₉ and the adjuvant CpG. K is the comparison of results after the fourth immunization for each group, wherein the best immunological effect is observed in the group of subcutaneous immunization with PP-3copy-Aβ1-6-loop1G₇₂-3copy-Aβ1-6-loop2S₁₄₉-3copy-Aβ1-6-loop3G₁₆₉ and the adjuvant CpG.

### Sequence Listing

SEQ ID NO: 1 is the amino acid sequence of a norovirus capsid P protein.
SEQ ID NOs: 2-8 are amino acid sequences of Aβ1-15 peptides from human, mouse, primate, rabbit, African clawed frog (*Xenopuslaevis*), rat and guinea pig.
SEQ ID NO: 9 is the nucleotide sequence encoding a norovirus P protein.
SEQ ID NO: 10 is the nucleotide sequence encoding an Aβ1-15 peptide from human.
SEQ ID NO: 11 is a site-directed mutation forward primer used in the process of obtaining pET26b-P protein-mEagI plasmid.
SEQ ID NO:12 is a site-directed mutation reverse primer used in the process of obtaining pET26b-P protein-mEagI plasmid.
SEQ ID NO: 13 is a site-directed mutation forward primer used in the process of obtaining pET26b-P protein-mEagI&mKpnI plasmid.
SEQ ID NO: 14 is a site-directed mutation reverse primer used in the process of obtaining pET26b-Pprotein-mEagI&mKpnI plasmid.
SEQ ID NO: 15-SEQ ID NO: 142 are nucleotide sequences encoding norovirus capsid P proteins of chimeric Aβ1-m peptides prepared in the examples of the invention.
SEQ ID NO: 143-SEQ ID NO: 152 are nucleotide sequences of DNA fragments of interest synthesized in the process of constructing recombinant plasmids expressing preferred 10 recombinant P proteins, corresponding to 10copy-Aβ1-16-loop1G₇₂, 1copy-Aβ1-6-loop2S₁₄₉, 10copy-Aβ1-6-loop2S₁₄₉, 20copy-Aβ1-6-loop3G₁₆₉, 3copy-Aβ1-12-loop2S₁₄₉-3copy-Aβ1-6-loop3G₁₆₉, 10copy-Aβ1-15 -loop1G₇₂-10copy-Aβ1-6-loop2S₁₄₉, 1copy-Ap1-6-loop1-G₇₂-10copy-Aβ1-6-loop3 G₁₆₉, 1copy-Aβ1-6-loop1G₇₂-1copy-Aβ1-6-loop2S₁₄₉-1copy-Aβ1-6-loop3G₁₆₉, 3copy-Aβ1-6-loop1G₇₂-3copy-Aβ1-6-loop2S₁₄₉-3copy-Aβ1-6-loop3G₁₆₉, and 10copy-Aβ1-6-loop1G₇₂-10copy-Aβ1-6-loop2S₁₄₉-10copy-Aβ1-6-loop3G₁₆₉, respectively.
SEQ ID NO: 153 is the nucleotide sequence of CpG adjuvant.

### Detailed Description of the Invention

### Definition

All the technical terms used in the present invention have the same meanings as those skilled in the art commonly understand, unless otherwise indicated.

An ordered and repetitive antigen array refers to multiple Aβ1-m peptides (m is an integer ranging from 6 to 15) repetitively and orderly arranged on the surface of a norovirus recombinant P particle.

P protein refers to the P protein in the norovirus capsid P proteins, which can self-assemble *in vitro* into a P particle. When used herein, P protein used at the gene level means a gene fragment, nucleotide sequence, plasmid, etc. encoding a P protein; P protein used at the protein level means a P protein monomer or dimer. In the accompanying figures, all schematic diagrams of protein show P protein, such as the plasmids encoding P proteins shown in Figure 1, 2 and 3, and the P protein monomer shown in the denatured electrophoresis of Figure 4.

P particle (abbreviated as PP) refers to a protein particle formed by the *in vitro* self-assembly of P protein in norovirus. The most common form of P particle is a tetracosamer. When used herein, P particle (PP) only used at the protein level means the form of polymer (such as a tetracosamer), including various proteins used for property detection and immunization, such as the polymer form shown in the denatured electrophoresis of Figure 4.

Pprotein-N1copy-Aβ1-m-loop1Ak1-N2copy-Aβ1-m-loop2Ak2-N3copy-Aβ1-m-loop 3Ak3 means that N1 copies of Aβ1-m are embedded behind the amino acid at position K2 into loop1 of P protein, N2 copies of Aβ1-m are embedded behind the amino acid at position K2 into loop2 of P protein, and N3 copies of Aβ1-m are embedded behind the amino acid at position K3 into loop3 of P protein, wherein m is an integer ranging from 6 to 15. Unless otherwise indicated, Aβ1-m is linked to P protein via a polypeptide linker having three glycines. For example, P protein-1copy-Aβ1-6-loop2T₁₄₈-1copy-Aβ1-6-loop2S₁₄₉-1copy-Aβ1-6-loop2N₁₅₀ means a P protein in which one copy of Aβ1-6 is respectively embedded behind the amino acid at position 148, behind the amino acid at position 149, and behind the amino acid at position 150 into loop2, and each Aβ1-6 is linked to P protein via an amino acid linker having three glycines encoded by GGA.

P protein-10copy-Aβ1-6-loop2S₁₄₉(GGA)₀ means that 10 copies of Aβ1-6 are embedded behind the amino acid at position 149 into loop2 of P protein, and there is no linker between Aβ1-6 and P protein, and no amino acid linker between each Aβ1-6, i.e. Aβ1-6 is directly linked to P protein or another Aβ1-6 via a peptide bond.

P protein-10copy-Aβ1-6-loop2S₁₄₉(GGC)₃ means that 10 copies of Aβ1-6 are embedded behind the amino acid at position 149 into loop2 of P protein, Aβ1-6 is linked to P protein via an amino acid linker having three glycines encoded by GGC, and each Aβ1-6 is linked to each other via an amino acid linker having three glycines encoded by GGC.

The technical solutions of the invention are further illustrated by the following examples; however, the present invention is not limited to these examples. Unless otherwise indicated, the formulations and devices used herein are all commercially available.

The present invention provides 127 recombinant P particles in which multiple copies of Aβ1-m peptides (m is an integer ranging from 6 to 15) are embedded into loop1, loop2 and/or loop3 of P protein. The P proteins from which said 127 recombinant P particles are formed are shown in Table 1.

**Table 1. The amino acid sequences of the recombinant P proteins from which said 127 recombinant P particles of the present invention are formed and the nucleotide sequences encoding the recombinant P proteins.**

| NO. | Recombinant P protein | Nucleotide Sequence encoding the Recombinant P protein |
|---|---|---|
| 1 | P protein-1copy-Aβ1-6-loop1I₇₀ | SEQ ID NO:15 |
| 2 | P protein-10copy-Aβ1-9-loop1I₇₀ | SEQ ID NO:16 |
| 3 | P protein-20copy-Aβ1-12-loop1I₇₀ | SEQ ID NO:17 |
| 4 | P protein-40copy-Aβ1-15-loop1I₇₀ | SEQ ID NO:18 |
| 5 | P protein-1copy-Aβ1-9-loop1A₇₁ | SEQ ID NO:19 |
| 6 | P protein-10copy-Aβ1-12-loop1A₇₁ | SEQ ID NO:20 |
| 7 | P protein-20copy-Aβ1-15-loop1A₇₁ | SEQ ID NO:21 |
| 8 | P protein-40copy-Aβ1-6-loop1A₇₁ | SEQ ID NO:22 |
| 9 | P protein-1copy-Aβ1-6-loop1G₇₂ | SEQ ID NO:23 |
| 10 | P protein-10copy-Aβ1-6-loop1G₇₂ | SEQ ID NO:24 |
| 11 | P protein-20copy-Aβ1-6-loop1G₇₂ | SEQ ID NO:25 |
| 12 | P protein-40copy-Aβ1-6-loop1G₇₂ | SEQ ID NO:26 |
| 13 | P protein-1copy-Aβ1-12-loop1T₇₃ | SEQ ID NO:27 |
| 14 | P protein-10copy-Aβ1-15-loop1T₇₃ | SEQ ID NO:28 |
| 15 | P protein-20copy-Aβ1-6-loop1T₇₃ | SEQ ID NO:29 |
| 16 | P protein-40copy-Aβ1-9-loop1T₇₃ | SEQ ID NO:30 |
| 17 | P protein-1copy-Aβ1-15-loop1Q₇₄ | SEQ ID NO:31 |
| 18 | P protein-10copy-Aβ1-6-loop1Q₇₄ | SEQ ID NO:32 |
| 19 | P protein-20copy-Aβ1-9-loop1Q₇₄ | SEQ ID NO:33 |
| 20 | P protein-40copy-Aβ1-12-loop1Q₇₄ | SEQ ID NO:34 |
| 21 | P protein-1copy-Aβ1-9-loop2T₁₄₈ | SEQ ID NO:35 |
| 22 | P protein-10copy-Aβ1-9-loop2T₁₄₈ | SEQ ID NO:36 |
| 23 | P protein-20copy-Aβ1-9-loop2T₁₄₈ | SEQ ID NO:37 |
| 24 | P protein-40copy-Aβ1-9-loop2T₁₄₈ | SEQ ID NO:38 |
| 25 | P protein-1copy-Aβ1-6-loop2S₁₄₉ | SEQ ID NO:39 |
| 26 | P protein-10copy-Aβ1-6-loop2S₁₄₉ | SEQ ID NO:40 |
| 27 | P protein-20copy-Aβ1-6-loop2S₁₄₉ | SEQ ID NO:41 |
| 28 | P protein-40copy-Aβ1-6-loop2S₁₄₉ | SEQ ID NO:42 |
| 29 | P protein-1copy-Aβ1-12-loop2N₁₅₀ | SEQ ID NO:43 |
| 30 | P protein-10copy-Aβ1-12-loop2N₁₅₀ | SEQ ID NO:44 |
| 31 | P protein-20copy-Aβ1-12-loop2N₁₅₀ | SEQ ID NO:45 |
| 32 | P protein-40copy-Aβ1-12-loop2N₁₅₀ | SEQ ID NO:46 |
| 33 | P protein-1copy-Aβ1-15-loop2D₁₅₁ | SEQ ID NO:47 |
| 34 | P protein-10copy-Aβ1-15-loop2D₁₅₁ | SEQ ID NO:48 |
| 35 | P protein-20copy-Aβ1-15-loop2D₁₅₁ | SEQ ID NO:49 |
| 36 | P protein-40copy-Aβ1-15-loop2D₁₅₁ | SEQ ID NO:50 |
| 37 | P protein-1copy-Aβ1-12-loop3D₁₆₈ | SEQ ID NO:51 |
| 38 | P protein-10copy-Aβ1-12-loop3D₁₆₈ | SEQ ID NO:52 |
| 39 | P protein-20copy-Aβ1-12-loop3D₁₆₈ | SEQ ID NO:53 |
| 40 | P protein-40copy-Aβ1-12-loop3D₁₆₈ | SEQ ID NO:54 |
| 41 | P protein-1copy-Aβ1-6-loop3G₁₆₉ | SEQ ID NO:55 |
| 42 | P protein-10copy-Aβ1-6-loop3G₁₆₉ | SEQ ID NO:56 |
| 43 | P protein-20copy-Aβ1-6-loop3G₁₆₉ | SEQ ID NO:57 |
| 44 | P protein-40copy-Aβ1-6-loop3G₁₆₉ | SEQ ID NO:58 |
| 45 | P protein-1copy-Aβ1-15-loop3S₁₇₀ | SEQ ID NO:59 |
| 46 | P protein-10copy-Aβ1-15-loop3S₁₇₀ | SEQ ID NO:60 |
| 47 | P protein-20copy-Aβ1-15-loop3S₁₇₀ | SEQ ID NO:61 |
| 48 | P protein-40copy-Aβ1-15-loop3S₁₇₀ | SEQ ID NO:62 |
| 49 | P protein-1copy-Aβ1-9-loop3T₁₇₁ | SEQ ID NO:63 |
| 50 | P protein-10copy-Aβ1-9-loop3T₁₇₁ | SEQ ID NO:64 |
| 51 | P protein-20copy-Aβ1-9-loop3T₁₇₁ | SEQ ID NO:65 |
| 52 | P protein-40copy-Aβ1-9-loop3T₁₇₁ | SEQ ID NO:66 |
| 53 | P protein-3copy-Aβ1-6-loop1G₇₂-3copy-Aβ1-6-loop2S₁₄₉ | SEQ ID NO:67 |
| 54 | P protein-10copy-Aβ1-9-loop1G₇₂-10copy-Aβ1-9-loop2S₁₄₉ | SEQ ID NO:68 |
| 55 | P protein-20copy-Aβ1-12-loop1G₇₂-20copy-Aβ1-12-loop2S₁₄₉ | SEQ ID NO:69 |
| 56 | P protein-40copy-Aβ1-15-loop1G₇₂-40copy-Aβ1-15-loop2S₁₄₉ | SEQ ID NO:70 |
| 57 | P protein-1copy-Aβ1-6-loop1I₇₀-1copy-Aβ1-12-loop2T₁₄₈ | SEQ ID NO:71 |
| 58 | P protein-3copy-Aβ1-9-loop1A₇₁-3copy-Aβ1-6-loop2S₁₄₉ | SEQ ID NO:72 |
| 59 | P protein-10copy-Aβ1-15-loop1G₇₂-10copy-Aβ1-6-loop2S₁₄₉ | SEQ ID NO:73 |
| 60 | P protein-20copy-Aβ1-9-loop1G₇₂-20copy-Aβ1-15-loop2N₁₅₀ | SEQ ID NO:74 |
| 61 | P protein-30copy-Aβ1-15-loop1T₇₃-30copy-Aβ1-12-loop2D₁₅₁ | SEQ ID NO:75 |
| 62 | P protein-40copy-Aβ1-12-loop1Q₇₄-40copy-Aβ1-9-loop2S₁₄₉ | SEQ ID NO:76 |
| 63 | P protein-1copy-Aβ1-6-loop1G₇₂-10copy-Aβ1-6-loop2S₁₄₉ | SEQ ID NO:77 |
| 64 | P protein-10copy-Aβ1-9-loop1G₇₂-20copy-Aβ1-9-loop2S₁₄₉ | SEQ ID NO:78 |
| 65 | P protein-20copy-Aβ1-12-loop1G₇₂-40copy-Aβ1-12-loop2S₁₄₉ | SEQ ID NO:79 |
| 66 | P protein-40copy-Aβ1-15-loop1G₇₂-10copy-Aβ1-15-loop2S₁₄₉ | SEQ ID NO:80 |
| 67 | P protein-1copy-Aβ1-6-loop1I₇₀-10copy-Aβ1-12-loop2T₁₄₈ | SEQ ID NO:81 |
| 68 | P protein-10copy-Aβ1-9-loop1A₇₁-20copy-Aβ1-6-loop2S₁₄₉ | SEQ ID NO:82 |
| 69 | P protein-20copy-Aβ1-15-loop1G₇₂-40copy-Aβ1-6-loop2S₁₄₉ | SEQ ID NO:83 |
| 70 | P protein-40copy-Aβ1-9-loop1G₇₂-20copy-Aβ1-15-loop2N₁₅₀ | SEQ ID NO:84 |
| 71 | P protein-20copy-Aβ1-15-loop1T₇₃-10copy-Aβ1-12-loop2D₁₅₁ | SEQ ID NO:85 |
| 72 | P protein-10copy-Aβ1-12-loop1Q₇₄-1copy-Aβ1-9-loop2S₁₄₉ | SEQ ID NO:86 |
| 73 | P protein-1copy-Aβ1-15-loop2S₁₄₉-1copy-Aβ1-15-loop3G₁₆₉ | SEQ ID NO:87 |
| 74 | P protein-10copy-Aβ1-12-loop2S₁₄₉-10copy-Aβ1-12-loop3G₁₆₉ | SEQ ID NO:88 |
| 75 | P protein-20copy-Aβ1-6-loop2S₁₄₉-20copy-Aβ1-6-loop3G₁₆₉ | SEQ ID NO:89 |
| 76 | P protein-40copy-Aβ1-9-loop2S₁₄₉-40copy-Aβ1-9-loop3G₁₆₉ | SEQ ID NO:90 |
| 77 | P protein-1copy-Aβ1-6-loop2T₁₄₈-1copy-Aβ1-9-loop3D₁₆₈ | SEQ ID NO:91 |
| 78 | P protein-3copy-Aβ1-12-loop2S₁₄₉-3copy-Aβ1-6-loop3G₁₆₉ | SEQ ID NO:92 |
| 79 | P protein-10copy-Aβ1-9-loop2S₁₄₉-10copy-Aβ1-15-loop3G₁₆₉ | SEQ ID NO:93 |
| 80 | P protein-20copy-Aβ1-15-loop2S₁₄₉-20copy-Aβ1-6-loop3G₁₆₉ | SEQ ID NO:94 |
| 81 | P protein-30copy-Aβ1-9-loop2N₁₅₀-30copy-Aβ1-12-loop3S₁₇₀ | SEQ ID NO:95 |
| 82 | P protein-40copy-Aβ1-15-loop2D₁₅₁-40copy-Aβ1-12-loop3T₁₇₁ | SEQ ID NO:96 |
| 83 | P protein-1copy-Aβ1-6-loop2S₁₄₉-10copy-Aβ1-6-loop3G₁₆₉ | SEQ ID NO:97 |
| 84 | P protein-10copy-Aβ1-9-loop2S₁₄₉-20copy-Aβ1-9-loop3G₁₆₉ | SEQ ID NO:98 |
| 85 | P protein-20copy-Aβ1-12-loop2S₁₄₉-40copy-Aβ1-12-loop3G₁₆₉ | SEQ ID NO:99 |
| 86 | P protein-40copy-Aβ1-15-loop2S₁₄₉-10copy-Aβ1-15-loop2G₁₆₉ | SEQ ID NO:100 |
| 87 | P protein-1copy-Aβ1-6-loop2T₁₄₈-10copy-Aβ1-9-loop3 D₁₆₈ | SEQ ID NO:101 |
| 88 | P protein-10copy-Aβ1-15-loop2N₁₅₀-20copy-Aβ1-6-loop3S₁₇₀ | SEQ ID NO:102 |
| 89 | P protein-20copy-Aβ1-12-loop2S₁₄₉-40copy-Aβ1-15-loop3G₁₆₉ | SEQ ID NO:103 |
| 90 | P protein-40copy-Aβ1-15-loop2D₁₅₁-20copy-Aβ1-12-loop3T₁₇₁ | SEQ ID NO:104 |
| 91 | P protein-20copy-Aβ1-12-loop2S₁₄₉-10copy-Aβ1-6-loop3G₁₆₉ | SEQ ID NO:105 |
| 92 | P protein-10copy-Aβ1-9-loop2S₁₄₉-1copy-Aβ1-15-loop3G₁₆₉ | SEQ ID NO:106 |
| 93 | P protein-1copy-Aβ1-15-loop1G₇₂-1copy-Aβ1-15-loop3G₁₆₉ | SEQ ID NO:107 |
| 94 | P protein-10copy-Aβ1-9-loop1G₇₂-10copy-Aβ1-9-loop3G₁₆₉ | SEQ ID NO:108 |
| 95 | P protein-20copy-Aβ1-6-loop1G₇₂-20copy-Aβ1-6-loop3G₁₆₉ | SEQ ID NO:109 |
| 96 | P protein-40copy-Aβ1-12-loop1G₇₂-40copy-Aβ1-12-loop3G₁₆₉ | SEQ ID NO:110 |
| 97 | P protein-1copy-Aβ1-6-loop1₁₇₀-1copy-Aβ1-9-loop3D₁₆₈ | SEQ ID NO:111 |
| 98 | P protein-3copy-Aβ1-6-loop1G₇₂-3copy-Aβ1-12-loop3G₁₆₉ | SEQ ID NO:112 |
| 99 | P protein-10copy-Aβ1-9-loop1A₇₁-10copy-Aβ1-15-loop3G₁₆₉ | SEQ ID NO:113 |
| 100 | P protein-20copy-Aβ1-12-loop1G₇₂-20copy-Aβ1-9-loop3S₁₇₀ | SEQ ID NO:114 |
| 101 | P protein-30copy-Aβ1-12-loop1Q₇₄-30copy-Aβ1-15-loop3T₁₇₁ | SEQ ID NO:115 |
| 102 | P protein-40copy-Aβ1-15-loop1T₇₃-40copy-Aβ1-6-loop3G₁₆₉ | SEQ ID NO:116 |
| 103 | P protein-1copy-Aβ1-6-loop1G₇₂-10copy-Aβ1-6-loop3G₁₆₉ | SEQ ID NO:117 |
| 104 | P protein-10copy-Aβ1-9-loop1G₇₂-20copy-Aβ1-9-loop3G₁₆₉ | SEQ ID NO:118 |
| 105 | P protein-20copy-Aβ1-12-loop1G₇₂-40copy-Aβ1-12-loop3G₁₆₉ | SEQ ID NO:119 |
| 106 | P protein-40copy-Aβ1-15-loop1G₇₂-10copy-Aβ1-15-loop3G₁₆₉ | SEQ ID NO:120 |
| 107 | P protein-1copy-Aβ1-6-loop1I₇₀-10copy-Aβ1-9-loop3D₁₆₈ | SEQ ID NO:121 |
| 108 | P protein-3copy-Aβ1-6-loop1G₇₂-10copy-Aβ1-12-loop3G₁₆₉ | SEQ ID NO:122 |
| 109 | P protein-10copy-Aβ1-12-loop1G₇₂-1copy-Aβ1-15-loop3S₁₇₀ | SEQ ID NO:123 |
| 110 | P protein-20copy-Aβ1-9-loop1A₇₁-40copy-Aβ1-15-loop3G₁₆₉ | SEQ ID NO:124 |
| 111 | P protein-40copy-Aβ1-9-loop1Q₇₄-20copy-Aβ1-12-loop3T₁₇₁ | SEQ ID NO:125 |
| 112 | P protein-30copy-Aβ1-15-loop1T₇₃-15copy-Aβ1-6-loop3G₁₆₉ | SEQ ID NO:126 |
| 113 | P protein-1copy-Aβ1-6-loop1I₇₀-10copy-Aβ1-9-loop2T₁₄₈-20copy -Aβ1-12-loop3D₁₆₈ | SEQ ID NO:127 |
| 114 | P protein-10copy-Aβ1-15-loop1A₇₁-20copy-Aβ1-12-loop2S₁₄₉ -40copy-Aβ1-9-loop3G₁₆₉ | SEQ ID NO:128 |
| 115 | P protein-1copy-Aβ1-6-loop1G₇₂-3copy-Aβ1-9-loop2N₁₅₀ -10copy-Aβ1-15-loop3S₁₇₀ | SEQ ID NO:129 |
| 116 | P protein-10copy-Aβ1-6-loop1Q₇₄-30copy-Aβ1-12-loop2D₁₅₁ -20copy-Aβ1-15-loop3T₁₇₁ | SEQ ID NO:130 |
| 117 | P protein-10copy-Aβ1-6-loop1T₇₃-10copy-Aβi-6-loop2S₁₄₉ -10copy-Aβ1-6-loop3G₁₆₉ | SEQ ID NO:131 |
| 118 | P protein-1copy-Aβ1-6-loop1G₇₂-1copy-Aβ1-6-loop2S₁₄₉ -1copy-Aβ1-6-loop3G₁₆₉ | SEQ ID NO:132 |
| 119 | P protein-3copy-Aβ1-6-loop1G₇₂-3copy-Aβ1-6-loop2S₁₄₉ -3copy-Aβ1-6-loop3G₁₆₉ | SEQ ID NO:133 |
| 120 | P protein-10copy-Aβ1-6-loop1 G₇₂-1 0copy-Aβ1-6-loop2S₁₄₉ -10copy-Aβ1-6-loop3G₁₆₉ | SEQ ID NO:134 |
| 121 | P protein-40copy-Aβ1-6-loop1G₇₂-40copy-Aβ1-6-loop2S₁₄₉ -40copy-Aβ1-6-loop3G₁₆₉ | SEQ ID NO:135 |
| 122 | P protein-10copy-Aβ1-6-loop2S₁₄₉(GGA)₀ | SEQ ID NO:136 |
| 123 | P protein-10copy-Aβ1-6-loop2S₁₄₉(GGA)₁ | SEQ ID NO:137 |
| 124 | P protein-10copy-Aβ1-6-loop2S₁₄₉(GGA)₅ | SEQ ID NO:138 |
| 125 | P protein-10copy-Aβ1-6-loop2S₁₄₉(GGA)₁₀ | SEQ ID NO:139 |
| 126 | P protein-10copy-Aβ1-6-loop2S₁₄₉(GGC)₃ | SEQ ID NO:140 |
| 127 | Pprotein-1copy-Aβ1-6-loop2T₁₄₈-1copy-Aβ1-6-loop2S₁₄₉-1copy-Aβ1-6-loop2N₁₅₀ | SEQ ID NO:141 |

The number of the recombinant P protein or recombinant P particle in the following tables respectively corresponds to the corresponding number of recombinant P protein in Table 1.

The present invention also provides a method for preparing said recombinant P protein particles, comprising the following steps:
1. Synthesizing artificially a DNA fragment comprising a DNA fragment encoding loop1, loop2 and/or loop3 domain(s) of a P protein embedded with multiple copies of Aβ1-m peptide;
2. Constructing pET26b-P protein plasmid (as shown in Figure 1A);
3. Carrying out site-directed mutation at the position before loop1 and behind loop3 of P protein by a point mutation method on condition that the amino acid sequence remains unchanged, to obtain the new restriction enzyme sites mKpnI and mEagI (as shown in Figure 1B);
4. According to various construction requirements, replacing multiple wild-type circular DNA in its entirety with the synthetic DNA fragment encoding loop1, loop2 and/or loop3 domain(s) of a P protein embedded with multiple copies of Aβ1-m peptide obtained in step 1 by using the restriction enzyme site SalI in the nucleic acid encoding P protein and the obtained enzyme sites mKpnI and mEagI in order to construct various recombinant P protein expression plasmids carrying multiple copies of Aβ1-m peptide respectively (as shown in Figure 1D-1M);
5. Transferring the expression plasmids obtained in step 4 into *Escherichia coli,* where the P protein embedded with an Aβ1-m immunogen is stably expressed and self-assembles into a P particle in the form of a tetracosamer.

Furthermore, according to the present invention, the following analysis and verification experiments were carried out:
1. Ten preferred protein vaccines were characterized by particle diameter determination and electron microscope analysis (as shown in Figure 4).
2. Experiments were carried out with different immune dosages and different immune adjuvants in a C57BL/6J mouse model, using PP-10copy-Aβ1-6-loop2S₁₄₉ protein vaccine. Results show that when CpG is used as the immune adjuvant, 25 µg protein vaccine could stimulate the mouse to produce the highest titer of a specific antibody against Aβ1-42, and meanwhile would not induce the mouse to produce T cell responses against Aβ1-42 (as shown in Figure 5).
3. Using PP-1copy-Aβ1-6-loop2S₁₄₉, PP-10copy-Aβ1-6-loop2S₁₄₉ and PP-3 copy-Aβ1-6-loop1G₇₂-3copy-Aβ1-6-loop2S₁₄₉-3copy-Aβ1-6-loop3G₁₆₉ as the immunogens, and CpG as the immune adjuvant, the immunological effects of the three protein vaccines were compared in mouse models, and the effects of both subcutaneous and nasal immunization on the immunological effects of the protein vaccines were analyzed (as shown in Figure 6). Experimental results show that compared with nasal immunization, subcutaneous immunization is more beneficial for the protein vaccines to induce antibodies.
4. Immunization was carried out with 127 candidate proteins. Various proteins were compared for their immunological effects, and the optimal candidate vaccine was screened. Results show that various vaccines can stimulate the mouse to produce a specific antibody against Aβ1-42 compared with the PBS control group; wherein the ten proteins, PP-10copy-Aβ1-6-loopG₇₂, PP-1copy-Aβ1-6-loop2S₁₄₉, PP-10copy-Aβ1-6-loop2S₁₄₉, PP-20copy-Aβ1-6-loop3G₁₆₉, PP-10copy-Aβ1-15-loop1G₇₂-10copy-Aβ1-6-loop2S₁₄₉, PP-3copy-Aβ1-12-loop2S₁₄₉-3copy-Aβ1-6-loop3G₁₆₉, PP-1copy-Aβ1-6-loop1G₇₂-10copy-Aβ1-6-loop3G₁₆₉, PP-1copy-Aβ1-6-loop1G₇₂-1copy-Aβ1-6-loop2S ₁₄₉-1copy-Aβ1-6-loop3G₁₆₉, PP-3copy-Aβ1-6-loop1G₇₂-3copy-Aβ1-6-loop2S₁₄₉-3copy-Aβ1-6-loop3G₁₆₉, PP-10copy-Aβ1-6-loop1G₇₂-10copy-Aβ1-6-loop2S₁₄₉-10copy-Aβ1-6-loop3G₁₆₉, have the optimal immunological effects and can stimulate the production of a high concentration of Aβ1-42.

### Example 1. Construction of pET26b-P protein plasmid

1 µL of Nde I enzyme and 1 µL of Xho I enzyme (purchased from Takara Corporation), and 5 µL of enzyme cleavage buffer (purchased from Takara Corporation) were respectively added to 4 µg of pET26b vector plasmid (purchased from Novagen Corporation) , and finally sterile water was added to the system to reach a final volume of 50 µL. Digestion was carried out at 37°C for 5 hours. Then the products were subjected to agarose gel electrophoresis, and recovered using a recovery column (purchased from Invitrogen) to obtain a plasmid vector having double enzyme-digested sticky ends.

A P protein nucleotide sequence as shown in SEQ ID NO: 9 was synthesized by gene synthetic method. The synthetic gene fragment was subjected to Nde I/Xho I double-enzyme digestion using the same method as mentioned above. The products were subjected to agarose gel electrophoresis, and recovered using a recovery column to obtain a gene fragment having double enzyme-digested sticky ends.

The above double enzyme-digested vector fragment and fragment of interest (with a molar ratio of 1:3, and a total volume of 15 µL) were mixed, and 0.75 µL of T4 ligase (purchased from Takara Corporation) and 1.5 µL of ligase buffer (purchased from Takara Corporation) were added. The ligation was carried out at 16 °C overnight to obtain a pET26b-P protein plasmid that can express a P protein particle, as shown in Figure 1A.

### Example 2. Construction of pET26b-P protein plasmid with restriction enzyme sites mKpnI and mEagI

The site-directed mutation method was as follows:
1. 5'CCGCCG3' was mutated to 5'CGGCCG3' by site-directed mutation method using the pET26b-P protein plasmid constructed in Example 1 to obtain pET26b-P protein-mEagI plasmid containing a mEagI restriction enzyme site without altering the amino acid sequence. The specific method was as follows: a pair of perfectly complementary bidirectional primers containing the mutation site was used:
   SEQ ID NO: 11 (forward):
      5'CGTTCACTTGGCTCCGGCCGTGGCTCCAACC3', and
   SEQ ID NO:12 (reverse):
      5'GGTTGGAGCCACGGCCGGAGCCAAGTGAACG3' ;
   the entire plasmid was subjected to PCR reaction, wherein the PCR reaction system was a KOD-Plus DNA polymerase system (purchased from TOYOBO Corporation), and the total volume of the reaction system was 50 µL (5 µL buffer, 0.2 mM dNTP, 1 mM magnesium sulfate, 0.3 µM upstream primer and 0.3 µM downstream primer, 50 ng template DNA, 1 µL KOD enzyme, and water which was added to a final volume of 50 µL). PCR was carried out in accordance with the reaction system instructions to obtain 20 µL of PCR products. 1 µL of DpnI enzyme (purchased from NEB Corporation) was added to the PCR products. Digestion was carried out at 37°C for 1 h. 10 µL of the digested products were added to Tran1-Blue competent cells (purchased from Peking TransGen Biotech, Ltd.), and placed on ice for 30 min. After that, the cells were subjected to heat shock at 42°C for 45 s, placed on ice for 2 min. and then were added to 600 µL of fluid LB medium without resistance, and recovered at 200 rpm at 37°C for 1 h. The culture broth was plated on a LB solid culture plate containing kanamycin (15 µg/mL), and were placed upside down at 37°C overnight. The obtained mutant plasmid clones were verified by sequencing. pET26b-P protein-mEagI plasmid is shown as Figure IB.
2. 5'GGCACA3' was mutated to 5'GGTACC3' using the pET26b-P protein-mEagI recombinant plasmid (as shown in Figure 1B) constructed in the previous step to obtain pET26b-P protein-mEagI&mKpnI plasmid containing both a mEagI restriction enzyme site and a mKpnI restriction enzyme site without altering the amino acid sequence. The specific method was as follows:
   a pair of perfectly complementary bidirectional primers containing the mutation site was used:
      SEQ ID NO: 13 (forward):
         5'GGTGTCCTGCTCGGTACCACCCAGCTCTCACC3', and
      SEQ ID NO: 14 (reverse):
         5'GGTGAGAGCTGGGTGGTACCGAGCAGGACACC3';
   and pET26b-P protein-mEagI&mKpnI plasmid was obtained by the same construction method as mentioned above and verified by sequencing. pET26b-P protein-mEagI&mKpnI plasmid is shown as in Figure 1C.

### Example 3. Synthesis of P particle loop gene fragments of interest embedded with human Aβ1-m genes

This step involves three different synthesis schemes in total:
The first is a P particle loop gene fragment of interest embedded with a human Aβ1-m gene located between a mKpnI restriction enzyme site and a SalI restriction enzyme site. That is, N1 copies of Aβ1-m gene are merely embedded into loop1. The P protein prepared by this method is abbreviated as P protein-N 1copy-Aβ1-m-loop 1.

The second is a P particle loop gene fragment of interest embedded with a human Aβ1-m gene located between a SalI restriction enzyme site and a mEagI restriction enzyme site. That is, (1) N2 copies of Aβ1-m gene are merely embedded into loop2, and the P protein prepared by this method is abbreviated as P protein-N2copy-Aβ1-m-loop2; (2) N3 copies of Aβ1-m gene are merely embedded into loop3, and the P protein prepared by this method is abbreviated as P protein-N3copy-Aβ1-m-loop3; and (3) N2 copies of Aβ1-m gene are embedded into loop2 and N3 copies of Aβ1-m gene are embedded into loop3, and the P protein prepared by this method is abbreviated as P protein-N2copy-Aβ1-m-loop2-N3copy-Aβ1-m-loop3, wherein each m is independently selected from an integer ranging from 1 to 40.

The third is a P particle loop gene fragment of interest embedded with a human Aβ1-m gene located between a mKpnI restriction enzyme site and a mEagI restriction enzyme site. That is, (1) N1 copies of Aβ1-m gene are embedded into loop1 and N2 copies of Aβ1-m gene are embedded into loop2, and the P protein prepared by this method is abbreviated as protein-N1copy-Aβ1-m-loop1-N2copy-Aβ1-m-loop2; (2) N1 copies of Aβ1-m gene are embedded into loop1 and N3 copies of Aβ1-m gene are embedded into loop3, and the P protein prepared by this method is abbreviated as P protein-N1copy-Aβ1-m-loop1-N3copy-Aβ1-m-loop3; and (3) N1 copies of Aβ1-m gene are embedded into loop1, N2 copies of Aβ1-m gene are embedded into loop2 and N3 copies of Aβ1-m gene are embedded into loop3, and the P protein prepared by this method is abbreviated as P protein-N1copy-Aβ1-m-loop1-N2copy-Aβ1-m-loop2-N3copy-Aβ1-m-loop3, wherein each m is independently selected from an integer ranging from 1 to 40.

The above three synthesis schemes are illustrated as follows:

### 3.1 Scheme I:

### 3.1.1

The loop1 gene fragment embedded with DNA encoding 10 copies of Aβ1-6 peptide of P protein-10copy-Aβ1-6-loop1G₇₂ (as shown in Figure 2B) was synthesized. The sequence of the synthetic gene fragment is shown as SEQ ID NO: 143.

### 3.2 Scheme II

### 3.2.1

The loop2 gene fragment embedded with DNA encoding one copy of Aβ1-6 peptide of P protein-1copy-Aβ1-6-loop2S₁₄₉ (as shown in Figure 2C) was synthesized. The sequence of the synthetic gene fragment is shown as SEQ ID NO: 144.

### 3.2.2

The loop2 gene fragment embedded with DNA encoding 10 copies of Aβ1-6 peptide of P protein-10copy-Aβ1-6-loop2S₁₄₉ (as shown in Figure 2D) was synthesized. The sequence of the synthetic gene fragment is shown as SEQ ID NO: 145.

### 3.2.3

The loop3 gene fragment embedded with DNA encoding 20 copies of Aβ1-6 peptide of P protein-20copy-Aβ1-6-loop3G₁₆₉ (as shown in Figure 2E) was synthesized. The sequence of the synthetic gene fragment is shown as SEQ ID NO: 146.

### 3.2.4

The loop2 gene fragment embedded with DNA encoding 3 copies of Aβ1-12 peptide and loop3 gene fragment embedded with DNA encoding 3 copies of Aβ1-6 peptide of P protein-3copy-Aβ1-12-loop2S₁₄₉-3copy-Aβ1-6-loop3G₁₆₉ (as shown in Figure 2G) were synthesized. The sequence of the synthetic gene fragments is shown as SEQ ID NO: 147.

### 3.3 Scheme III

### 3.3.1

The loop1 gene fragment embedded with DNA encoding 10 copies of Aβ1-15 peptide and loop2 gene fragment embedded with DNA encoding 10 copies of Aβ1-6 peptide of P protein-10copy-Aβ1-15-looplG₇₂-10copy-Aβ1-6-loop2S₁₄₉ (as shown in Figure 2F) were synthesized. The sequence of the synthetic gene fragments is shown as SEQ ID NO: 148.

### 3.3.2

The loop1 gene fragment embedded with DNA encoding 1 copy of Aβ1-6 peptide and loop3 gene fragment embedded with DNA encoding 10 copies of Aβ1-6 peptide of P protein-1copy-Aβ1-6-loop1G₇₂-10copy-Aβ1-6-loop3S₁₆₉ (as shown in Figure 2H) were synthesized. The sequence of the synthetic gene fragments is shown as SEQ ID NO: 149.

### 3.3.3

The loop123 gene fragment embedded respectively with DNA encoding 1 copy of Aβ1-6 peptide of P protein-1copy-Aβ1-6-loop1G₇₂-1copy-Aβ1-6-loop2S₁₄₉-1copy-Aβ1-6-loop3G₁₆₉ (as shown in Figure 2I) was synthesized. The sequence of the synthetic gene fragment is shown as SEQ ID NO: 150.

### 3.3.4

The loop1, loop2, loop3 gene fragments embedded respectively with DNA encoding 3 copies of Aβ1-6 peptide of P protein-3 copy-Aβ1-6-loop1G₇₂-3copy-Aβ1-6-loop2S₁₄₉-3copy-Aβ1-6-loop3G₁₆₉ (as shown in Figure 2J) were synthesized. The sequence of the synthetic gene fragments is shown as SEQ ID NO: 151.

### 3.3.5

The loop1, loop2, loop3 gene fragments embedded respectively with DNA encoding 10 copies of Aβ1-6 peptide of Pprotein-10copy-Aβ1-6-loop1G₇₂-10copy-Aβ1-6-loop2S₁₄₉-10copy-Aβ1-6-loop3G₁₆₉ (as shown in Figure 2K) were synthesized. The sequence of the synthetic gene fragments is shown as SEQ ID NO: 152.

Furthermore, in addition to the above 10 recombinant P proteins, synthetic methods of other 117 recombinant P proteins respectively involved the above three schemes, as specifically shown in Table 2.

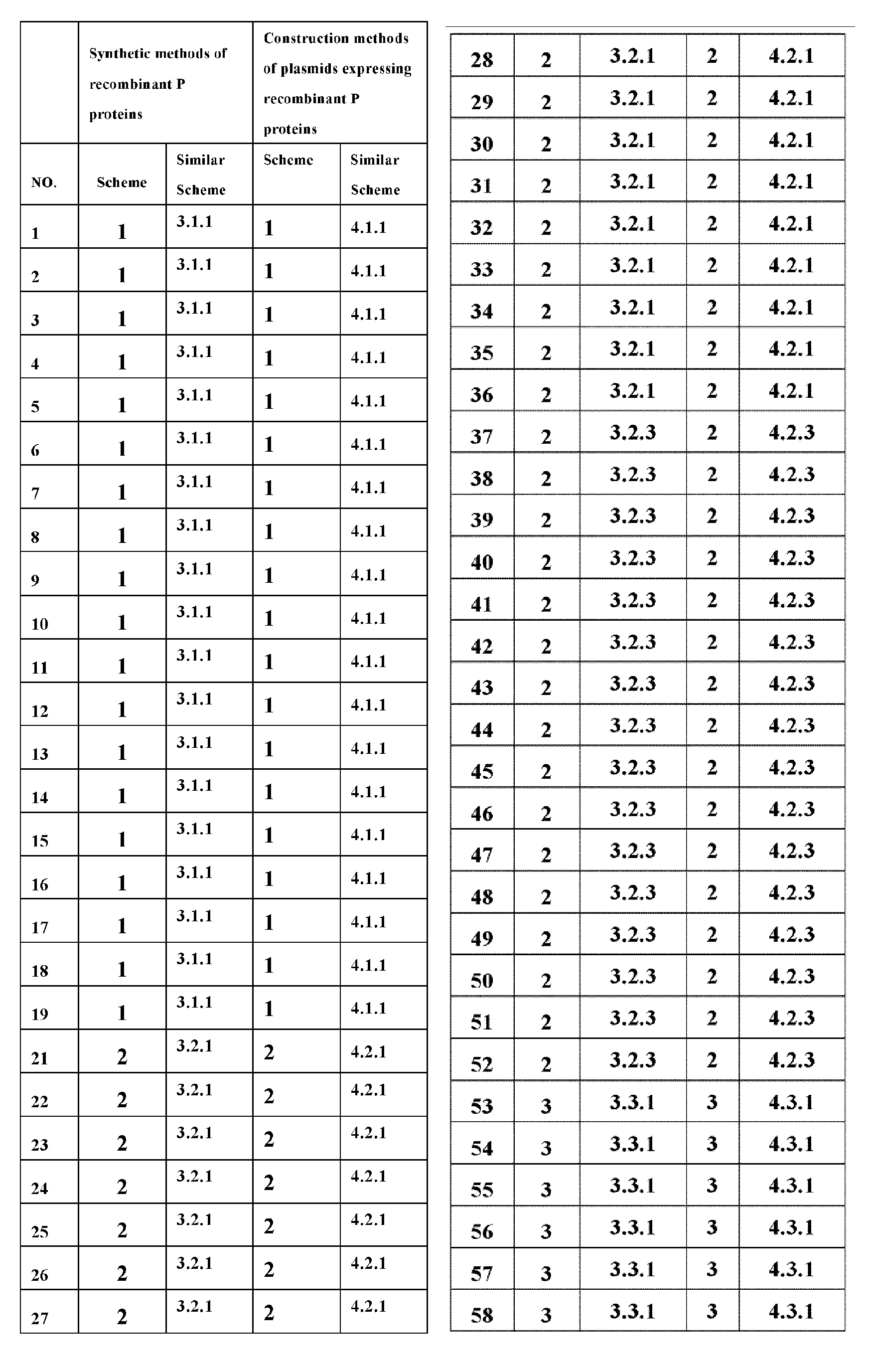

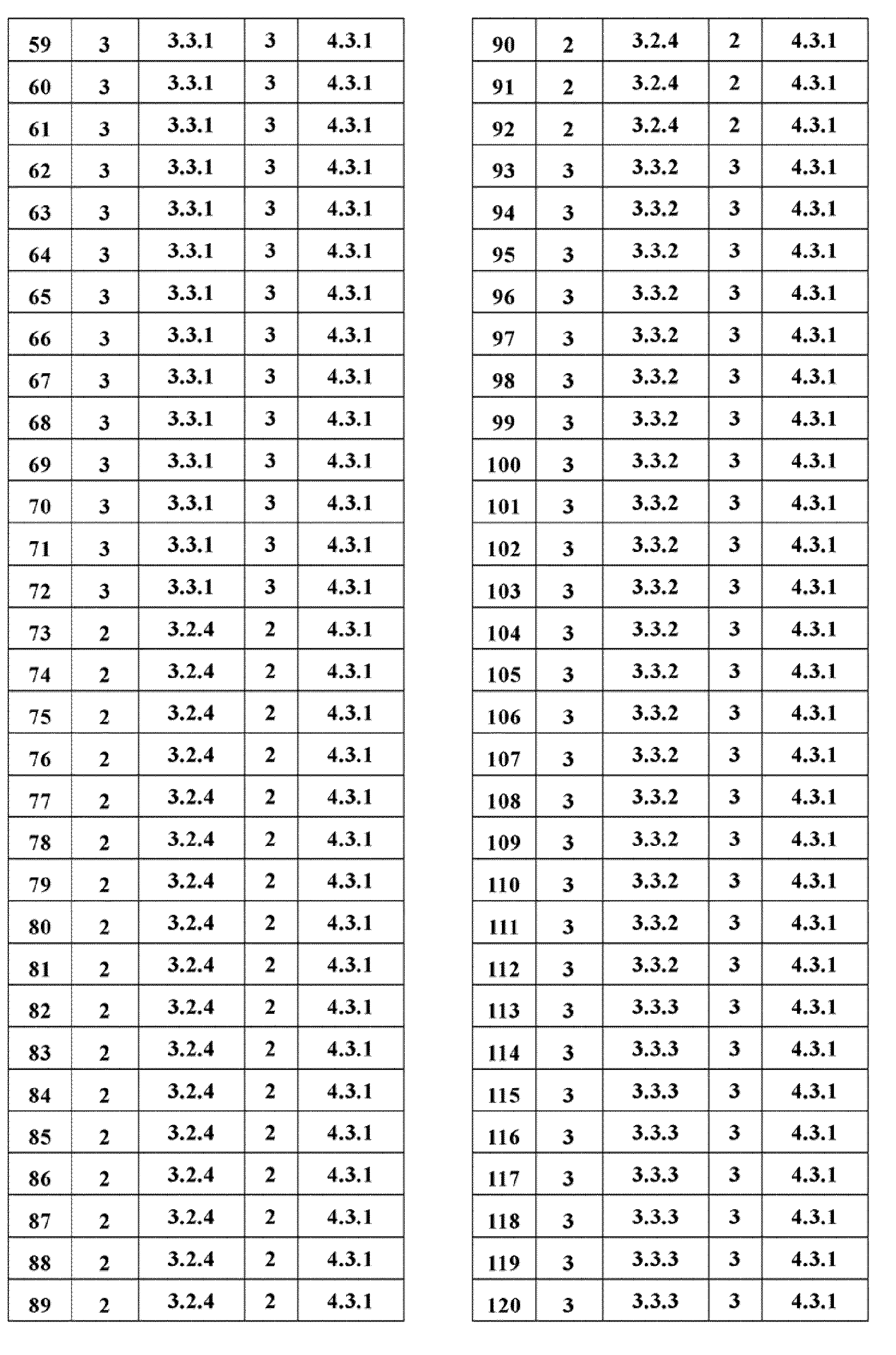

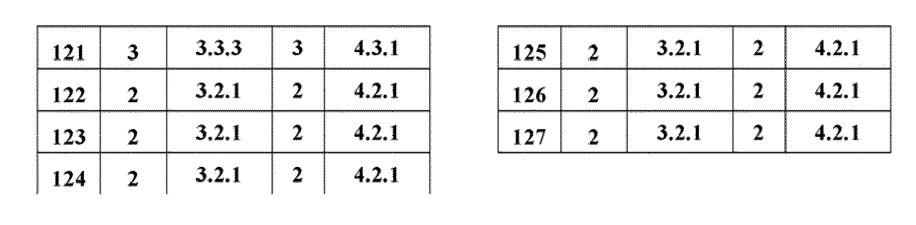

### Example 4. Construction of a pET26b vector stably expressing a P protein embedded with an Aβ1-m immunogen

Corresponding to Example 3, this step involves three different synthesis schemes in total:
The first, directed at Scheme I of Example 3, is a P particle loop gene fragment of interest embedded with a human Aβ1-m gene located between a mKpnI restriction enzyme site and a SalI restriction enzyme site. That is, N1 copies of Aβ1-m gene are merely embedded into loop1. The P protein prepared by this method is abbreviated as P protein-N1copy-Aβ1-m-loop1.

The second, directed at Scheme II of Example 3, is a P particle loop gene fragment of interest embedded with a human Aβ1-m gene located between a SalI restriction enzyme site and a mEagI restriction enzyme site. That is, (1) N2 copies of Aβ1-m gene are merely embedded into loop2, and the P protein prepared by this method is abbreviated as P protein-N2copy-Aβ1-m-loop2; (2) N3 copies of Aβ1-m gene are merely embedded into loop3, and the P protein prepared by this method is abbreviated as P protein-N3copy-Aβ1-m-loop3; and (3) N2 copies of Aβ1-m gene are embedded into loop2 and N3 copies of Aβ1-m gene are embedded into loop3, and the P protein prepared by this method is abbreviated as P protein-N2copy-Aβ1-m-loop2-N3 copy-Aβ1-m-loop3.

The third, directed at Scheme III of synthesizing a P protein embedded with an Aβ1-m immunogen in Example 3, is a P particle loop gene fragment of interest embedded with a human Aβ1-m gene located between a mKpnI restriction enzyme site and a mEagI restriction enzyme site. That is, (1) N1 copies of Aβ1-m gene are embedded into loop1 and N2 copies of Aβ1-m gene are embedded into loop2, and the P protein prepared by this method is abbreviated as P protein-N1copy-Aβ1-m-loop1-N2copy-Aβ1-m-loop2; (2) N1 copies of Aβ1-m gene are embedded into loop1 and N3 copies of Aβ1-m gene are embedded into loop3, and the P protein prepared by this method is abbreviated as P protein-N1copy-Aβ1-m-loop1-N3copy-Aβ1-m-loop3; and (3) N1 copies of Aβ1-m gene are embedded into loop1, N2 copies of Aβ1-m gene are embedded into loop2 and N3 copies of Aβ1-m gene are embedded into loop3, and the P protein prepared by this method is abbreviated as P protein-N1copy-Aβ1-m-loop1-N2copy-Aβ1-m-loop2-N3copy-Aβ1-m-loop3.

The above three synthesis schemes are illustrated as follows:

### 4.1 Scheme I:

The pET26b-P protein-mEagI-mKpnI plasmid vector obtained in Example 2 was subjected to SalI/KpnI double-enzyme digestion, using the mKpnI restriction enzyme site obtained by mutation and the SalI restriction enzyme site carried by the sequence itself, in order to excise the original loop1 region. The plasmid was recovered as the vector.

Meanwhile, the recombinant loop1 DNA fragment comprising multiple copies of the human Aβ1-m sequence synthesized in Example 3 was subjected to SalI/KpnI double-enzyme digestion in order to obtain the DNA fragment of interest.

The digested DNA fragment of interest was ligated to the digested vector in order to obtain a pET26b plasmid that can express a recombinant P protein embedded with an Aβ1-m immunogen. Finally, the plasmid was verified by sequencing, and thus a correct plasmid was obtained.

### 4.1.1 Construction of a plasmid vector expressing PP-10copy-Aβ1-6-loop1G₇₂ protein

1 µL of Sal I enzyme and 1 µL of Kpn I enzyme (purchased from Takara Corporation) and 5 µL of digestion buffer (purchased from Takara Corporation) were added respectively to 4 µg of pET26b-P protein-mEagI-mKpnI vector plasmid obtained in Example 2, and finally sterile water was added to the system to reach a final volume of 50 µL. Digestion was carried out at 37°C for 5 hours. The digested products were subjected to agarose gel electrophoresis, and recovered using gel recovery kit (purchased from Tiangen Biotech Co., Ltd.) to obtain a plasmid vector having sticky ends.

Meanwhile, the DNA fragment 10copy-Aβ1-6-loop1G₇₂ synthesized in Example 3 was subjected to SalI/KpnI double-enzyme digestion by the same method to obtain the gene fragment of interest.

The digested vector was mixed with the digested gene fragment of interest. 0.75 µL of T4 ligase (purchased from Takara Corporation) and 1.5 µL of ligase buffer (purchased from Takara Corporation) were added to the mixture. The ligation was carried out at 16°C overnight to obtain a plasmid vector expressing PP-10copy-Aβ1-6-loop1G₇₂ protein. The plasmid was verified by sequencing, as shown in Figure ID.

### 4.2 Scheme II

The pET26b-P protein-mEagI-mKpnI plasmid vector obtained in Example 2 was subjected to SalI/EagI double-enzyme digestion, using the mKpnI restriction enzyme site obtained by mutation and the SalI restriction enzyme site carried by the sequence itself, in order to excise the original loop2 and loop3 regions. The plasmid was recovered as the vector.

Meanwhile, the recombinant loop2 and loop3 DNA fragments comprising multiple copies of the human Aβ1-m sequence synthesized in Example 3 was subjected to SalI/EagI double-enzyme digestion to obtain the DNA fragment of interest.

The digested DNA fragment of interest was ligated to the digested vector to obtain a pET26b plasmid that can express a recombinant P protein embedded with an Aβ1-m immunogen. Finally, the plasmid was verified by sequencing, and thus a correct plasmid was obtained.

### 4.2.1 Construction of a plasmid vector expressing PP-1copy-Aβ1-6-loop2S₁₄₉ protein

1 µL of SalI enzyme and 1 µL of EagI enzyme (purchased from Takara Corporation) and an appropriate amount of digestion buffer (purchased from Takara Corporation) were respectively added to 4 µg of the pET26b-P protein-mEagI-mKpnI plasmid vector obtained in Example 2, , and finally sterile water was added to reach a final volume of 50 µL. Digestion was carried out at 37°C for 5 hours. The digested products were subjected to agarose gel electrophoresis, and recovered using gel recovery kit (purchased from Tiangen Biotech Co., Ltd.) to obtain a plasmid vector having sticky ends.

Meanwhile, the DNA fragment 1copy-Aβ1-6-loop2S₁₄₉ synthesized in Example 3 was subjected to SalI/KpnI double-enzyme digestion by the same method to obtain the gene fragment of interest.

The digested vector was mixed with the digested gene fragment of interest. 0.75 µL of T4 ligase (purchased from Takara Corporation) and 1.5 µL of ligase buffer (purchased from Takara Corporation) were added to the mixture. The ligation was carried out at 16°C overnight to obtain a plasmid vector expressing PP-1copy-Aβ1-6-loop2S₁₄₉ protein. The plasmid was verified by sequencing, as shown in Figure IE.

### 4.2.2 Construction of a plasmid vector expressing PP-10copy-Aβ1-6-loop2S₁₄₉ protein

A plasmid vector expressing PP-10copy-Aβ1-6-loop2S₁₄₉ protein was constructed, using the same method as 4.2.1. The plasmid is shown in Figure IF.

### 4.2.3 Construction of a plasmid vector expressing PP-20copy-Aβ1-6-loop3G₁₆₉ protein

A plasmid vector expressing PP-20copy-Aβ1-6-loop3G₁₆₉ protein was constructed, using the same method as 4.2.1. The plasmid is shown in Figure 1G.

### 4.2.4 Construction of a plasmid vector expressing PP-3copy-Aβ1-12-loopS₁₄₉-3copy-Aβ1-6-loop3G₁₆₉ protein

A plasmid vector expressing PP-3copy-Aβ1-12-loopS₁₄₉-3copy-Aβ1-6-loop3G₁₆₉ protein was constructed, using the same method as 4.2.1. The plasmid is shown in Figure 1I.

### 4.3 Scheme III

The pET26b-P protein-mEagI-mKpnI plasmid vector obtained in Example 2 was subjected to mKpnI/mEagI double-enzyme digestion, using the mEagI restriction enzyme site and the mKpnI restriction enzyme site obtained by mutation, in order to excise the original loop1, loop2 and loop3 regions. The plasmid was recovered as the vector.

Meanwhile, the recombinant loop1, loop2 and loop3 DNA fragments comprising multiple copies of the human Aβ1-m sequence synthesized in Example 3 was subjected to mKpnI/mEagI double-enzyme digestion in order to obtain the DNA fragment of interest.

The digested DNA fragment of interest was ligated to the digested vector in order to obtain a pET26b plasmid that can express a recombinant P protein embedded with an Aβ1-m immunogen. Finally, the plasmid was verified by sequencing, and thus a correct plasmid was obtained.

### 4.3.1 Construction of a plasmid vector expressing PP-3copy-Aβ1-6-loop1-G₇₂-3copy-Aβ1-6-loop2S₁₄₉-3copy-Aβ1-6-loop3G₁₆₉ protein

1 µL of KpnI enzyme and 1 µL of EagI enzyme (purchased from Takara Corporation) and an appropriate amount of digestion buffer (purchased from Takara Corporation) were added respectively to 4 µg of the pET26b-P protein-mEagI-mKpnI vector plasmid obtained in Example 2, and finally sterile water was added to reach a final volume of 50 µL. Digetsion was carried out at 37°C for 5 hours. The digested products were subjected to agarose gel electrophoresis, and recovered using gel recovery kit (purchased from Tiangen Biotech Co., Ltd.) to obtain a plasmid vector having sticky ends.

Meanwhile, the DNA fragment 3copy-Aβ1-6-loop1G₇₂-3copy-Aβ1-6-loop2S₁₄₉-3copy-Aβ1-6-loop3G₁₆₉ synthesized in Example 3 was subjected to EagI/KpnI double-enzyme digestion by the same method to obtain the gene fragment of interest.

The digested vector was mixed with the digested gene fragment of interest. 0.75 µL of T4 ligase (purchased from Takara Corporation) and 1.5 µL of ligase buffer (purchased from Takara Corporation) were added to the mixture. The ligation was carried out at 16°C overnight to obtain a plasmid vector expressing PP-1copy-Aβ1-6-loop2S₁₄₉ protein. The plasmid was verified by sequencing, as shown in Figure 1L.

### 4.3.2 Construction of a plasmid vector expressing PP-10copy-Aβ1-15-loop1G₇₂-10copy-Aβ1-6-loop2S₁₄₉ protein

A plasmid vector expressing PP-10copy-Aβ1-15-loopIG₇₂-10copy-Aβ1-6-loop2S₁₄₉ protein was constructed, using the same method as 4.3.1. The plasmid is shown in Figure 1H.

### 4.3.3 Construction of a plasmid vector expressing PP-1copy-Aβ1-6-loop1G₇₂-10copy-Aβ1-6-loop3G₁₆₉ protein

A plasmid vector expressing PP-1copy-Aβ1-6-loop1G₇₂-10copy-Aβ1-6-loop3G₁₆₉ protein was constructed, using the same method as 4.3.1. The plasmid is shown in Figure 1J.

### 4.3.4 Construction of a plasmid vector expressing PP-1copy-Aβ1-6-loop1G₇₂-1copy-Aβ1-6-loop2S₁₄₉-1copy-Aβ1-6-loop3G₁₆₉ protein

A plasmid vector expressing PP-1copy-Aβ1-6-loop1G₇₂-1copy-Aβ1-6-loop2S₁₄₉-1copy-Aβ1-6-loop3G₁₆₉ protein was constructed, using the same method as 4.3.1. The plasmid is shown in Figure 1K.

### 4.3.5 Construction of a plasmid vector expressing PP-10copy-Aβ1-6-loop1G₇₂-10copy-Aβ1-6-loop2S₁₄₉-10copy-Aβ1-6-loop3G₁₆₉ protein

A plasmid vector expressing PP-10copy-Aβ1-6-loop1G₇₂-10copy-Aβ1-6-loop2S₁₄₉-10copy-Aβ1-6-loop3G₁₆₉ protein was constructed, using the same method as 4.3.1. The plasmid is shown in Figure 1M.

Furthermore, in addition to the above 10 recombinant P proteins, construction methods of other 117 plasmids expressing recombinant P proteins respectively involve the above three schemes, specifically as shown in Table 2. The obtained recombinant plasmids are not shown.

### Example 5 Expression and purification of a P protein embedded with a human Aβ1-m immunogen

### 5.1 Expression of a recombinant P particle protein

1 µL of the recombinant plasmids prepared in the above examples were respectively added to 100 µL of *E. coli* BL21 competent cells (purchased from TransGen Corporation), ice-bathed for 30 min, heat shocked for 90 s in a water-bath at 42°C, and then ice-bathed for 2 min. 600 µL of LB medium was added to the mixture, and cultured at 180 rpm/min at 37°C for 1 h. The mixture was coated evenly on a LB solid medium containing kanamycin (15 µg/mL) resistance and cultured at 37°C for 24 h to obtain strains that can stably express recombinant proteins. A growing colony was picked and inoculated into 20 mL of LB medium. The mixture was cultured at 220 rpm at 37°C. When the OD value of the culture mixture reached 1.0, induction by Isopropyl β-D-Thiogalactoside (IPTG at a final concentration of 0.33 mmol/L) was carried out at 220 rpm at 16°C overnight. After the induction, the culture broth was centrifuged at 4000 rpm for 20 min. The supernatant was discarded, and the bacterial precipitates were resuspended with PBS. Centrifugation was conducted again at 4000 rpm for 20 min and the supernatant was discarded to obtain the bacterial precipitates containing proteins of interest.

### 5.2 Extraction and purification of recombinant P particle proteins

The bacterial precipitates obtained in 5.1 were resuspended by adding 20 mL of protein buffer (pH8.0, containing 50 mM Tris and 300 mM KCl). The bacteria were lysed by sonication on ice for 30 min. The mixture was centrifuged at 12,000 rpm at 4°C for 30 min. Subsequently, the supernatant was taken and allowed to pass through 0.45 µm filter membrane to obtain crude extracts of proteins.

The structures of recombinant P particle proteins are shown as Figure 3A-J.

The crude extracts of proteins were purified using a cation exchange column (purchased from GE Corporation). The specific scheme was as follows: First, the exchange column was rinsed with ultrapure water in a volume of about 100 mL, followed by equilibration with PB solution (pH 5.0) at a flow rate of 2 mL/min. Then 20 mL of the crude extracts of proteins were added to the exchange column at a flow rate of 1 ml/min. After the sample was completely loaded onto the column, the exchange column was rinsed with PB solution (pH 7.0) to remove protein impurities, and then eluted with PB solution containing 1 mol/L NaCl. The proteins at peak value were collected to obtain the proteins of interest.

The proteins were further purified by a hydrophobic chromatography column (purchased from GE Corporation). The specific scheme was as follows: First, the column was rinsed with ultrapure water, and then with PB solution (pH 7.0) at a flow rate of 2 mL/min. After the column was equilibrated well, the protein sample was loaded onto it. After the sample was completely loaded onto the column, the column was eluted by gradient with PB (pH 7.0) and 1 mol/L NaCl solution for 2 hours. The concentration of NaCl decreases from 1 mol/L to 0.1 mol/L. The proteins were collected at peak value.

The sizes of 10 P particle protein monomers were identified by reductive SDS-PAGE. The upper panels of Figures 4A-J respectively show that the size of PP-10copy-Aβ1-6-loop1G₇₂ protein is 45 KD; the size of PP-1copy-Aβ1-6-loop2S₁₄₉ protein is 37 KD; the size of PP-10copy-Aβ1-6-loopS₁₄₉ protein is 45 KD; the size of PP-20copy-Aβ1-6-loop3G₁₆₉ protein is 55 KD; the size of PP-10copy-Aβ1-15-loopIG₇₂-10copy-Aβ1-6-loop2S₁₄₉ protein is 66 KD; the size of PP-3copy-Aβ1-12-loop2S₁₄₉-3copy-Aβ1-6-loop3G₁₆₉ protein is 38 KD; the size of PP-1copy-Aβ1-6-loop1G₇₂-10copy-Aβ1-6-loop3G₁₆₉ protein is 47 KD; the size of PP-1copy-Aβ1-6-loop1G₇₂-1copy-Aβ1-6-loop2S ₁₄₉-1copy-Aβ1-6-loop3G₁₆₉ protein is 3 KD; the size of PP-3copy-Aβ1-6-loop1G₇₂-3copy-Aβ1-6-loop2S₁₄₉-3copy-Aβ1-6-loop3G₁₆₉ protein is 45 KD; and the size of PP-10copy-Aβ1-6-loop1G₇₂-10copy-Aβ1-6-loop2S₁₄₉-10copy-Aβ1-6-loop3G₁₆₉ is 66 KD

Then the tetracosamers of P protein particles were further isolated and purified using a Superdex 200 molecular sieve (purchased from GE Corporation). The procedures were as follows: The column was rinsed with ultrapure water at a flow rate of 1 mL/min for one volume of the column, and then again with about 120 mL of PB buffer (pH 5). After that, 2 mL of protein extraction solution was added to the column and washed with PB buffer at a flow rate of 1 mL/min. The proteins at peak value were collected to obtain the tetracosamers of P particle proteins. Three proteins were tested for their polymer structures by native polyacrylamide gel electrophoresis, as shown in the middle panels of Figures 4A-J. All ten protein bands are above 225 KDa, which indicates that recombinant proteins can self-assemble into tetracosamers of P protein particles, and they can remain their polymer form after being purified.

### Example 6 Characterization of a P protein particle embedded with a human Aβ1-m immunogen

Inventors further analyzed the particle diameter and morphology of 10 protein polymers. The particle diameter was tested using a nanoparticle diameter analyzer (purchased from Malvern Corporation) in accordance with the instructions of the manufacturer. The analysis results showed that there were particles having an average diameter of about 20 nm in the solution of the above 10 recombinant P particles. As shown in the lower panels of Figures 4A-J, PP-10copy-Aβ1-6-loop1G₇₂ protein particle has a particle diameter of 27.88 nm; PP-1copy-Aβ1-6-loop2S₁₄₉ protein particle has a particle diameter of 17.44 nm; PP-10copy-Aβ1-6-loop2S₁₄₉ protein particle has a particle diameter of 27.64 nm; PP-20copy-Aβ1-6-loop3G₁₆₉ protein particle has a particle diameter of 32.55 nm; PP-10copy-Aβ1-15-loopIG₇₂-10copy-Aβ1-6-loop2S₁₄₉ protein particle has a particle diameter of 35.88 nm; PP-3copy-Aβ1-12-loop2S₁₄₉-3copy-Aβ1-6-loop3G₁₆₉ protein particle has a particle diameter of 17.02 nm; PP-1copy-Aβ1-6-looplG₇₂-10copy-Aβ1-6-loop3G₁₆₉ protein particle has a particle diameter of 28.92 nm; PP-1copy-Aβ1-6-loop1G₇₂-1copy-Aβ1-6-loop2S₁₄₉-1copy-Aβ1-6-loop3G₁₆₉ protein particle has a particle diameter of 18.14 nm; PP-3 copy-Aβ1-6-loop1G₇₂-3copy-Aβ1-6-loop2S₁₄₉-3copy-Aβ1-6-loop3G₁₆₉ protein particle has a particle diameter of 25.56 nm; and PP-10copy-Aβ1-6-loop1G₇₂-10copy-Aβ1-6-loop2S₁₄₉-10copy-Aβ1-6-loop3G₁₆₉ protein particle has a particle diameter of 36.09 nm. Meanwhile, the results of electron microscope tests show that the recombinant P proteins are in the form of approximately spherical particles, and in the form of polymers. According to the ruler, 10 protein polymers mainly comprise particles of about 20 nm. Thus, all the above 10 recombinant P proteins can self-assemble *in vitro* to form tetracosamers of P particles. The sizes and P particle diameters of the 127 P particle proteins according to the invention are shown as Table 3.

**Table 3. The sizes and P particle diameters of 127 different forms of recombinant P particle proteins**

| **NO.** | **Size of Protein (KD)** | **P particle diameter nm)** |
|---|---|---|
| **1** | **36.2** | **17.75** |
| **2** | **48.4** | **31.23** |
| **3** | **68.2** | **36.37** |
| **4** | **114.4** | **39.86** |
| **5** | **36.6** | **16.98** |
| **6** | **51.7** | **31.53** |
| **7** | **74.8** | **38.13** |
| **8** | **74.8** | **38.2** |
| **9** | **36.2** | **16.98** |
| **10** | **45.1** | **27.88** |
| **11** | **55.0** | **32.07** |
| **12** | **74.8** | **37.89** |
| **13** | **36.9** | **17.89** |
| **14** | **55.0** | **31.99** |
| **15** | **55.0** | **31.68** |
| **16** | **88.0** | **39.54** |
| **17** | **37.2** | **17.89** |
| **18** | **45.1** | **18.03** |
| **19** | **61.6** | **34.66** |
| **20** | **101.2** | **39.75** |
| **21** | **36.5** | **16.77** |
| **22** | **48.4** | **31.15** |
| **23** | **61.6** | **34.15** |
| **24** | **88.0** | **39.65** |
| **25** | **36.2** | **17.44** |
| **26** | **45.1** | **27.64** |
| **27** | **55.0** | **31.97** |
| **28** | **74.8** | **38.17** |
| **29** | **36.9** | **17.95** |
| **30** | **51.7** | **31.67** |
| **31** | **68.2** | **36.34** |
| **32** | **101.2** | **39.67** |
| **33** | **37.2** | **18.01** |
| **34** | **55.0** | **32.01** |
| **35** | **74.8** | **38.22** |
| **36** | **114.4** | **39.92** |
| **37** | **36.9** | **18.02** |
| **38** | **51.7** | **31.42** |
| **39** | **68.2** | **36.41** |
| **40** | **101.2** | **40.11** |
| **41** | **36.2** | **17.51** |
| **42** | **45.1** | **27.61** |
| **43** | **55.0** | **32.55** |
| **44** | **74.8** | **38.21** |
| **45** | **37.2** | **18.13** |
| **46** | **55.0** | **31.87** |
| **47** | **74.8** | **38.17** |
| **48** | **114.4** | **40.17** |
| **49** | **36.5** | **18.16** |
| **50** | **48.4** | **31.26** |
| **51** | **61.6** | **36.18** |
| **52** | **88.0** | **39.18** |
| **53** | **41.5** | **25.06** |
| **54** | **61.9** | **34.09** |
| **55** | **101.5** | **40.06** |
| **56** | **193.9** | **45.3** |
| **57** | **38.2** | **19.2** |
| **58** | **42.5** | **26.2** |
| **59** | **65.2** | **35.88** |
| **60** | **101.5** | **39.83** |
| **61** | **144.4** | **43.7** |
| **62** | **154.3** | **44.01** |
| **63** | **46.4** | **30.31** |
| **64** | **75.1** | **31.88** |
| **65** | **134.5** | **42.67** |
| **66** | **134.5** | **43.12** |
| **67** | **53.0** | **31.99** |
| **68** | **68.5** | **36.28** |
| **69** | **114.7** | **39.67** |
| **70** | **127.9** | **40.02** |
| **71** | **91.6** | **39.77** |
| **72** | **53.4** | **31.89** |
| **73** | **49.4** | **18.24** |
| **74** | **68.5** | **36.44** |
| **75** | **75.1** | **38.17** |
| **76** | **141.1** | **42.55** |
| **77** | **37.8** | **17.1** |
| **78** | **43.5** | **17.02** |
| **79** | **68.5** | **35.21** |
| **80** | **94.9** | **39.62** |
| **81** | **124.6** | **41.41** |
| **82** | **180.7** | **45.23** |
| **83** | **46.4** | **28.31** |
| **84** | **75.1** | **38.21** |
| **85** | **134.5** | **42.71** |
| **86** | **134.5** | **41.93** |
| **87** | **49.7** | **31.19** |
| **88** | **75.1** | **38.38** |
| **89** | **147.7** | **44.22** |
| **90** | **147.7** | **43.91** |
| **91** | **78.4** | **39.4** |
| **92** | **50.7** | **31.87** |
| **93** | **39.4** | **18.31** |
| **94** | **61.9** | **34.67** |
| **95** | **75.1** | **38.09** |
| **96** | **167.5** | **45.8** |
| **97** | **37.8** | **18.45** |
| **98** | **43.4** | **27.67** |
| **99** | **68.5** | **36.38** |
| **100** | **94.9** | **40.01** |
| **101** | **144.4** | **43.96** |
| **102** | **154.3** | **44.38** |
| **103** | **46.4** | **28.92** |
| **104** | **75.1** | **38.22** |
| **105** | **134.5** | **42.51** |
| **106** | **134.5** | **42.79** |
| **107** | **49.7** | **31.33** |
| **108** | **55.0** | **32.09** |
| **109** | **54.0** | **32.91** |
| **110** | **141.1** | **43.68** |
| **111** | **121.3** | **40.03** |
| **112** | **109.8** | **38.88** |
| **113** | **83.1** | **39.44** |
| **114** | **141.5** | **43.21** |
| **115** | **60.6** | **35.87** |
| **116** | **134.9** | **42.57** |
| **117** | **65.6** | **36.12** |
| **118** | **38.8** | **18.14** |
| **119** | **44.8** | **25.56** |
| **120** | **65.6** | **36.09** |
| **121** | **154.7** | **44.11** |
| **122** | **41.5** | **20.45** |
| **123** | **42.7** | **24.24** |
| **124** | **47.5** | **28.99** |
| **125** | **53.6** | **32.01** |
| **126** | **45.1** | **27.64** |
| **127** | **38.9** | **18.15** |

### 3. Immunological effects of recombinant P particle protein vaccines

### 3.1 Determination of immune dosages and adjuvants of P particle protein vaccines

The PP-10copy-Aβ1-6-loop2S₁₄₉ protein vaccine was selected for use in female C57BL/6 mice aged 6-8 weeks (purchased from Beijing HFK Bioscience Co., LTD) to determine the immune dosage and immune adjuvant. The immune dosages were respectively 12.5 µg, 25 µg and 50 µg/animal, and were increased to 100 µL/animal with sterile PBS. Each group contained 6 mice. The immunization was performed by subcutaneous injection. The immune adjuvants were an aluminium adjuvant (purchased from Brenntag Biosector Corporation) and an CpG adjuvant that can specifically stimulate the body to produce humoral immunity (purchased from Takara Corporation). The nucleotide sequence of CpG adjuvant was as follows: TGTCGTCGTCGTTTGTCGTTTGTCGTT (SEQ ID NO: 153). The immunization was carried out at day 1, day 15 and day 29 (three times in total). Blood was taken on the day before every immunization. The mice were sacrificed two weeks after the third immunization. The recombinant P particle protein vaccine was tested for immunological responses of the induced humoral immunity and cellular immunity in mice. There were 7 test groups and 3 control groups. The immune dosage, adjuvant and immunizing antigen for each group are shown in Table 4.

**Table 4. Immunization scheme of PP-10copy-Aβ1-6-loop2S₁₄₉**

| **Group** | **Immune dosage (100 µl)** | **Adjuvant** | **Immunizing antigen** |
|---|---|---|---|
| **Negative control group** | **-** | **-** | **-** |
| | **-** | **aluminium adjuvant (200 µg)** | **PP-10copy-Aβ1-6-loop2S₁₄₉** |
| | **-** | **CpG (10µg)** | **-** |
| **Test group** | **12.5µg** | **-** | **PP-10copy-Aβ1-6-loop2S₁₄₉** |
| | **25µg** | **-** | **PP-10copy-Aβ1-6-loop2S₁₄₉** |
| | **50µg** | **-** | **PP-10copy-Aβ1-6-loop2S₁₄₉** |
| | **25µg** | **aluminium adjuvant (200 µg)** | **PP-10copy-Aβ1-6-loop2S₁₄₉** |
| | **25µg** | **CpG (10µg)** | **PP-10copy-Aβ1-6-loop2S₁₄₉** |
| | **25µg** | **aluminium adjuvant+CpG (10 µβ)** | **PP-10copy-Aβ1-6-loop2S₁₄₉** |
| | **100 µg** | **Freund's adjuvant (100µl)** | **Aβ1-42** |

### 3.1.1 Humoral immunity-ELISA detection experiment

Tails of the mice were cut and blood was taken on the day before every immunization. Blood samples were placed at 37°C for 2 h, then placed at 4°C for 1 h, and centrifuged at 3,000 rpm to take the supernatant serum. The serum was frozen for use. Aβ1-42 (purchased from GL Biochem (Shanghai) Ltd.) was used as antigen and formulated into 1 mg/mL solution with sterile PBS. The solution was diluted to 1 ng/µL with antigen coating solution and used for coating a 96-well plate (100 µL/well). The plate was coated at 4°C overnight. After each well was washed three times with 300 µL of PBST (pH 7.4, 0.01 mol/L PBS, containing 0.05% Tween-20), blocking solution (pH 7.4, 0.01 mol/L PBS, 20% fetal bovine serum) was added. Blocking was carried out at 37°C for 2 hours. Each well was washed three times with PBST. To the wells were added different dilution gradients (1:200, 1:800, 1:3200, 1:12800, 1:51200 and 1:204800) of antiserum (100 µL/well), and incubation was carried out at 37°C for 1 hour. Each well was washed three times with PBST. To the wells were added 0.3 µg/ml of HPR (horse radish peroxidase) goat-anti-mouse secondary antibody (purchased from Beijing Dingguochangsheng Biotechnology Co. LTD) (100 µL/well), and incubation was carried out at 37°C for 1 hour. Each well was washed three times with PBST. To the wells were added the substrate tetramethylbenzidine (TMB) (purchased from Tiangen Biotech Co., Ltd.) (100 µL/well), and color was developed in the dark for 25 min. 50 µL of 2M sulfuric acid was added to each well to terminate the reaction. Absorbance was detected at 450 nm using a microplate reader (purchased from Bio-red Corporation).

Experimental results are shown in Figure 5A. When CpG is used as immune adjuvant and the immune dosage is 25 µg, or the immune dosage is 50 µg and no immune adjuvant is used, PP-10copy-Aβ1-6-loop2-S₁₄₉ protein vaccine at all the shown dilutions can stimulate the mouse to produce relatively high titers of specific antibodies against Aβ42.

### 3.1.2 Cellular immunity-ELISPOT detection

A 96-well plate was coated with the monoclonal antibody against cytokine interferon γ (from elispot kit purchased from BD Corporation) in a concentration of 5 µg/mL (50 µL/well), and covered at 4°C overnight. After discarding the coating antibody and washing once with a complete medium containing 10% fetal bovine serum, 200 µL of this complete medium was added to each well. Blocking was carried out at 37°C for 1 hour, and then the medium was discarded. Mice used in the experiment were sacrificed by neck-pulling. Spleen cells of the mice were taken out and formulated into a cell suspension in a concentration of 10⁷/mL. The cell suspension was added to the coated 96-well plate (100 µL/well). 100 µL of 1 µg/mL specific antigen Aβ1-42 was added to each well. The plate was then cultured at 37°C in an incubator containing 5% CO₂ for 24 h to stimulate and activate the cells. 24 h later, the plate was washed two times with sterile water, and six times with sterile PBST (pH7.4, 0.01 mol/L PBS, containing 0.05% Tween-20) buffer to wash the cells away. 50 µL of 2 µg/mL antibody against interferon γ (from elispot kit purchased from BD Corporation) was added to each well and incubated at room temperature for 2 hours. The 96-well plate was washed, and horse radish peroxidase labeled biotin secondary antibody (from elispot kit purchased from BD Corporation) was added (50 µL/well). The plate was cultured at room temperature for 2 h, and washed four times with PBST, and two times with PBS. 50 µL of Elispot color developing solution (AEC substrate) was added to each well and reacted in the dark at room temperature for 5-60 min. The staining solution was discarded, and the plate was washed with distilled water. After being dried overnight, the sample was calculated for the number of activated cells using a microscope.

The results are shown in Figure 5B. In the T cell response-positive control group Aβ42 group, a large number of spots emerge, which demonstrates a strong T cell response. In the 25 µg protein+aluminium adjuvant group, a large number of spots also emerge, which demonstrates that the aluminium adjuvant could stimulate the body to produce a certain T cell response. Nevertheless, the 25 µg PP-10copy-Aβ1-6-loop2-S₁₄₉+CpG adjuvant group and 50 µg group have no or fewer positive spots, which demonstrates no evident T cell response occurring in the body, and as described in 3.1.1, this immunization strategy can stimulate the mice to produce the highest titer of specific antibodies against Aβ42. Considering the safety of vaccines, 25µg PP-10copy-Aβ1-6-loop2S₁₄₉+CpG adjuvant was selected as the optimal immunization strategy.

### 3.2 Comparison of immunological effects of three recombinant P particle protein vaccines

After the experiments for determining immune dosages and immune adjuvants of protein vaccines, the applicant first selected three representative recombinant proteins, adopted the strategy of a protein vaccine dosage of 25 µg/animal and CpG as the adjuvant, compared immunological effects of the three recombinant P particle protein vaccines in female C57BL/6 mice aged 6-8 weeks by the two immunization routes via nasal and subcutaneous injections in order to compare the effects of nasal and subcutaneous immunizations, and identify the differences in immunological effects of various proteins. Similar to the method in 3.1.1, immunization was carried out every two weeks and four times in total. Tails of the mice were cut and blood was taken before every immunization. Serum was tested by ELISA detection. The mice were divided into 7 test groups and 3 control groups. The immunogen and immunization route for each group are shown as in Figure 5.

**Table 5. Immunization schemes of 3 representative P particle proteins**

| **Group** | **Immunogen** | **immunization route** |
|---|---|---|
| **Control group** | **PBS** | **subcutaneous** |
| | **PBS** | **nasal** |
| | **CpG** | **subcutaneous** |
| | **CpG** | **nasal** |
| **test group** | **PP-leopy-Aβ1-6-loop2S₁₄₉+CpG** | **subcutaneous** |
| | **PP-10copy-Aβ1-6-loop2S₁₄₉+CpG** | **subcutaneous** |
| | **PP-3copy-Aβ1-6-loop1G₇₂-3copy-Aβ1-6-loop2S₁₄₉-3 copy-Aβ1-6-loop3S₁₆₉+CpG** | **subcutaneous** |
| | **PP-1copy-Aβ1-6-loop2S₁₄₉+CpG** | nasal |
| | **PP-1 0copy-Aβ1-6-loop2S₁₄₉+CpG** | **nasal** |
| | **PP-3copy-Aβ1-6-loop1G₇₂-3copy-Aβ1-6-loop2S₁₄₉-3 copy-Aβ1-6-loop3S₁₆₉+CpG** | **nasal** |

An ELISA plate was coated with Aβ1-42 as the antigen, using the method described in 3.1.1. A reaction was carried out using the immunized mouse serum as the primary antibody, HRP goat-anti-mouse antibody as the secondary antibody, TMB as the substrate, and sulfuric acid as the termination solution. After the termination of reaction, absorbance was detected at 450 nm using a microplate reader. The contents of antibodies in the serum were compared based on the absorbance. Experiment was carried out using PBS as the negative control, and the commercial antibody 6e10 (purchased from Covance Corporation) as the positive control, and using the sera before and after immunizing mice with three proteins as the test groups. Results are shown in Figure 6. All the three proteins can stimulate the mouse body to produce specific antibodies against Aβ42, which demonstrates that the vaccines of the present invention have good immunogenicity. Standard curve was plotted using the commercial antibody 6e10 as the positive control in order to calculate the concentration of antibodies. The comparison results of immunological effects are shown in Figure 6. The two immunization routes of the three proteins can all produce specific antibodies against Aβ42, the OD values of which are all significantly higher than those of the PBS group and CpG group. Moreover, with the increase of immunization times, the concentration of the antibody continuously increases and can reach the highest value at the fourth immunization. By comparison, it can be seen that the subcutaneous immunization has better effect than the nasal immunization.

### 3.3 Comparison of immunological effects of various recombinant P particle protein vaccines

The applicant determined that the adopted immune dosage was 25 µg/animal and the immunization route was subcutaneous injection by the experiments for determining immune dosages and immune adjuvants of protein vaccines, and immunological experiments of three representative recombinant proteins, and determined the immunological effects of 127 candidate vaccines in female C57BL/6 mice aged 6-8 weeks. Immunization procedures and method are as described in 3.2. Comparison results of immunological effects are shown in Table 6.

**Table 6 Concentrations of Aβ42 antibody produced by mice stimulated with protein vaccines having 127 different forms of P particles as the immunogens**

| **Group number** | **Concentration of the produced Aβ42 antibody after the fourth immunization (µg/ml)** |
|---|---|
| **1** | **94.88** |
| **2** | **90.28** |
| **3** | **64.55** |
| **4** | **29.09** |
| **5** | **85.62** |
| **6** | **77.10** |
| **7** | **65.75** |
| **8** | **55.98** |
| **9** | **101.79** |
| **10** | **153.34** |
| **11** | **103.01** |
| **12** | **63.67** |
| **13** | **38.57** |
| **14** | **40.87** |
| **15** | **48.89** |
| **16** | **34.82** |
| **17** | **89.98** |
| **18** | **111.76** |
| **19** | **77.09** |
| **20** | **38.78** |
| **21** | **105.87** |
| **22** | **162.36** |
| **23** | **55.38** |
| **24** | **44.70** |
| **25** | **162.78** |
| **26** | **189.83** |
| **27** | **137.62** |
| **28** | **82.09** |
| **29** | **187.23** |
| **30** | **83.42** |
| **31** | **56.67** |
| **32** | **54.31** |
| **33** | **87.47** |
| **34** | **77.21** |
| **35** | **68.47** |
| **36** | **42.12** |
| **37** | **98.09** |
| **38** | **67.92** |
| **39** | **21.83** |
| **40** | **34.01** |
| **41** | **108.53** |
| **42** | **145.61** |
| **43** | **158.96** |
| **44** | **43.57** |
| **45** | **76.37** |
| **46** | **64.89** |
| **47** | **56.17** |
| **48** | **29.02** |
| **49** | **68.78** |
| **50** | **75.26** |
| **51** | **77.15** |
| **52** | **39.58** |
| **53** | **130.09** |
| **54** | **144.98** |
| **55** | **36.75** |
| **56** | **24.98** |
| **57** | **99.90** |
| **58** | **78.79** |
| **59** | **178.46** |
| **60** | **71.14** |
| **61** | **88.82** |
| **62** | **22.19** |
| **63** | **109.70** |
| **64** | **142.06** |
| **65** | **20.08** |
| **66** | **19.98** |
| **67** | **111.49** |
| **68** | **109.15** |
| **69** | **41.78** |
| **70** | **39.75** |
| **71** | **96.58** |
| **72** | **123.43** |
| **73** | **169.92** |
| **74** | **178.83** |
| **75** | **120.47** |
| **76** | **21.10** |
| **77** | **102.80** |
| **78** | **197.09** |
| **79** | **95.03** |
| **80** | **76.78** |
| **81** | **55.87** |
| **82** | **23.56** |
| **83** | **139.92** |
| **84** | **159.80** |
| **85** | **25.73** |
| **86** | **33.09** |
| **87** | **87.82** |
| **88** | **58.97** |
| **89** | **27.41** |
| **90** | **24.46** |
| **91** | **73.45** |
| **92** | **82.67** |
| **93** | **168.21** |
| **94** | **116.81** |
| **95** | **82.39** |
| **96** | **43.08** |
| **97** | **142.97** |
| **98** | **158.03** |
| **99** | **103.64** |
| **100** | **59.82** |
| **101** | **21.11** |
| **102** | **11.87** |
| **103** | **165.27** |
| **104** | **66.72** |
| **105** | **47.86** |
| **106** | **34.56** |
| **107** | **150.98** |
| **108** | **75.73** |
| **109** | **99.17** |
| **110** | **45.43** |
| **111** | **31.25** |
| **112** | **67.87** |
| **113** | **155.87** |
| **114** | **101.23** |
| **115** | **135.15** |
| **116** | **79.75** |
| **117** | **202.44** |
| **118** | **215.93** |
| **119** | **245.12** |
| **120** | **222.76** |
| **121** | **23.89** |
| **122** | **149.78** |
| **123** | **152.29** |
| **124** | **166.09** |
| **125** | **113.11** |
| **126** | **189.83** |
| **127** | **160.08** |

In the above P particles, the 17 P particles, PP-10copy-Aβ1-6-loop1G₇₂, PP-1copy-Aβ1-6-loop2S₁₄₉, PP-10copy-Aβ1-6-loop2S₁₄₉, PP-10copy-Aβ1-6-loop3 G₁₆₉, PP-20copy-Aβ1-6-loop3 G₁₆₉, PP-3 copy-Aβ1-6-loop 1G₇₂-3 copy-Aβ1-6-loop2S₁₄₉, PP-10copy-Aβ1-15-loop1G₇₂-10copy-Aβ1-6-loop2S₁₄₉, PP-20cοpy-Aβ1-15-loop1G₇₂-40copy-Aβ1-6-loop2S₁₄₉, PP-3copy-Aβ1-12-loop2S₁₄₉-3copy-Aβ1-6-loop3G₁₆₉, PP-1copy-Aβ1-6-loop2S₁₄₉-10copy-Aβ1-6-loop3G₁₆₉, PP-20copy-Aβ1-12-loop2S₁₄₉-10copy-Aβ1-6-loop3G₁₆₉, PP-3copy-Aβ1-6-loop1G₇₂-3copy-Aβ1-12-loop3G₁₆₉, PP-1copy-Aβ1-6-loop1G₇₂-10copy-Aβ1-6-loop3G₁₆₉, PP-3 copy-Aβ1-6-loop1G₇₂-10copy-Aβ1-12-loop3G₁₆₉, PP-1copy-Aβ1-6-loop1G₇₂-1copy-Aβ1-6-loop2S₁₄₉-1copy-Aβ1-6-loop3G₁₆₉, PP-3 copy-Aβ1-6-loop1G₇₂-3copy-Aβ1-6-loop2S₁₄₉-3copy-Aβ1-6-loop3G₁₆₉, PP-10copy-Aβ1-6-loop1G₇₂-10copy-Aβ1-6-loop2S₁₄₉-10copy-Aβ1-6-loop3G₁₆₉, have good immunological effects and can induce the production of high concentrations of antibodies.

The standard curve is plotted in accordance with positive antibody 6e10, and its linear fitting curve is y=47.692x+0.2964, R2=0.99. According to this standard curve, the concentration of the antibody produced by induction can be calculated within the linear range, wherein PP-3 copy-Aβ1-6-loop1G₇₂-3copy-Aβ1-6-loop2S₁₄₉-3copy-Aβ1-6-loop3G₁₆₉ produce antibodies in a concentration of about 245.12 µg/mL after the fourth immunization.

Therefore, the present invention has good immunological effects and induces a high concentration of Aβ1-42 antibody in mouse serum after immunization. The present invention has very good therapeutical effects and is a protein vaccine with great potentials for treating AD.

## Claims

1. A recombinant P particle formed from a norovirus capsid P protein chimerized with amyloid beta (Aβ)₁₋ₘ peptide(s), wherein an Aβ₁₋ₘ peptide means the polypeptide consisting of the first m amino acids at the N terminal of the entire Aβ₁₋₄₂ protein, wherein m is an integer ranging from 6 to 15, the recombinant P particle forms an ordered and repetitive antigen array, the amino acid sequence of at least one of the Aβ₁₋ₘ peptide is embedded into loop1, loop2 and/or loop3 of the norovirus capsid P protein.

2. The recombinant P particle according to claim 1, wherein the amino acid sequence of the norovirus capsid P protein is as shown in SEQ ID NO: 1.

3. The recombinant P particle according to claim 1 or 2, wherein N₁ Aβ₁₋ₘ peptide sequences are embedded behind one or more amino acid sites selected from the group consisting of amino acids 70-74 of SEQ ID NO:1, i.e. 170, A71, G72, T73 and Q74; N₂ Aβ₁₋ₘ peptide sequences are embedded behind one or more amino acid sites selected from the group consisting of amino acids 148-151 of SEQ ID NO:1, i.e. T148, S149, N150 and D151; and N₃ Aβ₁₋ₘ peptide sequences are embedded behind one or more amino acid sites selected from the group consisting of amino acids 168-171 of SEQ ID NO:1, i.e. D168, G169, S170 and T171; wherein N₁, N₂ and N₃ are the number of copies inserted in to loop1, loop2 and loop3, which each are independently selected from an integer ranging from 0-40, and N₁+N₂+N₃≥1.

4. The recombinant P particle according to claims 1 to 3, wherein multiple consecutive Aβ₁₋ₘ peptide sequences embedded into the norovirus capsid P protein are linked directly or via a polypeptide linker, wherein the polypeptide linker is preferably (Gly)ₙ, preferably n being 1-10, and more preferably n being 3.

5. The recombinant P particle according to claims 1 to 3, wherein the Aβ₁₋ₘ peptide is linked to the norovirus capsid P protein directly or via a polypeptide linker, wherein the polypeptide linker is preferably (Gly)ₙ, preferably n being 1-10, and more preferably n being 3.

6. The recombinant P particle according to claims 1 to 3, wherein the Aβ₁₋ₘ peptide sequence is an amino acid sequence comprised in sequences selected from SEQ ID NOs: 2-8, and preferably, the Aβ₁₋ₘ peptide sequence is comprised in SEQ ID NO: 2.

7. The recombinant P particle according to claim 1, wherein the norovirus capsid P protein chimerized with Aβ₁₋ₘ peptide(s) is encoded by nucleic acid sequences of SEQ ID NO: 15-SEQ ID NO: 142; preferably, the norovirus capsid P protein chimerized with Aβ₁₋ₘ peptide(s) is encoded by a nucleic acid sequence of SEQ ID NO:24, SEQ ID NO:39, SEQ ID NO:40, SEQ ID NO:57, SEQ ID NO:73, SEQ ID NO:92, SEQ ID NO:117, SEQ ID NO:132, SEQ ID NO:133 or SEQ ID NO:134.

8. A nucleic acid encoding the recombinant P particle according to any one of claims 1-7, wherein preferably the nucleic acid has sequences of SEQ ID NO: 15-SEQ ID NO: 142, and more preferably, the nucleic acid has a sequence of SEQ ID NO:24, SEQ ID NO:39, SEQ ID NO:40, SEQ ID NO:57, SEQ ID NO:73, SEQ ID NO:92, SEQ ID NO:117, SEQ ID NO:132, SEQ ID NO:133 or SEQ ID NO:134.

9. A pharmaceutical composition comprising the recombinant P particle according to any one of claims 1-7 and a pharmaceutically acceptable carrier, wherein the pharmaceutical composition is preferably used in mammals, and more preferably used in human; preferably, the pharmaceutical composition is a vaccine, more preferably, the vaccine also comprises an adjuvant, and most preferably, the vaccine also comprises a CpG adjuvant or aluminum adjuvant; the vaccine is preferably administered for immunization subcutaneously or nasally, and more preferably administered subcutaneously.

10. The recombinant P particle according to any one of claims 1-7 for use in the treatment or prevention of Alzheimer's disease.

11. A method for preparing the recombinant P particle according to any one of claims 1-7, comprising the following steps:
i) obtaining an expression vector comprising a nucleic acid encoding a norovirus capsid P protein chimerized with Aβ₁₋ₘ peptide(s), wherein an Aβ1-m peptide means the polypeptide consisting of the first m amino acids at the N terminal of the entire Aβ₁₋₄₂ protein, wherein m is an integer ranging from 6 to 15;
ii) transferring the expression vector into a receptor cell;
iii) expressing the norovirus capsid P protein chimerized with Aβ₁₋ₘ peptide, and allowing it to self-assemble into a recombinant P particle,
and preferably, the method also comprises isolation and purification steps, and more preferably, the purification step includes using cation exchange chromatography and/or hydrophobic chromatography for purification.

## Patentansprüche

1. Rekombinantes P-Partikel, das aus einem Norovirus-Kapsid-P-Protein, das mit Amyloid-beta (Aβ)₁₋ₘ-Peptid(en) chimerisiert ist, gebildet ist, wobei ein Aβ₁₋ₘ-Peptid bedeutet, dass das Polypeptid aus den ersten m Aminosäuren an dem N-Terminus des gesamten Aβ₁₋₄₂-Proteins besteht, wobei m eine ganze Zahl im Bereich von 6 bis 15 ist, das rekombinante P-Partikel eine geordnete und wiederholte Antigenanordnung bildet, die Aminosäuresequenz von mindestens einem des Aβ₁₋ₘ-Peptids in Loop1, Loop2 und/oder Loop3 des Norovirus-Kapsid-P-Proteins eingebettet ist.

2. Rekombinantes P-Partikel nach Anspruch 1, wobei die Aminosäuresequenz des Norovirus-Kapsid-P-Proteins wie in SEQ.-ID Nr. 1 gezeigt ist.

3. Rekombinantes P-Partikel nach Anspruch 1 oder 2, wobei N₁ Aβ₁₋ₘ-Peptidsequenzen hinter einer oder mehreren Aminosäurestellen eingebettet sind, die aus der Gruppe ausgewählt sind, die aus Aminosäuren 70-74 der SEQ.-ID Nr. 1 besteht, d. h. 170, A71, G72, T73 und Q74; N₂ Aβ₁₋ₘ-Peptidsequenzen hinter einer oder mehreren Aminosäurestellen eingebettet sind, die aus der Gruppe ausgewählt sind, die aus Aminosäuren 148-151 der SEQ.-ID Nr. 1 besteht, d. h. T148, S149, N150 und D151; und N₃ Aβ₁₋ₘ-Peptidsequenzen hinter einer oder mehreren Aminosäurestellen eingebettet sind, die aus der Gruppe ausgewählt sind, die aus Aminosäuren 168-171 der SEQ.-ID Nr. 1 besteht, d. h. D168, G169, S170 und T171; wobei N₁, N₂ und N₃ die Anzahl von Kopien sind, die in den Loop1, Loop2 und Loop3 eingebaut sind, die jeweils unabhängig aus einer ganzen Zahl im Bereich von 0-40 ausgewählt sind, und Ni + N₂ + N_{3 ≥} 1.

4. Rekombinantes P-Partikel nach den Ansprüchen 1 bis 3, wobei mehrere aufeinanderfolgende Aβ₁₋ₘ-Peptidsequenzen, die in das Norovirus-Kapsid-P-Protein eingebettet sind, direkt oder über einen Polypeptid-Linker verknüpft sind, wobei der Polypeptid-Linker bevorzugt (Gly)ₙ ist, wobei bevorzugt n 1-10 ist und bevorzugter n 3 ist.

5. Rekombinantes P-Partikel nach den Ansprüchen 1 bis 3, wobei das Aβ₁₋ₘ-Peptid mit dem Norovirus-Kapsid-P-Protein direkt oder über einen Polypeptid-Linker verknüpft ist, wobei der Polypeptid-Linker bevorzugt (Gly)ₙ ist, wobei bevorzugt n 1-10 ist und bevorzugter n 3 ist.

6. Rekombinantes P-Partikel nach den Ansprüchen 1 bis 3, wobei die Aβ₁₋ₘ-Peptidsequenz eine Aminosäuresequenz ist, die in Sequenzen enthalten ist, die aus den SEQ.-ID Nr. 2-8 ausgewählt sind, und bevorzugt die Aβ₁₋ₘ-Peptidsequenz in SEQ.-ID Nr. 2 enthalten ist.

7. Rekombinantes P-Partikel nach Anspruch 1, wobei das Norovirus-Kapsid-P-Protein, das mit Aβ₁₋ₘ-Peptid(en) chimerisiert ist, durch Nukleinsäuresequenzen der SEQ.-ID Nr. 15 - SEQ.-ID Nr. 142 kodiert ist; bevorzugt das Norovirus-Kapsid-P-Protein, das mit Aβ₁₋ₘ-Peptid(en) chimerisiert ist, durch eine Nukleinsäuresequenz von SEQ.-ID Nr. 24, SEQ.-ID Nr. 39, SEQ.-ID Nr. 40, SEQ.-ID Nr. 57, SEQ.-ID Nr. 73, SEQ.-ID Nr. 92, SEQ.-ID Nr. 117, SEQ.-ID Nr. 132, SEQ.-ID Nr. 133 oder SEQ.-ID Nr. 134 kodiert ist.

8. Nukleinsäure, die das rekombinante P-Partikel nach einem der Ansprüche 1-7 kodiert, wobei bevorzugt die Nukleinsäure Sequenzen von SEQ.-ID Nr. 15 - SEQ.-ID Nr. 142 aufweist und bevorzugter die Nukleinsäure eine Sequenz von SEQ.-ID Nr. 24, SEQ.-ID Nr. 39, SEQ.-ID Nr. 40, SEQ.-ID Nr. 57, SEQ.-ID Nr. 73, SEQ.-ID Nr. 92, SEQ.-ID Nr. 117, SEQ.-ID Nr. 132, SEQ.-ID Nr. 133 oder SEQ.-ID Nr. 134 aufweist.

9. Pharmazeutische Zusammensetzung, die das rekombinante P-Partikel nach einem der Ansprüche 1-7 und einen pharmazeutisch verträglichen Träger umfasst, wobei die pharmazeutische Zusammensetzung bevorzugt in Säugern verwendet wird und bevorzugter in Menschen verwendet wird; bevorzugt die pharmazeutische Zusammensetzung ein Impfstoff ist, bevorzugter der Impfstoff auch ein Adjuvans umfasst und am bevorzugtesten der Impfstoff auch ein CpG-Adjuvans oder Aluminium-Adjuvans umfasst; der Impfstoff bevorzugt zur Immunisierung subkutan oder nasal verabreicht wird und bevorzugter subkutan verabreicht wird.

10. Rekombinantes P-Partikel nach einem der Ansprüche 1-7 für die Verwendung bei der Behandlung oder Prävention von Alzheimer-Krankheit.

11. Verfahren für die Herstellung des rekombinanten P-Partikel nach einem der Ansprüche 1-7, das die folgenden Schritte umfasst:
i) Erhalten eines Expressionsvektors, der eine Nukleinsäure umfasst, die ein Norovirus-Kapsid-P-Protein, das mit Aβ₁₋ₘ-Peptid(en) chimerisiert ist, kodiert, wobei ein Aβ1-m-Peptid bedeutet, dass das Polypeptid aus den ersten m Aminosäuren an dem N-Terminus des gesamten Aβ₁₋₄₂-Proteins besteht, wobei m eine ganze Zahl im Bereich von 6 bis 15 ist;
ii) Übertragen des Expressionsvektors in eine Empfängerzelle;
iii) Exprimieren des Norovirus-Kapsid-P-Proteins, das mit Aβ₁₋ₘ-Peptid chimerisiert ist, und Ermöglichen der Selbstorganisation dieses zu einem rekombinanten P-Partikel, und wobei bevorzugt das Verfahren auch Isolierungs- und Aufreinigungsschritte umfasst und bevorzugter der Aufreinigungsschritt die Verwendung von Kationenaustauschchromatographie und/oder hydrophober Chromatographie für die Aufreinigung einschließt.

## Revendications

1. Particule P recombinante formée d'une protéine P de capside de norovirus chimérisée avec un(des) peptide(s) bêta-amyloïde(s) (Aβ)₁₋ₘ, où un peptide Aβ₁₋ₘ signifie le polypeptide composé des m premiers acides aminés à la terminaison N de la protéine Aβ₁₋₄₂ entière, où m est un entier allant de 6 à 15, la particule P recombinante forme un arrangement d'antigènes ordonnés répétitifs, la séquence d'acides aminés d'au moins l'un du peptide Aβ₁₋ₘ est incorporée dans la bouclel, la boucle2 et/ou la boucle3 de la protéine P de capside de norovirus.

2. Particule P recombinante selon la revendication 1, dans laquelle la séquence d'acides aminés de la protéine P de capside de norovirus est présentée dans la SEQ ID NO : 1.

3. Particule P recombinante selon la revendication 1 ou 2, dans laquelle N₁_séquences du peptide Aβ₁₋ₘ sont incorporées derrière un ou plusieurs sites d'acides aminés sélectionnés parmi le groupe composé des acides aminés 70-74 de la SEQ ID NO : 1, c-à-d. 170, A71, G72, T73 et Q74 ; N₂ séquences du peptide Aβ₁₋ₘ sont incorporées derrière un ou plusieurs sites d'acides aminés sélectionnés parmi le groupe composé des acides aminés 148-151 de la SEQ ID NO : 1, c-à-d. T148, S149, N150 et D151 ; et N₃ séquences du peptide Aβ₁₋ₘ sont incorporées derrière un ou plusieurs sites d'acides aminés sélectionnés parmi le groupe composé des acides aminés 168-171 de la SEQ ID NO : 1, c-à-d. D168, G169, S170 et T171 ; où N₁, N₂ et N₃ sont le nombre de copies incorporées dans la bouclel, la boucle2 et la boucle3, qui sont sélectionnés chacun indépendamment parmi un entier allant de 0-40, et N₁+ N₂+ N₃≥1.

4. Particule P recombinante selon les revendications 1 à 3, dans laquelle de multiples séquences consécutives du peptide Aβ₁₋ₘ incorporées dans la protéine P de capside de norovirus sont liées directement ou via un lieur polypeptidique, où le lieur polypeptidique est de préférence (Gly)ₙ, n étant de préférence 1-10, et n étant plus préférentiellement 3.

5. Particule P recombinante selon les revendications 1 à 3, dans laquelle le peptide Aβ₁₋ₘ est lié directement à la protéine P de capside de norovirus ou via un lieur polypeptidique, où le lieur polypeptidique est de préférence (Gly)ₙ, n étant de préférence 1-10, et n étant plus préférentiellement 3.

6. Particule P recombinante selon les revendications 1 à 3, dans laquelle la séquence du peptide Aβ₁₋ₘ est une séquence d'acides aminés comprise dans les séquences sélectionnées parmi les SEQ ID NO : 2-8, et de préférence, la séquence du peptide Aβ₁₋ₘ est comprise dans la SEQ ID NO : 2.

7. Particule P recombinante selon la revendication 1, dans laquelle la protéine P de capside de norovirus chimérisée avec un(des) peptide(s) Aβ₁₋ₘ est codée par des séquences d'acide nucléique des SEQ ID NO : 15 - SEQ ID NO : 142 ; de préférence, la protéine P de capside de norovirus chimérisée avec un(des) peptide(s) Aβ₁₋ₘ est codée par une séquence d'acide nucléique de la SEQ ID NO : 24, SEQ ID NO : 39, SEQ ID NO : 40, SEQ ID NO : 57, SEQ ID NO : 73, SEQ ID NO : 92, SEQ ID NO : 117, SEQ ID NO : 132, SEQ ID NO : 133 ou SEQ ID NO : 134.

8. Acide nucléique codant la particule P recombinante selon l'une quelconque des revendications 1-7, où l'acide nucléique a de préférence des séquences des SEQ ID NO : 15 - SEQ ID NO : 142, et plus préférentiellement, l'acide nucléique a une séquence de la SEQ ID NO : 24, SEQ ID NO : 39, SEQ ID NO : 40, SEQ ID NO : 57, SEQ ID NO : 73, SEQ ID NO : 92, SEQ ID NO : 117, SEQ ID NO : 132, SEQ ID NO : 133 ou SEQ ID NO : 134.

9. Composition pharmaceutique comprenant la particule P recombinante selon l'une quelconque des revendications 1-7 et un véhicule pharmaceutiquement acceptable, où la composition pharmaceutique est utilisée de préférence chez des mammifères, et est utilisée plus préférentiellement chez l'être humain ; de préférence, la composition pharmaceutique est un vaccin, plus préférentiellement, le vaccin comprend aussi un adjuvant, et plus préférentiellement, le vaccin comprend aussi un adjuvant CpG ou un adjuvant à base d'aluminium, le vaccin est administré de préférence pour immunisation par voie sous-cutanée ou nasale, et administré plus préférentiellement par voie sous-cutanée.

10. Particule P recombinante, selon l'une quelconque des revendications 1-7, pour une utilisation dans le traitement ou la prévention de la maladie d'Alzheimer.

11. Procédé de préparation de la particule P recombinante selon l'une quelconque des revendications 1-7, comprenant les étapes suivantes :
i) obtenir un vecteur d'expression comprenant un acide nucléique codant une protéine P de capside de norovirus chimérisée avec un(des) peptide(s) Aβ₁₋ₘ, où un peptide Aβ₁₋ₘ signifie le polypeptide composé des m premiers acides aminés de la terminaison N de la protéine Aβ₁₋₄₂ entière, où m est un entier allant de 6 à 15 ;
ii) transférer le vecteur d'expression dans une cellule réceptrice ;
iii) exprimer la protéine P de capside de norovirus chimérisée avec un peptide Aβ₁₋ₘ, et lui permettre de s'auto-assembler en une particule P recombinante,
et le procédé comprend aussi de préférence des étapes d'isolement et de purification, et plus préférentiellement, l'étape de purification comprend l'utilisation de la chromographie échangeuse de cations et/ou de la chromographie hydrophobe pour purification.
